(19) Europäisches Patentamt
European Patent Office
Office européen des brevets

(11) EP 3 342 780 A1

(12) # EUROPEAN PATENT APPLICATION

(43) Date of publication:
**04.07.2018 Bulletin 2018/27**

(21) Application number: **16207553.5**

(22) Date of filing: **30.12.2016**

(51) Int Cl.:
***C07K 14/435*** *(2006.01)* ***A01N 57/18*** *(2006.01)*
***C12N 15/82*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
- **Dow AgroSciences LLC**
  **Indianapolis, IN 46268 (US)**
- **Fraunhofer-Gesellschaft zur Förderung der angewandten Forschung e.V.**
  **80686 München (DE)**

(72) Inventors:
- **Narva, Kenneth E.**
  **Indianapolis, IN 46268 (US)**
- **Worden, Sarah E.**
  **Indianapolis, IN 46268 (US)**
- **Frey, Meghan L.**
  **Indianapolis, IN 46268 (US)**
- **Rangasamy, Murugesan**
  **Indianapolis, IN 46268 (US)**
- **Gandra, Premchand**
  **Indianapolis, IN 46268 (US)**
- **Lo, Wendy**
  **Indianapolis, IN 46268 (US)**
- **Fishilevich, Elane**
  **Indianapolis, IN 46268 (US)**
- **Vilcinskas, Andreas**
  **35394 Gießen (DE)**
- **Knorr, Eileen**
  **35392 Gießen (DE)**

(74) Representative: **Zwicker, Jörk**
**ZSP Patentanwälte PartG mbB**
**Hansastraße 32**
**80686 München (DE)**

Remarks:
Claims filed after the date of receipt of the divisional application (Rule 68(4) EPC).

(54) **PRE-MRNA PROCESSING FACTOR 8 (PRP8) NUCLEIC ACID MOLECULES TO CONTROL INSECT PESTS**

(57) This disclosure concerns nucleic acid molecules and methods of use thereof for control of insect pests through RNA interference-mediated inhibition of target coding and transcribed noncoding sequences in insect pests, including coleopteran pests. The disclosure also concerns methods for making transgenic plants that express nucleic acid molecules useful for the control of insect pests, and the plant cells and plants obtained thereby.

**FIG. 1**

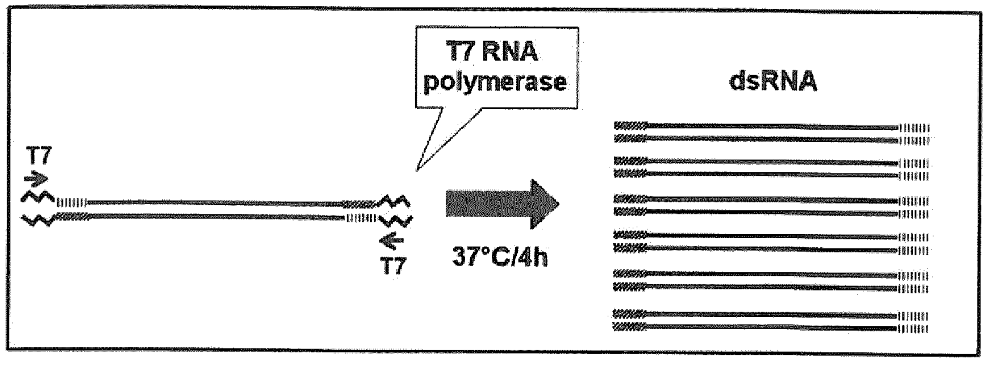


EP 3 342 780 A1

**Description**

## TECHNICAL FIELD OF THE DISCLOSURE

The present invention relates generally to genetic control of plant damage caused by insect pests (*e.g.*, coleopteran pests). In particular embodiments, the present invention relates to identification of target coding and non-coding polynucleotides, and the use of recombinant DNA technologies for post-transcriptionally repressing or inhibiting expression of target coding and non-coding polynucleotides in the cells of an insect pest to provide a plant protective effect.

## BACKGROUND

The western corn rootworm (WCR), *Diabrotica virgifera virgifera* LeConte, is one of the most devastating corn rootworm species in North America and is a particular concern in corn-growing areas of the Midwestern United States. The northern corn rootworm (NCR), *Diabrotica barberi* Smith and Lawrence, is a closely-related species that co-inhabits much of the same range as WCR. There are several other related subspecies of *Diabrotica* that are significant pests in the Americas: the Mexican corn rootworm (MCR), *D. virgifera zeae* Krysan and Smith; the southern corn rootworm (SCR), *D. undecimpunctata howardi* Barber; *D. balteata* LeConte; *D. undecimpunctata tenella; D. speciosa* Germar; and *D. u. undecimpunctata* Mannerheim. The United States Department of Agriculture has estimated that corn rootworms cause $1 billion in lost revenue each year, including $800 million in yield loss and $200 million in treatment costs.

Both WCR and NCR eggs are deposited in the soil during the summer. The insects remain in the egg stage throughout the winter. The eggs are oblong, white, and less than 0.004 inches in length. The larvae hatch in late May or early June, with the precise timing of egg hatching varying from year to year due to temperature differences and location. The newly hatched larvae are white worms that are less than 0.125 inches in length. Once hatched, the larvae begin to feed on corn roots. Corn rootworms go through three larval instars. After feeding for several weeks, the larvae molt into the pupal stage. They pupate in the soil, and then emerge from the soil as adults in July and August. Adult rootworms are about 0.25 inches in length.

Corn rootworm larvae complete development on corn and several other species of grasses. Larvae reared on yellow foxtail emerge later and have a smaller head capsule size as adults than larvae reared on corn. Ellsbury et al. (2005) Environ. Entomol. 34:627-34. WCR adults feed on corn silk, pollen, and kernels on exposed ear tips. If WCR adults emerge before corn reproductive tissues are present, they may feed on leaf tissue, thereby slowing plant growth and occasionally killing the host plant. However, the adults will quickly shift to preferred silks and pollen when they become available. NCR adults also feed on reproductive tissues of the corn plant, but in contrast rarely feed on corn leaves.

Most of the rootworm damage in corn is caused by larval feeding. Newly hatched rootworms initially feed on fine corn root hairs and burrow into root tips. As the larvae grow larger, they feed on and burrow into primary roots. When corn rootworms are abundant, larval feeding often results in the pruning of roots all the way to the base of the corn stalk. Severe root injury interferes with the roots' ability to transport water and nutrients into the plant, reduces plant growth, and results in reduced grain production, thereby often drastically reducing overall yield. Severe root injury also often results in lodging of corn plants, which makes harvest more difficult and further decreases yield. Furthermore, feeding by adults on the corn reproductive tissues can result in pruning of silks at the ear tip. If this "silk clipping" is severe enough during pollen shed, pollination may be disrupted.

Control of corn rootworms may be attempted by crop rotation, chemical insecticides, biopesticides (*e.g.,* the spore-forming gram-positive bacterium, *Bacillus thurfugiensis*), transgenic plants that express *Bt* toxins, or a combination thereof. Crop rotation suffers from the disadvantage of placing unwanted restrictions upon the use of farmland. Moreover, oviposition of some rootworm species may occur in soybean fields, thereby mitigating the effectiveness of crop rotation practiced with corn and soybean.

Chemical insecticides are the most heavily relied upon strategy for achieving corn rootworm control. Chemical insecticide use, though, is an imperfect corn rootworm control strategy; over $1 billion may be lost in the United States each year due to corn rootworm when the costs of the chemical insecticides are added to the costs of the rootworm damage that may occur despite the use of the insecticides. High populations of larvae, heavy rains, and improper application of the insecticide(s) may all result in inadequate corn rootworm control. Furthermore, the continual use of insecticides may select for insecticide-resistant rootworm strains, as well as raise significant environmental concerns due to the toxicity to non-target species.

European pollen beetles (PB) are serious pests in oilseed rape, both the larvae and adults feed on flowers and pollen. Pollen beetle damage to the crop can cause 20-40% yield loss. The primary pest species is *Meligethes aeneus.* Currently, pollen beetle control in oilseed rape relies mainly on pyrethroids which are expected to be phased out soon because of their environmental and regulatory profile. Moreover, pollen beetle resistance to existing chemical insecticides has been reported. Therefore, urgently needed are environmentally friendly pollen beetle control solutions with novel modes of action.

In nature, pollen beetles overwinter as adults in the soil or under leaf litter. In spring the adults emerge from hibernation and start feeding on flowers of weeds, and migrate onto flowering oilseed rape plants. The eggs are laid in oilseed rape flower buds. The larvae feed and develop in the buds and on the flowers. Late stage larvae find a pupation site in the soil. The second generation of adults emerge in July and August and feed on various flowering plants before finding sites for overwintering.

RNA interference (RNAi) is a process utilizing endogenous cellular pathways, whereby an interfering RNA (iRNA) molecule (e.g., a dsRNA molecule) that is specific for all, or any portion of adequate size, of a target gene results in the degradation of the mRNA encoded thereby. In recent years, RNAi has been used to perform gene "knockdown" in a number of species and experimental systems; for example, *Caenorhabditis elegans,* plants, insect embryos, and cells in tissue culture. *See, e.g.,* Fire et al. (1998) Nature 391:806-11; Martinez et al. (2002) Cell 110:563-74; McManus and Sharp (2002) Nature Rev. Genetics 3:737-47.

RNAi accomplishes degradation of mRNA through an endogenous pathway including the DICER protein complex. DICER cleaves long dsRNA molecules into short fragments of approximately 20 nucleotides, termed small interfering RNA (siRNA). The siRNA is unwound into two single-stranded RNAs: the passenger strand and the guide strand. The passenger strand is degraded, and the guide strand is incorporated into the RNA-induced silencing complex (RISC). Micro ribonucleic acids (miRNAs) are structurally very similar molecules that are cleaved from precursor molecules containing a polynucleotide "loop" connecting the hybridized passenger and guide strands, and they may be similarly incorporated into RISC. Post-transcriptional gene silencing occurs when the guide strand binds specifically to a complementary mRNA molecule and induces cleavage by Argonaute, the catalytic component of the RISC complex. This process is known to spread systemically throughout the organism despite initially limited concentrations of siRNA and/or miRNA in some eukaryotes such as plants, nematodes, and some insects.

Only transcripts complementary to the siRNA and/or miRNA are cleaved and degraded, and thus the knockdown of mRNA expression is sequence-specific. In plants, several functional groups of DICER genes exist. The gene silencing effect of RNAi persists for days and, under experimental conditions, can lead to a decline in abundance of the targeted transcript of 90% or more, with consequent reduction in levels of the corresponding protein. In insects, there are at least two DICER genes, where DICER1 facilitates miRNA-directed degradation by Argonaute1. Lee et al. (2004) Cell 117 (1):69-81. DICER2 facilitates siRNA-directed degradation by Argonaute2.

U.S. Patent 7,612,194 and U.S. Patent Publication Nos. 2007/0050860, 2010/0192265, and 2011/0154545 disclose a library of 9112 expressed sequence tag (EST) sequences isolated from *D.* v. *virgifera* LeConte pupae. It is suggested in U.S. Patent 7,612,194 and U.S. Patent Publication No. 2007/0050860 to operably link to a promoter a nucleic acid molecule that is complementary to one of several particular partial sequences of *D. v. virgifera* vacuolar-type $H^+$-ATPase (V-ATPase) disclosed therein for the expression of anti-sense RNA in plant cells. U.S. Patent Publication No. 2010/0192265 suggests operably linking a promoter to a nucleic acid molecule that is complementary to a particular partial sequence of a *D. v. virgifera* gene of unknown and undisclosed function (the partial sequence is stated to be 58% identical to C56C10.3 gene product in *C. elegans*) for the expression of anti-sense RNA in plant cells. U.S. Patent Publication No. 2011/0154545 suggests operably linking a promoter to a nucleic acid molecule that is complementary to two particular partial sequences of *D. v. virgifera* coatomer beta subunit genes for the expression of anti-sense RNA in plant cells. Further, U.S. Patent 7,943,819 discloses a library of 906 expressed sequence tag (EST) sequences isolated from *D. v. virgifera* LeConte larvae, pupae, and dissected midguts, and suggests operably linking a promoter to a nucleotide sequence that is complementary to a particular partial sequence of a *D. v. virgifera* charged multivesicular body protein 4b gene for the expression of double-stranded RNA in plant cells.

No further suggestion is provided in U.S. Patent 7,612,194, and U.S. Patent Publication Nos. 2007/0050860, 2010/0192265, and 2011/0154545 to use any particular sequence of the more than nine thousand sequences listed therein for RNA interference, other than the several particular partial sequences of V-ATPase and the particular partial sequences of genes of unknown function. Furthermore, none of U.S. Patent 7,612,194, and U.S. Patent Publication Nos. 2007/0050860, 2010/0192265, and 2011/0154545 provides any guidance as to which other of the over nine thousand sequences provided would be lethal, or even otherwise useful, in species of corn rootworm when used as dsRNA or siRNA. U.S. Patent 7,943,819 provides no suggestion to use any particular sequence of the more than nine hundred sequences listed therein for RNA interference, other than the particular partial sequence of a charged multivesicular body protein 4b gene. Furthermore, U.S. Patent 7,943,819 provides no guidance as to which other of the over nine hundred sequences provided would be lethal, or even otherwise useful, in species of corn rootworm when used as dsRNA or siRNA. U.S. Patent Application Publication No. U.S. 2013/040173 and PCT Application Publication No. WO 2013/169923 describe the use of a sequence derived from a *Diabrotica virgifera* Snf7 gene for RNA interference in maize. (Also disclosed in Bolognesi et al. (2012) PLOS ONE 7(10): e47534. doi:10.1371/journal.pone.0047534).

The overwhelming majority of sequences complementary to corn rootworm DNAs (such as the foregoing) do not provide a plant protective effect from species of corn rootworm when used as dsRNA or siRNA. For example, Baum et al. (2007) Nature Biotechnology 25:1322-1326, describe the effects of inhibiting several WCR gene targets by RNAi. These authors reported that 8 of the 26 target genes they tested were not able to provide experimentally significant coleopteran pest mortality at a very high iRNA (e.g., dsRNA) concentration of more than 520 ng/cm$^2$.

The authors of U.S. Patent 7,612,194 and U.S. Patent Publication No. 2007/0050860 made the first report of *in planta* RNAi in corn plants targeting the western corn rootworm. Baum et al. (2007) Nat. Biotechnol. 25(11): 322-6. These authors describe a high-throughput *in vivo* dietary RNAi system to screen potential target genes for developing transgenic RNAi maize. Of an initial gene pool of 290 targets, only 14 exhibited larval control potential. One of the most effective double-stranded RNAs (dsRNA) targeted a gene encoding vacuolar ATPase subunit A (V-ATPase), resulting in a rapid suppression of corresponding endogenous mRNA and triggering a specific RNAi response with low concentrations of dsRNA. Thus, these authors documented for the first time the potential for *in planta* RNAi as a possible pest management tool, while simultaneously demonstrating that effective targets could not be accurately identified *a priori*, even from a relatively small set of candidate genes.

## SUMMARY OF THE DISCLOSURE

Disclosed herein are nucleic acid molecules (*e.g.*, target genes, DNAs, dsRNAs, siRNAs, miRNAs, shRNAs, and hpRNAs), and methods of use thereof, for the control of insect pests, including, for example, coleopteran pests, such as *D. v. virgifera* LeConte (western corn rootworm, "WCR"); *D. barberi* Smith and Lawrence (northern corn rootworm, "NCR"); *D. u. howardi* Barber (southern corn rootworm, "SCR"); *D. v. zeae* Krysan and Smith (Mexican corn rootwonn, "MCR"); *D. balteata* LeConte; *D. u. tenella*; *D. speciosa* Germar; *D. u. undecimpunctata* Mannerheim, and *Meligethes aeneus* Fabricius (pollen beetle, "PB"). In particular examples, exemplary nucleic acid molecules are disclosed that may be homologous to at least a portion of one or more native nucleic acids in an insect pest.

In these and further examples, the native nucleic acid sequence maybe a target gene, the product of which may be, for example and without limitation: involved in a metabolic process; or involved in larval development. In some examples, post-transcriptional inhibition of the expression of a target gene by a nucleic acid molecule comprising a polynucleotide homologous thereto may be lethal to an insect pest or result in reduced growth and/or development of an insect pest. In specific examples, *pre-mRNA processing factor 8* (*prp8*) or a *prp8*homolog may be selected as a target gene for post-transcriptional silencing. In particular examples, a target gene useful for post-transcriptional inhibition is a *prp8* gene selected from the group consisting of *Diabrotica prp8* (*e.g.,* SEQ ID NO:1 (*prp8-1*) and SEQ ID NO:3 (*prp8-2*)) and *Meligethes prp8 (e.g.,* SEQ ID NO:5). An isolated nucleic acid molecule comprising the polynucleotide of SEQ ID NO: 1; the complement of SEQ ID NO: 1; SEQ ID NO:3; the complement of SEQ ID NO:3; SEQ ID NO:5; the complement of SEQ ID NO:5; and/or fragments of any of the foregoing (*e.g.*, SEQ ID NOs:7-12 and the complements thereof) is therefore disclosed herein.

Also disclosed are nucleic acid molecules comprising a polynucleotide that encodes a polypeptide that is at least about 85% identical to an amino acid sequence within a target gene product (for example, the product of a *prp8* gene). For example, a nucleic acid molecule may comprise a polynucleotide encoding a polypeptide that is at least 85% identical to *Diabrotica* PRP8 (*e.g.*, SEQ ID NO:2 and SEQ ID NO:4); *Meligethes* PRP8 (*e.g.*, SEQ ID NO:6); and/or an amino acid sequence within a product of a *prp8* gene. Further disclosed are nucleic acid molecules comprising a polynucleotide that is the reverse complement of a polynucleotide that encodes a polypeptide at least 85% identical to an amino acid sequence within a target gene product.

Also disclosed are cDNA polynucleotides that may be used for the production of iRNA (e.g., dsRNA, siRNA, shRNA, miRNA, and hpRNA) molecules that are complementary to all or part of an insect pest target gene, for example, an *prp8* gene. In particular embodiments, dsRNAs, siRNAs, shRNAs, miRNAs, and/or hpRNAs may be produced *in vitro* or *in vivo* by a genetically-modified organism, such as a plant or bacterium. In particular examples, cDNA molecules are disclosed that may be used to produce iRNA molecules that are complementary to all or part of a *prp8* gene *(e.g.,* SEQ ID NO:1, SEQ ID NO:3, and SEQ ID NO:5).

Further disclosed are means for inhibiting expression of *a prp8* gene in a *Diabrotica* pest, and means for providing *prp8*-mediated *Diabrotica* pest protection to a plant. A means for inhibiting expression of a *prp8* gene in a *Diabrotica* pest is a double-stranded RNA molecule, wherein one strand of the molecule consists of a polyribonucleotide selected from the group consisting of SEQ ID NOs:86-90; and the complements thereof. Functional equivalents of means for inhibiting expression of a *prp8* gene in a *Diabrotica* pest include double-stranded RNA molecules comprising a polyribonucleotide that is substantially homologous to all or part of a *Diabrotica prp8* gene comprising a nucleotide sequence selected from the group consisting of SEQ ID NOs:7-11. A means for providing *prp8*-mediated *Diabrotica* pest protection to a plant is a DNA molecule comprising a polynucleotide encoding a means for inhibiting expression of a *prp8* gene in a *Diabrotica* pest operably linked to a promoter, wherein the DNA molecule is capable of being integrated into the genome of a plant.

Also disclosed are means for inhibiting expression of a *prp8* gene in a *Meligethes* pest, and means for providing *prp8*-mediated *Meligethes* pest protection to a plant. A means for inhibiting expression of a *prp8* gene in a *Meligethes* pest is a double-stranded RNA molecule, wherein one strand of the molecule consists of the polyribonucleotide of SEQ ID NO:92; and the complements thereof. Functional equivalents of means for inhibiting expression of a *prp8* gene in a *Meligethes* pest include double-stranded RNA molecules comprising a polyribonucleotide that is substantially homologous to all or part of a *Meligethes prp8* gene comprising SEQ ID NO:12. A means for providing *prp8*-mediated *Meligethes* pest protection to a plant is a DNA molecule comprising a polynucleotide encoding a means for inhibiting expression of a *prp8* gene in a *Meligethes* pest operably linked to a promoter, wherein the DNA molecule is capable of being integrated into the genome of a plant

Additionally disclosed are methods for controlling a population of an insect pest *(e.g.,* a coleopteran pest), comprising providing to an insect pest *(e.g.,* a coleopteran pest) an iRNA *(e.g.,* dsRNA, siRNA, shRNA, miRNA, and hpRNA) molecule that functions upon being taken up by the pest to inhibit a biological function within the pest.

In some embodiments, methods for controlling a population of a coleopteran pest comprises providing to the coleopteran pest an iRNA molecule that comprises all or part of a polynucleotide selected from the group consisting of: SEQ ID NO:84; the complement of SEQ ID NO:84; SEQ ID NO:85; the complement of SEQ ID NO:85; SEQ ID NO:86; the complement of SEQ ID NO:86; SEQ ID NO:87; the complement of SEQ ID NO:87; SEQ ID NO:88; the complement of SEQ ID NO:88; SEQ ID NO:89; the complement of SEQ ID NO:89; SEQ ID NO:90; the complement of SEQ ID NO:90; SEQ ID NO:91; the complement of SEQ ID NO:91; SEQ ID NO:92; the complement of SEQ ID NO:92; a polynucleotide that hybridizes to a native coding polynucleotide of a *Diabrotica* organism *(e.g.,* WCR) comprising all or part of any of SEQ ID NOs:7-11; the complement of a polynucleotide that hybridizes to a native coding polynucleotide of a *Diabrotica* organism comprising all or part of any of SEQ ID NOs:7-11; a polynucleotide that hybridizes to a native coding polynucleotide of a *Meligethes* organism *(e.g.,* PB) comprising all or part of SEQ ID NO: 12; and the complement of a polynucleotide that hybridizes to a native coding polynucleotide of a *Meligethes* organism comprising all or part of SEQ ID NO: 12.

In particular embodiments, an iRNA that functions upon being taken up by an insect pest to inhibit a biological function within the pest is transcribed from a DNA comprising all or part of a polynucleotide selected from the group consisting of: SEQ ID NO:1; the complement of SEQ ID NO:1; SEQ ID NO:3; the complement of SEQ ID NO:3; SEQ ID NO:5; the complement of SEQ ID NO:5; a native coding polynucleotide of a *Diabrotica* organism *(e.g.,* WCR) comprising all or part of any of SEQ ID NOs:7-11; the complement of a native coding polynucleotide of a *Diabrotica* organism comprising all or part of any of SEQ ID NOs:7-11; a native coding polynucleotide of a *Meligethes* organism *(e.g.,* PB) comprising all or part of SEQ ID NO:12; and the complement of a native coding polynucleotide of a *Meligethes* organism comprising all or part of SEQ ID NO:12.

Also disclosed herein are methods wherein dsRNAs, siRNAs, shRNAs, miRNAs, and/or hpRNAs may be provided to an insect pest in a diet-based assay, or in genetically-modified plant cells expressing the dsRNAs, siRNAs, shRNAs, miRNAs, and/or hpRNAs. In these and further examples, the dsRNAs, siRNAs, shRNAs, miRNAs, and/or hpRNAs may be ingested by the pest. Ingestion of dsRNAs, siRNA, shRNAs, miRNAs, and/or hpRNAs of the invention may then result in RNAi in the pest, which in turn may result in silencing of a gene essential for viability of the pest and leading ultimately to mortality. In particular examples, a coleopteran pest controlled by use of nucleic acid molecules of the invention may be WCR, NCR, SCR, and/or PB.

The foregoing and other features will become more apparent from the following Detailed Description of several embodiments, which proceeds with reference to the accompanying **FIGs. 1**-**2**.

## BRIEF DESCRIPTION OF THE FIGURES

**FIG. 1** includes a depiction of a strategy used to generate dsRNA from a single transcription template with a single pair of primers.

**FIG. 2** includes a depiction of a strategy used to generate dsRNA from two transcription templates.

## DETAILED DESCRIPTION

### *I. Overview of several embodiments*

We developed RNA interference (RNAi) as a tool for insect pest management, using one of the most likely target pest species for transgenic plants that express dsRNA; the western corn rootworm. Thus far, most genes proposed as targets for RNAi in rootworm larvae do not actually achieve their purpose. Herein, we describe RNAi-mediated knockdown of *prp8* in the exemplary insect pests, western corn rootworm and pollen beetle, which is shown to have a lethal phenotype when, for example, iRNA molecules are delivered *via* ingested or injected *prp8* dsRNA. In embodiments herein, the ability to deliver *prp8* dsRNA by feeding to insects confers a RNAi effect that is very useful for insect (*e.g.*, coleopteran) pest management. By combining *prp8*-mediated RNAi with other useful RNAi targets, the potential to affect multiple target sequences, for example, in larval rootworms and pollen beetle, may increase opportunities to develop sustainable approaches to insect pest management involving RNAi technologies.

Disclosed herein are methods and compositions for genetic control of insect (*e.g.*, coleopteran) pest infestations. Methods for identifying one or more gene(s) essential to the lifecycle of an insect pest for use as a target gene for RNAi-mediated control of an insect pest population are also provided. DNA plasmid vectors encoding an RNA molecule may be designed to suppress one or more target gene(s) essential for growth, survival, and/or development. In some embodiments, the RNA molecule may be capable of forming dsRNA molecules. In some embodiments, methods are provided for post-transcriptional repression of expression or inhibition of a target gene *via* nucleic acid molecules that are complementary to a coding or non-coding sequence of the target gene in an insect pest. In these and further embodiments, a pest may ingest one or more dsRNA, siRNA, shRNA, miRNA, and/or hpRNA molecules transcribed from all or a portion of a nucleic acid molecule that is complementary to a coding or non-coding sequence of a target gene, thereby providing a plant-protective effect.

Thus, some embodiments involve sequence-specific inhibition of expression of target gene products, using dsRNA, siRNA, shRNA, miRNA and/or hpRNA that is complementary to coding and/or non-coding sequences of the target gene(s) to achieve at least partial control of an insect (*e.g.*, coleopteran) pest. Disclosed is a set of isolated and purified nucleic acid molecules comprising a polynucleotide, for example, as set forth in one of SEQ ID NOs:1, 3, 5, and fragments thereof. In some embodiments, a stabilized dsRNA molecule may be expressed from these polynucleotides, fragments thereof, or a gene comprising one or more of these polynucleotides, for the post-transcriptional silencing or inhibition of a target gene. In certain embodiments, isolated and purified nucleic acid molecules comprise all or part of any of SEQ ID NOs:1, 3, and 5 *(e.g.,* SEQ ID NOs: 7-12), and/or a complement thereof.

Some embodiments involve a recombinant host cell *(e.g.,* a plant cell) having in its genome at least one recombinant DNA encoding at least one iRNA (*e.g.*, dsRNA) molecule(s). In particular embodiments, an encoded dsRNA molecule(s) may be provided when ingested by an insect (*e.g.*, coleopteran) pest to post-transcriptionally silence or inhibit the expression of a target gene in the pest. The recombinant DNA may comprise, for example, any of SEQ ID NOs:1, 3, 5, and 7-12; fragments of any of SEQ ID NOs:1, 3, 5, and 7-12; and a polynucleotide consisting of a partial sequence of a gene comprising one or more of SEQ ID NOs:1, 3, 5, and 7-12; and/or complements thereof.

Some embodiments involve a recombinant host cell having in its genome a recombinant DNA encoding at least one iRNA (*e.g.*, dsRNA) molecule(s) comprising all or part of SEQ ID NO:84, SEQ ID NO:85 or SEQ ID NO:91 (*e.g.,* at least one polyribonucleotide selected from a group comprising SEQ ID NOs:86-90 and 92). When ingested by an insect (*e.g.*, coleopteran) pest, the iRNA molecule(s) may silence or inhibit the expression of a target *prp8* DNA (*e.g.,* a DNA comprising all or part of a polynucleotide selected from the group consisting of SEQ ID NOs:1, 3, 5, and 7-12) in the pest, and thereby result in cessation of growth, development, and/or feeding in the pest.

In some embodiments, a recombinant host cell having in its genome at least one recombinant DNA encoding at least one RNA molecule capable of forming a dsRNA molecule may be a transformed plant cell. Some embodiments involve transgenic plants comprising such a transformed plant cell. In addition to such transgenic plants, progeny plants of any transgenic plant generation, transgenic seeds, and transgenic plant products, are all provided, each of which comprises recombinant DNA(s). In particular embodiments, an RNA molecule capable of forming a dsRNA molecule may be expressed in a transgenic plant cell. Therefore, in these and other embodiments, a dsRNA molecule may be isolated from a transgenic plant cell. In particular embodiments, the transgenic plant is a plant selected from the group comprising corn (*Zea mays),* soybean (*Glycine max*), cotton, plants of the family *Poaceae*, and rapeseed (*Brassica sp.*).

Some embodiments involve a method for modulating the expression of a target gene in an insect (*e.g.*, coleopteran) pest cell. In these and other embodiments, a nucleic acid molecule may be provided, wherein the nucleic acid molecule comprises a polynucleotide encoding an RNA molecule capable of forming a dsRNA molecule. In particular embodiments, a polynucleotide encoding an RNA molecule capable of forming a dsRNA molecule may be operatively linked to a promoter, and may also be operatively linked to a transcription termination sequence. In particular embodiments, a method for modulating the expression of a target gene in an insect pest cell may comprise: (a) transforming a plant cell with a vector comprising a polynucleotide encoding an RNA molecule capable of forming a dsRNA molecule; (b) culturing the transformed plant cell under conditions sufficient to allow for development of a plant cell culture comprising a plurality of transformed plant cells; (c) selecting for a transformed plant cell that has integrated the vector into its genome; and (d) determining that the selected transformed plant cell comprises the RNA molecule capable of forming a dsRNA molecule encoded by the polynucleotide of the vector. A plant may be regenerated from a plant cell that has the vector integrated in its genome and comprises the dsRNA molecule encoded by the polynucleotide of the vector.

Thus, also disclosed is a transgenic plant comprising a vector having a polynucleotide encoding an RNA molecule capable of forming a dsRNA molecule integrated in its genome, wherein the transgenic plant comprises the dsRNA molecule encoded by the polynucleotide of the vector. In particular embodiments, expression of an RNA molecule capable of forming a dsRNA molecule in the plant is sufficient to modulate the expression of a target gene in a cell of an insect (e.g., coleopteran) pest that contacts the transformed plant or plant cell (for example, by feeding on the transformed plant, a part of the plant (e.g., root) or plant cell), such that growth and/or survival of the pest is inhibited. Transgenic plants disclosed herein may display resistance and/or enhanced tolerance to insect pest infestations. Particular transgenic plants may display resistance and/or enhanced protection from one or more coleopteran pest(s) selected from the group consisting of: WCR; NCR; SCR; MCR; *D. balteata* LeConte; *D. u. tenella; Meligethes aeneus* Fabricius; and *D. u. undecimpunctata* Mannerheim.

Also disclosed herein are methods for delivery of control agents, such as an iRNA molecule, to an insect (*e.g.*, coleopteran) pest. Such control agents may cause, directly or indirectly, an impairment in the ability of an insect pest population to feed, grow or otherwise cause damage in a host. In some embodiments, a method is provided comprising delivery of a stabilized dsRNA molecule to an insect pest to suppress at least one target gene in the pest, thereby causing RNAi and reducing or eliminating plant damage in a pest host. In some embodiments, a method of inhibiting expression of a target gene in the insect pest may result in cessation of growth, survival, and/or development in the pest.

In some embodiments, compositions *(e.g.,* a topical composition) are provided that comprise an iRNA *(e.g.,* dsRNA) molecule for use with plants, animals, and/or the environment of a plant or animal to achieve the elimination or reduction of an insect (*e.g*., coleopteran) pest infestation. In particular embodiments, the composition may be a nutritional composition or food source to be fed to the insect pest. Some embodiments comprise making the nutritional composition or food source available to the pest. Ingestion of a composition comprising iRNA molecules may result in the uptake of the molecules by one or more cells of the pest, which may in turn result in the inhibition of expression of at least one target gene in cell(s) of the pest. Ingestion of or damage to a plant or plant cell by an insect pest infestation may be limited or eliminated in or on any host tissue or environment in which the pest is present by providing one or more compositions comprising an iRNA molecule in the host of the pest.

The compositions and methods disclosed herein may be used together in combinations with other methods and compositions for controlling damage by insect (*e.g*., coleopteran) pests. For example, an iRNA molecule as described herein for protecting plants from insect pests may be used in a method comprising the additional use of one or more chemical agents effective against an insect pest, biopesticides effective against such a pest, crop rotation, recombinant genetic techniques that exhibit features different from the features of RNAi-mediated methods and RNAi compositions (*e.g*., recombinant production of proteins in plants that are harmful to an insect pest (*e.g., Bt* toxins and PIP-1 polypeptides (*See* U.S. Patent Publication No. US 2014/0007292 A1)), and/or recombinant expression of other iRNA molecules.

### *II. Abbreviations*

dsRNA double-stranded ribonucleic acid
EST expressed sequence tag
GI growth inhibition
NCBI National Center for Biotechnology Information
gDNA genomic deoxyribonucleic acid
iRNA inhibitory ribonucleic acid
ORF open reading frame
RNAi ribonucleic acid interference
miRNA micro ribonucleic acid
shRNA small hairpin ribonucleic acid
siRNA small inhibitory ribonucleic acid
hpRNA hairpin ribonucleic acid
UTR untranslated region
WCR western corn rootworm (*Diabrotica virgifera virgifera* LeConte)
NCR northern corn rootworm (*Diabrotica barberi* Smith and Lawrence)
MCR Mexican corn rootworm (*Diabrotica virgifera zeae* Krysan and Smith)
PB Pollen beetle (*Meligethes aeneus* Fabricius)
PCR polymerase chain reaction
qPCR quantitative polymerase chain reaction
RISC RNA-induced Silencing Complex
SCR southern corn rootworm (*Diabrotica undecimpunctata* howardi Barber)
SEM standard error of the mean
YFP yellow florescent protein

### *III. Terms*

In the description and tables which follow, a number of terms are used. In order to provide a clear and consistent understanding of the specification and claims, including the scope to be given such terms, the following definitions are provided:

Coleopteran pest: As used herein, the term "coleopteran pest" refers to pest insects of the order Coleoptera, including pest insects in the genus *Diabrotica,* which feed upon agricultural crops and crop products, including corn and other true grasses. In particular examples, a coleopteran pest is selected from a list comprising *D. v. virgifera* LeConte (WCR); *D. barberi* Smith and Lawrence (NCR); *D. u. howardi* (SCR); *D. v. zeae* (MCR); *D. balteata* LeConte; *D. u. tenella; D. u. undecimpunctata* Mannerheim; *D. speciosa* Germar; and *Meligethes aeneus* Fabricius (PB).

Contact (with an organism): As used herein, the term "contact with" or "uptake by" an organism (*e.g.*, a coleopteran pest), with regard to a nucleic acid molecule, includes internalization of the nucleic acid molecule into the organism, for example and without limitation: ingestion of the molecule by the organism (*e.g.*, by feeding); contacting the organism with a composition comprising the nucleic acid molecule; and soaking of organisms with a solution comprising the nucleic acid molecule.

Contig: As used herein the term "contig" refers to a DNA sequence that is reconstructed from a set of overlapping DNA segments derived from a single genetic source.

Corn plant: As used herein, the term "corn plant" refers to a plant of the species, *Zea mays* (maize).

Expression: As used herein, "expression" of a coding polynucleotide (for example, a gene or a transgene) refers to the process by which the coded information of a nucleic acid transcriptional unit (including, *e.g.*, gDNA or cDNA) is converted into an operational, non-operational, or structural part of a cell, often including the synthesis of a protein. Gene expression can be influenced by external signals; for example, exposure of a cell, tissue, or organism to an agent that increases or decreases gene expression. Expression of a gene can also be regulated anywhere in the pathway from DNA to RNA to protein. Regulation of gene expression occurs, for example, through controls acting on transcription, translation, RNA transport and processing, degradation of intermediary molecules such as mRNA, or through activation, inactivation, compartmentalization, or degradation of specific protein molecules after they have been made, or by combinations thereof. Gene expression can be measured at the RNA level or the protein level by any method known in the art, including, without limitation, northern blot, RT-PCR, western blot, or *in vitro, in situ*, or *in vivo* protein activity assay(s).

Genetic material: As used herein, the term "genetic material" includes all genes, and nucleic acid molecules, such as DNA and RNA.

Inhibition: As used herein, the term "inhibition," when used to describe an effect on a coding polynucleotide (for example, a gene), refers to a measurable decrease in the cellular level of mRNA transcribed from the coding polynucleotide and/or peptide, polypeptide, or protein product of the coding polynucleotide. In some examples, expression of a coding polynucleotide may be inhibited such that expression is approximately eliminated. "Specific inhibition" refers to the inhibition of a target coding polynucleotide without consequently affecting expression of other coding polynucleotides (*e.g.*, genes) in the cell wherein the specific inhibition is being accomplished.

Insect: As used herein with regard to pests, the term "insect pest" specifically includes coleopteran insect pests.

Isolated: An "isolated" biological component (such as a nucleic acid or protein) has been substantially separated, produced apart from, or purified away from other biological components in the cell of the organism in which the component naturally occurs (*i.e.*, other chromosomal and extra-chromosomal DNA and RNA, and proteins), while effecting a chemical or functional change in the component (*e.g.*, a nucleic acid may be isolated from a chromosome by breaking chemical bonds connecting the nucleic acid to the remaining DNA in the chromosome). Nucleic acid molecules and proteins that have been "isolated" include nucleic acid molecules and proteins purified by standard purification methods. The term also embraces nucleic acids and proteins prepared by recombinant expression in a host cell, as well as chemically-synthesized nucleic acid molecules, proteins, and peptides.

Nucleic acid molecule: As used herein, the term "nucleic acid molecule" may refer to a polymeric form of nucleotides, which may include both sense and anti-sense strands of RNA, cDNA, gDNA, and synthetic forms and mixed polymers of the above. A nucleotide or nucleobase may refer to a ribonucleotide, deoxyribonucleotide, or a modified form of either type of nucleotide. A "nucleic acid molecule" as used herein is synonymous with "nucleic acid" and "polynucleotide." A nucleic acid molecule is usually at least 10 bases in length, unless otherwise specified. By convention, the nucleotide sequence of a nucleic acid molecule is read from the 5' to the 3' end of the molecule. The "complement" of a nucleic acid molecule refers to a polynucleotide having nucleobases that may form base pairs with the nucleobases of the nucleic acid molecule (*i.e.*, A-T/U, and G-C).

Some embodiments include nucleic acids comprising a template DNA that is transcribed into an RNA molecule that is the complement of an mRNA molecule. In these embodiments, the complement of the nucleic acid transcribed into the mRNA molecule is present in the 5' to 3' orientation, such that RNA polymerase (which transcribes DNA in the 5' to 3' direction) will transcribe a nucleic acid from the complement that can hybridize to the mRNA molecule. Unless explicitly stated otherwise, or it is clear to be otherwise from the context, the term "complement" therefore refers to a polynucleotide having nucleobases, from 5' to 3', that may form base pairs with the nucleobases of a reference nucleic acid. Similarly, unless it is explicitly stated to be otherwise (or it is clear to be otherwise from the context), the "reverse complement" of a nucleic acid refers to the complement in reverse orientation. The foregoing is demonstrated in the following illustration:

| | |
|---|---|
| ATGATGATG | polynucleotide |
| TACTACTAC | "complement" of the polynucleotide |
| CATCATCAT | "reverse complement" of the polynucleotide |

Some embodiments of the invention may include hairpin RNA-forming RNAi molecules. In these RNAi molecules, both the complement of a nucleic acid to be targeted by RNA interference and the reverse complement may be found in the same molecule, such that the single-stranded RNA molecule may "fold over" and hybridize to itself over the region comprising the complementary and reverse complementary polynucleotides.

"Nucleic acid molecules" include all polynucleotides, for example: single- and double-stranded forms of DNA; single-stranded forms of RNA; and double-stranded forms of RNA (dsRNA). The term "nucleotide sequence" or "nucleic acid sequence" refers to both the sense and antisense strands of a nucleic acid as either individual single strands or in the duplex. The term "ribonucleic acid" (RNA) is inclusive of iRNA (inhibitory RNA), dsRNA (double stranded RNA), siRNA (small interfering RNA), shRNA (small hairpin RNA), mRNA (messenger RNA), miRNA (micro-RNA), hpRNA (hairpin RNA), tRNA (transfer RNAs, whether charged or discharged with a corresponding acylated amino acid), and cRNA (complementary RNA). The term "deoxyribonucleic acid" (DNA) is inclusive of cDNA, gDNA, and DNA-RNA hybrids. The terms "polynucleotide" and "nucleic acid," and "fragments" thereof will be understood by those in the art as a term that includes both gDNAs, ribosomal RNAs, transfer RNAs, messenger RNAs, operons, and smaller engineered polynucleotides that encode or may be adapted to encode, peptides, polypeptides, or proteins.

Oligonucleotide: An oligonucleotide is a short nucleic acid polymer. Oligonucleotides may be formed by cleavage of longer nucleic acid segments, or by polymerizing individual nucleotide precursors. Automated synthesizers allow the synthesis of oligonucleotides up to several hundred bases in length. Because oligonucleotides may bind to a complementary nucleic acid, they may be used as probes for detecting DNA or RNA. Oligonucleotides composed of DNA (oligodeoxyribonucleotides) may be used in PCR, a technique for the amplification of DNAs. In PCR, the oligonucleotide is typically referred to as a "primer," which allows a DNA polymerase to extend the oligonucleotide and replicate the complementary strand.

A nucleic acid molecule may include either or both naturally occurring and modified nucleotides linked together by naturally occurring and/or non-naturally occurring nucleotide linkages. Nucleic acid molecules may be modified chemically or biochemically, or may contain non-natural or derivatized nucleotide bases, as will be readily appreciated by those of skill in the art. Such modifications include, for example, labels, methylation, substitution of one or more of the naturally occurring nucleotides with an analog, internucleotide modifications (*e.g.*, uncharged linkages: for example, methyl phosphonates, phosphotriesters, phosphoramidates, carbamates, *etc.;* charged linkages: for example, phosphorothioates, phosphorodithioates, *etc.;* pendent moieties: for example, peptides; intercalators: for example, acridine, psoralen, *etc.;* chelators; alkylators; and modified linkages: for example, alpha anomeric nucleic acids, *etc.*). The term "nucleic acid molecule" also includes any topological conformation, including single-stranded, double-stranded, partially duplexed, triplexed, hairpinned, circular, and padlocked conformations.

As used herein with respect to DNA, the term "coding polynucleotide," "structural polynucleotide," or "structural nucleic acid molecule" refers to a polynucleotide that is ultimately translated into a polypeptide, *via* transcription and mRNA, when placed under the control of appropriate regulatory elements. With respect to RNA, the term "coding polynucleotide" refers to a polynucleotide that is translated into a peptide, polypeptide, or protein. The boundaries of a coding polynucleotide are determined by a translation start codon at the 5'-terminus and a translation stop codon at the 3'-terminus. Coding polynucleotides include, but are not limited to: gDNA; cDNA; EST; and recombinant polynucleotides.

As used herein, "transcribed non-coding polynucleotide" refers to segments of mRNA molecules such as 5'UTR, 3'UTR, and intron segments that are not translated into a peptide, polypeptide, or protein. Further, "transcribed non-coding polynucleotide" refers to a nucleic acid that is transcribed into an RNA that functions in the cell, for example, structural RNAs (*e.g.*, ribosomal RNA (rRNA) as exemplified by 5S rRNA, 5.8S rRNA, 16S rRNA, 18S rRNA, 23S rRNA, and 28S rRNA, and the like); transfer RNA (tRNA); and snRNAs such as U4, U5, U6, and the like. Transcribed non-coding polynucleotides also include, for example and without limitation, small RNAs (sRNA), which term is often used to describe small bacterial non-coding RNAs; small nucleolar RNAs (snoRNA); microRNAs (miRNA); small interfering RNAs (siRNA); Piwi-interacting RNAs (piRNA); and long non-coding RNAs. Further still, "transcribed non-coding polynucleotide" refers to a polynucleotide that may natively exist as an intragenic "spacer" in a nucleic acid and which is transcribed into an RNA molecule.

Lethal RNA interference: As used herein, the term "lethal RNA interference" refers to RNA interference that results in death or a reduction in viability of the subject individual to which, for example, a dsRNA, miRNA, siRNA, shRNA, and/or hpRNA is delivered.

Genome: As used herein, the term "genome" refers to chromosomal DNA found within the nucleus of a cell, and also refers to organelle DNA found within subcellular components of the cell. In some embodiments of the invention, a DNA molecule may be introduced into a plant cell, such that the DNA molecule is integrated into the genome of the plant cell. In these and further embodiments, the DNA molecule may be either integrated into the nuclear DNA of the plant cell, or integrated into the DNA of the chloroplast or mitochondrion of the plant cell. The term "genome," as it applies to bacteria, refers to both the chromosome and plasmids within the bacterial cell. In some embodiments of the invention, a DNA molecule may be introduced into a bacterium such that the DNA molecule is integrated into the genome of the bacterium. In these and further embodiments, the DNA molecule may be either chromosomally-integrated or located as or in a stable plasmid.

Sequence identity: The term "sequence identity" or "identity," as used herein in the context of two polynucleotides or polypeptides, refers to the residues in the sequences of the two molecules that are the same when aligned for maximum correspondence over a specified comparison window.

As used herein, the term "percentage of sequence identity" may refer to the value determined by comparing two optimally aligned sequences (*e.g.*, nucleic acid sequences or polypeptide sequences) of a molecule over a comparison window, wherein the portion of the sequence in the comparison window may comprise additions or deletions (*i.e.,* gaps) as compared to the reference sequence (which does not comprise additions or deletions) for optimal alignment of the two sequences. The percentage is calculated by determining the number of positions at which the identical nucleotide or amino acid residue occurs in both sequences to yield the number of matched positions, dividing the number of matched positions by the total number of positions in the comparison window, and multiplying the result by 100 to yield the percentage of sequence identity. A sequence that is identical at every position in comparison to a reference sequence is said to be 100% identical to the reference sequence, and vice-versa.

Methods for aligning sequences for comparison are well-known in the art. Various programs and alignment algorithms are described in, for example: Smith and Waterman (1981) Adv. Appl. Math. 2:482; Needleman and Wunsch (1970) J. Mol. Biol. 48:443; Pearson and Lipman (1988) Proc. Natl. Acad. Sci. U.S.A. 85:2444; Higgins and Sharp (1988) Gene 73:237-44; Higgins and Sharp (1989) CABIOS 5:151-3; Corpet et al. (1988) Nucleic Acids Res. 16:10881-90; Huang et al. (1992) Comp. Appl. Biosci. 8:155-65; Pearson et al. (1994) Methods Mol. Biol. 24:307-31; Tatiana et al. (1999) FEMS Microbiol. Lett. 174:247-50. A detailed consideration of sequence alignment methods and homology calculations can be found in, *e.g.,* Altschul et al. (1990) J. Mol. Biol. 215:403-10.

The National Center for Biotechnology Information (NCBI) Basic Local Alignment Search Tool (BLAST™; Altschul *et al.* (1990)) is available from several sources, including the National Center for Biotechnology Information (Bethesda, MD), and on the internet, for use in connection with several sequence analysis programs. A description of how to determine sequence identity using this program is available on the internet under the "help" section for BLAST™. For comparisons of nucleic acid sequences, the "Blast 2 sequences" function of the BLAST™ (Blastn) program may be employed using the default BLOSUM62 matrix set to default parameters. Nucleic acids with even greater sequence similarity to the sequences of the reference polynucleotides will show increasing percentage identity when assessed by this method.

Specifically hybridizable/Specifically complementary: As used herein, the terms "Specifically hybridizable" and "Specifically complementary" are terms that indicate a sufficient degree of complementarity such that stable and specific binding occurs between the nucleic acid molecule and a target nucleic acid molecule. Hybridization between two nucleic acid molecules involves the formation of an anti-parallel alignment between the nucleobases of the two nucleic acid molecules. The two molecules are then able to form hydrogen bonds with corresponding bases on the opposite strand to form a duplex molecule that, if it is sufficiently stable, is detectable using methods well known in the art. A polynucleotide need not be 100% complementary to its target nucleic acid to be specifically hybridizable. However, the amount of complementarity that must exist for hybridization to be specific is a function of the hybridization conditions used.

Hybridization conditions resulting in particular degrees of stringency will vary depending upon the nature of the hybridization method of choice and the composition and length of the hybridizing nucleic acids. Generally, the temperature of hybridization and the ionic strength (especially the $Na^+$ and/or $Mg^{++}$ concentration) of the hybridization buffer will determine the stringency of hybridization, though wash times also influence stringency. Calculations regarding hybridization conditions required for attaining particular degrees of stringency are known to those of ordinary skill in the art, and are discussed, for example, in Sambrook et al. (ed.) Molecular Cloning: A Laboratory Manual, 2nd ed., vol. 1-3, Cold Spring Harbor Laboratory Press, Cold Spring Harbor, NY, 1989, chapters 9 and 11; and Hames and Higgins (eds.) Nucleic Acid Hybridization, IRL Press, Oxford, 1985. Further detailed instruction and guidance with regard to the hybridization of nucleic acids may be found, for example, in Tijssen, "Overview of principles of hybridization and the strategy of nucleic acid probe assays," in Laboratory Techniques in Biochemistry and Molecular Biology-Hybridization with Nucleic Acid Probes, Part I, Chapter 2, Elsevier, NY, 1993; and Ausubel et al., Eds., Current Protocols in Molecular Biology, Chapter 2, Greene Publishing and Wiley-Interscience, NY, 1995.

As used herein, "stringent conditions" encompass conditions under which hybridization will only occur if there is less than 20% mismatch between the sequence of the hybridization molecule and a homologous polynucleotide within the target nucleic acid molecule. "Stringent conditions" include further particular levels of stringency. Thus, as used herein, "moderate stringency" conditions are those under which molecules with more than 20% sequence mismatch will not hybridize; conditions of "high stringency" are those under which sequences with more than 10% mismatch will not hybridize; and conditions of "very high stringency" are those under which sequences with more than 5% mismatch will not hybridize.

The following are representative, non-limiting hybridization conditions.

High Stringency condition (detects polynucleotides that share at least 90% sequence identity): Hybridization in 5x SSC buffer at 65 °C for 16 hours; wash twice in 2x SSC buffer at room temperature for 15 minutes each; and wash twice in 0.5x SSC buffer at 65 °C for 20 minutes each.

Moderate Stringency condition (detects polynucleotides that share at least 80% sequence identity): Hybridization in 5x-6x SSC buffer at 65-70 °C for 16-20 hours; wash twice in 2x SSC buffer at room temperature for 5-20 minutes each; and wash twice in 1x SSC buffer at 55-70 °C for 30 minutes each.

Non-stringent control condition (polynucleotides that share at least 50% sequence identity will hybridize): Hybridization in 6x SSC buffer at room temperature to 55 °C for 16-20 hours; wash at least twice in 2x-3x SSC buffer at room temperature to 55 °C for 20-30 minutes each.

As used herein, the term "substantially homologous," "substantially identical," or "substantial homology," with regard to a nucleic acid, refers to a polynucleotide having contiguous nucleobases that hybridize under stringent conditions to the reference nucleic acid. For example, nucleic acids that are substantially homologous to a reference nucleic acid of any of SEQ ID NOs:1, 3, 5, and 7-12 are those nucleic acids that hybridize under stringent conditions (*e.g.*, the Moderate Stringency conditions set forth, *supra*) to the reference nucleic acid. Substantially homologous polynucleotides may have at least 80% sequence identity. For example, substantially homologous polynucleotides may have from about 80% to 100% sequence identity, such as 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; and about 100%. The property of substantial homology is closely related to specific hybridization. For example, a nucleic acid molecule is specifically hybridizable when there is a sufficient degree of complementarity to avoid non-specific binding of the nucleic acid to non-target polynucleotides under conditions where specific binding is desired, for example, under stringent hybridization conditions.

As used herein, the term "ortholog" refers to a gene in two or more species that has evolved from a common ancestral nucleic acid, and may retain the same function in the two or more species.

As used herein, two nucleic acid molecules are said to exhibit "complete complementarity" when every nucleotide of a polynucleotide read in the 5' to 3' direction is complementary to every nucleotide of the other polynucleotide when read in the 3' to 5' direction. A polynucleotide that is complementary to a reference polynucleotide will exhibit a sequence identical to the reverse complement of the reference polynucleotide. These terms and descriptions are well defined in the art and are easily understood by those of ordinary skill in the art.

Operably linked: A first polynucleotide is operably linked with a second polynucleotide when the first polynucleotide is in a functional relationship with the second polynucleotide. When recombinantly produced, operably linked polynucleotides are generally contiguous, and, where necessary to join two protein-coding regions, in the same reading frame (*e.g.,* in a translationally fused ORF). However, nucleic acids need not be contiguous to be operably linked.

The term, "operably linked," when used in reference to a regulatory genetic element and a coding polynucleotide, means that the regulatory element affects the expression of the linked coding polynucleotide. "Regulatory elements," or "control elements," refer to polynucleotides that influence the timing and level/amount of transcription, RNA processing or stability, or translation of the associated coding polynucleotide. Regulatory elements may include promoters; translation leaders; introns; enhancers; stem-loop structures; repressor binding polynucleotides; polynucleotides with a termination sequence; polynucleotides with a polyadenylation recognition sequence; *etc.* Particular regulatory elements may be located upstream and/or downstream of a coding polynucleotide operably linked thereto. Also, particular regulatory elements operably linked to a coding polynucleotide may be located on the associated complementary strand of a double-stranded nucleic acid molecule.

Promoter: As used herein, the term "promoter" refers to a region of DNA that may be upstream from the start of transcription, and that may be involved in recognition and binding of RNA polymerase and other proteins to initiate transcription. A promoter may be operably linked to a coding polynucleotide for expression in a cell, or a promoter may be operably linked to a polynucleotide encoding a signal peptide which may be operably linked to a coding polynucleotide for expression in a cell. A "plant promoter" maybe a promoter capable of initiating transcription in plant cells. Examples of promoters under developmental control include promoters that preferentially initiate transcription in certain tissues, such as leaves, roots, seeds, fibers, xylem vessels, tracheids, or sclerenchyma. Such promoters are referred to as "tissue-preferred". Promoters which initiate transcription only in certain tissues are referred to as "tissue-specific". A "cell type-specific" promoter primarily drives expression in certain cell types in one or more organs, for example, vascular cells in roots or leaves. An "inducible" promoter may be a promoter which may be under environmental control. Examples of environmental conditions that may initiate transcription by inducible promoters include anaerobic conditions and the presence of light. Tissue-specific, tissue-preferred, cell type specific, and inducible promoters constitute the class of "non-constitutive" promoters. A "constitutive" promoter is a promoter which may be active under most environmental conditions or in most tissue or cell types.

Any inducible promoter can be used in some embodiments of the invention. *See* Ward et al. (1993) Plant Mol. Biol. 22:361-366. With an inducible promoter, the rate of transcription increases in response to an inducing agent. Exemplary inducible promoters include, but are not limited to: Promoters from the ACEI system that respond to copper; *In2* gene from maize that responds to benzenesulfonamide herbicide safeners; Tet repressor from Tn10; and the inducible promoter from a steroid hormone gene, the transcriptional activity of which may be induced by a glucocorticosteroid hormone (Schena et al. (1991) Proc. Natl. Acad. Sci. USA 88:0421).

Exemplary constitutive promoters include, but are not limited to: Promoters from plant viruses, such as the 35S promoter from Cauliflower Mosaic Virus (CaMV); promoters from rice actin genes; ubiquitin promoters; pEMU; MAS; maize H3 histone promoter; and the ALS promoter, *XbaI*/*NcoI* fragment 5' to the *Brassica napus ALS3* structural gene (or a polynucleotide similar to said *Xba1*/*NcoI* fragment) (International PCT Publication No. WO96/30530).

Additionally, any tissue-specific or tissue-preferred promoter may be utilized in some embodiments of the invention. Plants transformed with a nucleic acid molecule comprising a coding polynucleotide operably linked to a tissue-specific promoter may produce the product of the coding polynucleotide exclusively, or preferentially, in a specific tissue. Exemplary tissue-specific or tissue-preferred promoters include, but are not limited to: A seed-preferred promoter, such as that from the phaseolin gene; a leaf-specific and light-induced promoter such as that from *cab* or *rubisco*; an anther-specific promoter such as that from *LAT52*; a pollen-specific promoter such as that from *Zm13*; and a microspore-preferred promoter such as that from *apg.*

Transformation: As used herein, the term "transformation" or "transduction" refers to the transfer of one or more nucleic acid molecule(s) into a cell. A cell is "transformed" by a nucleic acid molecule transduced into the cell when the nucleic acid molecule becomes stably replicated by the cell, either by incorporation of the nucleic acid molecule into the cellular genome, or by episomal replication. As used herein, the term "transformation" encompasses all techniques by which a nucleic acid molecule can be introduced into such a cell. Examples include, but are not limited to: transfection with viral vectors; transformation with plasmid vectors; electroporation (Fromm et al. (1986) Nature 319:791-3); lipofection (Felgner et al. (1987) Proc. Natl. Acad. Sci. USA 84:7413-7); microinjection (Mueller et al. (1978) Cell 15:579-85); *Agrobacterium*-mediated transfer (Fraley et al. (1983) Proc. Natl. Acad. Sci. USA 80:4803-7); direct DNA uptake; and microprojectile bombardment (Klein et al. (1987) Nature 327:70).

Transgene: An exogenous nucleic acid. In some examples, a transgene may be a DNA that encodes one or both strand(s) of an RNA capable of forming a dsRNA molecule that comprises a polyribonucleotide that is complementary to a nucleic acid molecule found in a coleopteran pest. In further examples, a transgene may be a gene (*e.g.*, a herbicide-tolerance gene, a gene encoding an industrially or pharmaceutically useful compound, or a gene encoding a desirable agricultural trait). In these and other examples, a transgene may contain regulatory elements operably linked to a coding polynucleotide of the transgene (*e.g.*, a promoter).

Vector: A nucleic acid molecule as introduced into a cell, for example, to produce a transformed cell. A vector may include genetic elements that permit it to replicate in the host cell, such as an origin of replication. Examples of vectors include, but are not limited to: a plasmid; cosmid; bacteriophage; or virus that carries exogenous DNA into a cell. A vector may also include one or more genes, including ones that produce antisense molecules, and/or selectable marker genes and other genetic elements known in the art. A vector may transduce, transform, or infect a cell, thereby causing the cell to express the nucleic acid molecules and/or proteins encoded by the vector. A vector optionally includes materials to aid in achieving entry of the nucleic acid molecule into the cell *(e.g.,* a liposome, protein coating, *etc.*).

Yield: A stabilized yield of about 100% or greater relative to the yield of check varieties in the same growing location growing at the same time and under the same conditions. In particular embodiments, "improved yield" or "improving yield" means a cultivar having a stabilized yield of 105% or greater relative to the yield of check varieties in the same growing location containing significant densities of the coleopteran pests that are injurious to that crop growing at the same time and under the same conditions, which are targeted by the compositions and methods herein.

Unless specifically indicated or implied, the terms "a," "an," and "the" signify "at least one," as used herein.

Unless otherwise specifically explained, all technical and scientific terms used herein have the same meaning as commonly understood by those of ordinary skill in the art to which this disclosure belongs. Definitions of common terms in molecular biology can be found in, for example, Lewin's Genes X, Jones & Bartlett Publishers, 2009 (ISBN 10 0763766321); Krebs et al. (eds.), The Encyclopedia of Molecular Biology, Blackwell Science Ltd., 1994 (ISBN 0-632-02182-9); and Meyers R.A. (ed.), Molecular Biology and Biotechnology: A Comprehensive Desk Reference, VCH Publishers, Inc., 1995 (ISBN 1-56081-569-8). All percentages are by weight and all solvent mixture proportions are by volume unless otherwise noted. All temperatures are in degrees Celsius.

### *IV. Nucleic Acid Molecules Comprising an Insect Pest Sequence*

#### A. Overview

Described herein are nucleic acid molecules useful for the control of insect pests. In some examples, the insect pest is a coleopteran insect pest. Described nucleic acid molecules include target polynucleotides (*e.g.*, native genes, and non-coding polynucleotides), dsRNAs, siRNAs, shRNAs, hpRNAs, and miRNAs. For example, dsRNA, siRNA, miRNA, shRNA, and/or hpRNA molecules are described in some embodiments that may be specifically complementary to all or part of one or more native nucleic acids in a coleopteran pest. In these and further embodiments, the native nucleic acid(s) may be one or more target gene(s), the product of which may be, for example and without limitation: involved in a metabolic process or involved in larval development. Nucleic acid molecules described herein, when introduced into a cell comprising at least one native nucleic acid(s) to which the nucleic acid molecules are specifically complementary, may initiate RNAi in the cell, and consequently reduce or eliminate expression of the native nucleic acid(s). In some examples, reduction or elimination of the expression of a target gene by a nucleic acid molecule specifically complementary thereto may result in reduction or cessation of growth, development, and/or feeding in the coleopteran pest.

In some embodiments, at least one target gene in an insect pest may be selected, wherein the target gene comprises a coleopteran *prp8* polynucleotide. In particular examples, a target gene comprising a coleopteran *prp8* polynucleotide is selected, wherein the target gene comprises a *Diabrotica* polynucleotide selected from among SEQ ID NOs:1, 3, and 7-11. In particular examples, a target gene comprising a coleopteran *prp8* polynucleotide is selected, wherein the target gene comprises a *Meligethes* polynucleotide selected from among SEQ ID NOs:5 and 12.

In some embodiments, a target gene may be a nucleic acid molecule comprising a polynucleotide that can be reverse translated *in silico* to a polypeptide comprising a contiguous amino acid sequence that is at least about 85% identical (e.g., at least 84%, 85%, about 90%, about 95%, about 96%, about 97%, about 98%, about 99%, about 100%, or 100% identical) to the amino acid sequence of a protein product of a *prp8* polynucleotide. A target gene may be any *prp8* polynucleotide in an insect pest, the post-transcriptional inhibition of which has a deleterious effect on the growth and/or survival of the pest, for example, to provide a protective benefit against the pest to a plant. In particular examples, a target gene is a nucleic acid molecule comprising a polynucleotide that can be reverse translated *in silico* to a polypeptide comprising a contiguous amino acid sequence that is: (i) at least about 85% identical, about 90% identical, about 95% identical, about 96% identical, about 97% identical, about 98% identical, about 99% identical, about 100% identical, or 100% identical to the amino acid sequence of SEQ ID NO:2; (ii) at least about 85% identical, about 90% identical, about 95% identical, about 96% identical, about 97% identical, about 98% identical, about 99% identical, about 100% identical, or 100% identical to the amino acid sequence of SEQ ID NO:4, or (iii) at least about 85% identical, about 90% identical, about 95% identical, about 96% identical, about 97% identical, about 98% identical, about 99% identical, about 100% identical, or 100% identical to the amino acid sequence of SEQ ID NO: 6.

Provided according to the invention are DNAs, the expression of which results in an RNA molecule comprising a polynucleotide that is specifically complementary to all or part of a native RNA molecule that is encoded by a coding polynucleotide in an insect (e.g., coleopteran) pest. In some embodiments, after ingestion of the expressed RNA molecule by an insect pest, down-regulation of the coding polynucleotide in cells of the pest may be obtained. In particular embodiments, down-regulation of the coding polynucleotide in cells of the insect pest results in a deleterious effect on the growth and/or development of the pest.

In some embodiments, target polynucleotides include transcribed non-coding RNAs, such as 5'UTRs; 3'UTRs; spliced leaders; introns; outrons (*e.g.*, 5'UTR RNA subsequently modified in trans splicing); donatrons (*e.g.*, non-coding RNA required to provide donor sequences for *trans* splicing); and other non-coding transcribed RNA of target insect pest genes. Such polynucleotides may be derived from both mono-cistronic and poly-cistronic genes.

Thus, also described herein in connection with some embodiments are iRNA molecules (e.g., dsRNAs, siRNAs, miRNAs, shRNAs, and hpRNAs) that comprise at least one polynucleotide that is specifically complementary to all or part of a target nucleic acid in an insect (*e.g.*, coleopteran) pest. In some embodiments an iRNA molecule may comprise polynucleotide(s) that are complementary to all or part of a plurality of target nucleic acids; for example, 2, 3, 4, 5, 6, 7, 8, 9, 10, or more target nucleic acids. In particular embodiments, an iRNA molecule may be produced *in vitro,* or *in vivo* by a genetically-modified organism, such as a plant or bacterium. Also disclosed are cDNAs that may be used for the production of dsRNA molecules, siRNA molecules, miRNA molecules, shRNA molecules, and/or hpRNA molecules that are specifically complementary to all or part of a target nucleic acid in an insect pest. Further described are recombinant DNA constructs for use in achieving stable transformation of particular host targets. Transformed host targets may express effective levels of dsRNA, siRNA, miRNA, shRNA, and/or hpRNA molecules from the recombinant DNA constructs. Therefore, also described is a plant transformation vector comprising at least one polynucleotide operably linked to a heterologous promoter functional in a plant cell, wherein expression of the polynucleotide(s) results in an RNA molecule comprising a string of contiguous nucleobases that is specifically complementary to all or part of a target nucleic acid in an insect pest.

In particular examples, nucleic acid molecules useful for the control of insect (e.g., coleopteran) pests may include: all or part of a native nucleic acid isolated from *Diabrotica* comprising a *prp8* polynucleotide (*e.g.,* any of SEQ ID NOs:1, 3, and 7-11); DNAs that when expressed result in an RNA molecule comprising a polynucleotide that is specifically complementary to all or part of a native RNA molecule that is encoded by *Diabrotica prp8*; iRNA molecules (*e.g.,* dsRNAs, siRNAs, miRNAs, shRNAs, and hpRNAs) that comprise at least one polynucleotide that is specifically complementary to all or part of *Diabrotica prp8*; cDNAs that may be used for the production of dsRNA molecules, siRNA molecules, miRNA molecules, shRNA molecules, and/or hpRNA molecules that are specifically complementary to all or part of *Diabrotica prp8*; all or part of a native nucleic acid isolated from *Meligethes* comprising a *prp8* polynucleotide *(e.g.,* SEQ ID NOs:5 and 12); DNAs that when expressed result in an RNA molecule comprising a polynucleotide that is specifically complementary to all or part of a native RNA molecule that is encoded by *Meligethes prp8*; iRNA molecules *(e.g.,* dsRNAs, siRNAs, miRNAs, shRNAs, and hpRNAs) that comprise at least one polynucleotide that is specifically complementary to all or part of *Meligethes prp8*; cDNAs that may be used for the production of dsRNA molecules, siRNA molecules, miRNA molecules, shRNA molecules, and/or hpRNA molecules that are specifically complementary to all or part of *Meligethes pip8;* and recombinant DNA constructs for use in achieving stable transformation of particular host targets, wherein a transformed host target comprises one or more of the foregoing nucleic acid molecules.

**B. Nucleic Acid Molecules**

The present invention provides, *inter alia*, iRNA *(e.g.,* dsRNA, siRNA, miRNA, shRNA, and hpRNA) molecules that inhibit target gene expression in a cell, tissue, or organ of an insect (e.g., coleopteran) pest; and DNA molecules capable of being expressed as an iRNA molecule in a cell or microorganism to inhibit target gene expression in a cell, tissue, or organ of an insect pest.

Some embodiments of the invention provide an isolated nucleic acid molecule comprising at least one (e.g., one, two, three, or more) polynucleotide(s) selected from the group consisting of: SEQ ID NOs:1, 3, and 5; the complement of any of SEQ ID NOs:1, 3, and 5; a fragment of at least 15, at least 18, at least 20, at least 22, at least 25, at least 28, at least 30, at least 50, at least 100, preferably at least 15 contiguous nucleotides of SEQ ID NO:1, a fragment of at least 15, at least 18, at least 20, at least 22, at least 25, at least 28, at least 30, at least 50, at least 100, preferably at least 15 contiguous nucleotides of SEQ ID NO:3, and a fragment of at least 15, at least 18, at least 20, at least 22, at least 25, at least 28, at least 30, at least 50, at least 100, preferably at least 15 contiguous nucleotides of SEQ ID NO:5 *(e.g.,* any of SEQ ID NOs:7-12); the complement of a fragment of at least 15, at least 18, at least 20, at least 22, at least 25, at least 28, at least 30, at least 50, at least 100, preferably at least 15 contiguous nucleotides of SEQ ID NO:1, the complement of a fragment of at least 15, at least 18, at least 20, at least 22, at least 25, at least 28, at least 30, at least 50, at least 100, preferably at least 15 contiguous nucleotides of SEQ ID NO:3, and the complement of a fragment of at least 15, at least 18, at least 20, at least 22, at least 25, at least 28, at least 30, at least 50, at least 100, preferably at least 15 contiguous nucleotides of SEQ ID NO:5; a native coding polynucleotide of a *Diabrotica* organism (*e.g*., WCR) comprising any of SEQ ID NOs:7, 8, 9, 10, and 11; the complement of a native coding polynucleotide of a *Diabrotica* organism comprising any of SEQ ID NOs: 7, 8, 9, 10, and 11; a fragment of at least 15, at least 18, at least 20, at least 22, at least 25, at least 28, at least 30, at least 50, preferably at least 15 of a native coding polynucleotide of a *Diabrotica* organism comprising SEQ ID NO:7, a fragment of at least 15, at least 18, at least 20, at least 22, at least 25, at least 28, at least 30, at least 50, preferably at least 15 of a native coding polynucleotide of a *Diabrotica* organism comprising SEQ ID NO:8, a fragment of at least 15, at least 18, at least 20, at least 22, at least 25, at least 28, at least 30, at least 50, preferably at least 15 of a native coding polynucleotide of a *Diabrotica* organism comprising SEQ ID NO:9, a fragment of at least 15, at least 18, at least 20, at least 22, at least 25, at least 28, at least 30, at least 50, preferably at least 15 of a native coding polynucleotide of a *Diabrotica* organism comprising SEQ ID NO: 10, and/or a fragment of at least 15, at least 18, at least 20, at least 22, at least 25, at least 28, at least 30, at least 50, preferably at least 15 of a native coding polynucleotide of a *Diabrotica* organism comprising SEQ ID NO:11; the complement of a fragment of at least 15, at least 18, at least 20, at least 22, at least 25, at least 28, at least 30, at least 50, preferably at least 15 of a native coding polynucleotide of a *Diabrotica* organism comprising SEQ ID NO:7, the complement of a fragment of at least 15, at least 18, at least 20, at least 22, at least 25, at least 28, at least 30, at least 50, preferably at least 15 of a native coding polynucleotide of a *Diabrotica* organism comprising SEQ ID NO:8, the complement of a fragment of at least 15, at least 18, at least 20, at least 22, at least 25, at least 28, at least 30, at least 50, preferably at least 15 of a native coding polynucleotide of a *Diabrotica* organism comprising SEQ ID NO:9, the complement of a fragment of at least 15, at least 18, at least 20, at least 22, at least 25, at least 28, at least 30, at least 50, preferably at least 15 of a native coding polynucleotide of a *Diabrotica* organism comprising SEQ ID NO: 10, and/or the complement of a fragment of at least 15, at least 18, at least 20, at least 22, at least 25, at least 28, at least 30, at least 50, preferably at least 15 of a native coding polynucleotide of a *Diabrotica* organism comprising SEQ ID NO:11; a native coding polynucleotide of a *Meligethes* organism *(e.g.,* PB) comprising SEQ ID NO: 12; the complement of a native coding polynucleotide of a *Meligethes* organism comprising SEQ ID NO: 12; a fragment of at least 15, at least 18, at least 20, at least 22, at least 25, at least 28, at least 30, at least 50, preferably at least 15 of a native coding polynucleotide of a *Meligethes* organism comprising SEQ ID NO: 12; and the complement of a fragment of at least 15, at least 18, at least 20, at least 22, at least 25, at least 28, at least 30, at least 50, preferably at least 15 of a native coding polynucleotide of a *Meligethes* organism comprising SEQ ID NO: 12.

In particular embodiments, contact with or uptake by an insect (e.g., coleopteran) pest of an iRNA transcribed from the isolated polynucleotide inhibits the growth, development, and/or feeding of the pest. In some embodiments, contact with or uptake by the insect occurs *via* feeding on plant material comprising the iRNA. In some embodiments, contact with or uptake by the insect occurs *via* spraying of a plant comprising the insect with a composition comprising the iRNA.

In some embodiments, an isolated nucleic acid molecule of the invention may comprise at least one (*e.g.*, one, two, three, or more) polynucleotide(s) selected from the group consisting of: SEQ ID NO:84; the complement of SEQ ID NO:84; SEQ ID NO:85; the complement of SEQ ID NO:85; SEQ ID NO:86; the complement of SEQ ID NO:86; SEQ ID NO:87; the complement of SEQ ID NO:87; SEQ ID NO:88; the complement of SEQ ID NO:88; SEQ ID NO:89; the complement of SEQ ID NO:89; SEQ ID NO:90; the complement of SEQ ID NO:90; SEQ ID NO:91; the complement of SEQ ID NO:91; SEQ ID NO:92; the complement of SEQ ID NO:92; a fragment of at least 15, at least 18, at least 20, at least 22, at least 25, at least 28, at least 30, at least 50, at least 100, preferably at least 15 contiguous nucleotides of SEQ ID NO:84, a fragment of at least 15, at least 18, at least 20, at least 22, at least 25, at least 28, at least 30, at least 50, at least 100, preferably at least 15 contiguous nucleotides of SEQ ID NO:85, and/or a fragment of at least 15, at least 18, at least 20, at least 22, at least 25, at least 28, at least 30, at least 50, at least 100, preferably at least 15 contiguous nucleotides of SEQ ID NO:91; the complement of a fragment of at least 15, at least 18, at least 20, at least 22, at least 25, at least 28, at least 30, at least 50, at least 100, preferably at least 15 contiguous nucleotides of SEQ ID NO:84, the complement of a fragment of at least 15, at least 18, at least 20, at least 22, at least 25, at least 28, at least 30, at least 50, at least 100, preferably at least 15 contiguous nucleotides of SEQ ID NO:85, and/or the complement of a fragment of at least 15, at least 18, at least 20, at least 22, at least 25, at least 28, at least 30, at least 50, at least 100, preferably at least 15 contiguous nucleotides of SEQ ID NO:91; a native coding polynucleotide of a *Diabrotica* organism comprising any of SEQ ID NOs:86, 87, 88, 89 and 90; the complement of a native coding polynucleotide of a *Diabrotica* organism comprising any of SEQ ID NOs:86, 87, 88, 89 and 90; a fragment of at least 15, at least 18, at least 20, at least 22, at least 25, at least 28, at least 30, at least 50, preferably at least 15 of a native coding polynucleotide of a *Diabrotica* organism comprising SEQ ID NO: 86, a fragment of at least 15, at least 18, at least 20, at least 22, at least 25, at least 28, at least 30, at least 50, preferably at least 15 of a native coding polynucleotide of a *Diabrotica* organism comprising SEQ ID NO:87, a fragment of at least 15, at least 18, at least 20, at least 22, at least 25, at least 28, at least 30, at least 50, preferably at least 15 of a native coding polynucleotide of a *Diabrotica* organism comprising SEQ ID NO:88, a fragment of at least 15, at least 18, at least 20, at least 22, at least 25, at least 28, at least 30, at least 50, preferably at least 15 of a native coding polynucleotide of a *Diabrotica* organism comprising SEQ ID NO:89, and/or a fragment of at least 15, at least 18, at least 20, at least 22, at least 25, at least 28, at least 30, at least 50, preferably at least 15 of a native coding polynucleotide of a *Diabrotica* organism comprising SEQ ID NO:90; and the complement of a fragment of at least 15, at least 18, at least 20, at least 22, at least 25, at least 28, at least 30, at least 50, preferably at least 15 of a native coding polynucleotide of a *Diabrotica* organism comprising SEQ ID NO:86, the complement of a fragment of at least 15, at least 18, at least 20, at least 22, at least 25, at least 28, at least 30, at least 50, preferably at least 15 of a native coding polynucleotide of a *Diabrotica* organism comprising SEQ ID NO:87, the complement of a fragment of at least 15, at least 18, at least 20, at least 22, at least 25, at least 28, at least 30, at least 50, preferably at least 15 of a native coding polynucleotide of a *Diabrotica* organism comprising SEQ ID NO:88, the complement of a fragment of at least 15, at least 18, at least 20, at least 22, at least 25, at least 28, at least 30, at least 50, preferably at least 15 of a native coding polynucleotide of a *Diabrotica* organism comprising SEQ ID NO:89, and/or the complement of a fragment of at least 15, at least 18, at least 20, at least 22, at least 25, at least 28, at least 30, at least 50, preferably at least 15 of a native coding polynucleotide of a *Diabrotica* organism comprising SEQ ID NO:90; a native coding polynucleotide of a *Meligethes* organism comprising SEQ ID NO:92; the complement of a native coding polynucleotide of a *Meligethes* organism comprising SEQ ID NO:92; a fragment of at least 15, at least 18, at least 20, at least 22, at least 25, at least 28, at least 30, at least 50, preferably at least 15 of a native coding polynucleotide of a *Meligethes* organism comprising SEQ ID NO:92; and the complement of a fragment of at least 15, at least 18, at least 20, at least 22, at least 25, at least 28, at least 30, at least 50, preferably at least 15 of a native coding polynucleotide of a *Meligethes* organism comprising SEQ ID NO:92.

In particular embodiments, contact with or uptake by a coleopteran pest of the isolated polynucleotide inhibits the growth, development, and/or feeding of the pest. In some embodiments, contact with or uptake by the insect occurs *via* feeding on plant material or bait comprising the iRNA. In some embodiments, contact with or uptake by the coleopteran pest occurs *via* spraying of a plant comprising the insect with a composition comprising the iRNA.

In certain embodiments, dsRNA molecules provided by the invention comprise polynucleotides complementary to a transcript from a target gene comprising any of SEQ ID NOs:1, 3, 5, 7, 8, 9, 10, 11 and 12, and fragments thereof, the inhibition of which target gene in an insect pest results in the reduction or removal of a polypeptide or polynucleotide agent that is essential for the pest's growth, development, or other biological function. A selected polynucleotide may exhibit from about 80% to about 100% sequence identity to SEQ ID NO:1, from about 80% to about 100% sequence identity to SEQ ID NO:3, from about 80% to about 100% sequence identity to SEQ ID NO:5, from about 80% to about 100% sequence identity to SEQ ID NO:7, from about 80% to about 100% sequence identity to SEQ ID NO:8, from about 80% to about 100% sequence identity to SEQ ID NO:9, from about 80% to about 100% sequence identity to SEQ ID NO: 10, from about 80% to about 100% sequence identity to SEQ ID NO: 11, or from about 80% to about 100% sequence identity to SEQ ID NO:12; from about 80% to about 100% sequence identity to a contiguous fragment of any of SEQ ID NO:1, from about 80% to about 100% sequence identity to a contiguous fragment of any of SEQ ID NO:3, from about 80% to about 100% sequence identity to a contiguous fragment of any of SEQ ID NO:5, from about 80% to about 100% sequence identity to a contiguous fragment of any of SEQ ID NO:7, from about 80% to about 100% sequence identity to a contiguous fragment of any of SEQ ID NO:8, from about 80% to about 100% sequence identity to a contiguous fragment of any of SEQ ID NO:9, from about 80% to about 100% sequence identity to a contiguous fragment of any of SEQ ID NO:10, from about 80% to about 100% sequence identity to a contiguous fragment of any of SEQ ID NO:11, or from about 80% to about 100% sequence identity to a contiguous fragment of any of SEQ ID NO:12; and the complement of any of the foregoing. For example, a selected polynucleotide may exhibit 79%; 80%; about 81%; about 82%; about 83%; about 84%; about 85%; about 86%; about 87%; about 88%; about 89%; about 90%; about 91 %; about 92%; about 93%; about 94% about 95%; about 96%; about 97%; about 98%; about 98.5%; about 99%; about 99.5%; or about 100% sequence identity to SEQ ID NO:1, may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to SEQ ID NO:3, may exhibit 79%; 80%; about 81 %; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to SEQ ID NO:5, may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to SEQ ID NO:7, may exhibit 79%; 80%; about 81%; about 82%; about 83%; about 84%; about 85%; about 86%; about 87%; about 88%; about 89%; about 90%; about 91 %; about 92%; about 93%; about 94% about 95%; about 96%; about 97%; about 98%; about 98.5%; about 99%; about 99.5%; or about 100% sequence identity to SEQ ID NO:8, may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to SEQ ID NO:9, may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to SEQ ID NO:10, may exhibit 79%; 80%; about 81%; about 82%; about 83%; about 84%; about 85%; about 86%; about 87%; about 88%; about 89%; about 90%; about 91 %; about 92%; about 93%; about 94% about 95%; about 96%; about 97%; about 98%; about 98.5%; about 99%; about 99.5%; or about 100% sequence identity to SEQ ID NO:11 or may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to SEQ ID NO:12; may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to a contiguous fragment of SEQ ID NO:1, may exhibit 79%; 80%; about 81 %; about 82%; about 83%; about 84%; about 85%; about 86%; about 87%; about 88%; about 89%; about 90%; about 91 %; about 92%; about 93%; about 94% about 95%; about 96%; about 97%; about 98%; about 98.5%; about 99%; about 99.5%; or about 100% sequence identity to a contiguous fragment of SEQ ID NO:3, may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to a contiguous fragment of SEQ ID NO:5, may exhibit 79%; 80%; about 81 %; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to a contiguous fragment of SEQ ID NO:7, may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to a contiguous fragment of SEQ ID NO:8, may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to a contiguous fragment of SEQ ID NO:9, may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to a contiguous fragment of SEQ ID NO:10, may exhibit 79%; 80%; about 81%; about 82%; about 83%; about 84%; about 85%; about 86%; about 87%; about 88%; about 89%; about 90%; about 91%; about 92%; about 93%; about 94% about 95%; about 96%; about 97%; about 98%; about 98.5%; about 99%; about 99.5%; or about 100% sequence identity to a contiguous fragment ofSEQ ID NO:11, or may exhibit 79%; 80%; about 81%; about 82%; about 83%; about 84%; about 85%; about 86%; about 87%; about 88%; about 89%; about 90%; about 91 %; about 92%; about 93%; about 94% about 95%; about 96%; about 97%; about 98%; about 98.5%; about 99%; about 99.5%; or about 100% sequence identity to a contiguous fragment of SEQ ID NO:12; and the complement of any of the foregoing. In some examples, a dsRNA molecule is transcribed from a polynucleotide containing a sense nucleotide sequence that is substantially identical or identical to a contiguous fragment of any of SEQ ID NOs:1, 3, 5, 7, 8, 9, 10, 11, and 12; an antisense nucleotide sequence that is at least substantially the reverse complement of the sense nucleotide sequence; and an intervening nucleotide sequence positioned between the sense and the antisense sequences, such that the sense and antisense polyribonucleotides transcribed from the respective nucleotide sequences hybridize to form a "stem" structure in the dsRNA, and polyribonucleotide transcribed from the intervening sequence forms a "loop." Such a dsRNA molecule may be referred to as a hairpin RNA (hpRNA) molecule.

In some embodiments, a DNA molecule capable of being expressed as an iRNA molecule in a cell or microorganism to inhibit target gene expression may comprise a single polynucleotide that is specifically complementary to all or part of a native polynucleotide found in one or more target insect pest species *(e.g.,* a coleopteran pest species), or the DNA molecule can be constructed as a chimera from a plurality of such specifically complementary polynucleotides.

In some embodiments, a nucleic acid molecule may comprise a first and a second polynucleotide separated by a "spacer." A spacer may be a region comprising any sequence of nucleotides that facilitates secondary structure formation between the first and second polynucleotides, where this is desired. In one embodiment, the spacer is part of a sense or antisense coding polynucleotide for mRNA. The spacer may alternatively comprise any combination of nucleotides or homologues thereof that are capable of being linked covalently to a nucleic acid molecule. In some examples, the spacer may be an intron (*e.g.*, as ST-LS1 intron or a RTM1 intron).

For example, in some embodiments, the DNA molecule may comprise a polynucleotide coding for one or more different iRNA molecules, wherein each of the different iRNA molecules comprises a first polyribonucleotide and a second polyribonucleotide, wherein the first and second polyribonucleotides are complementary to each other. The first and second polyribonucleotides may be connected within an RNA molecule by a spacer. The spacer may constitute part of the first polyribonucleotide or the second polyribonucleotide. Expression of a RNA molecule comprising the first and second polyribonucleotides may lead to the formation of a dsRNA molecule by specific intramolecular base-pairing of the first and second polyribonucleotides. The first polyribonucleotide or the second polyribonucleotide may be substantially identical to a polyribonucleotide *(e.g.,* a transcript of a target gene or transcribed non-coding polynucleotide) native to an insect pest, a derivative thereof, or a complementary polynucleotide thereto.

dsRNA nucleic acid molecules comprise double strands of polymerized ribonucleotides, and may include modifications to either the phosphate-sugar backbone or the nucleoside. Modifications in RNA structure may be tailored to allow specific inhibition. In one embodiment, dsRNA molecules may be modified through a ubiquitous enzymatic process so that siRNA molecules may be generated. This enzymatic process may utilize an RNase III enzyme, such as DICER in eukaryotes, either *in vitro* or *in vivo*. *See* Elbashir et al. (2001) Nature 411:494-8; and Hamilton and Baulcombe (1999) Science 286(5441):950-2. DICER or functionally-equivalent RNase III enzymes cleave larger dsRNA strands and/or hpRNA molecules into smaller oligonucleotides (*e.g.*, siRNAs), each of which is about 19-25 nucleotides in length. The siRNA molecules produced by these enzymes have 2 to 3 nucleotide 3' overhangs, and 5' phosphate and 3' hydroxyl termini. The siRNA molecules generated by RNase III enzymes are unwound and separated into single-stranded RNA in the cell. The siRNA molecules then specifically hybridize with RNAs transcribed from a target gene, and both RNA molecules are subsequently degraded by an inherent cellular RNA-degrading mechanism. This process may result in the effective degradation or removal of the RNA encoded by the target gene in the target organism. The outcome is the post-transcriptional silencing of the targeted gene. In some embodiments, siRNA molecules produced by endogenous RNase III enzymes from heterologous nucleic acid molecules may efficiently mediate the down-regulation of target genes in insect pests.

In some embodiments, a nucleic acid molecule may include at least one non-naturally occurring polynucleotide that can be transcribed into a single-stranded RNA molecule capable of forming a dsRNA molecule *in vivo* through intermolecular hybridization. Such dsRNAs typically self-assemble, and can be provided in the nutrition source of an insect (e.g., coleopteran) pest to achieve the post-transcriptional inhibition of a target gene. In these and further embodiments, a nucleic acid molecule may comprise two different non-naturally occurring polynucleotides, each of which is specifically complementary to a different target gene in an insect pest. When such a nucleic acid molecule is provided as a dsRNA molecule to, for example, a coleopteran pest, the dsRNA molecule inhibits the expression of at least two different target genes in the pest.

**C. Obtaining Nucleic Acid Molecules**

A variety of polynucleotides in insect (e.g., coleopteran) pests may be used as targets for the design of nucleic acid molecules, such as iRNAs and DNA molecules encoding iRNAs. Selection of native polynucleotides is not, however, a straight-forward process. For example, only a small number of native polynucleotides in a coleopteran pest will be effective targets. It cannot be predicted with certainty whether a particular native polynucleotide can be effectively down-regulated by nucleic acid molecules of the invention, or whether down-regulation of a particular native polynucleotide will have a detrimental effect on the growth, viability, feeding, and/or survival of an insect pest. The vast majority of native coleopteran pest polynucleotides, such as ESTs isolated therefrom (for example, the coleopteran pest polynucleotides listed in U.S. Patent 7,612,194), do not have a detrimental effect on the growth and/or viability of the pest. Neither is it predictable which of the native polynucleotides that may have a detrimental effect on an insect pest are able to be used in recombinant techniques for expressing nucleic acid molecules complementary to such native polynucleotides in a host plant and providing the detrimental effect on the pest upon feeding without causing harm to the host plant.

In some embodiments, nucleic acid molecules (*e.g.*, dsRNA molecules to be provided in the host plant of an insect pest) target cDNAs that encode proteins or parts of proteins essential for pest development and/or survival, such as polypeptides involved in metabolic or catabolic biochemical pathways, cell division, energy metabolism, digestion, host plant recognition, and the like. As described herein, ingestion of compositions by a target pest organism containing one or more dsRNAs, at least one segment of which is specifically complementary to at least a substantially identical segment of RNA produced in the cells of the target pest organism, can result in the death or other inhibition of the target. A polynucleotide, either DNA or RNA, derived from an insect pest can be used to construct plant cells protected against infestation by the pests. The host plant of the coleopteran pest (*e.g., Z. mays* or *Brassica* sp.), for example, can be transformed to contain one or more polynucleotides derived from the coleopteran pest as provided herein. The polynucleotide transformed into the host may encode one or more RNAs that form into a dsRNA structure in the cells or biological fluids within the transformed host, thus making the dsRNA available if/when the pest forms a nutritional relationship with the transgenic host. This may result in the suppression of expression of one or more genes in the cells of the pest, and ultimately death or inhibition of its growth or development.

In particular embodiments, a gene is targeted that is essentially involved in the growth and development of an insect (e.g., coleopteran) pest. Other target genes for use in the present invention may include, for example, those that play important roles in pest viability, movement, migration, growth, development, infectivity, and establishment of feeding sites. A target gene may therefore be a housekeeping gene or a transcription factor. Additionally, a native insect pest polynucleotide for use in the present invention may also be derived from a homolog (e.g., an ortholog), of a plant, viral, bacterial or insect gene, the function of which is known to those of skill in the art, and the polynucleotide of which is specifically hybridizable with a target gene in the genome of the target pest. Methods of identifying a homolog of a gene with a known nucleotide sequence by hybridization are known to those of skill in the art.

In some embodiments, the invention provides methods for obtaining a nucleic acid molecule comprising a polynucleotide for producing an iRNA (e.g., dsRNA, siRNA, miRNA, shRNA, and hpRNA) molecule. One such embodiment comprises: (a) analyzing one or more target gene(s) for their expression, function, and phenotype upon dsRNA-mediated gene suppression in an insect (*e.g.*, coleopteran) pest; (b) probing a cDNA or gDNA library with a probe comprising all or a portion of a polynucleotide or a homolog thereof from a targeted pest that displays an altered (*e.g.*, reduced) growth or development phenotype in a dsRNA-mediated suppression analysis; (c) identifying a DNA clone that specifically hybridizes with the probe; (d) isolating the DNA clone identified in step (b); (e) sequencing the cDNA or gDNA fragment that comprises the clone isolated in step (d), wherein the sequenced nucleic acid molecule comprises all or a substantial portion of the RNA or a homolog thereof; and (f) chemically synthesizing all or a substantial portion of a gene, or an siRNA, miRNA, hpRNA, mRNA, shRNA, or dsRNA.

In further embodiments, a method for obtaining a nucleic acid fragment comprising a polynucleotide for producing a substantial portion of an iRNA (*e.g.*, dsRNA, siRNA, miRNA, shRNA, and hpRNA) molecule includes: (a) synthesizing first and second oligonucleotide primers specifically complementary to a portion of a native polynucleotide from a targeted insect (*e.g.,* coleopteran) pest; and (b) amplifying a cDNA or gDNA insert present in a cloning vector using the first and second oligonucleotide primers of step (a), wherein the amplified nucleic acid molecule comprises a substantial portion of a siRNA, miRNA, hpRNA, mRNA, shRNA, or dsRNA molecule.

Nucleic acids can be isolated, amplified, or produced by a number of approaches. For example, an iRNA (*e.g.*, dsRNA, siRNA, miRNA, shRNA, and hpRNA) molecule may be obtained by PCR amplification of a target polynucleotide (*e.g.*, a target gene or a target transcribed non-coding polynucleotide) derived from a gDNA or cDNA library, or portions thereof. DNA or RNA may be extracted from a target organism, and nucleic acid libraries may be prepared therefrom using methods known to those ordinarily skilled in the art. gDNA or cDNA libraries generated from a target organism may be used for PCR amplification and sequencing of target genes. A confirmed PCR product may be used as a template for *in vitro* transcription to generate sense and antisense RNA with minimal promoters. Alternatively, nucleic acid molecules may be synthesized by any of a number of techniques (*See, e.g.,* Ozaki et al. (1992) Nucleic Acids Research, 20: 5205-5214; and Agrawal et al. (1990) Nucleic Acids Research, 18: 5419-5423), including use of an automated DNA synthesizer (for example, a P.E. Biosystems, Inc. (Foster City, Calif.) model 392 or 394 DNA/RNA Synthesizer), using standard chemistries, such as phosphoramidite chemistry. *See, e.g.,* Beaucage et al. (1992) Tetrahedron, 48: 2223-2311; U.S. Patents 4,980,460, 4,725,677, 4,415,732, 4,458,066, and 4,973,679. Alternative chemistries resulting in non-natural backbone groups, such as phosphorothioate, phosphoramidate, and the like, can also be employed.

An RNA, dsRNA, siRNA, miRNA, shRNA, or hpRNA molecule of the present invention may be produced chemically or enzymatically by one skilled in the art through manual or automated reactions, or *in vivo* in a cell comprising a nucleic acid molecule comprising a polynucleotide encoding the RNA, dsRNA, siRNA, miRNA, shRNA, or hpRNA molecule. RNA may also be produced by partial or total organic synthesis- any modified ribonucleotide can be introduced by *in vitro* enzymatic or organic synthesis. An RNA molecule may be synthesized by a cellular RNA polymerase or a bacteriophage RNA polymerase (*e.g.*, T3 RNA polymerase, T7 RNA polymerase, and SP6 RNA polymerase). Expression constructs useful for the cloning and expression of polynucleotides are known in the art. *See, e.g.,* International PCT Publication No. WO97/32016; and U.S. Patents 5,593,874, 5,698,425, 5,712,135, 5,789,214, and 5,804,693. RNA molecules that are synthesized chemically or by *in vitro* enzymatic synthesis may be purified prior to introduction into a cell. For example, RNA molecules can be purified from a mixture by extraction with a solvent or resin, precipitation, electrophoresis, chromatography, or a combination thereof. Alternatively, RNA molecules that are synthesized chemically or by *in vitro* enzymatic synthesis may be used with no or a minimum of purification, for example, to avoid losses due to sample processing. The RNA molecules may be dried for storage or dissolved in an aqueous solution. The solution may contain buffers or salts to promote annealing, and/or stabilization of dsRNA molecule duplex strands.

In embodiments, a dsRNA molecule maybe formed by a single self-complementary RNA strand or from two complementary RNA strands. dsRNA molecules may be synthesized either *in vivo* or *in vitro.* An endogenous RNA polymerase of the cell may mediate transcription of the one or two RNA strands *in vivo*, or cloned RNA polymerase may be used to mediate transcription *in vivo* or *in vitro.* Post-transcriptional inhibition of a target gene in an insect pest may be host-targeted by specific transcription in an organ, tissue, or cell type of the host *(e.g.,* by using a tissue-specific promoter); stimulation of an environmental condition in the host *(e.g.,* by using an inducible promoter that is responsive to infection, stress, temperature, and/or chemical inducers); and/or engineering transcription at a developmental stage or age of the host *(e.g.,* by using a developmental stage-specific promoter). RNA strands that form a dsRNA molecule, whether transcribed *in vitro* or *in vivo*, may or may not be polyadenylated, and may or may not be capable of being translated into a polypeptide by a cell's translational apparatus.

**D. Recombinant Vectors and Host Cell Transformation**

In some embodiments, the invention also provides a DNA molecule for introduction into a cell *(e.g.,* a bacterial cell, a yeast cell, or a plant cell), wherein the DNA molecule comprises a polynucleotide that, upon expression to RNA and ingestion by an insect (*e.g.*, coleopteran) pest, achieves suppression of a target gene in a cell, tissue, or organ of the pest. Thus, some embodiments provide a recombinant nucleic acid molecule comprising a polynucleotide capable of being expressed as an iRNA (*e.g.*, dsRNA, siRNA, miRNA, shRNA, and hpRNA) molecule in a plant cell to inhibit target gene expression in an insect pest. In order to initiate or enhance expression, such recombinant nucleic acid molecules may comprise one or more regulatory elements, which regulatory elements may be operably linked to the polynucleotide capable of being expressed as an iRNA. Methods to express a gene suppression molecule in plants are known, and may be used to express a polynucleotide of the present invention. *See*, *e.g.,* International PCT Publication No. WO06/073727; and U.S. Patent Publication No. 2006/0200878 A1)

In specific embodiments, a recombinant DNA molecule of the invention may comprise a polynucleotide encoding an RNA that may form a dsRNA molecule. Such recombinant DNA molecules may encode RNAs that may form dsRNA molecules capable of inhibiting the expression of endogenous target gene(s) in an insect (*e.g*., coleopteran) pest cell upon ingestion. In many embodiments, a transcribed RNA may form a dsRNA molecule that may be provided in a stabilized form; *e.g.*, as a hairpin and stem and loop structure.

In some embodiments, one strand of a dsRNA molecule may be formed by transcription from a polynucleotide which is substantially homologous to a polynucleotide selected from the group consisting of SEQ ID NOs:1, 3, and 5; the complements of SEQ ID NOs:1, 3, and 5; a fragment of at least 15, at least 18, at least 20, at least 22, at least 25, at least 28, at least 30, at least 50, at least 100, preferably at least 15 contiguous nucleotides of SEQ ID NO:1, a fragment of at least 15, at least 18, at least 20, at least 22, at least 25, at least 28, at least 30, at least 50, at least 100, preferably at least 15 contiguous nucleotides of SEQ ID NO:3, and/or a fragment of at least 15, at least 18, at least 20, at least 22, at least 25, at least 28, at least 30, at least 50, at least 100, preferably at least 15 contiguous nucleotides of SEQ ID NO:5 *(e.g.,* SEQ ID NOs:7-12); the complement of a fragment of at least 15, at least 18, at least 20, at least 22, at least 25, at least 28, at least 30, at least 50, at least 100, preferably at least 15 contiguous nucleotides of SEQ ID NO: 1, the complement of a fragment of at least 15, at least 18, at least 20, at least 22, at least 25, at least 28, at least 30, at least 50, at least 100, preferably at least 15 contiguous nucleotides of SEQ ID NO:3, and/or the complement of a fragment of at least 15, at least 18, at least 20, at least 22, at least 25, at least 28, at least 30, at least 50, at least 100, preferably at least 15 contiguous nucleotides of SEQ ID NO:5; a native coding polynucleotide of a *Diabrotica* organism *(e.g.,* WCR) comprising any of SEQ ID NOs:7, 8, 9, 10 and 11; the complement of a native coding polynucleotide of a *Diabrotica* organism comprising any of SEQ ID NOs:7, 8, 9, 10 and 11; a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:7, a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:8, a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO: 9, a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:10, and/or a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO: 11; the complement of a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:7, the complement of a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:8, the complement of a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:9, the complement of a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:10, and/or the complement of a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:11; a native coding polynucleotide of a *Meligethes* organism (*e.g.,* PB) comprising SEQ ID NO: 12; the complement of a native coding polynucleotide of a *Meligethes* organism comprising SEQ ID NO:12; a a fragment of at least 15, at least 18, at least 20, at least 22, at least 25, at least 28, at least 30, at least 50, at least 100, preferably at least 15 contiguous nucleotides of a native coding polynucleotide of a *Meligethes* organism comprising SEQ ID NO: 12; and the complement of a fragment of at least 15, at least 18, at least 20, at least 22, at least 25, at least 28, at least 30, at least 50, at least 100, preferably at least 15 contiguous nucleotides of a native coding polynucleotide of a *Meligethes* organism comprising SEQ ID NO: 12.

In some embodiments, one strand of a dsRNA molecule may be formed by transcription from a polynucleotide that is substantially homologous to a polynucleotide selected from the group consisting of SEQ ID NOs:7, 8, 9, 10, 11, and 12; the complement of any of SEQ ID NOs:7, 8, 9, 10, 11 and 12; fragments of at least 15, at least 18, at least 20, at least 22, at least 25, at least 28, at least 30, at least 50, at least 100, preferably at least 15 contiguous nucleotides of any of SEQ ID NOs: 7, 8, 9, 10, 11, and 12; and the complements of fragments of at least 15, at least 18, at least 20, at least 22, at least 25, at least 28, at least 30, at least 50, at least 100, preferably at least 15 contiguous nucleotides of any of SEQ ID NOs: 7, 8, 9, 10, 11, and 12.

In particular embodiments, a recombinant DNA molecule encoding an RNA that may form a dsRNA molecule may comprise a coding region wherein at least two polynucleotides are arranged such that one polynucleotide is in a sense orientation, and the other polynucleotide is in an antisense orientation, relative to at least one promoter, wherein the sense polynucleotide and the antisense polynucleotide are linked or connected by a spacer of, for example, from about five (~5) to about one thousand (~1000) nucleotides. The spacer may form a loop between the sense and antisense polynucleotides. The sense polynucleotide or the antisense polynucleotide may be substantially homologous to a target gene (*e.g.,* a *prp8* gene comprising any of SEQ ID NOs:1, 3, 5, 7, 8, 9, 10, 11 and 12) or fragment thereof. In some embodiments, however, a recombinant DNA molecule may encode an RNA that may form a dsRNA molecule without a spacer. In embodiments, a sense coding polynucleotide and an antisense coding polynucleotide may be different lengths.

Polynucleotides identified as having a deleterious effect on an insect pest or a plant-protective effect with regard to the pest may be readily incorporated into expressed dsRNA molecules through the creation of appropriate expression cassettes in a recombinant nucleic acid molecule of the invention. For example, such polynucleotides may be expressed as a hairpin with stem and loop structure by taking a first segment corresponding to a target gene polynucleotide (*e.g.,* a *prp8* gene comprising any of SEQ ID NOs:1,3,5, and 7-12, and fragments of any of the foregoing); linking this polynucleotide to a second segment spacer region that is not homologous or complementary to the first segment; and linking this to a third segment, wherein at least a portion of the third segment is substantially complementary to the first segment. The transcript of such a construct forms a stem and loop structure by intramolecular base-pairing of the polyribonucleotide encoded by the first segment with the polyribonucleotide encoded by the third segment, wherein the loop structure forms comprising the polyribonucleotide encoded by the second segment. *See, e.g.,* U.S. Patent Publication Nos. 2002/0048814 and 2003/0018993; and International PCT Publication Nos. WO94/01550 and WO98/05770. A dsRNA molecule may be generated, for example, in the form of a double-stranded structure such as a stem-loop structure (*e.g.*, hairpin), whereby production of siRNA targeted for a native insect (*e.g.,* coleopteran) pest polynucleotide is enhanced by co-expression of a fragment of the targeted gene, for instance on an additional plant expressible cassette, that leads to enhanced siRNA production, or reduces methylation to prevent transcriptional gene silencing of the dsRNA hairpin promoter.

Certain embodiments of the invention include introduction of a recombinant nucleic acid molecule of the present invention into a plant (*i.e.,* transformation) to achieve insect *(e.g.,* coleopteran) pest-inhibitory levels of expression of one or more iRNA molecules. A recombinant DNA molecule may, for example, be a vector, such as a linear or a closed circular plasmid. The vector system may be a single vector or plasmid, or two or more vectors or plasmids that together contain the total DNA to be introduced into the genome of a host. In addition, a vector may be an expression vector. Polynucleotides of the invention can, for example, be suitably inserted into a vector under the control of a suitable promoter that functions in one or more hosts to drive expression of a linked coding polynucleotide or other DNA element. Many vectors are available for this purpose, and selection of the appropriate vector will depend mainly on the size of the nucleic acid to be inserted into the vector and the particular host cell to be transformed with the vector. Each vector contains various components depending on its function (*e.g*., amplification of DNA or expression of DNA) and the particular host cell with which it is compatible.

To impart protection from a coleopteran insect pest to a transgenic plant, a recombinant DNA may, for example, be transcribed into an iRNA molecule (*e.g.,* an RNA molecule that forms a dsRNA molecule) within the tissues or fluids of the recombinant plant. An iRNA molecule may comprise a polyribonucleotide that is substantially homologous and specifically hybridizable to a corresponding transcribed polyribonucleotide within an insect pest that may cause damage to the host plant species. The pest may contact the iRNA molecule that is transcribed in cells of the transgenic host plant, for example, by ingesting cells or fluids of the transgenic host plant that comprise the iRNA molecule. Thus, in particular examples, expression of a target gene is suppressed by the iRNA molecule within insect pests that infest the transgenic host plant. In some embodiments, suppression of expression of the target gene in a target coleopteran pest may result in the plant being protected against attack by the pest.

In order to enable delivery of iRNA molecules to an insect pest in a nutritional relationship with a plant cell that has been transformed with a recombinant nucleic acid molecule of the invention, expression (*i.e.,* transcription) of iRNA molecules in the plant cell is required. Thus, a recombinant nucleic acid molecule may comprise a polynucleotide of the invention operably linked to one or more regulatory elements, such as a heterologous promoter element that functions in a host cell, such as a bacterial cell wherein the nucleic acid molecule is to be amplified, and a plant cell wherein the nucleic acid molecule is to be expressed.

Promoters suitable for use in nucleic acid molecules of the invention include those that are inducible, viral, synthetic, or constitutive, all of which are well known in the art. Non-limiting examples describing such promoters include U.S. Patents 6,437,217 (maize RS81 promoter); 5,641,876 (rice actin promoter); 6,426,446 (maize RS324 promoter); 6,429,362 (maize PR-1 promoter); 6,232,526 (maize A3 promoter); 6,177,611 (constitutive maize promoters); 5,322,938, 5,352,605,5,359,142, and 5,530,196 (CaMV 35S promoter); 6,433,252 (maize L3 oleosin promoter); 6,429,357 (rice actin 2 promoter, and rice actin 2 intron); 6,294,714 (light-inducible promoters); 6,140,078 (salt-inducible promoters); 6,252,138 (pathogen-inducible promoters); 6,175,060 (phosphorous deficiency-inducible promoters); 6,388,170 (bidirectional promoters); 6,635,806 (gamma-coixin promoter); and U.S. Patent Publication No. 2009/757,089 (maize chloroplast aldolase promoter). Additional promoters include the nopaline synthase (NOS) promoter (Ebert et al. (1987) Proc. Natl. Acad. Sci. USA 84(16):5745-9) and the octopine synthase (OCS) promoters (which are carried on tumor-inducing plasmids of *Agrobacterium tumefaciens*); the caulimovirus promoters such as the cauliflower mosaic virus (CaMV) 19S promoter (Lawton et al. (1987) Plant Mol. Biol. 9:315-24); the CaMV 35S promoter (Odell et al. (1985) Nature 313:810-2; the figwort mosaic virus 35S-promoter (Walker et al. (1987) Proc. Natl. Acad. Sci. USA 84(19):6624-8); the sucrose synthase promoter (Yang and Russell (1990) Proc. Natl. Acad. Sci. USA 87:4144-8); the R gene complex promoter (Chandler et al. (1989) Plant Cell 1:1175-83); the chlorophyll a/b binding protein gene promoter; CaMV 35S (U.S. Patents 5,322,938, 5,352,605, 5,359,142, and 5,530,196); FMV 35S (U.S. Patents 6,051,753, and 5,378,619); a PC1SV promoter (U.S. Patent 5,850,019); the SCP1 promoter (U.S. Patent 6,677,503); and AGRtu.nos promoters (GenBank™ Accession No. V00087; Depicker et al. (1982) J. Mol. Appl. Genet. 1:561-73; Bevan et al. (1983) Nature 304:184-7).

In particular embodiments, nucleic acid molecules of the invention comprise a tissue-specific promoter, such as a root-specific promoter. Root-specific promoters drive expression of operably-linked coding polynucleotides exclusively or preferentially in root tissue. Examples of root-specific promoters are known in the art. *See, e.g.,* U.S. Patents 5,110,732; 5,459,252 and 5,837,848; and Opperman et al. (1994) Science 263:221-3; and Hirel et al. (1992) Plant Mol. Biol. 20:207-18. In some embodiments, a polynucleotide or fragment for coleopteran pest control according to the invention may be cloned between two root-specific promoters oriented in opposite transcriptional directions relative to the polynucleotide or fragment, and which are operable in a transgenic plant cell and expressed therein to produce RNA molecules in the transgenic plant cell that subsequently may form dsRNA molecules, as described, *supra.* The iRNA molecules expressed in plant tissues may be ingested by an insect pest so that suppression of target gene expression is achieved.

Additional regulatory elements that may optionally be operably linked to a nucleic acid include 5'UTRs located between a promoter element and a coding polynucleotide that function as a translation leader element. The translation leader element is present in fully-processed mRNA, and it may affect processing of the primary transcript, and/or RNA stability. Examples of translation leader elements include maize and petunia heat shock protein leaders (U.S. Patent 5,362,865), plant virus coat protein leaders, plant rubisco leaders, and others. *See, e.g.,* Turner and Foster (1995) Molecular Biotech. 3(3):225-36. Non-limiting examples of 5'UTRs include GmHsp (U.S. Patent 5,659,122); PhDnaK (U.S. Patent 5,362,865); AtAnt1; TEV (Carrington and Freed (1990) J. Virol. 64:1590-7); and AGRtunos (GenBank™ Accession No. V00087; and Bevan et al. (1983) Nature 304:184-7).

Additional regulatory elements that may optionally be operably linked to a nucleic acid also include 3' non-translated elements, 3' transcription termination regions, or polyadenylation regions. These are genetic elements located downstream of a polynucleotide, and include polynucleotides that provide polyadenylation signal, and/or other regulatory signals capable of affecting transcription or mRNA processing. The polyadenylation signal functions in plants to cause the addition of polyadenylate nucleotides to the 3' end of the mRNA precursor. The polyadenylation element can be derived from a variety of plant genes, or from T-DNA genes. A non-limiting example of a 3' transcription termination region is the nopaline synthase 3' region (nos 3'; Fraley et al. (1983) Proc. Natl. Acad. Sci. USA 80:4803-7). An example of the use of different 3' non-translated regions is provided in Ingelbrecht et al., (1989) Plant Cell 1:671-80. Non-limiting examples of polyadenylation signals include one from a *Pisum sativum* RbcS2 gene (Ps.RbcS2-E9; Coruzzi et al. (1984) EMBO J. 3:1671-9) and AGRtu.nos (GenBank™ Accession No. E01312).

Some embodiments may include a plant transformation vector that comprises an isolated and purified DNA molecule comprising at least one of the above-described regulatory elements operatively linked to one or more polynucleotides of the present invention. When expressed, the one or more polynucleotides result in one or more iRNA molecule(s) comprising a polyribonucleotide that is specifically complementary to all or part of a native RNA molecule in an insect pest. Thus, the polynucleotide(s) may comprise a segment encoding all or part of a polyribonucleotide present within a targeted RNA transcript in the insect pest, and may comprise inverted repeats of all or a part of a targeted pest transcript. A plant transformation vector may contain polynucleotides specifically complementary to more than one target polynucleotide, thus allowing production of more than one dsRNA for inhibiting expression of two or more genes in cells of one or more populations or species of target insect pests. Segments of polynucleotides specifically complementary to polynucleotides present in different genes can be combined into a single composite nucleic acid molecule for expression in a transgenic plant. Such segments may be contiguous or separated by a spacer.

In some embodiments, a plasmid of the present invention already containing at least one polynucleotide(s) of the invention can be modified by the sequential insertion of additional polynucleotide(s) in the same plasmid, wherein the additional polynucleotide(s) are operably linked to the same regulatory elements as the original at least one polynucleotide(s). In some embodiments, a nucleic acid molecule may be designed for the inhibition of multiple target genes. In some embodiments, the multiple genes to be inhibited can be obtained from the same insect (*e.g.,* coleopteran) pest species, which may enhance the effectiveness of the nucleic acid molecule. In other embodiments, the genes can be derived from different insect pests, which may broaden the range of pests against which the agent(s) is/are effective. When multiple genes are targeted for suppression or a combination of expression and suppression, a polycistronic DNA element can be engineered.

A recombinant nucleic acid molecule or vector of the present invention may comprise a selectable marker that confers a selectable phenotype on a transformed cell, such as a plant cell. Selectable markers may also be used to select for plants or plant cells that comprise a recombinant nucleic acid molecule of the invention. The marker may encode biocide resistance, antibiotic resistance (*e.g.,* kanamycin, Geneticin (G418), bleomycin, hygromycin, *etc.*), or herbicide tolerance (*e.g.,* glyphosate, *etc.*). Examples of selectable markers include, but are not limited to: a *neo* gene which codes for kanamycin resistance and can be selected for using kanamycin, G418, *etc.*; a *bar* gene which codes for bialaphos resistance; a mutant EPSP synthase gene which encodes glyphosate tolerance; a *nitrilase* gene which confers resistance to bromoxynil; a mutant acetolactate synthase (*ALS*) gene which confers imidazolinone or sulfonylurea resistance; and a methotrexate resistant *DHFR* gene. Multiple selectable markers are available that confer resistance to ampicillin, bleomycin, chloramphenicol, gentamycin, hygromycin, kanamycin, lincomycin, methotrexate, phosphinothricin, puromycin, spectinomycin, rifampicin, streptomycin and tetracycline, and the like. Examples of such selectable markers are illustrated in, *e.g.,* U.S. Patents 5,550,318; 5,633,435; 5,780,708 and 6,118,047.

A recombinant nucleic acid molecule or vector of the present invention may also include a screenable marker.

Screenable markers may be used to monitor expression. Exemplary screenable markers include a β-glucuronidase or *uidA* gene (GUS) which encodes an enzyme for which various chromogenic substrates are known (Jefferson et al. (1987) Plant Mol. Biol. Rep. 5:387-405); an R-locus gene, which encodes a product that regulates the production of anthocyanin pigments (red color) in plant tissues (Dellaporta et al. (1988) "Molecular cloning of the maize R-nj allele by transposon tagging with Ac." In 18th Stadler Genetics Symposium, P. Gustafson and R. Appels, eds. (New York: Plenum), pp. 263-82); a β-lactamase gene (Sutcliffe et al. (1978) Proc. Natl. Acad. Sci. USA 75:3737-41); a gene which encodes an enzyme for which various chromogenic substrates are known (*e.g.,* PADAC, a chromogenic cephalosporin); a luciferase gene (Ow et al. (1986) Science 234:856-9); an *xylE* gene that encodes a catechol dioxygenase that can convert chromogenic catechols (Zukowski et al. (1983) Gene 46(2-3):247-55); an amylase gene (Ikatu et al. (1990) Bio/Technol. 8:241-2); a tyrosinase gene which encodes an enzyme capable of oxidizing tyrosine to DOPA and dopaquinone which in turn condenses to melanin (Katz et al. (1983) J. Gen. Microbiol. 129:2703-14); and an α-galactosidase.

In some embodiments, recombinant nucleic acid molecules, as described, *supra,* may be used in methods for the creation of transgenic plants and expression of heterologous nucleic acids in plants to prepare transgenic plants that exhibit reduced susceptibility to insect (*e.g.,* coleopteran) pests. Plant transformation vectors can be prepared, for example, by inserting nucleic acid molecules encoding iRNA molecules into plant transformation vectors and introducing these into plants.

Suitable methods for transformation of host cells include any method by which DNA can be introduced into a cell, such as by transformation of protoplasts (*See, e.g.,* U.S. Patent 5,508,184), by desiccation/inhibition-mediated DNA uptake (*See, e.g.,* Potrykus et al. (1985) Mol. Gen. Genet. 199:183-8), by electroporation (*See, e.g.,* U.S. Patent 5,384,253), by agitation with silicon carbide fibers (*See, e.g.,* U.S. Patents 5,302,523 and 5,464,765), by *Agrobacterium*-mediated transformation (*See, e.g.,* U.S. Patents 5,563,055; 5,591,616; 5,693,512; 5,824,877; 5,981,840; and 6,384,301) and by acceleration of DNA-coated particles (*See, e.g.,* U.S. Patents 5,015,580; 5,550,318; 5,538,880; 6,160,208; 6,399,861; and 6,403,865), *etc.* Techniques that are particularly useful for transforming corn are described, for example, in U.S. Patents 7,060,876 and 5,591,616; and International PCT Publication WO95/06722. Through the application of techniques such as these, the cells of virtually any species may be stably transformed. In some embodiments, transforming DNA is integrated into the genome of the host cell. In the case of multicellular species, transgenic cells may be regenerated into a transgenic organism. Any of these techniques may be used to produce a transgenic plant, for example, comprising one or more nucleic acids encoding one or more iRNA molecules in the genome of the transgenic plant.

The most widely utilized method for introducing an expression vector into plants is based on the natural transformation system of *Agrobacterium. A. tumefaciens* and *A. rhizogenes* are plant pathogenic soil bacteria which genetically transform plant cells. The Ti and Ri plasmids of A. *tumefaciens* and *A. rhizogenes*, respectively, carry genes responsible for genetic transformation of the plant. The Ti (tumor-inducing)-plasmids contain a large segment, known as T-DNA, which is transferred to transformed plants. Another segment of the Ti plasmid, the Vir region, is responsible for T-DNA transfer. The T-DNA region is bordered by terminal repeats. In modified binary vectors, the tumor-inducing genes have been deleted, and the functions of the Vir region are utilized to transfer foreign DNA bordered by the T-DNA border elements. The T-region may also contain a selectable marker for efficient recovery of transgenic cells and plants, and a multiple cloning site for inserting polynucleotides for transfer such as a dsRNA encoding nucleic acid.

In particular embodiments, a plant transformation vector is derived from a Ti plasmid of *A. tumefaciens* (*See, e.g.,* U.S. Patents 4,536,475, 4,693,977, 4,886,937, and 5,501,967; and European Patent No. EP 0 122 791) or a Ri plasmid of *A. rhizagenes.* Additional plant transformation vectors include, for example and without limitation, those described by Herrera-Estrella et al. (1983) Nature 303:209-13; Bevan et al. (1983) Nature 304:184-7; Klee et al. (1985) Bio/Technol. 3:637-42; and in European Patent No. EP 0 120 516, and those derived from any of the foregoing. Other bacteria such as *Sinorhizobium, Rhizobium,* and *Mesorhizobium* that interact with plants naturally can be modified to mediate gene transfer to a number of diverse plants. These plant-associated symbiotic bacteria can be made competent for gene transfer by acquisition of both a disarmed Ti plasmid and a suitable binary vector.

After providing exogenous DNA to recipient cells, transformed cells are generally identified for further culturing and plant regeneration. In order to improve the ability to identify transformed cells, one may desire to employ a selectable or screenable marker gene, as previously set forth, with the transformation vector used to generate the transformant. In the case where a selectable marker is used, transformed cells are identified within the potentially transformed cell population by exposing the cells to a selective agent or agents. In the case where a screenable marker is used, cells may be screened for the desired marker gene trait.

Cells that survive the exposure to the selective agent, or cells that have been scored positive in a screening assay, may be cultured in media that supports regeneration of plants. In some embodiments, any suitable plant tissue culture media (*e.g.,* MS and N6 media) maybe modified by including further substances, such as growth regulators. Tissue maybe maintained on a basic medium with growth regulators until sufficient tissue is available to begin plant regeneration efforts, or following repeated rounds of manual selection, until the morphology of the tissue is suitable for regeneration (*e.g.*, at least 2 weeks), then transferred to media conducive to shoot formation. Cultures are transferred periodically until sufficient shoot formation has occurred. Once shoots are formed, they are transferred to media conducive to root formation. Once sufficient roots are formed, plants can be transferred to soil for further growth and maturation.

To confirm the presence of a nucleic acid molecule of interest (for example, a DNA encoding one or more iRNA molecules that inhibit target gene expression in a coleopteran pest) in the regenerating plants, a variety of assays may be performed. Such assays include, for example: molecular biological assays, such as Southern and northern blotting, PCR, and nucleic acid sequencing; biochemical assays, such as detecting the presence of a protein product, *e.g.,* by immunological means (ELISA and/or western blots) or by enzymatic function; plant part assays, such as leaf or root assays; and analysis of the phenotype of the whole regenerated plant.

Integration events may be analyzed, for example, by PCR amplification using, *e.g.,* oligonucleotide primers specific for a nucleic acid molecule of interest. PCR genotyping is understood to include, but not be limited to, polymerase-chain reaction (PCR) amplification of gDNA derived from isolated host plant callus tissue predicted to contain a nucleic acid molecule of interest integrated into the genome, followed by standard cloning and sequence analysis of PGR amplification products. Methods of PCR genotyping have been well described (for example, Rios, G. et al. (2002) Plant J. 32:243-53) and may be applied to gDNA derived from any plant species (*e.g., Z. mays,* cotton, soybean, and *B. napus*) or tissue type, including cell cultures.

A transgenic plant formed using *Agrobacterium*-dependent transformation methods typically contains a single recombinant DNA inserted into one chromosome. The polynucleotide of the single recombinant DNA is referred to as a "transgenic event" or "integration event". Such transgenic plants are heterozygous for the inserted exogenous polynucleotide. In some embodiments, a transgenic plant homozygous with respect to a transgene may be obtained by sexually mating (selfing) an independent segregant transgenic plant that contains a single exogenous gene to itself, for example a $T_0$ plant, to produce $T_1$ seed. One fourth of the $T_1$ seed produced will be homozygous with respect to the transgene. Germinating $T_1$ seed results in plants that can be tested for heterozygosity, typically using an SNP assay or a thermal amplification assay that allows for the distinction between heterozygotes and homozygotes (*i.e.,* a zygosity assay).

In particular embodiments, at least 2, 3, 4, 5, 6, 7, 8, 9 or 10 or more different iRNA molecules are produced in a plant cell that have an insect (*e.g.,* coleopteran) pest-inhibitory effect. The iRNA molecules (*e.g.,* dsRNA molecules) may be expressed from multiple nucleic acids introduced in different transformation events, or from a single nucleic acid introduced in a single transformation event. In some embodiments, a plurality of iRNA molecules are expressed under the control of a single promoter. In other embodiments, a plurality of iRNA molecules are expressed under the control of multiple promoters. Single iRNA molecules may be expressed that comprise multiple polynucleotides that are each homologous to different loci within one or more insect pests (for example, the loci defined by SEQ ID NOs:1, 3, and 5), both in different populations of the same species of insect pest, or in different species of insect pests.

In addition to direct transformation of a plant with a recombinant nucleic acid molecule, transgenic plants can be prepared by crossing a first plant having at least one transgenic event with a second plant lacking such an event. For example, a recombinant nucleic acid molecule comprising a polynucleotide that encodes an iRNA molecule may be introduced into a first plant line that is amenable to transformation to produce a transgenic plant, which transgenic plant may be crossed with a second plant line to introgress the polynucleotide that encodes the iRNA molecule into the second plant line.

In some aspects, seeds and commodity products produced by transgenic plants derived from transformed plant cells are included, wherein the seeds or commodity products comprise a detectable amount of a nucleic acid of the invention. In some embodiments, such commodity products may be produced, for example, by obtaining transgenic plants and preparing food or feed from them. Commodity products comprising one or more of the polynucleotides of the invention includes, for example and without limitation: meals, oils, crushed or whole grains or seeds of a plant, and any food product comprising any meal, oil, or crushed or whole grain of a recombinant plant or seed comprising one or more of the nucleic acid molecules of the invention. In particular examples, a commodity product is a bait composition or formulation comprising one or more of the nucleic acid molecules of the invention The detection of one or more of the polynucleotides of the invention in one or more commodity or commodity products is *de facto* evidence that the commodity or commodity product is produced from a transgenic plant designed to express one or more of the iRNA molecules of the invention for the purpose of controlling insect pests.

In some embodiments, a transgenic plant or seed comprising a nucleic acid molecule of the invention also may comprise at least one other transgenic event in its genome, including without limitation: a transgenic event from which is transcribed an iRNA molecule targeting a locus in a coleopteran pest other than the one defined by SEQ ID NO:1, SEQ ID NO:3, and SEQ ID NO:5, such as, for example, one or more loci selected from the group consisting of *Caf1-180* (U.S. Patent Application Publication No. 2012/0174258), *VatpaseC* (U.S. Patent Application Publication No. 2012/0174259), *Rho1* (U.S. Patent Application Publication No. 2012/0174260), *VatpaseH* (U.S. Patent Application Publication No. 2012/0198586), *PPI-87B* (U.S. Patent Application Publication No. 2013/0091600), *RPA70* (U.S. Patent Application Publication No. 2013/0091601), *RPS6* (U.S. Patent Application Publication No. 2013/0097730), *ROP* (U.S. Patent Application Publication No. 14/577,811), *RNA polymerase I1* (U.S. Patent Application Publication No. 62/133,214), *RNA polymerase II140* (U.S. Patent Application Publication No. 14/577,854), *RNA polymerase II215* (U.S. Patent Application Publication No. 62/133,202), *RNA polymerase II33* (U.S. Patent Application Publication No. 62/133,210), *transcription, elongation factor spt5* (U.S. Patent Application No. 62/168,613), *transcription elongation factor spt6* (U.S. Patent Application No. 62/168,606), *ncm* (U.S. Patent Application No. 62/095487), *dre4* (U.S. Patent Application No. 14/705,807), *COPI alpha* (U.S. Patent Application No. 62/063,199), *COPI beta* (U.S. Patent Application No. 62/063,203), *COPI gamma* (U.S. Patent Application No. 62/063,192), and *COPI delta* (U.S. Patent Application No. 62/063,216); a transgenic event from which is transcribed an iRNA molecule targeting a gene in an organism other than a coleopteran pest *(e.g.,* a plant-parasitic nematode); a gene encoding an insecticidal protein *(e.g.,* a *Bacillus thuringiensis* insecticidal protein, and a PIP-1 polypeptide); a herbicide tolerance gene *(e.g.,* a gene providing tolerance to glyphosate); and a gene contributing to a desirable phenotype in the transgenic plant, such as increased yield, altered fatty acid metabolism, or restoration of cytoplasmic male sterility. In particular embodiments, polynucleotides encoding iRNA molecules of the invention may be combined with other insect control and disease traits in a plant to achieve desired traits for enhanced control of plant disease and insect damage. In some examples, genes encoding pesticidal proteins may be combined, including, for example and without limitation: isolated or recombinant nucleic acid molecules encoding Alcaligenes Insecticidal Protein-1A and Alcaligenes Insecticidal Protein-1B (AfIP-1A and AfIP-1B) polypeptides (U.S. Patent Application Publication No. 2014/0033361); or isolated or recombinant nucleic acid molecules encoding PIP polypeptides (WO 2015038734). Combining insect control traits that employ distinct modes-of-action may provide protected transgenic plants with superior durability over plants harboring a single control trait, for example, because of the reduced probability that resistance to the trait(s) will develop in the field.

## *V. Target Gene Suppression in an Insects Pest*

### A. Overview

In some embodiments of the invention, at least one nucleic acid molecule useful for the control of insect (*e.g.,* coleopteran) pests may be provided to an insect pest, wherein the nucleic acid molecule leads to RNAi-mediated gene silencing in the pest. In particular embodiments, an iRNA molecule (*e.g.*, dsRNA, siRNA, miRNA, shRNA, and hpRNA) may be provided to a coleopteran pest. In some embodiments, a nucleic acid molecule useful for the control of insect pests may be provided to a pest by contacting the nucleic acid molecule with the pest. In these and further embodiments, a nucleic acid molecule useful for the control of insect pests may be provided in a feeding substrate of the pest, for example, a nutritional composition. In these and further embodiments, a nucleic acid molecule useful for the control of an insect pest maybe provided through ingestion of plant material comprising the nucleic acid molecule that is ingested by the pest. In certain embodiments, the nucleic acid molecule is present in plant material through expression of a recombinant nucleic acid introduced into the plant material, for example, by transformation of a plant cell with a vector comprising the recombinant nucleic acid and regeneration of a plant material or whole plant from the transformed plant cell.

In some embodiments, a pest is contacted with the nucleic acid molecule that leads to RNAi-mediated gene silencing in the pest through contact with a topical composition (*e.g.,* a composition applied by spraying) or an RNAi bait. RNAi baits are formed when the dsRNA is mixed with food or an attractant or both. When the pests eat the bait, they also consume the dsRNA. Baits may take the form of granules, gels, flowable powders, liquids, or solids. In particular embodiments, iRNA molecules targeting *prp8* may be incorporated into a bait formulation such as that described in U.S. Patent No. 8,530,440 which is hereby incorporated by reference. Generally, with baits, the baits are placed in or around the environment of the insect pest, for example, such that the insect pest can come into contact with, and/or be attracted to, the bait.

### B. RNAi-mediated Target Gene Suppression

In some embodiments, the invention provides iRNA molecules (*e.g.,* dsRNA, siRNA, miRNA, shRNA, and hpRNA) that maybe designed to target essential native polynucleotides *(e.g.,* essential genes) in the transcriptome of an insect pest (for example, a coleopteran *(e.g.,* WCR, SCR, NCR, and PB) pest), for example, by designing an iRNA molecule that comprises at least one strand comprising a polynucleotide that is specifically complementary to the target polynucleotide. The sequence of an iRNA molecule so designed may be identical to that of the target polynucleotide, or may incorporate mismatches that do not prevent specific hybridization between the iRNA molecule and its target polynucleotide.

iRNA molecules of the invention may be used in methods for gene suppression in an insect (*e.g.,* coleopteran) pest, thereby reducing the level or incidence of damage caused by the pest on a plant (for example, a protected transformed plant comprising an iRNA molecule). As used herein the term "gene suppression" refers to any of the well-known methods for reducing the levels of protein produced as a result of gene transcription to mRNA and subsequent translation of the mRNA, including the reduction of protein expression from a gene or a coding polynucleotide including post-transcriptional inhibition of expression and transcriptional suppression. Post-transcriptional inhibition is mediated by specific homology between all or a part of an mRNA transcribed from a gene targeted for suppression and the corresponding iRNA molecule used for suppression. Additionally, post-transcriptional inhibition refers to the substantial and measurable reduction of the amount of mRNA available in the cell for binding by ribosomes.

In some embodiments wherein an iRNA molecule is a dsRNA molecule, the dsRNA molecule may be cleaved by the enzyme, DICER, into short siRNA molecules (approximately 20 nucleotides in length). The double-stranded siRNA molecule generated by DICER activity upon the dsRNA molecule may be separated into two single-stranded siRNAs; the "passenger strand" and the "guide strand." The passenger strand may be degraded, and the guide strand may be incorporated into RISC. Post-transcriptional inhibition occurs by specific hybridization of the guide strand with a specifically complementary polynucleotide of an mRNA molecule, and subsequent cleavage by the enzyme, Argonaute (catalytic component of the RISC complex).

In other embodiments of the invention, any form of iRNA molecule may be used. Those of skill in the art will understand that dsRNA molecules typically are more stable during preparation and during the step of providing the iRNA molecule to a cell than are single-stranded RNA molecules, and are typically also more stable in a cell. Thus, while siRNA and miRNA molecules, for example, may be equally effective in some embodiments, a dsRNA molecule may be chosen due to its stability.

In particular embodiments, a nucleic acid molecule is provided that comprises a polynucleotide, which polynucleotide may be expressed *in vitro* to produce an iRNA molecule that comprises a polyribonucleotide that is substantially homologous to a polyribonucleotide of an RNA molecule encoded by a polynucleotide within the genome of an insect pest. In certain embodiments, the *in vitro* transcribed iRNA molecule may be a stabilized dsRNA molecule that comprises a stem-loop structure. After an insect pest contacts the *in vitro* transcribed iRNA molecule, post-transcriptional inhibition of a target gene in the pest (for example, an essential gene) may occur.

In some embodiments of the invention, expression of a nucleic acid molecule comprising at least 15 contiguous nucleotides (*e.g.,* at least 19 contiguous nucleotides) of a polynucleotide are used in a method for post-transcriptional inhibition of a target gene in an insect (*e.g.,* coleopteran) pest, wherein the polynucleotide is selected from the group consisting of: SEQ ID NO:1; the complement of SEQ ID NO:1; SEQ ID NO:3; the complement of SEQ ID NO:3; SEQ ID NO:5; the complement of SEQ ID NO:5; SEQ ID NO:7; the complement of SEQ ID NO:7; SEQ ID NO:8; the complement of SEQ ID NO:8; SEQ ID NO:9; the complement of SEQ ID NO:9; SEQ ID NO: 10; the complement of SEQ ID NO: 10; SEQ ID NO:11; the complement of SEQ ID NO:11; SEQ ID NO:12; the complement of SEQ ID NO:12; a fragment of at least 15, at least 18, at least 20, at least 22, at least 25, at least 28, at least 30, at least 50, at least 100, preferably at least 15 contiguous nucleotides of SEQ ID NO:1; the complement of a fragment of at least 15, at least 18, at least 20, at least 22, at least 25, at least 28, at least 30, at least 50, at least 100, preferably at least 15 contiguous nucleotides of SEQ ID NO: 1; a fragment of at least 15, at least 18, at least 20, at least 22, at least 25, at least 28, at least 30, at least 50, at least 100, preferably at least 15 contiguous nucleotides of SEQ ID NO:3; the complement of a fragment of at least 15 contiguous nucleotides of SEQ ID NO:3; a native coding polynucleotide of a *Diabrotica* organism comprising any of SEQ ID NOs:7, 8, 9, 10 and 11; the complement of a native coding polynucleotide of a *Diabrotica* organism comprising any of SEQ ID NOs:7, 8, 9, 10 and 11; a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:7, a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:8, a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:9, a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO: 10, and/or a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:11; the complement of a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:7, the complement of a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:8, the complement of a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:9, the complement of a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:10, and/or the complement of a fragment of at least 15, at least 18, at least 20, at least 22, at least 25, at least 28, at least 30, at least 50, at least 100, preferably at least 15 contiguous nucleotides of a native coding polynucleotide of a *Diabrotica* organism comprising SEQ ID NO:11; a fragment of at least 15, at least 18, at least 20, at least 22, at least 25, at least 28, at least 30, at least 50, at least 100, preferably at least 15 contiguous nucleotides of SEQ ID NO:5; the complement of a fragment of at least 15, at least 18, at least 20, at least 22, at least 25, at least 28, at least 30, at least 50, at least 100, preferably at least 15 contiguous nucleotides of SEQ ID NO:5; a native coding polynucleotide of a *Meligethes* organism comprising SEQ ID NO: 12; the complement of a native coding polynucleotide of a *Meligethes* organism comprising SEQ ID NO: 12; a fragment of at least 15, at least 18, at least 20, at least 22, at least 25, at least 28, at least 30, at least 50, at least 100, preferably at least 15 contiguous nucleotides of a native coding polynucleotide of a *Meligethes* organism comprising SEQ ID NO:12; and the complement of a fragment of at least 15, at least 18, at least 20, at least 22, at least 25, at least 28, at least 30, at least 50, at least 100, preferably at least 15 contiguous nucleotides of a native coding polynucleotide of a *Meligethes* organism comprising SEQ ID NO:12. In certain embodiments, expression of a nucleic acid molecule that is at least about 80% identical (*e.g.,* 79%, about 80%, about 81%, about 82%, about 83%, about 84%, about 85%, about 86%, about 87%, about 88%, about 89%, about 90%, about 91 %, about 92%, about 93%, about 94%, about 95%, about 96%, about 97%, about 98%, about 99%, about 100%, and 100%) with any of the foregoing may be used. In these and further embodiments, a nucleic acid molecule may be expressed that specifically hybridizes to a RNA molecule present in at least one cell of a coleopteran insect (*e.g., Diabrotica* and *Meligethes*) pest.

It is an important feature of some embodiments herein that the RNAi post-transcriptional inhibition system is able to tolerate sequence variations among target genes that might be expected due to genetic mutation, strain polymorphism, or evolutionary divergence. The introduced nucleic acid molecule may not need to be absolutely homologous to either a primary transcription product or a fully-processed mRNA of a target gene, so long as the introduced nucleic acid molecule is specifically hybridizable to either a primary transcription product or a fully-processed mRNA of the target gene. Moreover, the introduced nucleic acid molecule may not need to be full-length, relative to either a primary transcription product or a fully processed mRNA of the target gene.

Inhibition of a target gene using the iRNA technology of the present invention is sequence-specific; *i.e.,* polynucleotides substantially homologous to the iRNA molecule(s) are targeted for genetic inhibition. In some embodiments, an RNA molecule comprising a polynucleotide with a nucleotide sequence that is identical to that of a portion of a target gene may be used for inhibition. In these and further embodiments, an RNA molecule comprising a polynucleotide with one or more insertion, deletion, and/or point mutations relative to a target polynucleotide may be used. In particular embodiments, an iRNA molecule and a portion of a target gene may share, for example, at least from about 80%, at least from about 81%, at least from about 82%, at least from about 83%, at least from about 84%, at least from about 85%, at least from about 86%, at least from about 87%, at least from about 88%, at least from about 89%, at least from about 90%, at least from about 91 %, at least from about 92%, at least from about 93%, at least from about 94%, at least from about 95%, at least from about 96%, at least from about 97%, at least from about 98%, at least from about 99%, at least from about 100%, and 100% sequence identity. Alternatively, the duplex region of a dsRNA molecule may be specifically hybridizable with a portion of a target gene transcript. In specifically hybridizable molecules, a less than full length polynucleotide exhibiting a greater homology compensates for a longer, less homologous polynucleotide. The length of the polynucleotide of a duplex region of a dsRNA molecule that is identical to a portion of a target gene transcript may be at least about 25, 50, 100, 200, 300, 400, 500, or at least about 1000 bases. In some embodiments, a polynucleotide of greater than 20-100 nucleotides may be used. In particular embodiments, a polynucleotide of greater than about 200-300 nucleotides may be used. In particular embodiments, a polynucleotide of greater than about 500-1000 nucleotides may be used, depending on the size of the target gene.

In certain embodiments, expression of a target gene in a pest (*e.g.,* coleopteran) may be inhibited by at least 10%; at least 33%; at least 50%; or at least 80% within a cell of the pest, such that a Significant inhibition takes place. Significant inhibition refers to inhibition over a threshold that results in a detectable phenotype (*e.g.,* cessation of growth, cessation of feeding, cessation of development, induced mortality, *etc.*), or a detectable decrease in RNA and/or gene product corresponding to the target gene being inhibited. Although, in certain embodiments of the invention, inhibition occurs in substantially all cells of the pest, in other embodiments inhibition occurs only in a subset of cells expressing the target gene.

In some embodiments, transcriptional suppression is mediated by the presence in a cell of a dsRNA molecule exhibiting substantial sequence identity to a promoter DNA or the complement thereof to effect what is referred to as "promoter trans suppression." Gene suppression may be effective against target genes in an insect pest that may ingest or contact such dsRNA molecules, for example, by ingesting or contacting plant material containing the dsRNA molecules. dsRNA molecules for use in promoter trans suppression may be specifically designed to inhibit or suppress the expression of one or more homologous or complementary polynucleotides in the cells of the insect pest. Post-transcriptional gene suppression by antisense or sense oriented RNA to regulate gene expression in plant cells is disclosed in U.S. Patents 5,107,065; 5,759,829; 5,283,184; and 5,231,020.

**C. Expression of iRNA Molecules Provided to an Insect Pest**

Expression of iRNA molecules for RNAi-mediated gene inhibition in an insect *(e.g.,* coleopteran) pest may be carried out in any one of many *in vitro* or *in vivo* formats. The iRNA molecules may then be provided to an insect pest, for example, by contacting the iRNA molecules with the pest, or by causing the pest to ingest or otherwise internalize the iRNA molecules. Some embodiments include transformed host plants of a coleopteran pest, transformed plant cells, and progeny of transformed plants. The transformed plant cells and transformed plants may be engineered to express one or more of the iRNA molecules, for example, under the control of a heterologous promoter, to provide a pest-protective effect. Thus, when a transgenic plant or plant cell is consumed by an insect pest during feeding, the pest may ingest iRNA molecules expressed in the transgenic plants or cells. The polynucleotides of the present invention may also be introduced into a wide variety of prokaryotic and eukaryotic microorganism hosts to produce iRNA molecules. The term "microorganism" includes prokaryotic and eukaryotic species, such as bacteria and fungi.

Modulation of gene expression may include partial or complete suppression of such expression. In another embodiment, a method for suppression of gene expression in an insect *(e.g.,* coleopteran) pest comprises providing in the tissue of the host of the pest a gene-suppressive amount of at least one dsRNA molecule formed following transcription of a polynucleotide as described herein, at least one segment of which is complementary to an mRNA within the cells of the insect pest. A dsRNA molecule, including its modified form such as an siRNA, miRNA, shRNA, or hpRNA molecule, ingested by an insect pest may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from a *prp8* DNA molecule, for example, comprising a polynucleotide selected from the group consisting of SEQ ID NOs:1, 3, 5, 7, 8, 9, 10, 11 and 12, e.g. may be at least from about 80%, 81%, 82%, 83%, 84%, 85%, 86%, 87%, 88%, 89%, 90%, 91 %, 92%, 93%, 94%, 95%, 96%, 97%, 98%, 99%, or about 100% identical to an RNA molecule transcribed from a polynucleotide of SEQ ID NO: 1, may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from a polynucleotide of SEQ ID NO: 3, may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from a polynucleotide of SEQ ID NO: 5, may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from a polynucleotide of SEQ ID NO: 7, may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from a polynucleotide of SEQ ID NO: 8, may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from a polynucleotide of SEQ ID NO: 9, may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from a polynucleotide of SEQ ID NO: 10, and/or may be at least from about 80%, 81%, 82%, 83%, 84%, 85%, 86%, 87%, 88%, 89%, 90%, 91%, 92%, 93%, 94%, 95%, 96%, 97%, 98%, 99%, or about 100% identical to an RNA molecule transcribed from a polynucleotide of SEQ ID NO: 11. Isolated and substantially purified nucleic acid molecules including, but not limited to, non-naturally occurring polynucleotides and recombinant DNA constructs for providing dsRNA molecules are therefore provided, which suppress or inhibit the expression of an endogenous coding polynucleotide or a target coding polynucleotide in an insect pest when introduced thereto.

Particular embodiments provide a delivery system for the delivery of iRNA molecules for the post-transcriptional inhibition of one or more target gene(s) in an insect (*e.g.,* coleopteran) plant pest and control of a population of the plant pest. In some embodiments, the delivery system comprises ingestion of a host transgenic plant cell or contents of the host cell comprising RNA molecules transcribed in the host cell. In these and further embodiments, a transgenic plant cell or a transgenic plant is created that contains a recombinant DNA construct providing a stabilized dsRNA molecule of the invention. Transgenic plant cells and transgenic plants comprising nucleic acids encoding a particular iRNA molecule may be produced by employing recombinant DNA technologies (which basic technologies are well-known in the art) to construct a plant transformation vector comprising a polynucleotide encoding an iRNA molecule of the invention (*e.g.,* a stabilized dsRNA molecule); to transform a plant cell or plant; and to generate the transgenic plant cell or the transgenic plant that contains the transcribed iRNA molecule.

To impart protection from insect pests to a transgenic plant, a recombinant DNA molecule may, for example, be transcribed into an iRNA molecule, such as a dsRNA molecule, a siRNA molecule, a miRNA molecule, a shRNA molecule, or a hpRNA molecule. In some embodiments, a RNA molecule transcribed from a recombinant DNA molecule may form a dsRNA molecule within the tissues or fluids of the recombinant plant. Such a dsRNA molecule may comprise in part a polyribonucleotide that is identical to a corresponding polyribonucleotide transcribed from a DNA within an insect pest of a type that may infest the host plant. Expression of a target gene within the pest is suppressed by the dsRNA molecule, and the suppression of expression of the target gene in the pest results in the transgenic plant being protected against the pest. The modulatory effects of dsRNA molecules have been shown to be applicable to a variety of genes expressed in pests, including, for example, endogenous genes responsible for cellular metabolism or cellular transformation, including house-keeping genes; transcription factors; molting-related genes; and other genes which encode polypeptides involved in cellular metabolism or normal growth and development.

For transcription from a transgene *in vivo* or an expression construct, a regulatory region (*e.g.,* promoter, enhancer, silencer, and polyadenylation signal) may be used in some embodiments to transcribe the RNA strand (or strands). Therefore, in some embodiments, as set forth, *supra,* a polynucleotide for use in producing iRNA molecules may be operably linked to one or more promoter elements functional in a plant host cell. The promoter may be an endogenous promoter, normally resident in the host genome. The polynucleotide of the present invention, under the control of an operably linked promoter element, may further be flanked by additional elements that advantageously affect its transcription and/or the stability of a resulting transcript. Such elements maybe located upstream of the operably linked promoter, downstream of the 3' end of the expression construct, and may occur both upstream of the promoter and downstream of the 3' end of the expression construct.

Some embodiments provide methods for reducing the damage to a host plant (*e.g.*, a corn plant) caused by an insect (*e.g.,* coleopteran) pest that feeds on the plant, wherein the method comprises providing in the host plant a transformed plant cell expressing at least one nucleic acid molecule of the invention, wherein the nucleic acid molecule(s) functions upon being taken up by the pest(s) to inhibit the expression of a target polynucleotide within the pest(s), which inhibition of expression results in mortality and/or reduced growth of the pest(s), thereby reducing the damage to the host plant caused by the pest(s). In some embodiments, the nucleic acid molecule(s) comprise dsRNA molecules. In these and further embodiments, the nucleic acid molecule(s) comprise dsRNA molecules that each comprise more than one polyribonucleotide that is specifically hybridizable to a nucleic acid molecule expressed in a coleopteran pest cell. In some embodiments, the nucleic acid molecule(s) consist of one polynucleotide that is specifically hybridizable to a nucleic acid molecule expressed in an insect pest cell.

In some embodiments, a method for increasing the yield of a crop (*e.g.,* a corn crop and an oilseed rape crop) is provided, wherein the method comprises introducing into a plant at least one nucleic acid molecule comprising a polynucleotide of the invention; cultivating the plant to allow the expression of an iRNA molecule from the polynucleotide, wherein expression of the iRNA molecule inhibits insect pest damage and/or growth, thereby reducing or eliminating a loss of yield due to pest infestation. In some embodiments, the iRNA molecule is a dsRNA molecule. In these and further embodiments, the dsRNA molecules may each comprise more than one polyribonucleotide that is specifically hybridizable to a nucleic acid molecule expressed in an insect pest cell. Thus, specifically polyribonucleotides of a dsRNA molecule may be expressed from one or more nucleotide sequences within a polynucleotide of the invention.

In some embodiments, a method for modulating the expression of a target gene in an insect pest is provided, the method comprising: transforming a plant cell with a vector comprising a polynucleotide encoding at least one iRNA molecule of the invention, wherein the polynucleotide is operatively-linked to a promoter and a transcription termination element; culturing the transformed plant cell under conditions sufficient to allow for development of a plant cell culture including a plurality of transformed plant cells; selecting for transformed plant cells that have integrated the polynucleotide into their genomes; screening the transformed plant cells for expression of an iRNA molecule encoded by the integrated polynucleotide; selecting a transgenic plant cell that expresses the iRNA molecule; and feeding the selected transgenic plant cell to the insect pest. Plants may also be regenerated from transgenic plant cells that express an iRNA molecule encoded by the integrated nucleic acid molecule. In some embodiments, the iRNA molecule is a dsRNA molecule comprising a polyribonucleotide that is specifically hybridizable to the transcript of a target gene in the insect pest. In these and further embodiments, the dsRNA molecules comprise more than one polyribonucleotide that is transcribed from a nucleotide sequence within the polynucleotide encoding the dsRNA molecule.

iRNA molecules of the invention can be incorporated within the seeds of a plant species (*e.g.,* corn and canola), either as a product of expression from a recombinant gene incorporated into a genome of the plant cells, or as incorporated into a coating or seed treatment that is applied to the seed before planting. A plant cell comprising a recombinant gene is considered to be a transgenic event. Also included in embodiments of the invention are delivery systems for the delivery of iRNA molecules to insect (*e.g.,* coleopteran) pests. For example, the iRNA molecules of the invention may be directly introduced into the cells of a pest(s). Methods for introduction may include direct mixing of iRNA with plant tissue from a host for the insect pest(s), as well as application of compositions comprising iRNA molecules of the invention to host plant tissue. For example, iRNA molecules may be sprayed onto a plant surface. Alternatively, an iRNA molecule may be expressed by a microorganism, and the microorganism may be applied onto the plant surface, or introduced into a root or stem by a physical means such as an injection. As discussed, *supra,* a transgenic plant may also be genetically engineered to express at least one iRNA molecule in an amount sufficient to kill the insect pests known to infest the plant. iRNA molecules produced by chemical or enzymatic synthesis may also be formulated in a manner consistent with common agricultural practices, and used as spray-on or bait products for controlling plant damage by an insect pest. The formulations may include the appropriate adjuvants (*e.g.*, stickers and wetters) required for efficient foliar coverage, as well as UV protectants to protect iRNA molecules (*e.g.,* dsRNA molecules) from UV damage. Such additives are commonly used in the bioinsecticide industry, and are well known to those skilled in the art. Such applications may be combined with other spray-on insecticide applications (biologically based or otherwise) to enhance plant protection from the pests.

All references, including publications, patents, and patent applications, cited herein are hereby incorporated by reference to the extent they are not inconsistent with the explicit details of this disclosure, and are so incorporated to the same extent as if each reference were individually and specifically indicated to be incorporated by reference and were set forth in its entirety herein. The references discussed herein are provided solely for their disclosure prior to the filing date of the present application. Nothing herein is to be construed as an admission that the inventors are not entitled to antedate such disclosure by virtue of prior invention.

The following EXAMPLES are provided to illustrate certain particular features and/or aspects. These EXAMPLES should not be construed to limit the disclosure to the particular features or aspects described.

## EXAMPLES

### EXAMPLE 1: Materials and Methods

A number of dsRNA molecules (including those corresponding to *prp8-1* reg1 (SEQ ID NO:7), *prp8-2* reg1 (SEQ ID NO:8), *prp8-3* reg1 (SEQ ID NO:9), *prp8-3* v1 (SEQ ID NO: 10), and *prp8-3* v2 (SEQ ID NO:11), and were synthesized and purified using a MEGASCRIPT® T7 RNAi kit (LIFE TECHNOLOGIES, Carlsbad, CA) or T7 Quick High Yield RNA Synthesis Kit (NEW ENGLAND BIOLABS, Whitby, Ontario). The purified dsRNA molecules were prepared in TE buffer, and all bioassays contained a control treatment consisting of this buffer, which served as a background check for mortality or growth inhibition of WCR (*Diabrotica virgifera virgifera* LeConte). The concentrations of dsRNA molecules in the bioassay buffer were measured using a NANODROP™ 8000 spectrophotometer (THERMO SCIENTIFIC, Wilmington, DE).

Samples were tested for insect activity in bioassays conducted with neonate insect larvae on artificial insect diet. WCR eggs were obtained from CROP CHARACTERISTICS, INC. (Farmington, MN).

The bioassays were conducted in 128-well plastic trays specifically designed for insect bioassays (C-D INTERNATIONAL, Pitman, NJ). Each well contained approximately 1.0 mL of an artificial diet designed for growth of coleopteran insects. A 60 μL aliquot of dsRNA sample was delivered by pipette onto the surface of the diet of each well (40 $\mu Lcm^2$). dsRNA sample concentrations were calculated as the amount of dsRNA per square centimeter ($ng/cm^2$) of surface area (1.5 $cm^2$) in the well. The treated trays were held in a fume hood until the liquid on the diet surface evaporated or were absorbed into the diet.

Within a few hours of eclosion, individual larvae were picked up with a moistened camel hair brush and deposited on the treated diet (one or two larvae per well). The infested wells of the 128-well plastic trays were then sealed with adhesive sheets of clear plastic, and vented to allow gas exchange. Bioassay trays were held under controlled environmental conditions (28 °C, ~40% Relative Humidity, 16:8 (Light:Dark)) for 9 days, after which time the total number of insects exposed to each sample, the number of dead insects, and the weight of surviving insects were recorded. Average percent mortality and average growth inhibition were calculated for each treatment. Growth inhibition (GI) was calculated as follows:

$$GI = [1 - (TWIT/TNIT)/(TWIBC/TNIBC)],$$

where TWIT is the Total Weight of live Insects in the Treatment;
TNIT is the Total Number of Insects in the Treatment;
TWIBC is the Total Weight of live Insects in the Background Check (Buffer control); and
TNIBC is the Total Number of Insects in the Background Check (Buffer control).

The statistical analysis was done using JMP™ software (SAS, Cary, NC).

The $LC_{50}$ (Lethal Concentration) is defined as the dosage at which 50% of the test insects are killed. The $GI_{50}$ (Growth Inhibition) is defined as the dosage at which the mean growth *(e.g.* live weight) of the test insects is 50% of the mean value seen in Background Check samples.

Replicated bioassays demonstrated that ingestion of particular samples resulted in a surprising and unexpected mortality and growth inhibition of corn rootworm larvae.

### EXAMPLE 2: Identification of Candidate Target Genes

Insects from multiple stages of WCR (*Diabrotica virgifera virgifera* LeConte) development were selected for pooled transcriptome analysis to provide candidate target gene sequences for control by RNAi transgenic plant insect protection technology.

In one exemplification, total RNA was isolated from about 0,9 gm whole first-instar WCR larvae; (4 to 5 days post-hatch; held at 16 °C), and purified using the following phenol/TRI REAGENT®-based method (MOLECULAR RESEARCH CENTER, Cincinnati, OH):

Larvae were homogenized at room temperature in a 15 mL homogenizer with 10 mL of TRI REAGENT® until a homogenous suspension was obtained. Following 5 min. incubation at room temperature, the homogenate was dispensed into 1.5 mL microfuge tubes (1 mL per tube), 200 μL of chloroform was added, and the mixture was vigorously shaken for 15 seconds. After allowing the extraction to sit at room temperature for 10 min, the phases were separated by centrifugation at 12,000 x g at 4 °C. The upper phase (comprising about 0.6 mL) was carefully transferred into another sterile 1.5 mL tube, and an equal volume of room temperature isopropanol was added. After incubation at room temperature for 5 to 10 min, the mixture was centrifuged 8 min at 12,000 x g (4°C or 25 °C).

The supernatant was carefully removed and discarded, and the RNA pellet was washed twice by vortexing with 75% ethanol, with recovery by centrifugation for 5 min at 7,500 x g (4 °C or 25 °C) after each wash. The ethanol was carefully removed, the pellet was allowed to air-dry for 3 to 5 min, and then was dissolved in nuclease-free sterile water. RNA concentration was determined by measuring the absorbance (A) at 260 mn and 280 nm. A typical extraction from about 0.9 gm of larvae yielded over 1 mg of total RNA, with an $A_{260}/A_{280}$ ratio of 1.9. The RNA thus extracted was stored at -80 °C until further processed.

RNA quality was determined by running an aliquot through a 1 % agarose gel. The agarose gel solution was made using autoclaved 10x TAE buffer (Tris-acetate EDTA; 1x concentration is 0.04 M Tris-acetate, 1 mM EDTA (ethylenediamine tetra-acetic acid sodium salt), pH 8.0) diluted with DEPC (diethyl pyrocarbonate)-treated water in an autoclaved container. 1x TAE was used as the running buffer. Before use, the electrophoresis tank and the well-forming comb were cleaned with RNaseAway™ (INVITROGEN INC., Carlsbad, CA). Two μL of RNA sample were mixed with 8 μL of TE buffer (10 mM Tris HCl pH 7.0; 1 mM EDTA) and 10 μL of RNA sample buffer (NOVAGEN® Catalog No 70606; EMD4 Bioscience, Gibbstown, NJ). The sample was heated at 70 °C for 3 min, cooled to room temperature, and 5 μL (containing 1 μg to 2 μg RNA) were loaded per well. Commercially available RNA molecular weight markers were simultaneously run in separate wells for molecular size comparison. The gel was run at 60 volts for 2 hrs.

A normalized cDNA library was prepared from the larval total RNA by a commercial service provider (EUROFINS MWG Operon, Huntsville, AL), using random priming. The normalized larval cDNA library was sequenced at 1/2 plate scale by GS FLX 454 Titanium™ series chemistry at EUROFINS MWG Operon, which resulted in over 600,000 reads with an average read length of 348 bp. 350,000 reads were assembled into over 50,000 contigs. Both the unassembled reads and the contigs were converted into BLASTable databases using the publicly available program, FORMATDB (available from NCBI).

Total RNA and normalized cDNA libraries were similarly prepared from materials harvested at other WCR developmental stages. A pooled transcriptome library for target gene screening was constructed by combining cDNA library members representing the various developmental stages.

Candidate genes for RNAi targeting were hypothesized to be essential for survival and growth in pest insects. Selected target gene homologs were identified in the transcriptome sequence database, as described below. Full-length or partial sequences of the target genes were amplified by PCR to prepare templates for double-stranded RNA (dsRNA) production.

TBLASTN searches using candidate protein coding sequences were run against BLASTable databases containing the unassembled *Diabrotica* sequence reads or the assembled contigs. Significant hits to a *Diabrotica* sequence (defined as better than $e^{-20}$ for contigs homologies and better than $e^{-10}$ for unassembled sequence reads homologies) were confirmed using BLASTX against the NCBI non-redundant database. The results of this BLASTX search confirmed that the *Diabrotica* homolog candidate gene sequences identified in the TBLASTN search indeed comprised *Diabrotica* genes, or were the best hit to the non-*Diabrotica* candidate gene sequence present in the *Diabratica* sequences. In most cases, *Tribolium* candidate genes which were annotated as encoding a protein gave an unambiguous sequence homology to a sequence or sequences in the *Diabrotica* transcriptome sequences. In a few cases, it was clear that some of the *Diabrotica* contigs or unassembled sequence reads selected by homology to a non-*Diabrotica* candidate gene overlapped, and that the assembly of the contigs had failed to join these overlaps. In those cases, Sequencher™ v4.9 (GENE CODES CORPORATION, Ann Arbor, MI) was used to assemble the sequences into longer contigs.

Several candidate target genes encoding *Diabrotica prp8* (SEQ ID NO:1 and SEQ ID NO:3) were identified as genes that may lead to coleopteran pest mortality, inhibition of growth, inhibition of development, and/or inhibition of feeding in WCR.

The *Drosophilia prp8* gene consists of a NusG amino-terminal (NGN) domain and a C-terminal Kyprides-Onzonis-Woese (KOW) domain, acting as a dual transcriptional regulator that functions as both a negative and positive elongation factor. The NGN domain of *prp8* binds to RNAP whereas the KOW domain(s) recruits additional regulatory factors to RNAP. The KOW domain in eukaryotic is thought to allow the recruitment of a larger number of transcription factors. In addition, *prp8* may also participate in the regulation of pre-mRNA processing, as it interacts with the capping enzyme. Together with the small zinc-finger protein SPT4 (suppressor of Ty 4), *prp8* builds the heterodimeric complex DSIF (DRB (5,6-dichloro-1-β-D-ribofuranosylbenzimidazole) sensitivity-inducing factor).

The sequences SEQ ID NO:1 and SEQ ID NO:3 are novel. The sequences are not provided in public databases, and are not disclosed in PCT International Patent Publication No. WO/2011/025860; U.S. Patent Application No. 20070124836; U.S. Patent Application No. 20090306189; U.S. Patent Application No. US20070050860; U.S. Patent Application No. 20100192265; U.S. Patent 7,612,194; or U.S. Patent Application No. 2013192256. WCR *prp8-1* (SEQ ID NO:1) is somewhat related to a fragment of a sequence from *Tribolium castaneum* (GENBANK Accession No. XM_961838.2). WCR *prp8-2* (SEQ ID NO:3) is somewhat related to a fragment of a sequence from *Oryctolagus cuniculus* (GENBANK Accession No. N_144353.4). The closest homolog of the WCR PRPB-1 amino acid sequence (SEQ ID NO:2) is a *Tribolium casetanum* protein having GENBANK Accession No. XP_966931.1 (99% similar; 98% identical over the homology region). The closest homolog of the WCR PRP8-2 amino acid sequence (SEQ ID NO:4) is a *Gregarina niphandrodes* protein having GENBANK Accession No. XP_011131272.1 (85% similar; 76% identical over the homology region).

*Prp8* dsRNA transgenes can be combined with other dsRNA molecules to provide redundant RNAi targeting and synergistic RNAi effects. Transgenic corn events expressing dsRNA that targets *prp8* are useful for preventing root feeding damage by corn rootworm. *Prp8* dsRNA transgenes represent new modes of action for combining with *Bacillus thuringiensis* insecticidal protein technology in Insect Resistance Management gene pyramids to mitigate against the development of rootworm populations resistant to either of these rootworm control technologies.

## EXAMPLE 3: Amplification of Target Genes to produce dsRNA

Full-length or partial clones of sequences of a *Diabrotica* candidate gene, herein referred to as *prp8,* were used to generate PCR amplicons for dsRNA synthesis. Primers were designed to amplify portions of coding regions of each target gene by PCR. *See* **Table 1.** Where appropriate, a T7 phage promoter sequence (TTAATACGACTCACTATAGGGAGA; SEQ ID NO:13) was incorporated into the 5' ends of the amplified sense or antisense strands. *See* **Table 1.** Total RNA was extracted from WCR using TRIzol® (Life Technologies, Grand Island, NY), and was then used to make first-strand cDNA with SuperScriptIII® First-Strand Synthesis System and manufacturers Oligo dT primed instructions (Life Technologies, Grand Island, NY). First-strand cDNA was used as template for PCR reactions using opposing primers positioned to amplify all or part of the native target gene sequence. dsRNA was also amplified from a DNA clone comprising the coding region for a yellow fluorescent protein (YFP) (SEQ ID NO:14; Shagin et al. (2004) Mol. Biol. Evol. 21(5):841-50).

**Table 1.** Primers and Primer Pairs used to amplify portions of coding regions of exemplary *prp8* target gene and *YFP* negative control gene.

| | Gene ID | Primer ID | Sequence |
|---|---|---|---|
| **Pair 1** | *prp8-1* | Dvv-prp8-1_For | TTAATACGACTCACTATAGGGAGACAATTTACAAGATGTGTGGGATGTG (SEQ ID NO:15) |
| | | Dvv-prp8-1_Rev | TTAATACGACTCACTATAGGGAGACATTATTAGGATCTGGATGTTCTGTTAG (SEQ ID NO:16) |
| **Pair 2** | *prp8-2* | Dw-prp8-2_For | TTAATACGACTCACTATAGGGAGACGGCTTAATCCGCGGCCTCCAGTTCAGCAGTTTC (SEQ ID NO:17) |
| | | Dvv-prp8-2_Rev | TTAATACGACTCACTATAGGGAGACTTTGCCCCAACTCAGCTCAGCTAAAC (SEQ ID NO:18) |
| **Pair 3** | *prp8-3* | Dvv-prp8-3_For | TTAATACGACTCACTATAGGGAGACTAAGAATAACGTCGTTATAAACTACAAAGATATG (SEQ ID NO:19) |
| | | Dw-prp8-3_Rev | TTAATACGACTCACTATAGGGAGACATTATTAGGATCTGGATGTTCTGTTAGG (SEQ ID NO:20) |

(continued)

| | Gene ID | Primer ID | Sequence |
|---|---|---|---|
| **Pair 4** | *prp8-3 v1* | Dw-prp8-3_v1_For | TTAATACGACTCACTATAGGGAGACTAAGAATAACGTCGTTATAAAC (SEQ ID NO:21) |
| | | Dvv-prp8-3_v1_Rev | TTAATACGACTCACTATAGGGAGAGCAGATCCAAAACCAGACCATAATAC (SEQ ID NO:22) |
| **Pair 5** | *prp8-3 v2* | Dvv-prp8-3_v2_For | TTAATACGACTCACTATAGGGAGATGGCTGGGCCACCTCAAATG (SEQ ID NO:23) |
| | | Dw-prp8-3_v2_Rev | TTAATACGACTCACTATAGGGAGAGACATTATTAGGATCTGGATG (SEQ ID NO:24) |
| **Pair 6** | YFP | YFP-F_T7 | TTAATACGACTCACTATAGGGAGACACCATGGGCTCCAGCGGCGCCC (SEQ ID NO:34) |
| | | YFP-R_T7 | TTAATACGACTCACTATAGGGAGAAGATCTTGAAGGCGCTCTTCAGG (SEQ ID NO:37) |

**EXAMPLE 4: RNAi Constructs**

Template preparation by PCR and dsRNA synthesis. A strategy used to provide specific templates for *prp8* and *YFP* dsRNA production is shown in **FIG. 1.** Template DNAs intended for use in *prp8* dsRNA synthesis were prepared by PCR using the primer pairs in **Table 1** and (as PCR template) first-strand cDNA prepared from total RNA isolated from WCR eggs, first-instar larvae, or adults. For each selected *prp8* and *YFP* target gene region, PCR amplifications introduced a T7 promoter sequence at the 5' ends of the amplified sense and antisense strands (the *YFP* segment was amplified from a DNA clone of the *YFP* coding region). The two PCR amplified fragments for each region of the target genes were then mixed in approximately equal amounts, and the mixture was used as transcription template for dsRNA production. *See* FIG. 1. The sequences of the dsRNA templates amplified with the particular primer pairs were: SEQ ID NO:7 (*prp8-1* reg1), SEQ ID NO:8 (*prp8-2* reg1), SEQ ID NO:9 (*prp8-3* reg1), SEQ ID NO:10 (*prp8-3* v1), SEQ ID NO:11 (*prp8-3* v2), and SEQ ID NO:14 (YFP). Double-stranded RNA for insect bioassay was synthesized and purified using an AMBION® MEGASCRIPT® RNAi kit following the manufacturer's instructions (INVITROGEN) or HiScribe® T7 In Vitro Transcription Kit following the manufacturer's instructions (New England Biolabs, Ipswich, MA). The concentrations of dsRNAs were measured using a NANODROP™ 8000 spectrophotometer (THERMO SCIENTIFIC, Wilmington, DE).

Construction of plant transformation vectors. Entry vectors harboring a target gene construct for hairpin formation comprising segments of *prp8* (SEQ ID NO:1 and SEQ ID NO:3) are assembled using a combination of chemically synthesized fragments (DNA2.0, Menlo Park, CA) and standard molecular cloning methods. Intramolecular hairpin formation by RNA primary transcripts is facilitated by arranging (within a single transcription unit) two copies of the *prp8* target gene segment in opposite orientation to one another, the two segments being separated by a linker polynucleotide (*e.g.*, an ST-LS1 intron; Vancanneyt et al. (1990) Mol. Gen. Genet. 220(2):245-50). Thus, the primary mRNA transcript contains the two *prp8* gene segment sequences as large inverted repeats of one another, separated by the intron sequence. A copy of a maize ubiquitin 1 promoter (U.S. Patent 5,510,474) is used to drive production of the primary mRNA hairpin transcript, and a fragment comprising a 3' untranslated region from a maize peroxidase 5 gene (ZmPer5 3'UTR v2; U.S. Patent 6,699,984) is used to terminate transcription of the hairpin-RNA-expressing gene.

A negative control binary vector which comprises a gene that expresses a YFP hairpin dsRNA, is constructed by means of standard GATEWAY® recombination reactions with a typical binary destination vector and entry vector.

The binary destination vector comprises a herbicide tolerance gene (aryloxyalknoate dioxygenase; AAD-1 v3) (U.S. Patent 7,838,733(B2), and Wright et al. (2010) Proc. Natl. Acad. Sci. U.S.A. 107:20240-5) under the regulation of a sugarcane bacilliform badnavirus (ScBV) promoter (Schenk et al. (1999) Plant Molec. Biol. 39:1221-30). A synthetic 5'UTR sequence, comprised of sequences from a Maize Streak Virus (MSV) coat protein gene 5'UTR and intron 6 from a maize Alcohol Dehydrogenase 1 (ADH1) gene, is positioned between the 3' end of the SCBV promoter segment and the start codon of the AAD-1 coding region. A fragment comprising a 3' untranslated region from a maize lipase gene (ZmLip 3'UTR; U.S. Patent 7,179,902) is used to terminate transcription of the AAD-1 mRNA.

A further negative control binary vector, which comprises a gene that expresses a YFP protein, is constructed by means of standard GATEWAY® recombination reactions with a typical binary destination vector and entry vector. The binary destination vector comprises a herbicide tolerance gene (aryloxyalknoate dioxygenase; AAD-1 v3) (as above) under the expression regulation of a maize ubiquitin 1 promoter (as above) and a fragment comprising a 3' untranslated region from a maize lipase gene (ZmLip 3'UTR; as above). The entry vector comprises a YFP coding region (SEQ ID NO:27) under the expression control of a maize ubiquitin 1 promoter (as above) and a fragment comprising a 3' untranslated region from a maize peroxidase 5 gene (as above).

**EXAMPLE 5: Screening of Candidate Target Genes**

Synthetic dsRNA designed to inhibit target gene sequences identified in EXAMPLE 2 caused mortality and growth inhibition when administered to WCR in diet-based assays.

Replicated bioassays demonstrated that ingestion of dsRNA preparations derived from *prp8-2* reg1, *prp8-2* v1, and *prp8-2* v2 resulted in mortality and growth inhibition of western corn rootworm larvae. **Table 2** shows the results of diet-based feeding bioassays of WCR larvae following 9-day exposure to *prp8-2* reg1, *prp8-2* v1, and *prp8-2* v2 dsRNA, as well as the results obtained with a negative control sample of dsRNA prepared from a yellow fluorescent protein (YFP) coding region (SEQ ID NO:27). **Table 3** shows the $LC_{50}$ and $GI_{50}$ results of exposure to *prp8-2* v1 and *prp8-2* v2 dsRNA.

**Table 2.** Results of *prp8* dsRNA diet feeding assays obtained with western corn rootworm larvae after 9 days of feeding. ANOVA analysis found significance differences in Mean % Mortality and Mean % Growth Inhibition (GI). Means were separated using the Tukey-Kramer test.

| Gene Name | Dose (ng/cm$^2$) | N | Mean (%Mortality) ± SEM* | Mean (GI) ± SEM |
|---|---|---|---|---|
| ***prp8-3*** | 500 | 6 | 68.90 ± 8.63 (A) | 0.78 ± 0.17 (A) |
| ***prp8-3* v1** | 500 | 20 | 58.50±6.08 (A) | 0.73±0.08 (A) |
| ***prp8-3* v2** | 500 | 20 | 58.67±6.52 (A) | 0.75±0.07 (A) |
| **TE**** | 0 | 20 | 11.23 ± 2.73 (B) | 0.01 ± 0.04 (B) |
| **WATER** | 0 | 20 | 9.57 ± 2.66 (B) | -0.01 ± 0.03 (B) |
| **YFP***** | 500 | 18 | 5.40 ± 0.98 (B) | -0.0 ± 0.04 (B) |

*SEM =Standard Error of the Mean. Letters in parentheses designate statistical levels. Levels not connected by same letter are significantly different (P<0.05).
**TE = Tris HCl (1 mM) plus EDTA (0.1 mM) buffer, pH7.2.
***YFP = Yellow Fluorescent Protein

**Table 3.** Summary of oral potency of *prp8* dsRNA on WCR larvae (ng/cm$^2$).

| Gene Name | $LC_{50}$ | Range | $GI_{50}$ | Range |
|---|---|---|---|---|
| *prp8-3* v1 | 14.85 | 9,86 - 22.15 | 2.61 | 1.57 - 4.33 |
| *prp8-3* v2 | 20.88 | 13.34 - 32.84 | 1.29 | 0.46 - 3.60 |

It has previously been suggested that certain genes of *Diabrotica* spp. may be exploited for RNAi-mediated insect control. *See* U.S. Patent Publication No. 2007/0124836, which discloses 906 sequences, and U.S. Patent No. 7,612,194, which discloses 9,112 sequences. However, it was determined that many genes suggested to have utility for RNAi-mediated insect control are not efficacious in controlling *Diabfotica.* It was also determined that sequence *prp8-2* reg1*, prp8-2* v1, and *prp8-2* v2 dsRNA provide surprising and unexpected superior control of *Diabrotica,* compared to other genes suggested to have utility for RNAi-mediated insect control.

For example, *annexin, beta spectrin* 2, and *mtRP-L4* were each suggested in U.S. Patent 7,612,194 to be efficacious in RNAi-mediated insect control. SEQ ID NO:28 is the DNA sequence of *annexin* region 1 (Reg 1) and SEQ ID NO:29 is the DNA sequence of *annexin* region 2 (Reg 2). SEQ ID NO:30 is the DNA sequence of *beta spectrin* 2 region 1 (Reg 1) and SEQ ID NO:31 is the DNA sequence of *beta spectrin* 2 region 2 (Reg2). SEQ ID NO:32 is the DNA sequence of *mtRP-L4* region 1 (Reg 1) and SEQ ID NO:33 is the DNA sequence of *mtRP-L4* region 2 (Reg 2). A YFP sequence (SEQ ID NO:14) was also used to produce dsRNA as a negative control.

Each of the aforementioned sequences was used to produce dsRNA by the methods of EXAMPLE 3. The strategy used to provide specific templates for dsRNA production is shown in **FIG. 2.** Template DNAs intended for use in dsRNA synthesis were prepared by PCR using the primer pairs in Table 4 and (as PCR template) first-strand cDNA prepared from total RNA isolated from WCR first-instar larvae. (YFP was amplified from a DNA clone.) For each selected target gene region, two separate PCR amplifications were performed. The first PCR amplification introduced a T7 promoter sequence at the 5' end of the amplified sense strands. The second reaction incorporated the T7 promoter sequence at the 5' ends of the antisense strands. The two PCR amplified fragments for each region of the target genes were then mixed in approximately equal amounts, and the mixture was used as transcription template for dsRNA production. *See* **FIG. 2.** Double-stranded RNA was synthesized and purified using an AMBION® MEGAscript® RNAi kit following the manufacturer's instructions (INVITROGEN). The concentrations of dsRNAs were measured using a NANODROP™ 8000 spectrophotometer (THERMO SCIENTIFIC, Wilmington, DE) and the dsRNAs were each tested by the same diet-based bioassay methods described above. **Table 4** lists the sequences of the primers used to produce the *annexin* Reg1, *annexin* Reg2, *beta spectrin* 2 Reg1, *beta spectrin* 2 Reg2, *mtRP-L4* Reg1, *mtRP-L4* Reg2, and YFP dsRNA molecules. **Table 5** presents the results of diet-based feeding bioassays of WCR larvae following 9-day exposure to these dsRNA molecules. Replicated bioassays demonstrated that ingestion of these dsRNAs resulted in no mortality or growth inhibition of western corn rootworm larvae above that seen with control samples of TE buffer, Water, or YFP protein.

**Table 4.** Primers and Primer Pairs used to amplify portions of coding regions of genes.

| | Gene (Region) | Primer ID | Sequence |
|---|---|---|---|
| **Pair 6** | YFP | YFP-F T7 | TTAATACGACTCACTATAGGGAGACACCATGGGCTC CAGCGGCGCCC (SEQ ID NO:34) |
| | YFP | YFP-R | AGATCTTGAAGGCGCTCTTCAGG (SEQ ID NO:35) |
| **Pair 7** | YFP | YFP-F | CACCATGGGCTCCAGCGGCGCCC (SEQ ID NO:36) |
| | YFP | YFP-R_T7 | TTAATACGACTCACTATAGGGAGAAGATCTTGAAGG CGCTCTTCAGG (SEQ ID NO:37) |
| **Pair 8** | Annexin (Reg 1) | Ann-F1_T7 | TTAATACGACTCACTATAGGGAGAGCTCCAACAGTG GTTCCTTATC (SEQ ID NO:38) |
| | Annexin (Reg 1) | Ann-R1 | CTAATAATTCTTTTTTAATGTTCCTGAGG (SEQ ID NO:39) |
| **Pair 9** | Annexin (Reg 1) | Ann-F1 | GCTCCAACAGTGGT'I'CCTTATC (SEQ ID NO:40) |
| | Annexin (Reg 1) | Ann-R1_T7 | TTAATACGACTCACTATAGGGAGACTAATAATTCTT TTTTAATGTTCCTGAGG (SEQ ID NO:41) |
| **Pair 10** | Annexin (Reg 2) | Ann-F2_T7 | TTAATACGACTCACTATAGGGAGATTGTTACAAGCT GGAGAACTTCTC (SEQ ID NO:42) |
| | Annexin (Reg 2) | Ann-R2 | CTTAACCAACAACGGCTAATAAGG (SEQ ID NO:43) |
| **Pair 11** | Annexin (Reg 2) | Ann-F2 | TTGTTACAAGCTGGAGAACTTCTC (SEQ ID NO:44) |
| | Annexin (Reg 2) | Ann-R2_T7 | TTAATACGACTCACTATAGGGAGACTTAACCAACAA CGGCTAATAAGG (SEQ ID NO:45) |
| **Pair 12** | Beta-spect2 (Reg 1) | Betasp2-F1_T7 | TTAATACGACTCACTATAGGGAGAAGATGTTGGCTG CATCTAGAGAA (SEQ ID NO:46) |
| | Beta-spect2 (Reg 1) | Betasp2-R1 | GTCCATTCGTCCATCCACTGCA (SEQ ID NO:47) |

(continued)

| | Gene (Region) | Primer ID | Sequence |
|---|---|---|---|
| **Pair 13** | Beta-spect2 (Reg 1) | Betasp2-F1 | AGATGTTGGCTGCATCTAGAGAA (SEQ ID NO:48) |
| | Beta-spect2 (Reg 1) | Betasp2-R1_T7 | TTAATACGACTCACTATAGGGAGAGTCCATTCGTCC ATCCACTGCA (SEQ ID NO:49) |
| **Pair 14** | Beta-spect2 (Reg 2) | Betasp2-F2_T7 | TTAATACGACTCACTATAGGGAGAGCAGATGAACAC CAGCGAGAAA (SEQ ID NO:50) |
| | Beta-spect2 (Reg 2) | Betasp2-R2 | CTGGGCAGCTTCTTGTTTCCTC (SEQ ID NO:51) |
| **Pair 15** | Beta-spect2 (Reg 2) | Betasp2-F2 | GCAGATGAACACCAGCGAGAAA (SEQ ID NO:52) |
| | Beta-spect2 (Reg 2) | Betasp2-R2_T7 | TTAATACGACTCACTATAGGGAGACTGGGCAGCTTC TTGTTTCCTC (SEQ ID NO:53) |
| **Pair 16** | mtRP-L4 (Reg 1) | L4-F1_T7 | TTAATACGACTCACTATAGGGAGAAGTGAAATGTTA GCAAATATAACATCC (SEQ ID NO:54) |
| | mtRP-L4 (Reg 1) | L4-R1 | ACCTCTCACTTCAAATCTTGACTTTG (SEQ ID NO:55) |
| **Pair 17** | mtRP-L4 (Reg 1) | L4-F1 | AGTGAAATGTTAGCAAATATAACATCC (SEQ ID NO:56) |
| | mtRP-L4 (Reg 1) | L4-R1_T7 | TTAATACGACTCACTATAGGGAGAACCTCTCACTTC AAATCTTGACTTTG (SEQ ID NO:57) |
| **Pair 18** | mtRP-L4 (Reg 2) | L4-F2_T7 | TTAATACGACTCACTATAGGGAGACAAAGTCAAGAT TTGAAGTGAGAGGT (SEQ ID NO:58) |
| | mtRP-L4 (Reg 2) | L4-R2 | CTACAAATAAAACAAGAAGGACCCC (SEQ ID NO:59) |
| **Pair 19** | mtRP-L4 (Reg 2) | L4-F2 | CAAAGTCAAGATTTGAAGTGAGAGGT (SEQ ID NO:60) |
| | mtRP-L4 (Reg 2) | L4-R2_T7 | TTAATACGACTCACTATAGGGAGACTACAAATAAAA CAAGAAGGACCCC (SEQ ID NO:61) |

**Table 5.** Results of diet feeding assays obtained with western corn rootworm larvae after 9 days.

| Gene Name | Dose (ng/cm$^2$) | Mean Live Larval Weight (mg) | Mean % Mortality | Mean Growth Inhibition |
|---|---|---|---|---|
| ***annexin*-Reg 1** | 1000 | 0.545 | 0 | -0.262 |
| ***annexin*-Reg 2** | 1000 | 0.565 | 0 | -0.301 |
| ***beta spectrin2* Reg 1** | 1000 | 0.340 | 12 | -0.014 |
| ***beta spectrin2* Reg** 2 | 1000 | 0.465 | 18 | -0.367 |
| ***mtRP-L4* Reg 1** | 1000 | 0.305 | 4 | -0.168 |
| ***mtRP-L4* Reg 2** | 1000 | 0.305 | 7 | -0.180 |
| **TE buffer*** | 0 | 0.430 | 13 | 0.000 |
| **Water** | 0 | 0.535 | 12 | 0.000 |

(continued)

| Gene Name | Dose (ng/cm$^2$) | Mean Live Larval Weight (mg) | Mean % Mortality | Mean Growth Inhibition |
|---|---|---|---|---|
| **YFP**** | 1000 | 0.480 | 9 | -0.386 |
| *TE = Tris HCl (10 mM) plus EDTA (1 mM) buffer, pH8.<br>**YFP = Yellow Fluorescent Protein | | | | |

**EXAMPLE 6: Production of Transgenic Maize Tissues Comprising Insecticidal dsRNAs**

*Agrobacterium*-mediated Transformation Transgenic maize cells, tissues, and plants that produce one or more insecticidal dsRNA molecules (for example, at least one dsRNA molecule including a dsRNA molecule targeting a gene comprising *prp8* (*e.g.,* SEQ ID NO:1 and SEQ ID NO:3)) through expression of a chimeric gene stably-integrated into the plant genome are produced following *Agrobacterium*-mediated transformation. Maize transformation methods employing superbinary or binary transformation vectors are known in the art, as described, for example, in U.S. Patent 8,304,604, which is herein incorporated by reference in its entirety. Transformed tissues are selected by their ability to grow on Haloxyfop-containing medium and are screened for dsRNA production, as appropriate. Portions of such transformed tissue cultures may be presented to neonate corn rootworm larvae for bioassay, essentially as described in EXAMPLE 1.

*Agrobacterium* Culture Initiation. Glycerol stocks of *Agrobacterium* strain DAt13192 cells (PCT International Publication No. WO 2012/016222A2) harboring a binary transformation vector described above (EXAMPLE 4) are streaked on AB minimal medium plates (Watson, et al. (1975) J. Bacteriol. 123:255-264) containing appropriate antibiotics, and are grown at 20 °C for 3 days. The cultures are then streaked onto YEP plates (gm/L: yeast extract, 10; Peptone, 10; NaCl, 5) containing the same antibiotics and are incubated at 20 °C for 1 day.

*Agrobacterium* culture. On the day of an experiment, a stock solution of Inoculation Medium and acetosyringone is prepared in a volume appropriate to the number of constructs in the experiment and pipetted into a sterile, disposable, 250 mL flask. Inoculation Medium (Frame et al. (2011) Genetic Transformation Using Maize Immature Zygotic Embryos. IN Plant Embryo Culture Methods and Protocols: Methods in Molecular Biology. T. A. Thorpe and E. C. Yeung, (Eds), Springer Science and Business Media, LLC. pp 327-341) contains: 2.2 gm/L MS salts; 1X ISU Modified MS Vitamins (Frame *et al., ibid.*) 68.4 gm/L sucrose; 36 gm/L glucose; 115 mg/L L-proline; and 100 mg/L myo-inositol; at pH 5.4.) Acetosyringone is added to the flask containing Inoculation Medium to a final concentration of 200 μM from a 1 M stock solution in 100% dimethyl sulfoxide, and the solution is thoroughly mixed.

For each construct, 1 or 2 inoculating loops-full of *Agrobacterium* from the YEP plate are suspended in 15 mL Inoculation Medium/acetosyringone stock solution in a sterile, disposable, 50 mL centrifuge tube, and the optical density of the solution at 550 nm ($OD_{550}$) is measured in a spectrophotometer. The suspension is then diluted to $OD_{550}$ of 0.3 to 0.4 using additional Inoculation Medium/acetosyringone mixtures. The tube of *Agrobacterium* suspension is then placed horizontally on a platform shaker set at about 75 rpm at room temperature and shaken for 1 to 4 hours while embryo dissection is performed.

Ear sterilization and embryo isolation. Maize immature embryos are obtained from plants of *Zea mays* inbred line B104 (Hallauer et al. (1997) Crop Science 37:1405-1406), grown in the greenhouse and self- or sib-pollinated to produce ears. The ears are harvested approximately 10 to 12 days post-pollination. On the experimental day, de-husked ears are surface-sterilized by immersion in a 20% solution of commercial bleach (ULTRA CLOROX® Germicidal Bleach, 6.15% sodium hypochlorite; with two drops of TWEEN 20) and shaken for 20 to 30 min, followed by three rinses in sterile deionized water in a laminar flow hood. Immature zygotic embryos (1.8 to 2.2 mm long) are aseptically dissected from each ear and randomly distributed into microcentrifuge tubes containing 2.0 mL of a suspension of appropriate *Agrobacterium* cells in liquid Inoculation Medium with 200 μM acetosyringone, into which 2 μL of 10% BREAK-THRU® S233 surfactant (EVONIK INDUSTRIES; Essen, Germany) is added. For a given set of experiments, embryos from pooled ears are used for each transformation.

*Agrobacterium* co-cultivation. Following isolation, the embryos are placed on a rocker platform for 5 minutes. The contents of the tube are then poured onto a plate of Co-cultivation Medium, which contains 4.33 gm/L MS salts; 1X ISU Modified MS Vitamins; 30 gm/L sucrose; 700 mg/L L-proline; 3.3 mg/L Dicamba in KOH (3,6-dichloro-o-anisic acid or 3,6-dichloro-2-methoxybenzoic acid); 100 mg/L myo-inositol; 100 mg/L Casein Enzymatic Hydrolysate; 15 mg/L $AgNO_3$; 200 μM acetosyringone in DMSO; and 3 gm/L GELZAN™, at pH 5.8. The liquid *Agrobacteriun* suspension is removed with a sterile, disposable, transfer pipette. The embryos are then oriented with the scutellum facing up using sterile forceps with the aid of a microscope. The plate is closed, sealed with 3M™ MICROPORE™ medical tape, and placed in an incubator at 25 °C with continuous light at approximately 60 μmol $m^{-2}s^{-1}$ of Photosynthetically Active

Radiation (PAR).

Callus Selection and Regeneration of Transgenic Events. Following the Co-Cultivation period, embryos are transferred to Resting Medium, which is composed of 4.33 gm/L MS salts; 1X ISU Modified MS Vitamins; 30 gm/L sucrose; 700 mg/L L-proline; 3.3 mg/L Dicamba in KOH; 100 mg/L myo-inositol; 100 mg/L Casein Enzymatic Hydrolysate; 15 mg/L $AgNO_3$; 0.5 gm/L MES (2-(N-morpholino)ethanesulfonic acid monohydrate; PHYTOTECHNOLOGIES LABR.; Lenexa, KS); 250 mg/L Carbenicillin; and 2.3 gm/L GELZAN™; at pH 5.8. No more than 36 embryos are moved to each plate. The plates are placed in a clear plastic box and incubated at 27 °C with continuous light at approximately 50 μmol $m^{-2}s^{-1}$ PAR for 7 to 10 days. Callused embryos are then transferred (<18/plate) onto Selection Medium I, which is comprised of Resting Medium (above) with 100 nM R-Haloxyfop acid (0.0362 mg/L; for selection of calli harboring the *AAD-I* gene). The plates are returned to clear boxes and incubated at 27 °C with continuous light at approximately 50 μmol $m^{-2}s^{-1}$ PAR for 7 days. Callused embryos are then transferred (<12/plate) to Selection Medium II, which is comprised of Resting Medium (above) with 500 nM R-Haloxyfop acid (0.181 mg/L). The plates are returned to clear boxes and incubated at 27 °C with continuous light at approximately 50 μmol $m^{-2}s^{-1}$ PAR for 14 days. This selection step allows transgenic callus to further proliferate and differentiate.

Proliferating, embryogenic calli are transferred (<9/plate) to Pre-Regeneration medium. Pre-Regeneration Medium contains 4.33 gm/L MS salts; 1X ISU Modified MS Vitamins; 45 gm/L sucrose; 350 mg/L L-proline; 100 mg/L myo-inositol; 50 mg/L Casein Enzymatic Hydrolysate; 1.0 mg/L $AgNO_3$; 0.25 gm/L MES; 0.5 mg/L naphthaleneacetic acid in NaOH; 2.5 mg/L abscisic acid in ethanol; 1 mg/L 6-benzylaminopurine; 250 mg/L Carbenicillin; 2.5 gm/L GELZAN™; and 0.181 mg/L Haloxyfop acid; at pH 5.8. The plates are stored in clear boxes and incubated at 27 °C with continuous light at approximately 50 μmol $m^{-2}s^{-1}$ PAR for 7 days. Regenerating calli are then transferred (<6/plate) to Regeneration Medium in PHYTATRAYS™ (SIGMA-ALDRICH) and incubated at 28 °C with 16 hours light/8 hours dark per day (at approximately 160 μmol $m^{-2}s^{-1}$ PAR) for 14 days or until shoots and roots develop. Regeneration Medium contains 4.33 gm/L MS salts; 1X ISU Modified MS Vitamins; 60 gm/L sucrose; 100 mg/L myo-inositol; 125 mg/L Carbenicillin; 3 gm/L GELLAN™ gum; and 0.181 mg/L R-Haloxyfop acid; at pH 5.8. Small shoots with primary roots are then isolated and transferred to Elongation Medium without selection. Elongation Medium contains 4.33 gm/L MS salts; 1XISU Modified MS Vitamins; 30 gm/L sucrose; and 3.5 gm/L GELRITE™: at pH 5.8.

Transformed plant shoots selected by their ability to grow on medium containing Haloxyfop are transplanted from PHYTATRAYS™ to small pots filled with growing medium (PROMIX BX; PREMIER TECH HORTICULTURE), covered with cups or HUMI-DOMES (ARCO PLASTICS), and then hardened-off in a CONVIRON growth chamber (27 °C day/24 °C night, 16-hour photoperiod, 50-70% RH, 200 μmol $m^{-2}s^{-1}$ PAR). In some instances, putative transgenic plantlets are analyzed for transgene relative copy number by quantitative real-time PCR assays using primers designed to detect the *AAD1* herbicide tolerance gene integrated into the maize genome. Further, RNA qPCR assays are used to detect the presence of the linker sequence in expressed dsRNAs of putative transformants. Selected transformed plantlets are then moved into a greenhouse for further growth and testing.

Transfer and establishment of $T_0$ plants in the greenhouse for bioassay and seed production. When plants reach the V3-V4 stage, they are transplanted into IE CUSTOM BLEND (PROFILE/METRO MIX 160) soil mixture and grown to flowering in the greenhouse (Light Exposure Type: Photo or Assimilation; High Light Limit: 1200 PAR; 16-hour day length; 27 °C day/24 °C night).

Plants to be used for insect bioassays are transplanted from small pots to TINUS™ 350-4 ROOTPAINERS® (SPENCER-LEMAIRE INDUSTRIES, Acheson, Alberta, Canada;) (one plant per event per ROOTRAINER®). Approximately four days after transplanting to ROOTRAINERS®, plants are infested for bioassay.

Plants of the $T_1$ generation are obtained by pollinating the silks of $T_0$ transgenic plants with pollen collected from plants of non-transgenic elite inbred line B104 or other appropriate pollen donors, and planting the resultant seeds. Reciprocal crosses are performed when possible.

**EXAMPLE 7: Molecular Analyses of Transgenic Maize Tissues**

Molecular analyses (*e.g.* RNA qPCR) of maize tissues are performed on samples from leaves and roots that were collected from greenhouse grown plants on the same days that root feeding damage is assessed.

Results of RNA qPCR assays for the Per5 3'UTR are used to validate expression of hairpin transgenes. (A low level of Per5 3'UTR detection is expected in non-transformed maize plants, since there is usually expression of the endogenous Per5 gene in maize tissues.) Results of RNA qPCR assays for intervening sequence between repeat sequences (which is integral to the formation of dsRNA hairpin molecules) in expressed RNAs are used to validate the presence of hairpin transcripts. Transgene RNA expression levels are measured relative to the RNA levels of an endogenous maize gene.

DNA qPCR analyses to detect a portion of the *A4D1* coding region in gDNA are used to estimate transgene insertion copy number. Samples for these analyses are collected from plants grown in environmental chambers. Results are compared to DNA qPCR results of assays designed to detect a portion of a single-copy native gene, and simple events (having one or two copies of *prp8* transgenes) are advanced for further studies in the greenhouse.

Additionally, qPCR assays designed to detect a portion of the spectinomycin-resistance gene (*SpecR;* harbored on the binary vector plasmids outside of the T-DNA) are used to determine if the transgenic plants contain extraneous integrated plasmid backbone sequences.

RNA transcript expression level: Per 5 3'UTR qPCR. Callus cell events or transgenic plants are analyzed by real time quantitative PCR (qPCR) of the Per 5 3'UTR sequence to determine the relative expression level of the full length hairpin transcript, as compared to the transcript level of an internal maize gene (for example, GENBANK Accession No. BT069734), which encodes a TIP41-like protein (*i.e.* a maize homolog of GENBANK Accession No. AT4G34270; having a tBLASTX score of 74% identity; SEQ ID NO:62). RNA is isolated using an RNeasy™ 96 kit (QIAGEN, Valencia, CA). Following elution, the total RNA is subjected to a DNase1 treatment according to the kit's suggested protocol. The RNA is then quantified on a NANODROP 8000 spectrophotometer (THERMO SCIENTIFIC) and the concentration is normalized to 25 ng/μL. First strand cDNA is prepared using a HIGH CAPACITY cDNA SYNTHESIS KIT (INVITROGEN) in a 10 μL reaction volume with 5 μL denatured RNA, substantially according to the manufacturer's recommended protocol. The protocol is modified slightly to include the addition of 10 μL of 100 μM T20VN oligonucleotide (IDT) (TTTTTTTTTTTTTTTTTTTTVN, where V is A, C, or G, and N is A, C, G, or T; SEQ ID NO:63) into the 1 mL tube of random primer stock mix, in order to prepare a working stock of combined random primers and oligo dT.

Following cDNA synthesis, samples are diluted 1:3 with nuclease-free water, and stored at -20 °C until assayed.

Separate real-time PCR assays for the Per5 3' UTR and TIP41-like transcript are performed on a LIGHTCYCLER™ 480 (ROCHE DIAGNOSTICS, Indianapolis, IN) in 10 μL reaction volumes. For the Per5 3'UTR assay, reactions are run with Primers P5U76S (F) (SEQ ID NO:64) and P5U76A (R) (SEQ ID NO:65), and a ROCHE UNIVERSAL PROBE™ (UPL76; Catalog No. 4889960001; labeled with FAM). For the TIP41-like reference gene assay, primers TIPmxF (SEQ ID NO:66) and TIPmxR (SEQ ID NO:67), and Probe HXTIP (SEQ ID NO:68) labeled with HEX (hexachlorofluorescein) are used.

All assays include negative controls of no-template (mix only). For the standard curves, a blank (water in source well) is also included in the source plate to check for sample cross-contamination. Primer and probe sequences are set forth in **Table 6.** Reaction components recipes for detection of the various transcripts are disclosed in **Table 7,** and PCR reactions conditions are summarized in **Table 8.** The FAM (6-Carboxy Fluorescein Amidite) fluorescent moiety is excited at 465 nm and fluorescence is measured at 510 nm; the corresponding values for the HEX (hexachlorofluorescein) fluorescent moiety are 533 nm and 580 nm.

**Table 6.** Oligonucleotide sequences used for molecular analyses of transcript levels in transgenic maize.

| Target | Oligonucleotide | Sequence |
|---|---|---|
| Per5 3'UTR | P5U76S (F) | TTGTGATGTTGGTGGCGTAT (SEQ ID NO:64) |
| Per5 3'UTR | P5U76A (R) | TGTTAAATAAAACCCCAAAGATCG (SEQ ID NO:65) |
| Per5 3'UTR | Roche UPL76 (FAM-Probe) | Roche Diagnostics Catalog Number 488996001 ** |
| TIP41 | TIPmxF | TGAGGGTAATGCCAACTGGTT (SEQ ID NO:66) |
| TIP41 | TIPmxR | GCAATGTAACCGAGTGTCTCTCAA (SEQ ID NO:67) |
| TIP41 | HXTIP (HEX-Probe) | TTTTTGGCTTAGAGTTGATGGTGTACTGATGA (SEQ ID NO:68) |
| *TIP41-ike protein.<br>**NAv Sequence Not Available from the supplier. | | |

**Table 7.** PCR reaction recipes for transcript detection.

| | Per5 3'UTR | TIP-like Gene |
|---|---|---|
| **Component** | **Final Concentration** | |
| Roche Buffer | 1 X | 1X |
| P5U76S (F) | 0.4 μM | 0 |
| P5U7GA (R) | 0.4 μM | 0 |
| Roche UPL7G (FAM) | 0.2 μM | 0 |
| HEXtipZM F | 0 | 0.4 μM |

(continued)

| | Per5 3'UTR | TIP-like Gene |
|---|---|---|
| Component | Final Concentration | |
| HEXtipZM R | 0 | 0.4 μM |
| EXtipZMP (HEX) | 0 | 0.2 μM |
| cDNA (2.0 μL) | NA | NA |
| Water | To 10 μL | To 10 μL |

**Table 8.** Thermocycler conditions for RNA qPCR.

| Per5 3'UTR and TIP41-like Gene Detection | | | |
|---|---|---|---|
| **Process** | **Temp.** | **Time** | **No. Cycles** |
| Target Activation | 95 °C | 10 min | 1 |
| Denature | 95 °C | 10 sec | 40 |
| Extend | 60 °C | 40 sec | |
| Acquire FAM or HEX | 72 °C | 1 sec | |
| Cool | 40 °C | 10 sec | 1 |

Data are analyzed using LIGHTCYCLER™ Software v1.5 by relative quantification using a second derivative max algorithm for calculation of Cq values according to the supplier's recommendations. For expression analyses, expression values are calculated using the ΔΔCt method (*i.e.,* 2-(Cq TARGET - Cq REF)), which relies on the comparison of differences of Cq values between two targets, with the base value of 2 being selected under the assumption that, for optimized PCR reactions, the product doubles every cycle.

Transcript size and integrity: Northern Blot Assay. In some instances, additional molecular characterization of the transgenic plants is obtained by the use of Northern Blot (RNA blot) analysis to determine the molecular size of the *prp8* hairpin dsRNA in transgenic plants expressing a *prp8* hairpin dsRNA.

All materials and equipment are treated with RNaseZAP (AMBION/INVITROGEN) before use. Tissue samples (100 mg to 500 mg) are collected in 2 mL SAFELOCK EPPENDORF tubes, disrupted with a KLECKO™ tissue pulverizer (GARCIA MANUFACTURING, Visalia, CA) with three tungsten beads in 1 mL TRIZOL (INVITROGEN) for 5 min, then incubated at room temperature (RT) for 10 min. Optionally, the samples are centrifuged for 10 min at 4 °C at 11,000 rpm and the supernatant is transferred into a fresh 2 mL SAFELOCK EPPENDORF tube. After 200 μL chloroform are added to the homogenate, the tube is mixed by inversion for 2 to 5 min, incubated at RT for 10 minutes, and centrifuged at 12,000 x g for 15 min at 4 °C. The top phase is transferred into a sterile 1.5 mL EPPENDORF tube, 600 μL of 100% isopropanol are added, followed by incubation at RT for 10 min to 2 hr, and then centrifuged at 12,000 x g for 10 min at 4 °C to 25 °C. The supernatant is discarded and the RNA pellet is washed twice with 1 mL 70% ethanol, with centrifugation at 7,500 x g for 10 min at 4 °C to 25 °C between washes. The ethanol is discarded and the pellet is briefly air dried for 3 to 5 min before resuspending in 50 μL of nuclease-free water.

Total RNA is quantified using the NANODROP 8000® (THERMO-FISHER) and samples are normalized to 5 μg/10 μL. 10 μL of glyoxal (AMBION/INVITROGEN) are then added to each sample. Five to 14 ng of DIG RNA standard marker mix (ROCHE APPLIED SCIENCE, Indianapolis, IN) are dispensed and added to an equal volume of glyoxal. Samples and marker RNAs are denatured at 50 °C for 45 min and stored on ice until loading on a 1.25% SEAKEM GOLD agarose (LONZA, Allendale, NJ) gel in NORTHERNMAX 10 X glyoxal running buffer (AMBION/INVITROGEN). RNAs are separated by electrophoresis at 65 volts/30 mA for 2 hours and 15 minutes.

Following electrophoresis, the gel is rinsed in 2X SSC for 5 min and imaged on a GEL DOC station (BIORAD, Hercules, CA), then the RNA is passively transferred to a nylon membrane (MILLIPORE) overnight at RT, using 10X SSC as the transfer buffer (20X SSC consists of 3 sodium chloride and 300 mM trisodium citrate, pH 7.0). Following the transfer, the membrane is rinsed in 2X SSC for 5 minutes, the RNA is UV-crosslinked to the membrane (AGILENT/STRATAGENE), and the membrane is allowed to dry at room temperature for up to 2 days.

The membrane is pre-hybridized in ULTRAHYB™ buffer (AMBION/INVITROGEN) for 1 to 2 hr. The probe consists of a PCR amplified product containing the sequence of interest, (for example, the antisense sequence portion of SEQ ID NOs:7-11, as appropriate) labeled with digoxygenin by means of a ROCHE APPLIED SCIENCE DIG procedure.

Hybridization in recommended buffer is overnight at a temperature of 60 °C in hybridization tubes. Following hybridization, the blot is subjected to DIG washes, wrapped, exposed to film for 1 to 30 minutes, then the film is developed, all by methods recommended by the supplier of the DIG kit.

Transgene copy number determination. Maize leaf pieces approximately equivalent to 2 leaf punches are collected in 96-well collection plates (QIAGEN). Tissue disruption is performed with a KLECKO™ tissue pulverizer (GARCIA MANUFACTURING, Visalia, CA) in BIOSPRINT96 AP1 lysis buffer (supplied with a BIOSPRINT96 PLANT KIT; QIAGEN) with one stainless steel bead. Following tissue maceration, gDNA is isolated in high throughput format using a BIOSPRINT96 PLANT KIT and a BIOSPRINT96 extraction robot. gDNA is diluted 2:3 DNA:water prior to setting up the qPCR reaction.

qPCR analysis. Transgene detection by hydrolysis probe assay is performed by real-time PCR using a LIGHTCYCLE®480 system. Oligonucleotides to be used in hydrolysis probe assays to detect the linker sequence, or to detect a portion of the SpecR gene (*i.e.* the spectinomycin resistance gene borne on the binary vector plasmids; SEQ ID NO:69; SPC1 oligonucleotides in **Table 9),** are designed using LIGHTCYCLER® PROBE DESIGN SOFTWARE 2.0. Further, oligonucleotides to be used in hydrolysis probe assays to detect a segment of the *AAD-1* herbicide tolerance gene (SEQ ID NO:70 GAAD1 oligonucleotides in **Table 9)** are designed using PRIMER EXPRESS software (APPLIED BIOSYSTEMS). **Table 9** shows the sequences of the primers and probes. Assays are multiplexed with reagents for an endogenous maize chromosomal gene (Invertase (SEQ ID NO:71; GENBANK Accession No: U16123; referred to herein as IVR1), which serves as an internal reference sequence to ensure gDNA is present in each assay. For amplification, LIGHTCYCLER®480 PROBES MASTER mix (ROCHE APPLIED SCIENCE) is prepared at 1x final concentration in a 10 μL volume multiplex reaction containing 0.4 μM of each primer and 0.2 μM of each probe **(Table 10).** A two-step amplification reaction is performed as outlined in **Table 11.** Fluorophore activation and emission for the FAM- and HEX-labeled probes are as described above; CY5 conjugates are excited maximally at 650 nm and fluoresce maximally at 670 nm.

Cp scores (the point at which the fluorescence signal crosses the background threshold) are determined from the real time PCR data using the fit points algorithm (LIGHTCYCLER® SOFTWARE release 1.5) and the Relative Quant module (based on the ΔΔCt method). Data are handled as described previously (above; RNA qPCR).

**Table 9.** Sequences of primers and probes (with fluorescent conjugate) used for gene copy number determinations and binary vector plasmid backbone detection.

| Name | Sequence |
|---|---|
| GAAD1-F | TGTTCGGTTCCCTCTACCAA (SEQ ID NO:72) |
| GAAD1-R | CAACATCCATCACCTTGACTGA (SEQ ID NO:73) |
| GAAD1-P (FAM) | CACAGAACCGTCGCTTCAGCAACA (SEQ ID NO:74) |
| IVR1-F | TGGCGGACGACGACTTGT (SEQ ID NO:75) |
| IVR1-R | AAAGTTTGGAGGCTGCCGT (SEQ ID NO:76) |
| IVR1-P (HEX) | CGAGCAGACCGCCGTGTACTTCTACC (SEQ ID NO:77) |
| SPC1A | CTTAGCTGGATA.ACGCCAC (SEQ ID NO:78) |
| SPC1S | GACCGTAAGGCTTGATGAA (SEQ ID NO:79) |
| TQSPEC(CY5*) | CGAGATTCTCCGCGCTGTAGA (SEQ ID NO:80) |
| ST-LS1-F | GTATGTTTCTGCTTCTACCTTTGAT (SEQ ID NO:81) |
| ST-LS1-R | CCATGTTTTGGTCATATATTAGAAAAGTT (SEQ ID NO:82) |
| ST-LS1-P (FAM) | AGTAATATAGTATTTCAAGTATTTTTTTCAAAAT (SEQ ID NO:83) |
| CY5 = Cyanine-5 | |

**Table 10.** Reaction components for gene copy number analyses and plasmid backbone detection.

| Component | Amt. (μL) | Stock | Final Conc'n |
|---|---|---|---|
| 2x Buffer | 5.0 | 2x | 1x |
| Appropriate Forward Primer | 0.4 | 10 μM | 0.4 |
| Appropriate Reverse Primer | 0.4 | 10 μM | 0.4 |

(continued)

| Component | Amt. (μL) | Stock | Final Conc'n |
|---|---|---|---|
| Appropriate Probe | 0.4 | 5 μM | 0.2 |
| IVR1-Forward Primer | 0.4 | 10 μM | 0.4 |
| IVR1-Reverse Primer | 0.4 | 10 μM | 0.4 |
| IVR1-Probe | 0.4 | 5 μM | 0.2 |
| $H_2O$ | 0.6 | NA* | NA |
| gDNA | 2.0 | ND** | ND |
| **Total** | 10.0 | | |
| *NA = Not Applicable<br>**ND = Not Determined | | | |

**Table 11.** Thermocycler conditions for DNA qPCR.

| Genomic copy number analyses | | | |
|---|---|---|---|
| **Process** | **Temp.** | **Time** | **No. Cycles** |
| Target Activation | 95 °C | 10 min | 1 |
| Denature | 95 °C | 10 sec | 40 |
| Extend & Acquire FAM, HEX, or CY5 | 60 °C | 40 sec | |
| Cool | 40 °C | 10 sec | 1 |

**EXAMPLE 8: Bioassay of Transgenic Maize**

Insect Bioassays. Bioactivity of dsRNA of the subject invention produced in plant cells is demonstrated by bioassay methods. *See, e.g.,* Baum et al. (2007) Nat. Biotechnol. 25(11):1322-1326. One is able to demonstrate efficacy, for example, by feeding various plant tissues or tissue pieces derived from a plant producing an insecticidal dsRNA to target insects in a controlled feeding environment. Alternatively, extracts are prepared from various plant tissues derived from a plant producing the insecticidal dsRNA, and the extracted nucleic acids are dispensed on top of artificial diets for bioassays as previously described herein. The results of such feeding assays are compared to similarly conducted bioassays that employ appropriate control tissues from host plants that do not produce an insecticidal dsRNA, or to other control samples. Growth and survival of target insects on the test diet is reduced compared to that of the control group.

Insect Bioassays with Transgenic Maize Events. Two western corn rootworm larvae (1 to 3 days old) hatched from washed eggs are selected and placed into each well of the bioassay tray. The wells are then covered with a "PULL N' PEEL " tab cover (BIO-CV-16, BIO-SERV) and placed in a 28 °C incubator with an 18 hr/6 hr light/dark cycle. Nine days after the initial infestation, the larvae are assessed for mortality, which is calculated as the percentage of dead insects out of the total number of insects in each treatment. The insect samples are frozen at -20 °C for two days, then the insect larvae from each treatment are pooled and weighed. The percent of growth inhibition is calculated as the mean weight of the experimental treatments divided by the mean of the average weight of two control well treatments. The data are expressed as a Percent Growth Inhibition (of the negative controls). Mean weights that exceed the control mean weight are normalized to zero.

Insect bioassays in the greenhouse. Western corn rootworm (WCR, *Diabrotica virgifeaa virgifera* LeConte) eggs are received in soil from CROP CHARACTERISTICS (Farmington, MN). WCR eggs are incubated at 28 °C for 10 to 11 days. Eggs are washed from the soil, placed into a 0.15% agar solution, and the concentration is adjusted to approximately 75 to 100 eggs per 0.25 mL aliquot. A hatch plate is set up in a Petri dish with an aliquot of egg suspension to monitor hatch rates.

The soil around the maize plants growing in ROOTRANERS® is infested with 150 to 200 WCR eggs. The insects are allowed to feed for 2 weeks, after which time a "Root Rating" is given to each plant. A Node-Injury Scale is utilized for grading, essentially according to Oleson et al. (2005) J. Econ. Entomol. 98:1-8. Plants passing this bioassay, showing reduced injury, are transplanted to 5-gallon pots for seed production. Transplants are treated with insecticide to prevent further rootworm damage and insect release in the greenhouses. Plants are hand pollinated for seed production.

Seeds produced by these plants are saved for evaluation at the Ti and subsequent generations of plants.

Greenhouse bioassays include two kinds of negative control plants. Transgenic negative control plants are generated by transformation with vectors harboring genes designed to produce a yellow fluorescent protein (YFP) or a *YFP* hairpin dsRNA (*See* EXAMPLE 4). Non-transformed negative control plants are grown from seeds of parental corn varieties from which the transgenic plants were produced. Bioassays are conducted on two separate dates, with negative controls included in each set of plant materials.

**EXAMPLE 9: Transgenic *Zea mays* Comprising Coleopteran Pest Sequences**

10-20 transgenic To *Zea mays* plants are generated as described in EXAMPLE 6. A further 10-20 $T_1$ *Zea mays* independent lines expressing hairpin dsRNA for an RNAi construct are obtained for corn rootworm challenge. Hairpin dsRNA comprise a portion of SEQ ID NO:1 and/or SEQ ID NO:3. Additional hairpin dsRNAs are derived, for example, from coleopteran pest sequences such as, for example, *Caf1-180* (U.S. Patent Application Publication No. 2012/0174258), *VatpaseC* (U.S. Patent Application Publication No. 2012/0174259), *Rho1* (U.S. Patent Application Publication No. 2012/0174260), *VatpaseH* (U.S. Patent Application Publication No. 2012/0198586), *PPI-87B* (U.S. Patent Application Publication No. 2013/0091600), *RPA70* (U.S. Patent Application Publication No. 2013/0091601), *RPS6* (U.S. Patent Application Publication No. 2013/0097730), *ROP* (U.S. Patent Application Publication No. 14/577,811), *RNA polymerase I1* (U.S. Patent Application Publication No. 62/133,214), *RNA polymerase II140* (U.S. Patent Application Publication No. 14/577,854), *RNA polymerase II215* (U.S. Patent Application Publication No. 62/133,202), *RNA polymerase II33* (U.S. Patent Application Publication No. 62/133,210), *transcription elongation factor spt5* (U.S. Patent Application No. 62/168,613), *transcription elongation factor spt6* (U.S. Patent Application No. 62/168,606), *ncm* (U.S. Patent Application No. 62/095487), *dre4* (U.S. Patent Application No. 14/705,807), *COPI alpha* (U.S. Patent Application No. 62/063,199), *COPI beta* (U.S. Patent Application No. 62/063,203), *COPI gamma* (U.S. Patent Application No. 62/063,192), and *COPI delta* (U.S. Patent Application No. 62/063,216). These are confirmed through RT-PCR or other molecular analysis methods.

Total RNA preparations from selected independent $T_1$ lines are optionally used for RT-PCR with primers designed to bind in the linker of the hairpin expression cassette in each of the RNAi constructs. In addition, specific primers for each target gene in an RNAi construct are optionally used to amplify and confirm the production of the pre-processed mRNA required for siRNA production *in planta.* The amplification of the desired bands for each target gene confirms the expression of the hairpin RNA in each transgenic *Zea mays* plant. Processing of the dsRNA hairpin of the target genes into siRNA is subsequently optionally confirmed in independent transgenic lines using RNA blot hybridizations.

Moreover, RNAi molecules having mismatch sequences with more than 80% sequence identity to target genes affect corn rootworms in a way similar to that seen with RNAi molecules having 100% sequence identity to the target genes. The pairing of mismatch sequence with native sequences to form a hairpin dsRNA in the same RNAi construct delivers plant-processed siRNAs capable of affecting the growth, development, and viability of feeding coleopteran pests.

*In planta* delivery of dsRNA, siRNA, or miRNA corresponding to target genes and the subsequent uptake by coleopteran pests through feeding results in down-regulation of the target genes in the coleopteran pest through RNA-mediated gene silencing. When the function of a target gene is important at one or more stages of development, the growth and/or development of the coleopteran pest is affected, and in the case of at least one of WCR, NCR, SCR, MCR, *D. balteata* LeConte, *D. speciosa* Germar, *D. u. tenella,* and *D. u. undecimpunctata* Mannerheim, leads to failure to successfully infest, feed, and/or develop, or leads to death of the coleopteran pest. The choice of target genes and the successful application of RNAi are then used to control coleopteran pests.

Phenotypic comparison of transgenic RNAi lines and nontransfonned *Zea mays.* Target coleopteran pest genes or sequences selected for creating hairpin dsRNA have no similarity to any known plant gene sequence. Hence, it is not expected that the production or the activation of (systemic) RNAi by constructs targeting these coleopteran pest genes or sequences will have any deleterious effect on transgenic plants. However, development and morphological characteristics of transgenic lines are compared with non-transformed plants, as well as those of transgenic lines transformed with an "empty" vector having no hairpin-expressing gene. Plant root, shoot, foliage and reproduction characteristics are compared. There is no observable difference in root length and growth patterns of transgenic and non-transformed plants. Plant shoot characteristics such as height, leaf numbers and sizes, time of flowering, floral size and appearance are similar. In general, there are no observable morphological differences between transgenic lines and those without expression of target iRNA molecules when cultured *in vitro* and in soil in the glasshouse.

**EXAMPLE 10: Transgenic *Zea mays* Comprising a Coleopteran Pest Sequence and Additional RNAi Constructs**

A transgenic *Zea mays* plant comprising a heterologous coding sequence in its genome that is transcribed into an iRNA molecule that targets an organism other than a coleopteran pest is secondarily transformed *via Agrobacterium* or WHISKERS™ methodologies (*see* Petolino and Arnold (2009) Methods Mol. Biol. 526:59-67) to produce one or more insecticidal dsRNA molecules (for example, at least one dsRNA molecule including a dsRNA molecule targeting a gene comprising SEQ ID NO:1 and/or SEQ ID NO:3). Plant transformation plasmid vectors prepared essentially as described in EXAMPLE 4 are delivered *via Agrobacterium* or WHISKERS™-mediated transformation methods into maize suspension cells or immature maize embryos obtained from a transgenic Hi II or B104 *Zea mays* plant comprising a heterologous coding sequence in its genome that is transcribed into an iRNA molecule that targets an organism other than a coleopteran pest.

**EXAMPLE 11: Transgenic *Zea mays* Comprising an RNAi Construct and Additional Coleopteran Pest Control Sequences**

A transgenic *Zea mays* plant comprising a heterologous coding sequence in its genome that is transcribed into an iRNA molecule that targets a coleopteran pest organism (for example, at least one dsRNA molecule including a dsRNA molecule targeting a gene comprising SEQ ID NO:1 and/or SEQ ID NO:3) is secondarily transformed *via Agrobacterium* or WHISKERS™ methodologies (*see* Petolino and Arnold (2009) Methods Mol. Biol. 526:59-67) to produce one or more insecticidal protein molecules, for example, Cry3, Cry34 and Cry35 insecticidal proteins. Plant transformation plasmid vectors prepared essentially as described in EXAMPLE 4 are delivered *via Agrobacterium* or WHISKERS™-mediated transformation methods into maize suspension cells or immature maize embryos obtained from a transgenic B104 *Zea mays* plant comprising a heterologous coding sequence in its genome that is transcribed into an iRNA molecule that targets a coleopteran pest organism. Doubly-transformed plants are obtained that produce iRNA molecules and insecticidal proteins for control of coleopteran pests.

**EXAMPLE 12: Pollen Beetle Transcriptome**

Insects. Larvae and adult pollen beetles were collected from fields with flowering rapeseed plants (Giessen, Germany). Young adult beetles (each per treatment group: n = 20; 3 replicates) were challenged by injecting a mixture of two different bacteria (*Staphylococcus aureus* and *Pseudomonas aeruginosa*), one yeast (*Saccharomyces cerevisiae*) and bacterial LPS. Bacterial cultures were grown at 37 °C with agitation, and the optical density was monitored at 600 nm (OD600). The cells were harvested at OD600 ~1 by centrifugation and resuspended in phosphate-buffered saline. The mixture was introduced ventrolaterally by pricking the abdomen of pollen beetle imagoes using a dissecting needle dipped in an aqueous solution of 10 mg/ml LPS (purified *E. coli* endotoxin; SIGMA, Taufkirchen, Germany) and the bacterial and yeast cultures. Along with the immune challenged beetles, naïve beetles, and larvae were collected (n = 20 per and 3 replicates each) at the same time point.

RNA isolation. Total RNA was extracted 8 h after immunization from frozen beetles and larvae using TriReagent (Molecular Research Centre, Cincinnati, OH, USA) and purified using the RNeasy Micro Kit (Qiagen, Hilden, Germany) in each case following the manufacturers' guidelines. The integrity of the RNA was verified using an Agilent 2100 Bioanalyzer and a RNA 6000 Nano Kit (Agilent Technologies, Palo Alto, CA, USA). The quantity of RNA was determined using a Nanodrop ND-1000 spectrophotometer. RNA was extracted from each of the adult immune-induced treatment groups, adult control groups, and larval groups individually and equal amounts of total RNA were subsequently combined in one pool per sample (immune-challenged adults, control adults and larvae) for sequencing.

Transcriptome information. RNA-Seq data generation and assembly Single-read 100-bp RNA-Seq was carried out separately on 5 μg total RNA isolated from immune-challenged adult beetles, naive (control) adult beetles, and untreated larvae. Sequencing was carried out by EUROFINS MWG Operon using the Illumina HiSeq-2000 platform. This yielded 20.8 million reads for the adult control beetle sample, 21.5 million reads for the LPS-challenged adult beetle sample and 25.1 million reads for the larval sample. The pooled reads (67.5 million) were assembled using *Velvet*/*Oases* assembler software (Schulz et al. (2012) Bioinformatics. 28:1086-92; Zerbino and Birney (2008) Genome Res. 18:821-9). The transcriptome contained 55,648 sequences.

Pollen beetle *prp8* identification. A tblastn search of the transcriptome was used to identify matching contigs. As a query the peptide sequence of *prp8* from *Drosophilia* was used (Genbank NP_652610).

**EXAMPLE 13: Mortality of Pollen Beetle following treatment with *prp8* iRNA**

Gene-specific primers including the T7 polymerase promoter sequence at the 5' end were used to create PCR products of approximately 424 bp by PCR (SEQ ID NO:12). PCR fragments were cloned in the pGEM T easy vector according to the manufacturer's protocol and sent to a sequencing company to verify the sequence. The dsRNA was then produced by the T7 RNA polymerase (MEGAscript® RNAi Kit, Applied Biosystems) from a PCR construct generated from the sequenced plasmid according to the manufacturer's protocol.

Injection of ~100 nL dsRNA (1 μg/ul) into adult beetles was performed with a micromanipulator under a dissecting stereomicroscope (n = 10, 3 biological replications). Animals were anaesthetized on ice before they were affixed to double-stick tape. Controls received the same volume of water. All controls in all stages could not be tested due to a lack of animals. Controls were performed on a different date due to the limited availability of insects. Pollen beetles were maintained in Petri dishes with dried pollen and a wet tissue.

**Table 12.** Results of *M. aeneus* adult pollen beetle injection bioassay (Percentage of survival mean ± standard deviation (SD), n = 3 groups of 10).

| Treatment | % Survival Mean ± SD | | | | |
|---|---|---|---|---|---|
| | **Day 0** | **Day 2** | **Day 4** | **Day 6** | **Day 8** |
| *prp8* | 100 ± 0 | 100 ± 0 | 100 ± 0 | 97 ± 6 | 77 ± 32 |
| Control | 100 ± 0 | 100 ± 0 | 100 ± 0 | 100 ± 0 | 93 ± 6 |
| | **Day 10** | **Day 12** | **Day 14** | **Day 16** | |
| *prp8* | 73 ± 29 | 43 ± 25 | 37 ± 29 | 23 ± 15 | |
| Control | 93 ± 6 | 83 ± 6 | 80 ± 0 | 67 ± 6 | |

Feeding Bioassay: Beetles were kept without access to water in empty falcon tubes 24 h before treatment. A droplet of dsRNA (~5 μL) was placed in a small Petri dish, and 5 to 8 beetles were added to the Petri dish. Animals were observed under a stereomicroscope, and those that ingested dsRNA containing diet solution were selected for the bioassay. Beetles were transferred into petri dishes with dried pollen and a wet tissue. Controls received the same volume of water. All controls in all stages could not be tested due to a lack of animals. Controls were performed on a different date due to the limited availability of insects.

**Table 13.** Results of *M. aeneus* adult feeding bioassay (Percentage of survival mean ± standard deviation (SD), n = 3 groups of 10).

| Treatment | % Survival Mean ± SD | | | | |
|---|---|---|---|---|---|
| | **Day 0** | **Day 2** | **Day 4** | **Day 6** | **Day 8** |
| *prp8* | 100 ± 0 | 100 ± 0 | 100 ± 0 | 97 ± 6 | 87 ± 15 |
| Control | 100 ± 0 | 100 ± 0 | 100 ± 0 | 90 ± 10 | 8 ± 12 |
| | **Day 10** | **Day 12** | **Day 14** | **Day 16** | |
| *prp8* | 73 ± 15 | 60 ± 10 | 53 ± 15 | 47 ± 21 | |
| Control | 87 ± 12 | 87 ± 12 | 87 ± 12 | 87 ± 12 | |

**Table 14.** Results of *M. aeneus* adult feeding bioassay (Percentage of survival mean ± standard deviation (SD), n = 3 groups of 10).

| Treatment | % Survival Mean ± SD | | | | |
|---|---|---|---|---|---|
| | **Day 0** | **Day 2** | **Day 4** | **Day 6** | **Day 8** |
| *prp8* | 100 ± 0 | 97 ± 6 | 97 ± 6 | 93 ± 6 | 93 ± 6 |
| Control | 100 ± 0 | 100 ± 0 | 97 ± 6 | 97 ± 6 | 97 ± 6 |
| | **Day 10** | **Day 12** | **Day 14** | **Day 16** | |
| *prp8* | 90 ± 10 | 77 ± 21 | 77 ± 21 | 73 ± 23 | |
| Control | 97 ± 6 | 90 ± 0 | 90 ± 0 | 90 ± 0 | |

**EXAMPLE 14: *Agrobacterium*-mediated Transformation of Canola Hypocotyls**

*Agrobacterium* Preparation. The *Agrobacterium* strain containing the binary plasmid is streaked out on YEP media (Bacto Peptone™ 20.0 gm/L and Yeast Extract 10.0 gm/L) plates containing streptomycin (100 mg/ml) and spectinomycin (50 mg/mL) and incubated for 2 days at 28°C. The propagated *Agrobacterium* strain containing the binary plasmid is scraped from the 2-day streak plate using a sterile inoculation loop. The scraped *Agrobacterium* strain containing the binary plasmid is then inoculated into 150 mL modified YEP liquid with streptomycin (100 mg/ml) and spectinomycin (50 mg/ml) into sterile 500 mL baffled flask(s) and shaken at 200 rpm at 28°C. The cultures are centrifuged and resuspended in M-medium (LS salts, 3% glucose, modified B5 vitamins, 1 $\mu$M kinetin, 1 $\mu$M 2,4-D, pH 5.8) and diluted to the appropriate density (50 Klett Units as measured using a spectrophotometer) prior to transformation of canola hypocotyls.

Canola Transformation. *Seed germination:* Canola seeds (var. NEXERA 710™) are surface-sterilized in 10% Glorox™ for 10 minutes and rinsed three times with sterile distilled water (seeds are contained in steel strainers during this process). Seeds are planted for germination on ½ MS Canola medium (1/2 MS, 2% sucrose, 0.8% agar) contained in Phytatrays™ (25 seeds per Phytatray™) and placed in a Percival™ growth chamber with growth regime set at 25°C, photoperiod of 16 hours light and 8 hours dark for 5 days of germination.

*Pre-treatment:* On day 5, hypocotyl segments of about 3 mm in length are aseptically excised, the remaining root and shoot sections are discarded (drying of hypocotyl segments is prevented by immersing the hypocotyls segments into 10 mL of sterile milliQ™ water during the excision process). Hypocotyl segments are placed horizontally on sterile filter paper on callus induction medium, MSK1D1 (MS, 1 mg/L kinetin, 1 mg/L 2,4-D, 3.0% sucrose, 0.7% phytagar) for 3 days pre-treatment in a Percival™ growth chamber with growth regime set at 22-23°C, and a photoDeriod of 16 hours light, 8 hours dark.

*Co-cultivation with Agrobacterium:* The day before *Agrobacterium* co-cultivation, flasks of YEP medium containing the appropriate antibiotics, are inoculated with the *Agrobacterium* strain containing the binary plasmid. Hypocotyl segments are transferred from filter paper callus induction medium, SK1D1 to an empty 100 x 25 mm Petri™ dishes containing 10 mL of liquid M-medium to prevent the hypocotyl segments from drying. A spatula is used at this stage to scoop the segments and transfer the segments to new medium. The liquid M-medium is removed with a pipette and 40 mL of *Agrobacterium* suspension is added to the Petri™ dish (500 segments with 40 mL of *Agrobacterium* solution). The hypocotyl segments are treated for 30 minutes with periodic swirling of the Petri™ dish so that the hypocotyl segments remained immersed in the *Agrobacterium* solution. At the end of the treatment period, the *Agrabacterium* solution is pipetted into a waste beaker; autoclaved and discarded (the *Agrobacterium* solution is completely removed to prevent *Agrobacterium* overgrowth). The treated hypocotyls are transferred with forceps back to the original plates containing MSK1D1 media overlaid with filter paper (care is taken to ensure that the segments did not dry). The transformed hypocotyl segments and non-transformed control hypocotyl segments are returned to the Percival™ growth chamber under reduced light intensity (by covering the plates with aluminum foil), and the treated hypocotyl segments are co-cultivated with *Agrobacterium* for 3 days.

*Callus induction on selection medium:* After 3 days of co-cultivation, the hypocotyl segments are individually transferred with forceps onto callus induction medium, MSK1D1H1 (MS, 1 mg/L kinetin, 1 mg/L 2,4-D, 0.5 gm/L MES, 5 mg/L $AgNO_3$, 300 mg/L Timentin™, 200 mg/L carbenicillin, 1 mg/L Herbiace™, 3% sucrose, 0.7% phytagar) with growth regime set at 22-26°C. The hypocotyl segments are anchored on the medium but are not deeply embedded into the medium.

*Selection and shoot regeneration:* After 7 days on callus induction medium, the callusing hypocotyl segments are transferred to Shoot Regeneration Medium 1 with selection, MSB3Z1H1 (MS, 3 mg/L BAP, 1 mg/L zeatin, 0.5 gm/L MES, 5 mg/L $AgNO_3$, 300 mg/L Timentin™, 200 mg/L carbenicillin, 1 mg/L Herbiace™, 3% sucrose, 0.7% phytagar). After 14 days, the hypocotyl segments which develop shoots are transferred to Regeneration Medium 2 with increased selection, MSB3Z1H3 (MS, 3 mg/L BAP, 1 mg/L Zeatin, 0.5 gm/L MES, 5 mg/L $AgNO_3$, 300 mg/l Timentin™, 200 mg/L carbenicillin, 3 mg/L Herbiace™, 3% sucrose, 0.7% phytagar) with growth regime set at 22-26 °C.

*Shoot elongation:* After 14 days, the hypocotyl segments that develop shoots are transferred from Regeneration Medium 2 to shoot elongation medium, MSMESH5 (MS, 300 mg/L Timentin™, 5 mg/l Herbiace™, 2% sucrose, 0.7% TC Agar) with growth regime set at 22-26 °C. Shoots that are already elongated are isolated from the hypocotyl segments and transferred to MSMESH5. After 14 days the remaining shoots which have not elongated in the first round of culturing on shoot elongation medium are transferred to fresh shoot elongation medium, MSMESH5. At this stage all remaining hypocotyl segments which do not produce shoots are discarded.

*Root induction:* After 14 days of culturing on the shoot elongation medium, the isolated shoots are transferred to MSMEST medium (MS, 0.5 g/L MES, 300 mg/L Timentin™, 2% sucrose, 0.7% TC Agar) for root induction at 22-26 °C. Any shoots which do not produce roots after incubation in the first transfer to MSMEST medium are transferred for a second or third round of incubation on MSMEST medium until the shoots develop roots.

### Example 12: *prp8* dsRNA in Insect Management

*Prp8* dsRNA transgenes are combined with other dsRNA molecules in transgenic plants to provide redundant RNAi targeting and synergistic RNAi effects. Transgenic plants including, for example and without limitation, corn, soybean, and cotton expressing dsRNA that targets *prp8* are useful for preventing feeding damage by coleopteran insects. *Prp8* dsRNA transgenes are also combined in plants with *Bacillus thuringiensis* insecticidal protein technology to represent new modes of action in Insect Resistance Management gene pyramids. When combined with other dsRNA molecules that target insect pests and/or with insecticidal proteins in transgenic plants, a synergistic insecticidal effect is observed that also mitigates the development of resistant insect populations.

While the present disclosure may be susceptible to various modifications and alternative forms, specific embodiments have been described by way of example in detail herein. However, it should be understood that the present disclosure is not intended to be limited to the particular forms disclosed. Rather, the present disclosure is to cover all modifications, equivalents, and alternatives falling within the scope of the present disclosure as defined by the following appended claims and their legal equivalents.

The present invention pertains to the following embodiments:

1. An isolated nucleic acid molecule comprising at least one polynucleotide operably linked to a heterologous promoter, wherein the polynucleotide comprises a nucleotide sequence selected from the group consisting of:

SEQ ID NO:5; the complement of SEQ ID NO:5; a fragment of at least 15 contiguous nucleotides of SEQ ID NO:5; the complement of a fragment of at least 15 contiguous nucleotides of SEQ ID NO:5; a native coding sequence of a *Meligethes* organism comprising SEQ ID NO:12; the complement of a native coding sequence of a *Meligethes* organism comprising SEQ ID NO:12; a fragment of at least 15 contiguous nucleotides of a native coding sequence of a *Meligethes* organism comprising SEQ ID NO: 12; and the complement of a fragment of at least 15 contiguous nucleotides of a native coding sequence of a *Meligethes* organism comprising SEQ ID NO: 12.

2. The nucleic acid molecule of item 1, wherein the nucleotide sequence is selected from the group consisting of SEQ ID NO:5, SEQ ID NO:12, and the complements of the foregoing.
3. The nucleic acid molecule of item 1 or 2, wherein the molecule is a vector.
4. The nucleic acid molecule of any of items 1 to 3, wherein the organism is selected from the group consisting *ofD.* v. *virgifera* LeConte; *D. barberi* Smith and Lawrence; *D. u. howardi; D.* v. *zeae; D. balteata* LeConte; *D. u. tenella; D. u. undecimpunctata* Mannerheim; *D. speciosa* Germar; and *Meligethes aeneus* Fabricius (Pollen Beetle).
5. A ribonucleic acid (RNA) molecule encoded the nucleic acid molecule of any of items 1 to, wherein the RNA molecule comprises a polyribonucleotide encoded by the polynucleotide.
6. The RNA molecule of item 5, wherein the molecule is a double-stranded ribonucleic acid (dsRNA) molecule.
7. The dsRNA molecule of item 6, wherein contacting the molecule with a coleopteran pest inhibits the expression of an endogenous nucleic acid molecule that is specifically complementary to the polyribonucleotide.
8. The dsRNA molecule of item 7, wherein contacting the molecule with the coleopteran pest kills or inhibits the growth and/or feeding of the pest.
9. The dsRNA molecule of any of items 6 to 8, comprising a first, a second, and a third polyribonucleotide, wherein the first polyribonucleotide is encoded by the nucleotide sequence, wherein the third polyribonucleotide is linked to the first polyribonucleotide by the second polyribonucleotide, and wherein the third polyribonucleotide is substantially the reverse complement of the first polyribonucleotide, such that the first and the third polyribonucleotides hybridize when transcribed into a ribonucleic acid to form the dsRNA.
10. The RNA molecule of item 5, wherein the RNA molecule is a single-stranded ribonucleic acid molecule of between about 15 and about 30 nucleotides in length.
11. The vector of item 3, wherein the heterologous promoter is functional in a plant cell, and wherein the vector is a plant transformation vector.
12. A cell comprising the nucleic acid molecule of any of items 1 to 4.
13. The cell of item 12, wherein the cell is a prokaryotic cell.
14. The cell of item 12, wherein the cell is a eukaryotic cell.
15. The cell of item 14, wherein the cell is a plant cell.
16. A plant comprising the plant cell of item 15.
17. A part of the plant of item 16, wherein the plant part comprises the plant cell.
18. The plant part of item 17, wherein the plant part is a seed.
19. A food product or commodity product produced from the plant of item 16, wherein the product comprises a detectable amount of the nucleic acid molecule.
20. The plant of item 16, wherein a cell of the plant comprises a double-stranded ribonucleic acid (dsRNA) molecule encoded by the polynucleotide.
21. The plant cell of item 15, wherein the cell is a *Zea mays, Brassica* sp., or *Poaceae* cell.
22. The plant of item 16, wherein the plant is *Zea mays, Brassica* sp., or a plant of the family *Poaceae,*
23. The plant of item 20, wherein the dsRNA molecule inhibits the expression of an endogenous polynucleotide that is specifically complementary to the polyribonucleotide when a coleopteran pest ingests a part of the plant.
24. The nucleic acid molecule of any of items 1 to 4, further comprising at least one additional polynucleotide operably linked to a heterologous promoter, wherein the additional polynucleotide encodes an RNA molecule.
25. The nucleic acid molecule of item 24, wherein the heterologous promoter is functional in a plant cell, and wherein the molecule is a plant transformation vector.
26. A method for controlling an insect pest population, the method comprising providing an agent comprising a ribonucleic acid (RNA) molecule that functions upon contact with the insect pest to inhibit a biological function within the pest, wherein the RNA is specifically hybridizable with a polynucleotide selected from the group consisting of SEQ ID NOs:91 and 92; the complement of any of SEQ ID NOs: 91 and 92; a fragment of at least 15 contiguous nucleotides of any of SEQ ID NOs: 91 and 92; the complement of a fragment of at least 15 contiguous nucleotides of any of SEQ ID NOs: 91 and 92; a transcript of SEQ ID NO: 5; and the complement of a transcript of SEQ ID NO: 5.
27. The method according to item 26, wherein the RNA molecule is a double-stranded RNA (dsRNA) molecule.
28. The method according to item 26 or 27, wherein providing the agent comprises contacting the insect pest with a sprayable composition comprising the agent.
29. The method according to item 26, wherein providing the agent comprises feeding a plant cell comprising the RNA molecule to the insect pest.
30. A method for controlling a coleopteran pest population, the method comprising:

providing an agent comprising a first and a second polyribonucleotide that functions upon contact with the coleopteran pest to inhibit a biological function within the coleopteran pest, wherein the first polyribonucleotide comprises a nucleotide sequence having from about 90% to about 100% sequence identity to from about 15 to about 30 contiguous nucleotides of a polyribonucleotide selected from the group consisting of SEQ ID NOs: 91 and 92, and wherein the first polyribonucleotide is specifically hybridized to the second polyribonucleotide.

31. A method for controlling a coleopteran pest population, the method comprising:

providing in a host plant of a coleopteran pest a plant cell comprising the nucleic acid molecule of item 1, wherein the polynucleotide is expressed to produce a ribonucleic acid (dsRNA) molecule that functions upon contact with a coleopteran pest belonging to the population to inhibit the expression of a target sequence within the coleopteran pest and results in decreased growth and/or survival of the coleopteran pest or pest population, relative to development of the same pest species on a plant of the same host plant species that does not comprise the polynucleotide.

32. The method according to item 31, wherein the coleopteran pest population is reduced relative to a population of the same pest species infesting a host plant of the same host plant species lacking a plant cell comprising the nucleic acid molecule.
33. A method of controlling a coleopteran pest infestation in a plant, the method comprising providing in the diet of a coleopteran pest infesting the plant a ribonucleic acid (RNA) molecule that is specifically hybridizable with a polyribonucleotide selected from the group consisting of:

SEQ ID NOs: 91 and 92;
the complement of any of SEQ ID NOs: 91 and 92;
a fragment of at least 15 contiguous nucleotides of any of SEQ ID NOs: 91 and 92;
the complement of a fragment of at least 15 contiguous nucleotides of any of NOs: 91 and 92;
a transcript of any of SEQ ID NO: 5;
the complement of a transcript of any of SEQ ID NO: 5;
a fragment of at least 15 contiguous nucleotides of a transcript of SEQ ID NO: 5; and
the complement of a fragment of at least 15 contiguous nucleotides of a transcript of SEQ ID NO: 5.

34. The method according to item 33, wherein the diet comprises a plant cell comprising a polynucleotide that is transcribed to express the polyribonucleotide.
35. The method according to item 33 or 34, wherein the RNA molecule is a double-stranded RNA (dsRNA) molecule.
36. A method for improving the yield of a crop, the method comprising:

cultivating in the crop a plant comprising the nucleic acid molecule of item 1 to allow the expression of the polynucleotide.

37. The method according to item 36, wherein the plant is *Zea may, Brassica* sp., or a plant of the family *Poaceae.*

38. The method according to item 36 or 37, wherein expression of the polynucleotide produces an RNA molecule that suppresses a target gene in a coleopteran pest that has contacted a portion of the plant, thereby inhibiting the development or growth of the coleopteran pest and loss of yield due to infection by the coleopteran pest.
39. A method for producing a transgenic plant cell, the method comprising:

transforming a plant cell with the plant transformation vector of item 11;
culturing the transformed plant cell under conditions sufficient to allow for development of a plant cell culture comprising a plurality of transformed plant cells;
selecting for transformed plant cells that have integrated the polynucleotide into their genomes;
screening the transformed plant cells for expression of a ribonucleic acid (RNA) molecule encoded by the polynucleotide; and
selecting a plant cell that expresses the RNA.

40. The method according to item 39, wherein the RNA molecule is a double-stranded RNA molecule.
41. A method for producing a coleopteran pest-resistant transgenic plant, the method comprising:

regenerating a transgenic plant from a transgenic plant cell comprising the nucleic acid molecule of any of items 1 to 4, wherein expression of a ribonucleic acid (RNA) molecule encoded by the polynucleotide is sufficient to modulate the expression of a target gene in the coleopteran pest when it contacts the RNA molecule.

42. A method for producing a transgenic plant cell, the method comprising:

transforming a plant cell with a vector comprising a means for providing *prp8*-inediated *Diabrotica* pest protection to a plant;
culturing the transformed plant cell under conditions sufficient to allow for development of a plant cell culture comprising a plurality of transformed plant cells;
selecting for transformed plant cells that have integrated the means for providing *prp8*-mediated *Diabrotica* pest protection to a plant into their genomes;
screening the transformed plant cells for expression of a means for inhibiting expression of a *prp8* gene in a *Diabrotica* pest; and
selecting a plant cell that expresses the means for inhibiting expression of a *prp8* gene in a *Diabrotica* pest.

43. A method for producing a transgenic plant, the method comprising:

regenerating a transgenic plant from the transgenic plant cell produced by the method according to item 42, wherein plant cells of the plant comprise the means for inhibiting expression of a *prp8* gene in a *Diabrotica* pest.

44. The method according to item 43, wherein expression of the means for inhibiting expression of a *prp8* gene in a *Diabrotica* pest is sufficient to modulate the expression of a target *prp8* gene in a *Diabrotica* pest that infests the transgenic plant.
45. A plant comprising means for inhibiting expression of a *prp8* gene in a *Diabratica* pest.
46. A method for producing a transgenic plant cell, the method comprising:

transforming a plant cell with a vector comprising a means for providing *prp8*-mediated *Meligethes* pest protection to a plant;
culturing the transformed plant cell under conditions sufficient to allow for development of a plant cell culture comprising a plurality of transformed plant cells;
selecting for transformed plant cells that have integrated the means for providing *prp8*-mediated *Meligethes* pest protection to a plant into their genomes;
screening the transformed plant cells for expression of a means for inhibiting expression of a *prp8* gene in a *Meligethes* pest; and
selecting a plant cell that expresses the means for inhibiting expression of a *prp8* gene in a *Meligetlaes* pest.

47. A method for producing a transgenic plant, the method comprising:

regenerating a transgenic plant from the transgenic plant cell produced by the method according to item 46, wherein plant cells of the plant comprise the means for inhibiting expression of *a prp8* gene in a *Meligethes* pest.

48. The method according to item 47, wherein expression of the means for inhibiting expression of a *prp8* gene in a *Meligethes* pest is sufficient to modulate the expression of a target *prp8* gene in a *Meligethes* pest that infests the transgenic plant.

49. A plant comprising means for inhibiting expression of a *prp8* gene in a *Meligethes* pest.

50. The nucleic acid molecule of any of items 1 to 4, further comprising a polynucleotide encoding an insecticidal polypeptide from *Bacillus thuringiensis, Alcaligenes* spp., or *Pseudomonas* spp.

51. The nucleic acid molecule of item 50, wherein the insecticidal polypeptide is selected from the group of *B, thuringiensis* insecticidal polypeptides consisting of Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A, and Cyt2C.

52. The plant cell of item 15, wherein the cell comprises a polynucleotide encoding an insecticidal polypeptide from *Bacillus thuringiensis, Alcaligenes* spp., or *Pseudomonas* spp.

53. The plant cell of item 52, wherein the insecticidal polypeptide is selected from the group of *B, thuringiensis* insecticidal polypeptides consisting of Cry1B, Coy1I, Cry3, Cry7A, Cry8, Cry9D, Cry14, Cry18, Cry22, Cry23, Cry34, Cry35, Cry36, Cry37, Cry43, Cry55, Cyt1A, and Cyt2C.

54. The plant of item 16, wherein a cell of the plant comprises a polynucleotide encoding an insecticidal polypeptide from *Bacillus thuringiensis, Alcaligenes* spp., or *Pseudomonas* spp.

55. The plant of item 54, wherein the insecticidal polypeptide is selected from the group of *B. thuringiensis* insecticidal polypeptides consisting of Cry1B, Acry1I, Cry2A, Cry3, Cry7A, Cry8, Cry9D, Cry14, Cry18, Cry22, Cry23, Cry34, Cry35, Cry36, Cry37, Cry43, Cry55, Cyt1A, and Cyt2C.

56. The method according to item 30, wherein the plant cell comprises a polynucleotide encoding an insecticidal polypeptide from *Bacillus thuringiensis, Alcaligenes* spp., or *Pseudomonas* spp.

57. The method according to item 56, wherein the insecticidal polypeptide is selected from the group of *B. thuringiensis* insecticidal polypeptides consisting of Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A, and Cyt2C.

## Claims

**1.** An isolated nucleic acid molecule comprising at least one polynucleotide operably linked to a heterologous promoter, wherein the polynucleotide comprises a nucleotide sequence selected from the group consisting of:

SEQ ID NO: 5; the complement of SEQ ID NO: 5; a fragment of at least 15 contiguous nucleotides of SEQ ID NO: 5; the complement of a fragment of at least 15 contiguous nucleotides of SEQ ID NO: 5; a native coding sequence of a *Meligethes* organism comprising SEQ ID NO: 12; the complement of a native coding sequence of a *Meligethes* organism comprising SEQ ID NO: 12; a fragment of at least 15 contiguous nucleotides of a native coding sequence of a *Meligethes* organism comprising SEQ ID NO: 12; and the complement of a fragment of at least 15 contiguous nucleotides of a native coding sequence of a *Meligethes* organism comprising SEQ ID NO: 12.

**2.** The nucleic acid molecule of claim 1, wherein

(a) the nucleotide sequence is selected from the group consisting of SEQ ID NO: 5, SEQ ID NO: 12, and the complements of the foregoing; and/or
(b) the molecule is a vector, wherein preferably the heterologous promoter is functional in a plant cell, and wherein the vector is a plant transformation vector; and/or
(c) the organism is selected from the group consisting ofD. v. *virgifera* LeConte; *D. barberi* Smith and Lawrence; *D. u. howardi; D.* v. *zeae; D. balteata* LeConte; *D. u. tenella; D. u. undecimpunctata* Mannerheim; *D. speciosa* Germar; and *Meligethes aeneus* Fabricius (Pollen Beetle); and/or
(d) further comprising at least one additional polynucleotide operably linked to a heterologous promoter, wherein the additional polynucleotide encodes an RNA molecule, wherein preferably the heterologous promoter is functional in a plant cell, and wherein the molecule is a plant transformation vector; and/or
(e) further comprising a polynucleotide encoding an insecticidal polypeptide from *Bacillus thuringiensis, Alcaligenes* spp., or *Pseudomonas* spp. wherein preferably the insecticidal polypeptide is selected from the group of *B. thuringiensis* insecticidal polypeptides consisting of Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A, and Cyt2C.

**3.** A ribonucleic acid (RNA) molecule encoding the nucleic acid molecule of claim 1, wherein the RNA molecule comprises a polyribonucleotide encoded by the polynucleotide, wherein preferably

(i) the molecule is a double-stranded ribonucleic acid (dsRNA) molecule, preferably

(a) comprising a first, a second, and a third polyribonucleotide, wherein the first polyribonucleotide is encoded by the nucleotide sequence, wherein the third polyribonucleotide is linked to the first polyribonucleotide by the second polyribonucleotide, and wherein the third polyribonucleotide is substantially the reverse complement of the first polyribonucleotide, such that the first and the third polyribonucleotides hybridize when transcribed into a ribonucleic acid to form the dsRNA;
and/or
b) wherein contacting the molecule with a coleopteran pest inhibits the expression of an endogenous nucleic acid molecule that is specifically complementary to the polyribonucleotide, preferably contacting the molecule with the coleopteran pest kills or inhibits the growth and/or feeding of the pest;

and/or
(ii) the RNA molecule is a single-stranded ribonucleic acid molecule of between about 15 and about 30 nucleotides in length.

**4.** A cell comprising the nucleic acid molecule of claim 1, wherein the cell is preferably:

(a) a prokaryotic cell, or
(b) a eukaryotic cell, wherein the eukaryotic cell is preferably a plant cell, wherein the plant cell is preferably a *Zea may, Brassica* sp., or *Poaceae* plant cell.

**5.** A plant comprising the plant cell of claim 3, alternative (b), wherein preferably:

(a) the plant is *Zea mays*, *Brassica* sp., or a plant of the family *Poaceae;* and/or
(b) a cell of the plant comprises a double-stranded ribonucleic acid (dsRNA) molecule encoded by the polynucleotide, wherein preferably the dsRNA molecule inhibits the expression of an endogenous polynucleotide that is specifically complementary to the polyribonucleotide when a coleopteran pest ingests a part of the pla.

**6.** A part of the plant of claim 4, wherein the plant part comprises the plant cell, wherein the plant part is preferably a seed.

**7.** A food product or commodity product produced from the plant of claim 4, wherein the product comprises a detectable amount of the nucleic acid molecule.

**8.** A method for controlling an insect pest population, the method comprising providing an agent comprising a ribonucleic acid (RNA) molecule that functions upon contact with the insect pest to inhibit a biological function within the pest, wherein the RNA is specifically hybridizable with a polynucleotide selected from the group consisting of SEQ ID NOs:91 and 92; the complement of any of SEQ ID NOs: 91 and 92; a fragment of at least 15 contiguous nucleotides of any of SEQ ID NOs: 91 and 92; the complement of a fragment of at least 15 contiguous nucleotides of any of SEQ ID NOs: 91 and 92; a transcript of SEQ ID NO: 5; and the complement of a transcript of SEQ ID NO: 5, wherein preferably (a) the RNA molecule is a double-stranded RNA (dsRNA) molecule;
and/or

(b) providing the agent comprises contacting the insect pest with a sprayable composition comprising the agent;
and/or
(c) providing the agent comprises feeding a plant cell comprising the RNA molecule to the insect pest.

**9.** A method for controlling a

(a) coleopteran pest population, the method comprising:

(i) providing an agent comprising a first and a second polyribonucleotide that functions upon contact with the coleopteran pest to inhibit a biological function within the coleopteran pest, wherein the first polyribonucleotide comprises a nucleotide sequence having from about 90% to about 100% sequence identity to from about 15 to about 30 contiguous nucleotides of a polyribonucleotide selected from the group consisting of SEQ ID NOs: 91 and 92, and wherein the first polyribonucleotide is specifically hybridized to the second polyribonucleotide;
or
(ii) providing in a host plant of a coleopteran pest a plant cell comprising the nucleic acid molecule of claim 1, wherein the polynucleotide is expressed to produce a ribonucleic acid (dsRNA) molecule that functions upon contact with a coleopteran pest belonging to the population to inhibit the expression of a target sequence within the coleopteran pest and results in decreased growth and/or survival of the coleopteran pest or pest population, relative to development of the same pest species on a plant of the same host plant species that does not comprise the polynucleotide; wherein preferably the coleopteran pest population is reduced relative to a population of the same pest species infesting a host plant of the same host plant species lacking a plant cell comprising the nucleic acid molecule;

or
(b) coleopteran pest infestation in a plant, the method comprising providing in the diet of a coleopteran pest infesting the plant a ribonucleic acid (RNA) molecule that is specifically hybridizable with a polyribonucleotide selected from the group consisting of:

SEQ ID NOs: 91 and 92; the complement of any of SEQ ID NOs: 91 and 92; a fragment of at least 15 contiguous nucleotides of any of SEQ ID NOs: 91 and 92; the complement of a fragment of at least 15 contiguous nucleotides of any of NOs: 91 and 92; a transcript of any of SEQ ID NO: 5; the complement of a transcript of any of SEQ ID NO: 5; a fragment of at least 15 contiguous nucleotides of a transcript of SEQ ID NO: 5; and the complement of a fragment of at least 15 contiguous nucleotides of a transcript of SEQ ID NO: 5, wherein preferably

(i) the diet comprises a plant cell comprising a polynucleotide that is transcribed to express the polyribonucleotide; and/or
(ii) the RNA molecule is a double-stranded RNA (dsRNA) molecule.

**10.** A method for improving the yield of a crop, the method comprising:

cultivating in the crop a plant comprising the nucleic acid molecule of claim 1 to allow the expression of the, wherein preferably

(i) the plant is *Zea may, Brassica* sp., or a plant of the family *Poaccae;* and/or
(ii) expression of the polynucleotide produces an RNA molecule that suppresses a target gene in a coleopteran pest that has contacted a portion of the plant, thereby inhibiting the development or growth of the coleopteran pest and loss of yield due to infection by the coleopteran pest.

**11.** A method for producing a transgenic plant cell, the method comprising:

(a) transforming a plant cell with the plant transformation vector of claim 2, alternative (b); culturing the transformed plant cell under conditions sufficient to allow for development of a plant cell culture comprising a plurality of transformed plant cells; selecting for transformed plant cells that have integrated the polynucleotide into their gnomes;
screening the transformed plant cells for expression of a ribonucleic acid (RNA) molecule encoded by the polynucleotide; and
selecting a plant cell that expresses the RNA, wherein preferably the RNA molecule is a double-stranded RNA molecule;
or
(b) transforming a plant cell with a vector comprising a means for providing *prp8*-mediated *Diabrotica* pest protection to a plant;
culturing the transformed plant cell under conditions sufficient to allow for development of a plant cell culture comprising a plurality of transformed plant cells; selecting for transformed plant cells that have integrated the means for providing *prp8*-mediated *Diabrotica* pest protection to a plant into their genomes;
screening the transformed plant cells for expression of a means for inhibiting expression of a *prp8* gene in a *Diabrotica* pest; and
selecting a plant cell that expresses the means for inhibiting expression of a *prp8* gene in a *Diabrotica* pest.

**12.** A method for producing

(a) a coleopteran pest-resistant transgenic plant, the method comprising:

regenerating a transgenic plant from a transgenic plant cell comprising the nucleic acid molecule of claim 1, wherein expression of a ribonucleic acid (RNA) molecule encoded by the polynucleotide is sufficient to modulate the expression of a target gene in the coleopteran pest when it contacts the RNA molecule;

or
(b) a transgenic plant, the method comprising:

regenerating a transgenic plant from the transgenic plant cell produced by the method according to claim 11, alternative (b), wherein plant cells of the plant comprise the means for inhibiting expression of a *prp8* gene in a *Diabrotica* pest, wherein preferably expression of the means for inhibiting expression *of a prp8* gene in a *Diabrotica* pest is sufficient to modulate the expression of a target *prp8* gene in a *Diabrotica* pest that infests the transgenic plant;

or

(c) a transgenic plant, the method comprising: regenerating a transgenic plant from the transgenic plant cell produced by the method according to claim 10, alternative (b), wherein plant cells of the plant comprise the means for inhibiting expression of a *prp8* gene in a *Meligethes* pest, wherein preferably expression of the means for inhibiting expression of a *prp8* gene in a *Meligethes* pest is sufficient to modulate the expression of a target *prp8* gene in a *Meligethes* pest that infests the transgenic plant.

**13.** A plant comprising means for inhibiting expression of a *prp8* gene in a *Diabrotica* pest or *Meligethes* pest.

**14.** The plant cell of claim 3, wherein the cell comprises a polynucleotide encoding an insecticidal polypeptide from *Bacillus thuringiensis, Alcaligenes* spp., or *Pseudomonas* spp., wherein preferably the insecticidal polypeptide is selected from the group *of B. thuringiensis* insecticidal polypeptides consisting of CrylB, Cry1I, Cry3, Cry7A, Cry8, Cry9D, Cry14, Cry18, Cry22, Cry23, Cry34, Cry35, Cry36, Cry37, Cry43, Cry55, Cyt1A, and Cyt2C.

**15.** The plant of claim 4, wherein a cell of the plant comprises a polynucleotide encoding an insecticidal polypeptide from *Bacillus thuringiensis, Alcaligenes* spp., or *Pseudomonas* spp., wherein preferably the insecticidal polypeptide is selected from the group of *B. thuringiensis* insecticidal polypeptides consisting of Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A, and Cyt2C.

**16.** The method according to claim 9, alternative (a), wherein the plant cell comprises a polynucleotide encoding an insecticidal polypeptide from *Bacillus thuringiensis, Alcaligenes* spp., or *Pseudomonas* spp., wherein preferably the insecticidal polypeptide is selected from the group of *B. thuringiensis* insecticidal polypeptides consisting of Cry1B, Cry1I, Cry2A, Cry3, Cry7A, Cry8, Cry9D, Cry14, Cry18, Cry22, Cry23, Cry34, Cry35, Cry36, Cry37, Cry43, Cry55, Cyt1A, and Cyt2C.

FIG. 1

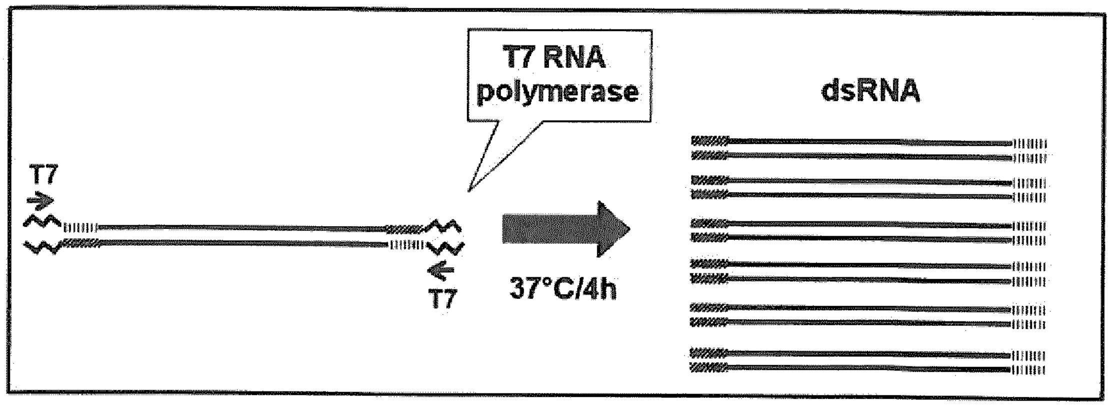

FIG. 2

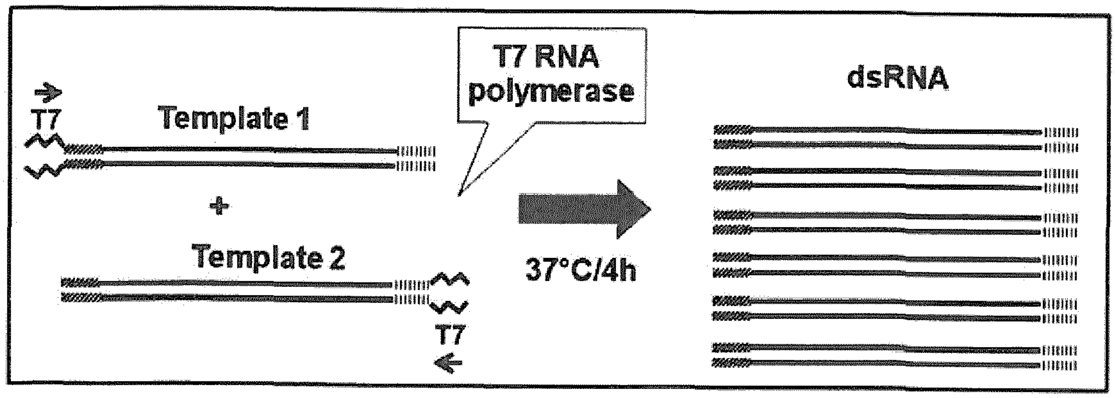

Europäisches Patentamt
European Patent Office
Office européen des brevets

# EUROPEAN SEARCH REPORT

Application Number
EP 16 20 7553

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/118762 A1 (MONSANTO TECHNOLOGY LLC [US]) 28 July 2016 (2016-07-28) * paragraph [00228]; claims 1-3,19; sequence 94 * | 1-16 | INV. C07K14/435 A01N57/18 C12N15/82 |
| X | WO 2015/010026 A2 (MONSANTO TECHNOLOGY LLC [US]) 22 January 2015 (2015-01-22) * claim 1; sequence 94 * | 1-16 | |
| X | US 2016/230186 A1 (BAUM JAMES A [US] ET AL) 11 August 2016 (2016-08-11) * paragraph [0179]; claim 1; sequence 7269 * | 1-13 | |
| E | WO 2017/011771 A1 (DOW AGROSCIENCES LLC [US]; FRAUNHOFER-GESELLSCHAFT ZUR FÖRDERUNG DER A) 19 January 2017 (2017-01-19) * claims 1,8,51-55 * | 1-16 | |

TECHNICAL FIELDS SEARCHED (IPC)

C07K
A01N
C12N

The present search report has been drawn up for all claims

1

| Place of search | Date of completion of the search | Examiner |
|---|---|---|
| Munich | 10 May 2017 | Obel, Nicolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 20 7553

This annex lists the patent family members relating to the patent documents cited in the above-mentioned European search report.
The members are as contained in the European Patent Office EDP file on
The European Patent Office is in no way liable for these particulars which are merely given for the purpose of information.

10-05-2017

| Patent document cited in search report | Publication date | Patent family member(s) | Publication date |
|---|---|---|---|
| WO 2016118762 A1 | 28-07-2016 | NONE | |
| WO 2015010026 A2 | 22-01-2015 | CA 2918387 A1 | 22-01-2015 |
| | | CN 105980567 A | 28-09-2016 |
| | | EP 3030663 A2 | 15-06-2016 |
| | | JP 2016532440 A | 20-10-2016 |
| | | US 2015143580 A1 | 21-05-2015 |
| | | US 2015203867 A1 | 23-07-2015 |
| | | WO 2015010026 A2 | 22-01-2015 |
| US 2016230186 A1 | 11-08-2016 | NONE | |
| WO 2017011771 A1 | 19-01-2017 | US 2017016024 A1 | 19-01-2017 |
| | | WO 2017011771 A1 | 19-01-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7612194 B
- US 20070050860 A
- US 20100192265 A
- US 20110154545 A
- US 7943819 B
- US 2013040173 A
- WO 2013169923 A
- US 20140007292 A1
- WO 9630530 A
- US 4980460 A
- US 4725677 A
- US 4415732 A
- US 4458066 A
- US 4973679 A
- WO 9732016 A
- US 5593874 A
- US 5698425 A
- US 5712135 A
- US 5789214 A
- US 5804693 A
- WO 06073727 A
- US 20060200878 A1
- US 20020048814 A
- US 20030018993 A
- WO 9401550 A
- WO 9805770 A
- US 6437217 B
- US 5641876 A
- US 6426446 B
- US 6429362 B
- US 6232526 B
- US 6177611 B
- US 5322938 A
- US 5352605 A
- US 5359142 A
- US 5530196 A
- US 6433252 B
- US 6429357 B
- US 6294714 B
- US 6140078 A
- US 6252138 B
- US 6175060 B
- US 6388170 B
- US 6635806 B
- US 2009757089 A
- US 6051753 A
- US 5378619 A
- US 5850019 A
- US 6677503 B
- US 5110732 A
- US 5459252 A
- US 5837848 A
- US 5362865 A
- US 5659122 A
- US 5550318 A
- US 5633435 A
- US 5780708 A
- US 6118047 A
- US 5508184 A
- US 5384253 A
- US 5302523 A
- US 5464765 A
- US 5563055 A
- US 5591616 A
- US 5693512 A
- US 5824877 A
- US 5981840 A
- US 6384301 B
- US 5015580 A
- US 5538880 A
- US 6160208 A
- US 6399861 B
- US 6403865 B
- US 7060876 B
- WO 9506722 A
- US 4536475 A
- US 4693977 A
- US 4886937 A
- US 5501967 A
- EP 0122791 A
- EP 0120516 A
- US 20120174258 A
- US 20120174259 A
- US 20120174260 A
- US 20120198586 A
- US 20130091600 A
- US 20130091601 A
- US 20130097730 A
- US 577811 A
- US 62133214 A
- US 577854 A
- US 62133202 A
- US 62133210 A
- US 62168613 A
- US 62168606 A
- US 62095487 A
- US 705807 A

- US 62063199 A
- US 62063203 A
- US 62063192 A
- US 62063216 A
- US 20140033361 A
- WO 2015038734 A
- US 8530440 B
- US 5107065 A
- US 5759829 A
- US 5283184 A
- US 5231020 A
- WO 2011025860 A
- US 20070124836 A
- US 20090306189 A
- US 2013192256 A
- US 5510474 A
- US 6699984 B
- US 7838733 B
- US 7179902 B
- US 8304604 B
- WO 2012016222 A2

**Non-patent literature cited in the description**


- **ELLSBURY et al.** *Environ. Entomol.,* 2005, vol. 34, 627-34
- **FIRE et al.** *Nature,* 1998, vol. 391, 806-11
- **MARTINEZ et al.** *Cell,* 2002, vol. 110, 563-74
- **MCMANUS ; SHARP.** *Nature Rev. Genetics,* 2002, vol. 3, 737-47
- **LEE et al.** *Cell,* 2004, vol. 117 (1), 69-81
- **BOLOGNESI et al.** *PLOS ONE,* 2012, vol. 7 (10), e47534
- **BAUM et al.** *Nature Biotechnology,* 2007, vol. 25, 1322-1326
- **BAUM et al.** *Nat. Biotechnol.,* 2007, vol. 25 (11), 322-6
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. U.S.A.,* 1988, vol. 85, 2444
- **HIGGINS ; SHARP.** *Gene,* 1988, vol. 73, 237-44
- **HIGGINS ; SHARP.** *CABIOS,* 1989, vol. 5, 151-3
- **CORPET et al.** *Nucleic Acids Res.,* 1988, vol. 16, 10881-90
- **HUANG et al.** *Comp. Appl. Biosci.,* 1992, vol. 8, 155-65
- **PEARSON et al.** *Methods Mol. Biol.,* 1994, vol. 24, 307-31
- **TATIANA et al.** *FEMS Microbiol. Lett.,* 1999, vol. 174, 247-50
- **ALTSCHUL et al.** *J. Mol. Biol.,* 1990, vol. 215, 403-10
- Molecular Cloning: A Laboratory Manual. Cold Spring Harbor Laboratory Press, 1989, vol. 1-3
- Nucleic Acid Hybridization. IRL Press, 1985
- Overview of principles of hybridization and the strategy of nucleic acid probe assays. **TIJSSEN.** Laboratory Techniques in Biochemistry and Molecular Biology-Hybridization with Nucleic Acid Probes. Elsevier, 1993
- Current Protocols in Molecular Biology. Greene Publishing and Wiley-Interscience, 1995
- **WARD et al.** *Plant Mol. Biol.,* 1993, vol. 22, 361-366
- **SCHENA et al.** *Proc. Natl. Acad. Sci. USA,* 1991, vol. 88, 0421
- **FROMM et al.** *Nature,* 1986, vol. 319, 791-3
- **FELGNER et al.** *Proc. Natl. Acad. Sci. USA,* 1987, vol. 84, 7413-7
- **MUELLER et al.** *Cell,* 1978, vol. 15, 579-85
- **FRALEY et al.** *Proc. Natl. Acad. Sci. USA,* 1983, vol. 80, 4803-7
- **KLEIN et al.** *Nature,* 1987, vol. 327, 70
- Lewin's Genes X. Jones & Bartlett Publishers, 2009
- The Encyclopedia of Molecular Biology. Blackwell Science Ltd, 1994
- Molecular Biology and Biotechnology: A Comprehensive Desk Reference. VCH Publishers, Inc, 1995
- **ELBASHIR et al.** *Nature,* 2001, vol. 411, 494-8
- **HAMILTON ; BAULCOMBE.** *Science,* 1999, vol. 286 (5441), 950-2
- **OZAKI et al.** *Nucleic Acids Research,* 1992, vol. 20, 5205-5214
- **AGRAWAL et al.** *Nucleic Acids Research,* 1990, vol. 18, 5419-5423
- **BEAUCAGE et al.** *Tetrahedron,* 1992, vol. 48, 2223-2311
- **EBERT et al.** *Proc. Natl. Acad. Sci. USA,* 1987, vol. 84 (16), 5745-9
- **LAWTON et al.** *Plant Mol. Biol.,* 1987, vol. 9, 315-24
- **ODELL et al.** *Nature,* 1985, vol. 313, 810-2
- **WALKER et al.** *Proc. Natl. Acad. Sci. USA,* 1987, vol. 84 (19), 6624-8
- **YANG ; RUSSELL.** *Proc. Natl. Acad. Sci. USA,* 1990, vol. 87, 4144-8
- **CHANDLER et al.** *Plant Cell,* 1989, vol. 1, 1175-83
- **DEPICKER et al.** *J. Mol. Appl. Genet.,* 1982, vol. 1, 561-73
- **BEVAN et al.** *Nature,* 1983, vol. 304, 184-7

- **OPPERMAN et al.** *Science,* 1994, vol. 263, 221-3
- **HIREL et al.** *Plant Mol. Biol.,* 1992, vol. 20, 207-18
- **TURNER ; FOSTER.** *Molecular Biotech.,* 1995, vol. 3 (3), 225-36
- **CARRINGTON ; FREED.** *J. Virol.,* 1990, vol. 64, 1590-7
- **INGELBRECHT et al.** *Plant Cell,* 1989, vol. 1, 671-80
- **CORUZZI et al.** *EMBO J.,* 1984, vol. 3, 1671-9
- **JEFFERSON et al.** *Plant Mol. Biol. Rep.,* 1987, vol. 5, 387-405
- Molecular cloning of the maize R-nj allele by transposon tagging with Ac. **DELLAPORTA et al.** Stadler Genetics Symposium. Plenum, 1988, 263-82
- **SUTCLIFFE et al.** *Proc. Natl. Acad. Sci. USA,* 1978, vol. 75, 3737-41
- **OW et al.** *Science,* 1986, vol. 234, 856-9
- **ZUKOWSKI et al.** *Gene,* 1983, vol. 46 (2-3), 247-55
- **IKATU et al.** *Bio/Technol.,* 1990, vol. 8, 241-2
- **KATZ et al.** *J. Gen. Microbiol.,* 1983, vol. 129, 2703-14
- **POTRYKUS et al.** *Mol. Gen. Genet.,* 1985, vol. 199, 183-8
- **ESTRELLA et al.** *Nature,* 1983, vol. 303, 209-13
- **KLEE et al.** *Bio/Technol.,* 1985, vol. 3, 637-42
- **RIOS, G. et al.** *Plant J.,* 2002, vol. 32, 243-53
- **SHAGIN et al.** *Mol. Biol. Evol.,* 2004, vol. 21 (5), 841-50
- **VANCANNEYT et al.** *Mol. Gen. Genet.,* 1990, vol. 220 (2), 245-50
- **WRIGHT et al.** *Proc. Natl. Acad. Sci. U.S.A.,* 2010, vol. 107, 20240-5
- **SCHENK et al.** *Plant Molec. Biol.,* 1999, vol. 39, 1221-30
- **WATSON et al.** *J. Bacteriol.,* 1975, vol. 123, 255-264
- Genetic Transformation Using Maize Immature Zygotic Embryos. **FRAME et al.** IN Plant Embryo Culture Methods and Protocols: Methods in Molecular Biology. Springer Science and Business Media, LLC, 2011, 327-341
- **HALLAUER et al.** *Crop Science,* 1997, vol. 37, 1405-1406
- **BAUM et al.** *Nat. Biotechnol.,* 2007, vol. 25 (11), 1322-1326
- **OLESON et al.** *J. Econ. Entomol.,* 2005, vol. 98, 1-8
- **PETOLINO ; ARNOLD.** *Methods Mol. Biol.,* 2009, vol. 526, 59-67
- **SCHULZ et al.** *Bioinformatics,* 2012, vol. 28, 1086-92
- **ZERBINO ; BIRNEY.** *Genome Res.,* 2008, vol. 18, 821-9

SEQUENCE LISTING

<110> Dow AgroSciences LLC
Fraunhofer-Gesellschaft zur Förderung der angewandten Forschung e.V.

<120> PRE-MRNA PROCESSING FACTOR 8 (PRP8) NUCLEIC ACID MOLECULES TO CONTROL COLEOPTERAN PESTS

<130> 2971-P13049.1US (79511-US-NP)

<160> 92

<170> PatentIn version 3.5

<210> 1
<211> 3884
<212> DNA
<213> Diabrotica virgifera

<400> 1
caagttacta gtgaatctgt tctatttctt ctccgtgctc caccctcttt agtccgaaaa 60

aatgcctgcg cctgctgctg ctgaaaatgg agcgcccagg acggaactgc aagagctcca 120

gctcaaagct gggcaagtta cagatgagtc cctggaaagc acaaggcgta tgttggctct 180

ctgcgaagag agtaccgatg ctggcacgaa gacattggag atggtccacc atcaaggcga 240

acaattggac cggatcgagg atggaatgga ccagatcaac accgatatgc gagaggctga 300

aaagaatttg actggaatgg aaaaatgctg tggcctttgt gtgttaccat gtcaaaaggg 360

ctcatcgttc aaagaagacg aaggaacgtg gaagggcaac gacgacggaa aagtcgtcaa 420

caaccaaccc caacgaatga tggacgatcg gaacggaatg ggccctcaag gcggatacat 480

cggcaggatc acgaacgacg cgcgagagga cgaaatggaa gaaaacgtcg gccaagtcaa 540

caccatgatc ggtaatctgc gtaacatggc tatcgatatg ggttcggagt tggaaaatca 600

aaataggcaa atcgatcgta tcaatctcaa gggtgaatcc aacgcgacga ggatagaggt 660

ggccaaccag cgggcacatg accttctcaa gtagacacaa cacacaaaaa cactgaaaag 720

tttttacttt ctatcatttt ttgcgattcc aactcgttcc tactgggtac ttgaaacaat 780

taaatatcta ttgctttttt agctacttaa ttagtcagtg ttcaataata tataaatgct 840

gtttcgtaac taacaaaact agaataatcg tcgtggtgac ataatcatga aaagtttata 900

aaacatctat aatttggtcc tttcacgtca ttattttcaa ttttacgacg ttttagaatg 960

gttcctaaga cggttaacgt tttaataaca aacgtatact ctgtttcatt aaacaatcgt 1020

ttctgtagct ttaattgtta taaattagcg atgatgttac aggtaccaaa aggcagtgtt 1080

gccaattatt tatcagtctg tgtattagaa gtaatgaatc ataagaatcc ggctcgaagg 1140

accaaatgct tatgtaggaa atattcttga agaaaatggt cttgcttaga tctgcccatt 1200

tcgtgggaac gaacctatat ttacgtgtag gccgatcgag tgaaagttac aaaattgcgt 1260

```
tcgcatacgt ttttaagagg cgcaataaat cagataaagt atcaagaggt tgtgtgtcaa     1320
aatagcagta ttaccaatta ctaattagtc tttgtgctag tatttgagtt ggcatacact     1380
gttgtgtgga aaactgaaca atacgctatt catttgaaga actgcttcaa ataatagcaa     1440
ataatagaag tatagatcgg agaaacaccg taaaaaatcg ggcaaaaagt tgctttatat     1500
aaaaattcaa tgaacgagga agtttatcat taagtggact ttggaagaga actggaagga     1560
aactgttcag tcagtcacga agttatatgt gataataaat gtttaatctt taaatttgaa     1620
aacaaaaccg ccaatttaat gatagagatc ataaaggatc ttaaatacta cgaaaaaaat     1680
tgtcagttct atcgagtaag tttcagtttg atttcatttg ccttatttat ggccctattt     1740
ggaaatgtaa gcgttaatgc ccattgccca ttgcccatgc cattttcatg gtaaatacta     1800
tttaaattag tagcattgta gtttatgttg gtatattgta tgaaaaaatc aaatttatta     1860
ctattttata actaaaaagc tatagaaacg acgaatatta ttatcagagg taaatgcaac     1920
gattcaaaga aggcaaaaag ttaggttaag atacctgtca ctgatattat aaccagaatc     1980
tcttggtcgt ttgttaatta tacttaaatc attgttccac gttgttaaag gcacataaag     2040
agtaagtatc ttcgaccaaa gttatcagaa ctcttctatc tttcataata tatcttagca     2100
ataacagctt caacgtgaaa ggatcaacta atattccaca tattgtgtac ataaaaaaca     2160
ctgattattt tacaataata ttgagctctc actgcaacag ttgttgttct ttggtttgaa     2220
ttgttttgta gaattttcga acacgttcac tgccagtgtg tatcgaaaac gcacttgaaa     2280
actcgggtaa caattatctg atgctaggtg attggttatc acataatcag cagtgaacat     2340
gagagttgtt gataaaaaaa ccaaataaaa ataaatgcct caaatctttt tttttttcaa     2400
gagaacaaat ttaaaactaa acttcagaaa ctctaccttc ttgttcggtg acgaaattta     2460
cgcatataat ctgccttttt tccagtattg tcgccaatat taaggcagtc gtcttatcat     2520
acaagttttt ttggtctttt tggcgttacc ttcgacttag tgatgataat aagctattca     2580
aatttaaaaa cagattttag tcactgggtg ctagatcggg acatttcaac ggttttgatg     2640
atttctttat aatcctaaat tttgaatttt tccagttgca ctgagacatt agatactata     2700
catctttttg tttgtacttt tacattaaac aatacttact acagattgat tatgtcacca     2760
tgacagtact tgaacaaacg ctattcaatt tttatattta gaaagagtat aaagttgagg     2820
gaccaggttg aaaggtacag tgttgaatag tataacagcc gcatgattga attttatctg     2880
taaatattac tttaattaat tatggcgcta gatttttgtt ctgtttgttt tctattaata     2940
aatatttaaa ttttataccg atgtttaatt tggtaggtct aattctagct gtagaattaa     3000
aaagtttaag tgggtaaata cgagaaccga gtgcgttaac gtagaacttg aaccatatct     3060
atccaaagca ctgtatttta gtgtgtatat ccctaacaat tagattacta gttttttca     3120
```

taaaacgcaa cttataccga acaaaaatta ttacatgatg tcatttcagc ctaaagcgag 3180

taagacagta atgccaactg tcatgtgtca aatgtcataa aagtcatgta taaaagttca 3240

gccgaacaat ttccaaatat gaacagttgt tggcgaccat gaaaaagatc tgtcagagtt 3300

gaatttatgt cgaaaacacg acattttaga atttttgtag gtgtttttat tctgtagaag 3360

ttgcccgttc atcacatata tacattatca taatttaatc tataccgtaa cgaatacatc 3420

ataacgcttc aggtatttta ttaaaaatct cttgaatgat gacaatatat gtaacagatt 3480

gagtggtaaa tgtctttttt ttgtaatttt tttggtaggt aatcgttttt tattcacgaa 3540

actaagtatg aaaaagctaa gactaagtgg aataacattt ttaaaatatg atttacaaat 3600

atattttgtg tatggctttt catcagtgta attaaatcgt tttaataaat atagttggtt 3660

tagacgttgt caaacataaa gacgtttgac aatgtctaca cgaaccattc tttttgaggt 3720

tacctctgtg cctgatttgt caaattgtca tctgtgcctt gccactcttg gcagtaaatg 3780

tatatccgta cccagtactg tcaatttact tcgttgtttg ttctgttctt tttgtaataa 3840

gttggttcat taataggaca tttcaacgat tctcatttgt ttcg 3884

<210> 2
<211> 2364
<212> PRT
<213> Diabrotica virgifera

<400> 2

Met Ser Leu Pro Pro Tyr Leu Leu Gly Pro Asn Pro Trp Ala Thr Met
1 5 10 15

Met Ala Gln Gln His Leu Ala Ala Ala His Ala Gln Ala Gln Ala Ala
20 25 30

Ala Ala Gln Ala His Ala His Ala Leu Gln Gln Gln Met Pro Pro Pro
35 40 45

His Pro Lys Pro Asp Ile Ile Thr Glu Asp Lys Leu Gln Glu Lys Ala
50 55 60

Leu Lys Trp His Gln Leu Gln Ser Lys Arg Phe Ala Asp Lys Arg Lys
65 70 75 80

Leu Gly Phe Val Glu Ala Gln Lys Glu Asp Met Pro Pro Glu His Ile
85 90 95

Arg Lys Ile Ile Arg Asp His Gly Asp Met Ser Ser Arg Lys Tyr Arg
100 105 110

His Asp Lys Arg Val Tyr Leu Gly Ala Leu Lys Tyr Met Pro His Ala

```
        115                 120                 125

Val Met Lys Leu Leu Glu Asn Met Pro Met Pro Trp Glu Gln Ile Arg
    130                 135                 140

Asp Val Lys Val Leu Tyr His Ile Thr Gly Ala Ile Thr Phe Val Asn
145                 150                 155                 160

Glu Ile Pro Trp Val Cys Glu Pro Ile Tyr Ile Ala Gln Trp Gly Thr
                165                 170                 175

Met Trp Ile Met Met Arg Arg Glu Lys Arg Asp Arg Arg His Phe Lys
            180                 185                 190

Arg Met Arg Phe Pro Pro Phe Asp Asp Glu Glu Pro Pro Leu Asp Tyr
        195                 200                 205

Ala Asp Asn Val Leu Asp Val Glu Pro Leu Glu Ala Ile Gln Ile Glu
    210                 215                 220

Leu Asp Ala Asp Glu Asp Ser Ala Ile Ala Lys Trp Phe Tyr Asp His
225                 230                 235                 240

Lys Pro Leu Val Gly Thr Lys Tyr Val Asn Gly Leu Thr Tyr Arg Lys
                245                 250                 255

Trp Asn Leu Ser Leu Pro Ile Met Ala Thr Leu Tyr Arg Leu Ala Asn
            260                 265                 270

Gln Leu Leu Thr Asp Leu Val Asp Asp Asn Tyr Phe Tyr Leu Phe Asp
        275                 280                 285

Thr Lys Ser Phe Phe Thr Ala Lys Ala Leu Asn Met Ala Ile Pro Gly
    290                 295                 300

Gly Pro Lys Phe Glu Pro Leu Ile Lys Asp Met Asn Pro Ala Asp Glu
305                 310                 315                 320

Asp Trp Asn Glu Phe Asn Asp Ile Asn Lys Ile Ile Ile Arg Gln Pro
                325                 330                 335

Ile Arg Thr Glu Tyr Arg Ile Ala Phe Pro Tyr Leu Tyr Asn Asn Met
            340                 345                 350

Pro His Phe Val His Leu Ser Trp Tyr His Ala Pro Asn Val Val Tyr
        355                 360                 365
```

```
Ile Lys Thr Glu Asp Pro Asp Leu Pro Ala Phe Tyr Phe Asp Pro Leu
    370                 375                 380

Ile Asn Pro Ile Ser His Arg His Ala Val Lys Ser Leu Glu Pro Leu
385                 390                 395                 400

Pro Asp Asp Asp Glu Glu Tyr Ile Leu Pro Glu Phe Val Gln Pro Phe
                405                 410                 415

Leu Gln Glu Thr Pro Leu Tyr Thr Asp Asn Thr Ala Asn Gly Ile Ser
            420                 425                 430

Leu Leu Trp Ala Pro Arg Pro Phe Asn Met Arg Ser Gly Arg Cys Arg
        435                 440                 445

Arg Ala Ile Asp Val Pro Leu Val Lys Pro Trp Tyr Met Glu His Cys
    450                 455                 460

Pro Pro Gly Gln Pro Val Lys Val Arg Val Ser Tyr Gln Lys Leu Leu
465                 470                 475                 480

Lys Tyr Tyr Val Leu Asn Ala Leu Lys His Arg Pro Pro Lys Ala Gln
                485                 490                 495

Lys Lys Arg Tyr Leu Phe Arg Ser Phe Lys Ser Thr Lys Phe Phe Gln
            500                 505                 510

Thr Thr Thr Leu Asp Trp Val Glu Ala Gly Leu Gln Val Cys Arg Gln
        515                 520                 525

Gly Tyr Asn Met Leu Asn Leu Leu Ile His Arg Lys Asn Leu Asn Tyr
    530                 535                 540

Leu His Leu Asp Tyr Asn Phe Asn Leu Lys Pro Val Lys Thr Leu Thr
545                 550                 555                 560

Thr Lys Glu Arg Lys Lys Ser Arg Phe Gly Asn Ala Phe His Leu Cys
                565                 570                 575

Arg Glu Ile Leu Arg Leu Thr Lys Leu Ile Ile Asp Ser His Val Gln
            580                 585                 590

Tyr Arg Leu Asn Asn Val Asp Ala Phe Gln Leu Ala Asp Gly Leu Gln
        595                 600                 605

Tyr Ile Phe Ala His Val Gly Gln Leu Thr Gly Met Tyr Arg Tyr Lys
    610                 615                 620
```

```
Tyr Lys Leu Met Arg Gln Ile Arg Met Cys Lys Asp Leu Lys His Leu
625                 630                 635                 640

Ile Tyr Tyr Arg Phe Asn Thr Gly Pro Val Gly Lys Gly Pro Gly Cys
                645                 650                 655

Gly Phe Trp Ala Pro Gly Trp Arg Val Trp Leu Phe Phe Met Arg Gly
            660                 665                 670

Ile Thr Pro Leu Leu Glu Arg Trp Leu Gly Asn Leu Leu Ser Arg Gln
        675                 680                 685

Phe Glu Gly Arg His Ser Lys Gly Val Ala Lys Thr Val Thr Lys Gln
    690                 695                 700

Arg Val Glu Ser His Phe Asp Leu Glu Leu Arg Ala Ser Val Met His
705                 710                 715                 720

Asp Ile Val Asp Met Met Pro Glu Gly Ile Lys Gln Asn Lys Ala Arg
                725                 730                 735

Thr Ile Leu Gln His Leu Ser Glu Ala Trp Arg Cys Trp Lys Ala Asn
            740                 745                 750

Ile Pro Trp Lys Val Pro Gly Leu Pro Ile Pro Ile Glu Asn Met Ile
        755                 760                 765

Leu Arg Tyr Val Lys Met Lys Ala Asp Trp Trp Thr Asn Thr Ala His
    770                 775                 780

Tyr Asn Arg Glu Arg Ile Arg Arg Gly Ala Thr Val Asp Lys Thr Val
785                 790                 795                 800

Cys Lys Lys Asn Leu Gly Arg Leu Thr Arg Leu Tyr Leu Lys Ala Glu
                805                 810                 815

Gln Glu Arg Gln His Asn Tyr Leu Lys Asp Gly Pro Tyr Ile Ser Pro
            820                 825                 830

Glu Glu Ala Val Ala Ile Tyr Thr Thr Thr Val His Trp Leu Glu Ser
        835                 840                 845

Arg Arg Phe Ala Pro Ile Pro Phe Pro Pro Leu Ser Tyr Lys His Asp
    850                 855                 860

Thr Lys Leu Leu Ile Leu Ala Leu Glu Arg Leu Lys Glu Ala Tyr Ser
865                 870                 875                 880
```

```
Val Lys Ser Arg Leu Asn Gln Ser Gln Arg Glu Glu Leu Gly Leu Ile
                885                 890                 895

Glu Gln Ala Tyr Asp Asn Pro His Glu Ala Leu Ser Arg Ile Lys Arg
            900                 905                 910

His Leu Leu Thr Gln Arg Ala Phe Lys Glu Val Gly Ile Glu Phe Met
        915                 920                 925

Asp Leu Tyr Ser His Leu Ile Pro Val Tyr Asp Val Glu Pro Leu Glu
    930                 935                 940

Lys Ile Thr Asp Ala Tyr Leu Asp Gln Tyr Leu Trp Tyr Glu Ala Asp
945                 950                 955                 960

Lys Arg Arg Leu Phe Pro Pro Trp Ile Lys Pro Ala Asp Thr Glu Pro
                965                 970                 975

Pro Pro Leu Leu Val Tyr Lys Trp Cys Gln Gly Ile Asn Asn Leu Gln
            980                 985                 990

Asp Val Trp Asp Val Asn Glu Gly  Glu Cys Asn Val Leu  Leu Glu Ser
        995                 1000                 1005

Lys Phe  Glu Lys Leu Tyr Glu  Lys Ile Asp Leu Thr  Leu Leu Asn
    1010                 1015                 1020

Arg Leu  Leu Arg Leu Ile Val  Asp His Asn Ile Ala  Asp Tyr Met
    1025                 1030                 1035

Thr Ala  Lys Asn Asn Val Val  Ile Asn Tyr Lys Asp  Met Asn His
    1040                 1045                 1050

Thr Asn  Ser Tyr Gly Ile Ile  Arg Gly Leu Gln Phe  Ala Ser Phe
    1055                 1060                 1065

Ile Thr  Gln Tyr Tyr Gly Leu  Val Leu Asp Leu Leu  Val Leu Gly
    1070                 1075                 1080

Leu Gln  Arg Ala Ser Glu Met  Ala Gly Pro Pro Gln  Met Pro Asn
    1085                 1090                 1095

Asp Phe  Leu Thr Phe Gln Asp  Val Gln Ser Glu Thr  Cys His Pro
    1100                 1105                 1110

Ile Arg  Leu Tyr Cys Arg Tyr  Val Asp Arg Ile His  Met Phe Phe
```

1115 1120 1125

Arg Phe Ser Ala Glu Glu Ala Lys Asp Leu Ile Gln Arg Tyr Leu
1130 1135 1140

Thr Glu His Pro Asp Pro Asn Asn Glu Asn Ile Val Gly Tyr Asn
1145 1150 1155

Asn Lys Lys Cys Trp Pro Arg Asp Ala Arg Met Arg Leu Met Lys
1160 1165 1170

His Asp Val Asn Leu Gly Arg Ala Val Phe Trp Asp Ile Lys Asn
1175 1180 1185

Arg Leu Pro Arg Ser Val Thr Thr Ile Gln Trp Glu Asn Ser Phe
1190 1195 1200

Val Ser Val Tyr Ser Lys Asp Asn Pro Asn Leu Leu Phe Asn Met
1205 1210 1215

Ser Gly Phe Glu Cys Arg Ile Leu Pro Lys Cys Arg Thr Gln His
1220 1225 1230

Glu Glu Phe Thr His Arg Asp Gly Val Trp Asn Leu Gln His Glu
1235 1240 1245

Gly Ser Lys Glu Arg Thr Ala Gln Cys Phe Leu Arg Val Asp Asp
1250 1255 1260

Glu Ser Met Ser Arg Phe His Asn Arg Val Arg Gln Ile Leu Met
1265 1270 1275

Ala Ser Gly Ser Thr Thr Phe Thr Lys Ile Val Asn Lys Trp Asn
1280 1285 1290

Thr Ala Leu Ile Gly Leu Met Thr Tyr Phe Arg Glu Ala Val Val
1295 1300 1305

Asn Thr Gln Glu Leu Leu Asp Leu Leu Val Lys Cys Glu Asn Lys
1310 1315 1320

Ile Gln Thr Arg Ile Lys Ile Gly Leu Asn Ser Lys Met Pro Ser
1325 1330 1335

Arg Phe Pro Pro Val Val Phe Tyr Thr Pro Lys Glu Leu Gly Gly
1340 1345 1350

```
Leu Gly  Met Leu Ser Met Gly  His Val Leu Ile Pro  Gln Ser Asp
    1355                 1360                 1365

Leu Arg  Trp Ser Lys Gln Thr  Asp Val Gly Ile Thr  His Phe Arg
    1370                 1375                 1380

Ser Gly  Ile Ser His Asp Glu  Asp Gln Leu Ile Pro  Asn Leu Tyr
    1385                 1390                 1395

Arg Tyr  Ile Gln Pro Trp Glu  Ser Glu Phe Ile Asp  Ser Gln Arg
    1400                 1405                 1410

Val Trp  Ala Glu Tyr Ala Leu  Lys Arg Gln Glu Ala  Asn Ala Gln
    1415                 1420                 1425

Asn Arg  Arg Leu Thr Leu Glu  Asp Leu Glu Asp Ser  Trp Asp Arg
    1430                 1435                 1440

Gly Ile  Pro Arg Ile Asn Thr  Leu Phe Gln Lys Asp  Arg His Thr
    1445                 1450                 1455

Leu Ala  Tyr Asp Lys Gly Trp  Arg Ile Arg Thr Glu  Phe Lys Gln
    1460                 1465                 1470

Tyr Gln  Val Leu Lys Gln Asn  Pro Phe Trp Trp Thr  His Gln Arg
    1475                 1480                 1485

His Asp  Gly Lys Leu Trp Asn  Leu Asn Asn Tyr Arg  Thr Asp Met
    1490                 1495                 1500

Ile Gln  Ala Leu Gly Gly Val  Glu Gly Ile Leu Glu  His Thr Leu
    1505                 1510                 1515

Phe Lys  Gly Thr Tyr Phe Pro  Thr Trp Glu Gly Leu  Phe Trp Glu
    1520                 1525                 1530

Lys Ala  Ser Gly Phe Glu Glu  Ser Met Lys Tyr Lys  Lys Leu Thr
    1535                 1540                 1545

Asn Ala  Gln Arg Ser Gly Leu  Asn Gln Ile Pro Asn  Arg Arg Phe
    1550                 1555                 1560

Thr Leu  Trp Trp Ser Pro Thr  Ile Asn Arg Ala Asn  Val Tyr Val
    1565                 1570                 1575

Gly Phe  Gln Val Gln Leu Asp  Leu Thr Gly Ile Phe  Met His Gly
    1580                 1585                 1590
```

```
Lys Ile  Pro Thr Leu Lys Ile  Ser Leu Ile Gln Ile  Phe Arg Ala
    1595                 1600                 1605

His Leu  Trp Gln Lys Val His  Glu Ser Ile Val Met  Asp Leu Cys
    1610                 1615                 1620

Gln Val  Phe Asp Gln Glu Leu  Asp Ala Leu Glu Ile  Glu Thr Val
    1625                 1630                 1635

Gln Lys  Glu Thr Ile His Pro  Arg Lys Ser Tyr Lys  Met Asn Ser
    1640                 1645                 1650

Ser Cys  Ala Asp Ile Leu Leu  Phe Ser Ala Tyr Lys  Trp Asn Val
    1655                 1660                 1665

Ser Arg  Pro Ser Leu Leu Ala  Asp Thr Lys Asp Thr  Met Asp Asn
    1670                 1675                 1680

Thr Thr  Thr Gln Lys Tyr Trp  Ile Asp Val Gln Leu  Arg Trp Gly
    1685                 1690                 1695

Asp Tyr  Asp Ser His Asp Val  Glu Arg Tyr Ala Arg  Ala Lys Phe
    1700                 1705                 1710

Leu Asp  Tyr Thr Thr Asp Asn  Met Ser Ile Tyr Pro  Ser Pro Thr
    1715                 1720                 1725

Gly Val  Leu Ile Ala Ile Asp  Leu Ala Tyr Asn Leu  His Ser Ala
    1730                 1735                 1740

Tyr Gly  Asn Trp Phe Pro Gly  Cys Lys Pro Leu Ile  Gln Gln Ala
    1745                 1750                 1755

Met Ala  Lys Ile Met Lys Ala  Asn Pro Ala Leu Tyr  Val Leu Arg
    1760                 1765                 1770

Glu Arg  Ile Arg Lys Ala Leu  Gln Leu Tyr Ser Ser  Glu Pro Thr
    1775                 1780                 1785

Glu Pro  Tyr Leu Ser Ser Gln  Asn Tyr Gly Glu Leu  Phe Ser Asn
    1790                 1795                 1800

Gln Ile  Ile Trp Phe Val Asp  Asp Thr Asn Val Tyr  Arg Val Thr
    1805                 1810                 1815

Ile His  Lys Thr Phe Glu Gly  Asn Leu Thr Thr Lys  Pro Ile Asn
    1820                 1825                 1830
```

```
Gly Ala  Ile Phe Ile Phe Asn  Pro Arg Thr Gly Gln  Leu Phe Leu
    1835                 1840                 1845

Lys Ile  Ile His Thr Ser Val  Trp Ala Gly Gln Lys  Arg Leu Gly
    1850                 1855                 1860

Gln Leu  Ala Lys Trp Lys Thr  Ala Glu Glu Val Ala  Ala Leu Ile
    1865                 1870                 1875

Arg Ser  Leu Pro Val Glu Glu  Gln Pro Lys Gln Ile  Ile Val Thr
    1880                 1885                 1890

Arg Lys  Gly Met Leu Asp Pro  Leu Glu Val His Leu  Leu Asp Phe
    1895                 1900                 1905

Pro Asn  Ile Val Ile Lys Gly  Ser Glu Leu Gln Leu  Pro Phe Gln
    1910                 1915                 1920

Ala Cys  Leu Lys Ile Glu Lys  Phe Gly Asp Leu Ile  Leu Lys Ala
    1925                 1930                 1935

Thr Glu  Pro Gln Met Val Leu  Phe Asn Leu Tyr Asp  Asp Trp Leu
    1940                 1945                 1950

Lys Thr  Ile Ser Ser Tyr Thr  Ala Phe Ser Arg Leu  Ile Leu Ile
    1955                 1960                 1965

Leu Arg  Ala Leu His Val Asn  Thr Glu Arg Thr Lys  Val Ile Leu
    1970                 1975                 1980

Lys Pro  Asp Lys Thr Thr Ile  Thr Glu Leu His His  Ile Trp Pro
    1985                 1990                 1995

Thr Leu  Ser Asp Asp Glu Trp  Ile Lys Val Glu Val  Gln Leu Lys
    2000                 2005                 2010

Asp Leu  Ile Leu Ala Asp Tyr  Gly Lys Lys Asn Asn  Val Asn Val
    2015                 2020                 2025

Ala Ser  Leu Thr Gln Ser Glu  Ile Arg Asp Ile Ile  Leu Gly Met
    2030                 2035                 2040

Glu Ile  Ser Ala Pro Ser Ala  Gln Arg Gln Gln Ile  Ala Glu Ile
    2045                 2050                 2055

Glu Lys  Gln Thr Lys Glu Gln  Ser Gln Leu Thr Ala  Thr Thr Thr
```

2060 2065 2070

Lys Thr Val Asn Lys His Gly Asp Glu Ile Ile Thr Ser Thr Thr
2075 2080 2085

Ser Asn Tyr Glu Thr Gln Thr Phe Ser Ser Lys Thr Glu Trp Arg
2090 2095 2100

Val Arg Ala Ile Ser Ala Thr Asn Leu His Leu Arg Thr Asn His
2105 2110 2115

Ile Tyr Val Ser Ser Asp Asp Ile Lys Glu Thr Gly Tyr Thr Tyr
2120 2125 2130

Ile Leu Pro Lys Asn Val Leu Lys Lys Phe Val Thr Ile Ser Asp
2135 2140 2145

Leu Arg Ala Gln Ile Cys Ala Phe Leu Tyr Gly Val Ser Pro Pro
2150 2155 2160

Asp Asn Pro Gln Val Lys Glu Leu Arg Cys Leu Val Leu Ala Pro
2165 2170 2175

Gln Trp Gly Thr His Gln Thr Val His Val Pro Asn Thr Pro Pro
2180 2185 2190

Asn His Pro Phe Leu Lys Asp Met Glu Pro Leu Gly Trp Ile His
2195 2200 2205

Thr Gln Pro Asn Glu Leu Pro Gln Leu Ser Pro Gln Asp Ile Thr
2210 2215 2220

Asn His Ala Lys Leu Met Ser Asp Asn Thr Thr Trp Asp Gly Glu
2225 2230 2235

Lys Thr Ile Ile Ile Thr Cys Ser Phe Thr Pro Gly Ser Cys Ser
2240 2245 2250

Leu Thr Ala Tyr Lys Leu Thr Pro Ser Gly Phe Glu Trp Gly Arg
2255 2260 2265

Gln Asn Thr Asp Lys Gly Asn Asn Pro Lys Gly Tyr Leu Pro Ser
2270 2275 2280

His Tyr Glu Lys Val Gln Met Leu Leu Ser Asp Arg Phe Leu Gly
2285 2290 2295

Phe Phe Met Val Pro Ala Gln Gly Ser Trp Asn Tyr Asn Phe Met
2300 2305 2310

Gly Val Arg His Asp Pro Ser Met Lys Tyr Glu Leu Gln Leu Ala
2315 2320 2325

Asn Pro Lys Glu Phe Tyr His Glu Val His Arg Pro Ala His Phe
2330 2335 2340

Leu Asn Phe Ser Ala Leu Glu Asp Gly Asp Gly Ala Gly Ala Asp
2345 2350 2355

Arg Glu Asp Ala Phe Ala
2360

<210> 3
<211> 9518
<212> DNA
<213> Diabrotica virgifera

<400> 3
tgaaagaatc gatcacctcc ccaaaaaaac acatacctgc ttcccagatc ggatgatgat 60

cgtcacccac tatgggaccg tcagctccac aaggtgcaag aacagtctgt gtttttggcc 120

gtgaacttct ttgaggcgac ctgtacgagt acgagagcgc tccctcacgt gggatttcgg 180

ttacatcgtc ctttagtccg caaaacgtcg tcaccggaac tttggaatga gggttgatgc 240

tcaaaaatcc acaattatac gacaagcatt tatctagacc atcgttgacg tttgtgtaat 300

tcgtgtgatg tccttttgaa catgcataaa gcatgttaag cacaggtgtg aacccctctt 360

tcgttggtag gcgctcctta ggaattacca atgaactttc gccagaattt gggttcgaaa 420

agattgtgtc cgagaattca cagctaacaa attcagtcgg attagtagtc gtcgcgttat 480

agctgatgaa gccgcattcc gggtcaagag agcacgtggc gagcgcgatc taaggtgaca 540

actatgtcgg agcagagtct tagagcctca cagatattgt cggcttatcc tgcaatacga 600

taaatctttt gcaactcttg aaacaacata ccagaccttt gagagatttc cggcccgtac 660

aggggacatc aacattcttt aatacgagtg atgtgatctc tggagtttgg ggctcagtct 720

cgccataaca agcggtactg aagacataaa aagaggctaa actgcgcatt gagcacacgc 780

gtgtcttgga catgaaggcc cgacaaatga tctccgaagt tgagctttaa atattgtgaa 840

ggcgggggat gagctcaaat gggccaggta gtagcaagaa catgaatggc aggaagccgg 900

aaatgcctcc agaggctctg aggaagataa ttgcagatca tggcgacatg agtagccgga 960

agtttcgcca agataagaga gtttaccttg gagcgctgaa gtatgtaccc catgctgttt 1020

acaaactctt agagaatcta cccatgcctt gggagcaagt gagaaacgta aaagtcttgt 1080

atcacacaac tggggcaatc tcttttgtga acgagatacc ttgggtagtc gagccgattt 1140

ttctggccca gtggggaaca atgtggataa tgatgcgacg tgagaaacgc gatcgccgtc 1200
atttcaaacg tatgagattt ccgcctttcg atgacgaaga gcctccactt gattacgccg 1260
acaacatatt agaccaacag cccctcgacg caatacaaat ggagctggac gctgaggaag 1320
acgctccagt gatagactgg ttttacgatc accaacctct ccaatacgat tctaattacc 1380
tcgcaggtcc caaataccga agatggcgtc tcgatttgaa ccaaatgagc gtcctgtata 1440
gattagccca tcaacttctg tctgatatca ttgatgacaa ttacttttac ctatttgatc 1500
tgaaatcatt ctttacagcc aaagcgctaa accttgccat tcccggtggg ccaaagtttg 1560
agcccctggt ccgcgatgtc gctgatgatt cggattggaa cacatttaat aacattgaca 1620
agataatcgt tcggcataaa atccgtacgg aatataaaat tgcattcccc tatctctaca 1680
atgacaggcc attcaaagtt tctttgagta aatatcattc tccgactgtg gtgtttgtga 1740
agcaagagga ggtcgaccaa cctgcattct actttgaccc tctcctgtat ccaatacctg 1800
cctatcgaac taaaaccgac aagtatttct gccaaactat cgaaagttca atagacgatg 1860
acttccttca ggagcttaac agctttgcgt caagcgccag cgcaggcatt ggatccgctg 1920
atagtctact ccagccgctt ttgtttgagg cgcctttgca gaccgacaca acatatggag 1980
gtataacatt gctgtgggct ccaagaccct tcaacataag atccgggttg accaggagag 2040
ctcaagatat tccactagtt cagtcctggt tccgagagca ctgcccaggt gcttcgacct 2100
atccggtgaa agttcgcgtc tcttatcaga agcttctcaa aacttgggta ctgagccatc 2160
tcagaagtcg tccgcctaag gcaatgaaga agcgcaatct cctgagacta tttaaaaaca 2220
ccaaattctt tcaatgtact gaaactgatt gggtggaggt tggtctgcac gtgtgccgcc 2280
aaggatataa tatgctcaat ctcctgattc atcgccgaaa tctaaactac cttcatctgg 2340
attataattt caatctgaag cccattaaaa cattgaccac taaagaacga aaaaagagtc 2400
gtttcggaaa tgcgttccat ctatgtcgcg agattctacg tctcaccaaa ttgattgttg 2460
actctcacgt ccagtaccgg ctggggaata tagatgcata tcaactggca gatggcttac 2520
aatacatatt ctgccacgtc ggtcaattga catccatgta tcgatacaaa taccggctta 2580
tgcgacaggt tcggctgtgc aaggatctca agcatctaat atattacaga ttcaacaccg 2640
gccaagtggg taaaggccca ggctgcggat tctggttgcc ctcatatcgt gtctggttgt 2700
tctttctgcg cgggatttta cctttattgg agagatggtt gggtaatcta ttggctcgtc 2760
agtttgaagg tcgaaacttg cgcggtcaag caaaatccgt cacgaagcaa cgagtggaag 2820
tctacttcga tttagagcta cgagctgctg tgatgcatga tctgctagat atgatgccag 2880
aaggaatccg agcaaacaaa gccaaaattg tacttcagca tctcagcgaa gcctggagat 2940
gttggaaggc gaatattccc tggaaggtcg ccgggattcc agctccggtg gaaaacatta 3000

ttctgagata tgtaaaacta aaatctgact ggtggacgaa tgccgcatat ttcaatcggg 3060
agagaattag acgtggagca actgtggaca agactgtgtg caaaaagaac ttggggcggc 3120
tcactcgttt gtggttgaag tcagagcaag aacgtcaaca tgggtacatg aaggatggtc 3180
cctatctaac cagtgaggag gcggtggcga tttacactac aatggtacat tggttggatt 3240
tgcgaaaatt cactcatatc ccatttcctc cattgaacta taaacacgac acaaaacttc 3300
tgattctcgc tctggagcgc ttgagggaca catacgccgt gaagacacga ctgaatcaag 3360
ttcagcgtga agagttgggt ctaatcgaac acgcgtacga taatcctcat gaggccatat 3420
cgcgaataaa acgacattta ttgactcaac gagccttcaa agacgccagt gttgagttca 3480
tggatctcta ctcgcattta gtacctgtat acgagatcga tccactagaa aaaatcaccg 3540
acgcttacct cgaccagtat ttatggtacg agtctgacct ccgccacctc ttcccaccgt 3600
ggataaaacc gagcgatcac gagcctctgc ctctgctgct ctataaatgg tcaaacaata 3660
taaataattt ggactcgata tgggaacatg acgacggttc ctgcgttgcc atgatgcaaa 3720
cgaagttgaa gaagattttc gagaaaattg atctcaccct tctcaataga ttgctgagat 3780
tgatagttga ccataatctc gctgattaca tgaccgcgaa aaacaacatt cggctgatct 3840
tcaaggacat gtcccataca aattattacg gcttaatccg cggcctccag ttcagcagtt 3900
tcatattcca atattatgct ctggtcatag atcttctgat tttagggctg acgcgagcca 3960
atgaacttgc cggcagtata ggtggcggcg gaggcggagg tttcgctaat ctcaaagatc 4020
gcgaaacgga gataaaacat cccatccgct tgtattgccg atatatagat gaaatatgga 4080
tctgcttcaa attcaccaaa gaggagtctc gtagcttgat tcaaaggtat ttgacggaga 4140
atccaaccgc tagtcagcag ctctccactg aagaaggcat cgactacccc atcaaaaagt 4200
gttggcctaa agactgccga atgagaaaaa tgaaattcga cgttaatatc ggacgagccg 4260
ttttctggga gattcagaaa cgtctaccga gaagtttagc tgagctgagt tggggcaaag 4320
atgctggaga ctcgacatcg tttgtgtcag tctatagtgt caataacccc aatcttctgt 4380
ttagcatggg cggctttgag gtccgaatcc tgccaaaagt tcgaggtggg actagtatgg 4440
gaactgggag cagttcacaa ggcgtatggc gtttacaaaa ctatctgacc aaggagacga 4500
cagcgtattg ttacattaga gttggtgacg aagccatacg taacttcgaa aatcgaattc 4560
ggcagattct gatgtcatcc ggctcggcaa cgttcacaaa ggtggcaaac aaatggaata 4620
cagctctgat cagccttgtg agttatttca gagaggcgat aatatatacg gaggatctcc 4680
tcgatctgtt ggtgaaatgt gaaaacaaaa tacaaacgag aatcaagatc ggtttgaata 4740
gtaaaatgcc gtcgaggttc ccccccgttg tgttctacac gcccaaagag ctcggcggct 4800
tgggcatgct ttccatgggg cacatcctta tccctcaatc tgacttgcgc tatatgaagc 4860
agaccaatga ttataccatc acccatttcc gctcgggaat gactcacgac gaagatcagt 4920

tgatacccaa tctctataga tacatccaga catgggaaag tgagttcatc gacagtcagc 4980
gagtttggtc ggaatataac atcaagagat ttgaagcaac cactaacggc ggcgccggtt 5040
caagtggcgg cagcggcggg agtcgcagac tgactttgga agacgtagag gagaactggg 5100
atcatggtat tccccgtatt aatacgttgt ttcagaaaga tcgacacacg ctgtgctacg 5160
ataagggctg gagattacgt caagagttta agcaatatca gatcctgcgg agcaatccat 5220
tctggtggac aaatatcaag cacgatggaa aattgtggaa tctcaacaac tatagaactg 5280
atatgatcca agctttgggc ggagttgagg gcattttgga acacacgctt ttcaaaggaa 5340
cttacttcca gacatgggaa ggtctattct gggaaaagtc tagtggcttc gaggaatcca 5400
tgaaatataa gaagttgaca aacgcgcaaa gaagtgggtt aaatcaaata cctaatcgga 5460
ggttcaccct ctggtggagt ccaacgatca atcggtcaaa tatctatgtt ggattccaag 5520
tccaattaga tctcacagga attttcatgc acggcaaaat cccaaccctc aagatcagct 5580
tgattcaaat cttccgcgcg catctttggc agaagattca tgagtcagtt atcatggatc 5640
tctgtcagat tttggatctc gaaattgaat ctttaggaat ccacacagtt aagaaagaaa 5700
ctatccatcc tcgaaaaagt tacaagatga atagctcttg tgcagatatc attttgtact 5760
cgtcgtacaa atggaacatc agcaatgtgc ctacacttct atcagccaac gcaaacgcat 5820
cggcctcatc aaccacctca accataagtt ggcttgatct tcaactccga tggggggatt 5880
acgactcgca cgacatcgaa agatactgcc ggtccaagta tcttgattac gtcaacgaca 5940
gcatgtctat ttatccgtcg aataccggag ttcttctggg catagatttg gcttacaata 6000
tgtacagcgg atttggaata tggattgacg gcttaaagga attggtccgt acgggcatgc 6060
gcaagatcat caaatcgaat ccgagtttgt atgtcttgag agaacgaata aggaaaggct 6120
tacaactgta tagctcggag ccgacagagc caaatcttga gtcttctaac tatggtgaac 6180
tgttcacctc taacggcccc aatacttggt tcgtcgatga tactaatgtt tatagggtta 6240
caattcacaa aactttcgag ggaaatttaa caaccaagcc gacgaatggg gccattgtta 6300
tcatcaaccc agtgactggc cagttgtttc tgaagattat acatactagt gtatggtcag 6360
gtcagaaacg cttgagtcaa ttggcgaagt ggaagaccgc tgaggaaatc accagtctca 6420
tccggtcttt gcctattgaa gaacaaccca agcagattat agtgaccaga aagggcatgc 6480
tggacccctt ggaagtacat ctgctagatt ttcctaacat cataatcaaa ggttccgagt 6540
tggcattgcc attccaaagt ctcatgaagt tggagaagtt ctcagatctc attctaaaag 6600
ctacaaaacc agatatggtt ctctttaacc tctatgatga ttggcttcaa aacatttcag 6660
catacactgc attttccaga ttgattcttc tactccgctc attgcacgtg aatcccgaga 6720
agaccaagat catcttgagg ccggatagat ccattatcac caaaccacac catatatggc 6780

ctaccattaa gaatgaggac tggaagaaga ttgaagttca attgaccgac ctaattctga 6840
ctgattactc caaggcaaat aatgtcgcta tcagctcact cacccagaca gaaatacgtg 6900
atatcattct aggtatggat ctccaaccac caagcctgca gagacaacaa atcgccgaga 6960
tcggaggcga gacgtccaac aatggagtgg cgttgtctgc ttcaggtatc actgcaacga 7020
ctacgagtac tactaatatc agtggtgacg caatgatcgt cactacccag agtcctcatg 7080
aacaacagat gttcttgagt aaaactgact ggagagttcg ggcgatgaac agcgggtcct 7140
tgtatttgag agctgagaag atttatatcg atgatgacgc gagagatgag acgatcactg 7200
gtacatcaag tactgcaacc tcggacggat ttacgtatac tattccacat aatcttatta 7260
ggctatttct tggggccgcg gatttgagaa ctcgaattgg cgcatacata tttggcacaa 7320
catctgccaa aatcctcttt gtgaaagaga tcaagacctt cgttatggtt ccgcaatcca 7380
attcacatga aaaagtggat tttgtcgaca tgttaccaga tcatcctatt ctcaaagaac 7440
ttgaaccatt gggatgggta caaactactg ccactggatc aaagccatct ctccacgata 7500
tcacattcac agctgctcta ctctcggacg gtccatgtca gatgcctagg ctcgatccta 7560
atgcttgtgt aatgctgttt gtcgctttga cgcaaggaag ttgcacgttg agcggttaca 7620
gattgactcc cgcagggctc gagtgggcta gtggcattac ggcaacaata caggcggagg 7680
tagctcctca gtatattgag aaaacccaat tgctggtctc ggataataca gccggattct 7740
ttatggtgcc agatgacgga ttttggaatt tcgctttcat gggcgtaaga ttcaacaaga 7800
aaacccctta caatttggta ttgaacgttc cgaaatcctt ctgtgatgaa ttgcatcgac 7860
ctaatcattt cttgcaattt gctcaactgg aagcgctgga tgagtccgat ggcgttgaag 7920
ccgaagactg gttagattag atcggacacg cgtgtgcgcg cgcaaatata gataaatgcg 7980
cgtgttgact agatttttgc ctcttgcctc agtggcattc gcagtcaatg ttgagccttc 8040
gcatcaagtc atgacgcaag atactggagg agctgtatca aacgtgctgg gaagcatcaa 8100
gagtcgatcc aaacagctgg cccaaagcat tcccgggtcg tcgatagcta gctgtttgac 8160
ttcctcaaat ccggaacttt gcaagaaaca ggttcgcttc gagcatgatt tgagaggact 8220
catgttgaaa ggtaccaccg atctggcttc catgcaatct ctcaagcaaa aattaacggt 8280
gcctagcgcc tatggcctgg acgccgctca agctaatgac atttttcatc aactgataaa 8340
ggagcttcac tttgatcagc aggcctacga attggtcact aatgcagcaa aagcaacgac 8400
gccgatgagc ccgagtatct cgcttccgac agtggcaccc ataccgatca acgcaggtgt 8460
gggcgctgcg gcagtgagtc ccggcatagc gaccgcaatt agccccttcg ccacaacatc 8520
ggtgagcaca ttggctccct cttctggagt cttaaatgct gcggccctta cgaccgcggc 8580
gccgacggcg agcacactga ttgcaagtgt ctccaccact gcctcgacgg cacactaaat 8640
ttcatttttt attggaaagc taatgttcgt tgctctagtt tacggaatca gttctgctgc 8700

attggtgctg gaaacaaagg ggattttgag agcttgttca gacaagttga aggttctggc 8760

cttacaacag agcgtcatag cgttatgcta cgtgatcttg agcactgtga atgcacacaa 8820

aaatggcaca cacggctctg gattgtggag ttttcaggac ttcaaacgag cgataccggt 8880

gacactagct tttctcagca tgcaggcaac tcagatgatt tgcctcgcca attcgagtat 8940

gggtagctac gtggtcgcga aagcaagttg tctgacattt aatatactgc tgttcggctg 9000

tctgattgtg acaattggcg ttgtgctccc tgtttgtaat agtcgagcgc actgcacaaa 9060

gtctgggttt tgcgcgggct tgatgtcttc cctggcgcaa gctgctttca tgcttctgtc 9120

atccgttgcg actaaaagac attttgcagc agcgccgatg aaactcctcg gtcattacac 9180

attctcggct gttgtagtat tatgggctat cctctggctt cgtgggtact ccgatgattc 9240

gacttgccag accagggggc ttttgacacg cataatctgg tccggtatta tcaatgtagt 9300

tgtggccatg agcgcaatgc gatgtttaaa aaacagtcat ccagttgcat tgaacatgat 9360

cagtttcgtc aaatccgttt tacagatttg ctgcgctgct ttgttctacg gagaccgccc 9420

caacagaaca gaaataatgg gcgtggcatt tgttctaggt ggaagtgcag tctactcgtg 9480

cggccgattt ttcatcaaag aaacagactg agtgccct 9518

<210> 4
<211> 2363
<212> PRT
<213> Diabrotica virgifera

<400> 4

Met Ser Ser Asn Gly Pro Gly Ser Ser Lys Asn Met Asn Gly Arg Lys
1 5 10 15

Pro Glu Met Pro Pro Glu Ala Leu Arg Lys Ile Ile Ala Asp His Gly
20 25 30

Asp Met Ser Ser Arg Lys Phe Arg Gln Asp Lys Arg Val Tyr Leu Gly
35 40 45

Ala Leu Lys Tyr Val Pro His Ala Val Tyr Lys Leu Leu Glu Asn Leu
50 55 60

Pro Met Pro Trp Glu Gln Val Arg Asn Val Lys Val Leu Tyr His Thr
65 70 75 80

Thr Gly Ala Ile Ser Phe Val Asn Glu Ile Pro Trp Val Val Glu Pro
85 90 95

Ile Phe Leu Ala Gln Trp Gly Thr Met Trp Ile Met Met Arg Arg Glu
100 105 110

```
Lys Arg Asp Arg Arg His Phe Lys Arg Met Arg Phe Pro Pro Phe Asp
        115                 120                 125

Asp Glu Glu Pro Pro Leu Asp Tyr Ala Asp Asn Ile Leu Asp Gln Gln
    130                 135                 140

Pro Leu Asp Ala Ile Gln Met Glu Leu Asp Ala Glu Glu Asp Ala Pro
145                 150                 155                 160

Val Ile Asp Trp Phe Tyr Asp His Gln Pro Leu Gln Tyr Asp Ser Asn
                165                 170                 175

Tyr Leu Ala Gly Pro Lys Tyr Arg Arg Trp Arg Leu Asp Leu Asn Gln
            180                 185                 190

Met Ser Val Leu Tyr Arg Leu Ala His Gln Leu Leu Ser Asp Ile Ile
        195                 200                 205

Asp Asp Asn Tyr Phe Tyr Leu Phe Asp Leu Lys Ser Phe Phe Thr Ala
    210                 215                 220

Lys Ala Leu Asn Leu Ala Ile Pro Gly Gly Pro Lys Phe Glu Pro Leu
225                 230                 235                 240

Val Arg Asp Val Ala Asp Asp Ser Asp Trp Asn Thr Phe Asn Asn Ile
                245                 250                 255

Asp Lys Ile Ile Val Arg His Lys Ile Arg Thr Glu Tyr Lys Ile Ala
            260                 265                 270

Phe Pro Tyr Leu Tyr Asn Asp Arg Pro Phe Lys Val Ser Leu Ser Lys
        275                 280                 285

Tyr His Ser Pro Thr Val Val Phe Val Lys Gln Glu Glu Val Asp Gln
    290                 295                 300

Pro Ala Phe Tyr Phe Asp Pro Leu Leu Tyr Pro Ile Pro Ala Tyr Arg
305                 310                 315                 320

Thr Lys Thr Asp Lys Tyr Phe Cys Gln Thr Ile Glu Ser Ser Ile Asp
                325                 330                 335

Asp Asp Phe Leu Gln Glu Leu Asn Ser Phe Ala Ser Ser Ala Ser Ala
            340                 345                 350

Gly Ile Gly Ser Ala Asp Ser Leu Leu Gln Pro Leu Leu Phe Glu Ala
```

355 360 365

Pro Leu Gln Thr Asp Thr Thr Tyr Gly Gly Ile Thr Leu Leu Trp Ala
370 375 380

Pro Arg Pro Phe Asn Ile Arg Ser Gly Leu Thr Arg Arg Ala Gln Asp
385 390 395 400

Ile Pro Leu Val Gln Ser Trp Phe Arg Glu His Cys Pro Gly Ala Ser
405 410 415

Thr Tyr Pro Val Lys Val Arg Val Ser Tyr Gln Lys Leu Leu Lys Thr
420 425 430

Trp Val Leu Ser His Leu Arg Ser Arg Pro Pro Lys Ala Met Lys Lys
435 440 445

Arg Asn Leu Leu Arg Leu Phe Lys Asn Thr Lys Phe Phe Gln Cys Thr
450 455 460

Glu Thr Asp Trp Val Glu Val Gly Leu His Val Cys Arg Gln Gly Tyr
465 470 475 480

Asn Met Leu Asn Leu Leu Ile His Arg Arg Asn Leu Asn Tyr Leu His
485 490 495

Leu Asp Tyr Asn Phe Asn Leu Lys Pro Ile Lys Thr Leu Thr Thr Lys
500 505 510

Glu Arg Lys Lys Ser Arg Phe Gly Asn Ala Phe His Leu Cys Arg Glu
515 520 525

Ile Leu Arg Leu Thr Lys Leu Ile Val Asp Ser His Val Gln Tyr Arg
530 535 540

Leu Gly Asn Ile Asp Ala Tyr Gln Leu Ala Asp Gly Leu Gln Tyr Ile
545 550 555 560

Phe Cys His Val Gly Gln Leu Thr Ser Met Tyr Arg Tyr Lys Tyr Arg
565 570 575

Leu Met Arg Gln Val Arg Leu Cys Lys Asp Leu Lys His Leu Ile Tyr
580 585 590

Tyr Arg Phe Asn Thr Gly Gln Val Gly Lys Gly Pro Gly Cys Gly Phe
595 600 605

```
Trp Leu Pro Ser Tyr Arg Val Trp Leu Phe Phe Leu Arg Gly Ile Leu
    610                 615                 620

Pro Leu Leu Glu Arg Trp Leu Gly Asn Leu Leu Ala Arg Gln Phe Glu
625                 630                 635                 640

Gly Arg Asn Leu Arg Gly Gln Ala Lys Ser Val Thr Lys Gln Arg Val
                645                 650                 655

Glu Val Tyr Phe Asp Leu Glu Leu Arg Ala Ala Val Met His Asp Leu
            660                 665                 670

Leu Asp Met Met Pro Glu Gly Ile Arg Ala Asn Lys Ala Lys Ile Val
        675                 680                 685

Leu Gln His Leu Ser Glu Ala Trp Arg Cys Trp Lys Ala Asn Ile Pro
    690                 695                 700

Trp Lys Val Ala Gly Ile Pro Ala Pro Val Glu Asn Ile Ile Leu Arg
705                 710                 715                 720

Tyr Val Lys Leu Lys Ser Asp Trp Trp Thr Asn Ala Ala Tyr Phe Asn
                725                 730                 735

Arg Glu Arg Ile Arg Arg Gly Ala Thr Val Asp Lys Thr Val Cys Lys
            740                 745                 750

Lys Asn Leu Gly Arg Leu Thr Arg Leu Trp Leu Lys Ser Glu Gln Glu
        755                 760                 765

Arg Gln His Gly Tyr Met Lys Asp Gly Pro Tyr Leu Thr Ser Glu Glu
    770                 775                 780

Ala Val Ala Ile Tyr Thr Thr Met Val His Trp Leu Asp Leu Arg Lys
785                 790                 795                 800

Phe Thr His Ile Pro Phe Pro Pro Leu Asn Tyr Lys His Asp Thr Lys
                805                 810                 815

Leu Leu Ile Leu Ala Leu Glu Arg Leu Arg Asp Thr Tyr Ala Val Lys
            820                 825                 830

Thr Arg Leu Asn Gln Val Gln Arg Glu Glu Leu Gly Leu Ile Glu His
        835                 840                 845

Ala Tyr Asp Asn Pro His Glu Ala Ile Ser Arg Ile Lys Arg His Leu
    850                 855                 860
```

Leu Thr Gln Arg Ala Phe Lys Asp Ala Ser Val Glu Phe Met Asp Leu
865 870 875 880

Tyr Ser His Leu Val Pro Val Tyr Glu Ile Asp Pro Leu Glu Lys Ile
885 890 895

Thr Asp Ala Tyr Leu Asp Gln Tyr Leu Trp Tyr Glu Ser Asp Leu Arg
900 905 910

His Leu Phe Pro Pro Trp Ile Lys Pro Ser Asp His Glu Pro Leu Pro
915 920 925

Leu Leu Leu Tyr Lys Trp Ser Asn Asn Ile Asn Asn Leu Asp Ser Ile
930 935 940

Trp Glu His Asp Asp Gly Ser Cys Val Ala Met Met Gln Thr Lys Leu
945 950 955 960

Lys Lys Ile Phe Glu Lys Ile Asp Leu Thr Leu Leu Asn Arg Leu Leu
965 970 975

Arg Leu Ile Val Asp His Asn Leu Ala Asp Tyr Met Thr Ala Lys Asn
980 985 990

Asn Ile Arg Leu Ile Phe Lys Asp Met Ser His Thr Asn Tyr Tyr Gly
995 1000 1005

Leu Ile Arg Gly Leu Gln Phe Ser Ser Phe Ile Phe Gln Tyr Tyr
1010 1015 1020

Ala Leu Val Ile Asp Leu Leu Ile Leu Gly Leu Thr Arg Ala Asn
1025 1030 1035

Glu Leu Ala Gly Ser Ile Gly Gly Gly Gly Gly Gly Gly Phe Ala
1040 1045 1050

Asn Leu Lys Asp Arg Glu Thr Glu Ile Lys His Pro Ile Arg Leu
1055 1060 1065

Tyr Cys Arg Tyr Ile Asp Glu Ile Trp Ile Cys Phe Lys Phe Thr
1070 1075 1080

Lys Glu Glu Ser Arg Ser Leu Ile Gln Arg Tyr Leu Thr Glu Asn
1085 1090 1095

Pro Thr Ala Ser Gln Gln Leu Ser Thr Glu Glu Gly Ile Asp Tyr
1100 1105 1110

```
Pro Ile  Lys Lys Cys Trp Pro  Lys Asp Cys Arg Met  Arg Lys Met
    1115                 1120                 1125

Lys Phe  Asp Val Asn Ile Gly  Arg Ala Val Phe Trp  Glu Ile Gln
    1130                 1135                 1140

Lys Arg  Leu Pro Arg Ser Leu  Ala Glu Leu Ser Trp  Gly Lys Asp
    1145                 1150                 1155

Ala Gly  Asp Ser Thr Ser Phe  Val Ser Val Tyr Ser  Val Asn Asn
    1160                 1165                 1170

Pro Asn  Leu Leu Phe Ser Met  Gly Gly Phe Glu Val  Arg Ile Leu
    1175                 1180                 1185

Pro Lys  Val Arg Gly Gly Thr  Ser Met Gly Thr Gly  Ser Ser Ser
    1190                 1195                 1200

Gln Gly  Val Trp Arg Leu Gln  Asn Tyr Leu Thr Lys  Glu Thr Thr
    1205                 1210                 1215

Ala Tyr  Cys Tyr Ile Arg Val  Gly Asp Glu Ala Ile  Arg Asn Phe
    1220                 1225                 1230

Glu Asn  Arg Ile Arg Gln Ile  Leu Met Ser Ser Gly  Ser Ala Thr
    1235                 1240                 1245

Phe Thr  Lys Val Ala Asn Lys  Trp Asn Thr Ala Leu  Ile Ser Leu
    1250                 1255                 1260

Val Ser  Tyr Phe Arg Glu Ala  Ile Ile Tyr Thr Glu  Asp Leu Leu
    1265                 1270                 1275

Asp Leu  Leu Val Lys Cys Glu  Asn Lys Ile Gln Thr  Arg Ile Lys
    1280                 1285                 1290

Ile Gly  Leu Asn Ser Lys Met  Pro Ser Arg Phe Pro  Pro Val Val
    1295                 1300                 1305

Phe Tyr  Thr Pro Lys Glu Leu  Gly Gly Leu Gly Met  Leu Ser Met
    1310                 1315                 1320

Gly His  Ile Leu Ile Pro Gln  Ser Asp Leu Arg Tyr  Met Lys Gln
    1325                 1330                 1335

Thr Asn  Asp Tyr Thr Ile Thr  His Phe Arg Ser Gly  Met Thr His
```

1340 1345 1350

Asp Glu Asp Gln Leu Ile Pro Asn Leu Tyr Arg Tyr Ile Gln Thr
1355 1360 1365

Trp Glu Ser Glu Phe Ile Asp Ser Gln Arg Val Trp Ser Glu Tyr
1370 1375 1380

Asn Ile Lys Arg Phe Glu Ala Thr Thr Asn Gly Gly Ala Gly Ser
1385 1390 1395

Ser Gly Gly Ser Gly Gly Ser Arg Arg Leu Thr Leu Glu Asp Val
1400 1405 1410

Glu Glu Asn Trp Asp His Gly Ile Pro Arg Ile Asn Thr Leu Phe
1415 1420 1425

Gln Lys Asp Arg His Thr Leu Cys Tyr Asp Lys Gly Trp Arg Leu
1430 1435 1440

Arg Gln Glu Phe Lys Gln Tyr Gln Ile Leu Arg Ser Asn Pro Phe
1445 1450 1455

Trp Trp Thr Asn Ile Lys His Asp Gly Lys Leu Trp Asn Leu Asn
1460 1465 1470

Asn Tyr Arg Thr Asp Met Ile Gln Ala Leu Gly Gly Val Glu Gly
1475 1480 1485

Ile Leu Glu His Thr Leu Phe Lys Gly Thr Tyr Phe Gln Thr Trp
1490 1495 1500

Glu Gly Leu Phe Trp Glu Lys Ser Ser Gly Phe Glu Glu Ser Met
1505 1510 1515

Lys Tyr Lys Lys Leu Thr Asn Ala Gln Arg Ser Gly Leu Asn Gln
1520 1525 1530

Ile Pro Asn Arg Arg Phe Thr Leu Trp Trp Ser Pro Thr Ile Asn
1535 1540 1545

Arg Ser Asn Ile Tyr Val Gly Phe Gln Val Gln Leu Asp Leu Thr
1550 1555 1560

Gly Ile Phe Met His Gly Lys Ile Pro Thr Leu Lys Ile Ser Leu
1565 1570 1575

Ile Gln Ile Phe Arg Ala His Leu Trp Gln Lys Ile His Glu Ser
1580 1585 1590

Val Ile Met Asp Leu Cys Gln Ile Leu Asp Leu Glu Ile Glu Ser
1595 1600 1605

Leu Gly Ile His Thr Val Lys Lys Glu Thr Ile His Pro Arg Lys
1610 1615 1620

Ser Tyr Lys Met Asn Ser Ser Cys Ala Asp Ile Ile Leu Tyr Ser
1625 1630 1635

Ser Tyr Lys Trp Asn Ile Ser Asn Val Pro Thr Leu Leu Ser Ala
1640 1645 1650

Asn Ala Asn Ala Ser Ala Ser Ser Thr Thr Ser Thr Ile Ser Trp
1655 1660 1665

Leu Asp Leu Gln Leu Arg Trp Gly Asp Tyr Asp Ser His Asp Ile
1670 1675 1680

Glu Arg Tyr Cys Arg Ser Lys Tyr Leu Asp Tyr Val Asn Asp Ser
1685 1690 1695

Met Ser Ile Tyr Pro Ser Asn Thr Gly Val Leu Leu Gly Ile Asp
1700 1705 1710

Leu Ala Tyr Asn Met Tyr Ser Gly Phe Gly Ile Trp Ile Asp Gly
1715 1720 1725

Leu Lys Glu Leu Val Arg Thr Gly Met Arg Lys Ile Ile Lys Ser
1730 1735 1740

Asn Pro Ser Leu Tyr Val Leu Arg Glu Arg Ile Arg Lys Gly Leu
1745 1750 1755

Gln Leu Tyr Ser Ser Glu Pro Thr Glu Pro Asn Leu Glu Ser Ser
1760 1765 1770

Asn Tyr Gly Glu Leu Phe Thr Ser Asn Gly Pro Asn Thr Trp Phe
1775 1780 1785

Val Asp Asp Thr Asn Val Tyr Arg Val Thr Ile His Lys Thr Phe
1790 1795 1800

Glu Gly Asn Leu Thr Thr Lys Pro Thr Asn Gly Ala Ile Val Ile
1805 1810 1815

```
Ile Asn  Pro Val Thr Gly Gln  Leu Phe Leu Lys Ile  Ile His Thr
    1820                 1825                 1830

Ser Val  Trp Ser Gly Gln Lys  Arg Leu Ser Gln Leu  Ala Lys Trp
    1835                 1840                 1845

Lys Thr  Ala Glu Glu Ile Thr  Ser Leu Ile Arg Ser  Leu Pro Ile
    1850                 1855                 1860

Glu Glu  Gln Pro Lys Gln Ile  Ile Val Thr Arg Lys  Gly Met Leu
    1865                 1870                 1875

Asp Pro  Leu Glu Val His Leu  Leu Asp Phe Pro Asn  Ile Ile Ile
    1880                 1885                 1890

Lys Gly  Ser Glu Leu Ala Leu  Pro Phe Gln Ser Leu  Met Lys Leu
    1895                 1900                 1905

Glu Lys  Phe Ser Asp Leu Ile  Leu Lys Ala Thr Lys  Pro Asp Met
    1910                 1915                 1920

Val Leu  Phe Asn Leu Tyr Asp  Asp Trp Leu Gln Asn  Ile Ser Ala
    1925                 1930                 1935

Tyr Thr  Ala Phe Ser Arg Leu  Ile Leu Leu Leu Arg  Ser Leu His
    1940                 1945                 1950

Val Asn  Pro Glu Lys Thr Lys  Ile Ile Leu Arg Pro  Asp Arg Ser
    1955                 1960                 1965

Ile Ile  Thr Lys Pro His His  Ile Trp Pro Thr Ile  Lys Asn Glu
    1970                 1975                 1980

Asp Trp  Lys Lys Ile Glu Val  Gln Leu Thr Asp Leu  Ile Leu Thr
    1985                 1990                 1995

Asp Tyr  Ser Lys Ala Asn Asn  Val Ala Ile Ser Ser  Leu Thr Gln
    2000                 2005                 2010

Thr Glu  Ile Arg Asp Ile Ile  Leu Gly Met Asp Leu  Gln Pro Pro
    2015                 2020                 2025

Ser Leu  Gln Arg Gln Gln Ile  Ala Glu Ile Gly Gly  Glu Thr Ser
    2030                 2035                 2040

Asn Asn  Gly Val Ala Leu Ser  Ala Ser Gly Ile Thr  Ala Thr Thr
    2045                 2050                 2055
```

```
Thr Ser  Thr Thr Asn Ile Ser  Gly Asp Ala Met Ile  Val Thr Thr
    2060                 2065                 2070

Gln Ser  Pro His Glu Gln Gln  Met Phe Leu Ser Lys  Thr Asp Trp
    2075                 2080                 2085

Arg Val  Arg Ala Met Asn Ser  Gly Ser Leu Tyr Leu  Arg Ala Glu
    2090                 2095                 2100

Lys Ile  Tyr Ile Asp Asp Asp  Ala Arg Asp Glu Thr  Ile Thr Gly
    2105                 2110                 2115

Thr Ser  Ser Thr Ala Thr Ser  Asp Gly Phe Thr Tyr  Thr Ile Pro
    2120                 2125                 2130

His Asn  Leu Ile Arg Leu Phe  Leu Gly Ala Ala Asp  Leu Arg Thr
    2135                 2140                 2145

Arg Ile  Gly Ala Tyr Ile Phe  Gly Thr Thr Ser Ala  Lys Asn Pro
    2150                 2155                 2160

Leu Val  Lys Glu Ile Lys Thr  Phe Val Met Val Pro  Gln Ser Asn
    2165                 2170                 2175

Ser His  Glu Lys Val Asp Phe  Val Asp Met Leu Pro  Asp His Pro
    2180                 2185                 2190

Ile Leu  Lys Glu Leu Glu Pro  Leu Gly Trp Val Gln  Thr Thr Ala
    2195                 2200                 2205

Thr Gly  Ser Lys Pro Ser Leu  His Asp Ile Thr Phe  Thr Ala Ala
    2210                 2215                 2220

Leu Leu  Ser Asp Gly Pro Cys  Gln Met Pro Arg Leu  Asp Pro Asn
    2225                 2230                 2235

Ala Cys  Val Met Leu Phe Val  Ala Leu Thr Gln Gly  Ser Cys Thr
    2240                 2245                 2250

Leu Ser  Gly Tyr Arg Leu Thr  Pro Ala Gly Leu Glu  Trp Ala Ser
    2255                 2260                 2265

Gly Ile  Thr Ala Thr Ile Gln  Ala Glu Val Ala Pro  Gln Tyr Ile
    2270                 2275                 2280

Glu Lys  Thr Gln Leu Leu Val  Ser Asp Asn Thr Ala  Gly Phe Phe
```

2285 2290 2295

Met Val Pro Asp Asp Gly Phe Trp Asn Phe Ala Phe Met Gly Val
2300 2305 2310

Arg Phe Asn Lys Lys Thr Pro Tyr Asn Leu Val Leu Asn Val Pro
2315 2320 2325

Lys Ser Phe Cys Asp Glu Leu His Arg Pro Asn His Phe Leu Gln
2330 2335 2340

Phe Ala Gln Leu Glu Ala Leu Asp Glu Ser Asp Gly Val Glu Ala
2345 2350 2355

Glu Asp Trp Leu Asp
2360

<210> 5
<211> 7089
<212> DNA
<213> Meligethes aeneus

<400> 5
atgtctttgc caccttattt actgggccca aatccctggt taatggctca acagcagtta 60

gctgcagctc atgctcaagc tcaggctgct gctgctcaag ctcatgccca agctttacaa 120

caacaaattc ctcctccaca cccaaaatca gatattataa cagaggataa acttcaagaa 180

aaagcccaaa aatggcatca gttacaatca aaaaggtttg cagacaaaag aaagttgggt 240

tttgtagaag cccaaaaaga agatatgcca ccagagcata ttagaaaaat tataagggat 300

catggggata tgagcagtcg taaatacaga catgataaaa gagtttattt aggagcttta 360

aaatatatgc cccatgcagt tatgaaatta ttggaaaata tgcctatgcc ttgggagcaa 420

attagagatg tgaaagtttt atatcacatt acaggtgcta ttacatttgt aaatgaaatt 480

ccttgggttg ttgagcctat ttatattgct caatggggta ctatgtggat catgatgaga 540

agagaaaaac gggaccgtag acattttaaa agaatgaggt ttcctccttt tgatgatgaa 600

gaacctcctc tagattatgc tgataatgtt ttagatgttg aacctctaga agcaattcaa 660

attgaactag atgctgaaga agactctgca attgcaaagt ggttctacga tcacaaacca 720

cttgttggtt ccaaatttgt taatggctcc acatacagaa agtggaattt atccttgcca 780

atgatggcta ctttgtatcg gttggcaaat caattactta ctgatttagt agatacaaat 840

tatttttatt tgtttgatac aaaaagtttt tttactgcta aagctttaaa tatggctata 900

ccaggagggc caaaatttga accccttatt aaagatatga atccagctga tgaagattgg 960

aatgaattta atgatatcaa caaaattata attcgtcaac ctattagaac tgaatatagg 1020

atagcttttc cttacttgta taataatatg ccacattttg tacatctatc ctggtatcat 1080
gcacctaacg ttgtttacat aaaaaccgaa gatcctgatt tgcccgcgtt ttattttgat 1140
cccccttatca accccatatc tcatagacac gcggtgaaga gtttagaacc tttacccgag 1200
gatgacgagg aatatatttt acctgaaacg gtacaaccat tcttgcaaga aacgcctctg 1260
tataccgaca atactgctaa tggtatcgct ctactttggg cgccacgacc gtttaatatg 1320
agatcaggca gatgcagaag ggcgatagac gtaccattag taaaatcttg gtacatggag 1380
catgttccac caggtcaacc tgtgaaagtc cgcgtcagtt accaaaaatt actgaaatat 1440
tacgtactta acgctttaaa acacagagcc cccaaacctc agaaaaagcg gtatctgttt 1500
agatcattta aatcaacaaa attctttcaa accacaaccc ttgattgggt tgaagctggt 1560
ttacaagtct gcagacaagg ttataacatg ttaaatctgt taattcatag aaagaatttg 1620
aattacttac atttggatta caatttcaac ttgaaacctg ttaaaacatt gacaaccaag 1680
gagagaaaaa agtcgcgttt tggaaacgct ttccacttgt gccgtgaaat tcttcgtcta 1740
acaaaattaa taattgattc ccacgtacaa tatagattaa ataacgtgga cgcttttcaa 1800
ctgtctgacg gtttacaata catattcgcc catgtcggcc aacttactgg aatgtacaga 1860
tacaaataca aacttatgag gcaaatccgc atgtgcaaag atcttaagca tctggtgtac 1920
tatcgtttta acacaggtcc agttggtaaa ggtcctggtt gtggaatctg ggctccaggt 1980
tggagagtgt ggctgttctt tatgaggggt attactccgc ttcttgaaag atggctgggt 2040
aacttgttgt cgcgtcagtt cgagggtcgt cactctaaag gtgtggcaaa aacggtcacc 2100
aagcaaaggg ttgagtctca ctttgatctt gagttgagag catctgttat gcatgatatt 2160
gttgatatga tgcctgaagg cataaaacag aacaaggcta gaacaatttt gcagcatttg 2220
tccgaagctt ggagatgttg gaaggcgaat attccctgga aagtacctgg gctaccaatt 2280
cccatcgaaa acatgatcct tcgttacgtt aaaatgaaag ccgattggtg gacaaacacc 2340
gctcactata acagagaaag aatacgtaga ggagccactg tcgataaaac cgtctgcaaa 2400
aagaacttag gtcgcttaac caggctgtac cttaaagctg aacaagaaag acaacataac 2460
taccttaaag acggtcctta catttcccct gaagaagccg ttgccattta tacaactact 2520
gtgcattggt tggagtccag aagattcgct cccatacctt ttccaccgct ttcctataaa 2580
cacgacacca aactgctaat tttggctttg gaaaggctta aagaagcata cagcgtaaaa 2640
tccaggttaa atcaaagtca aagagaagaa cttggtctca ttgaacaagc ctacgataat 2700
ccacacgaag ctttatcgag aatcaaacgt catttgttga cacaaagagc ttttaaagaa 2760
acggggattg aatttatgga tttgtatagc cacttgatac cagtgtatga tgttgaaccc 2820
ttagaaaaaa ttacagatgc ctatttagat caatacettt ggtatgaagc cgataaaaga 2880
agattgtttc cgccatggat caaacctgca gacacggaac caccgccgtt acttgtatac 2940

aaatggtgtc aaggcataaa caaccttcag gaggtttggg acgtaaacga gggcgaatgt 3000
aacgttttgc tggaatcgaa atttgaaaaa ctttatgaaa aaatcgattt gaccctgctg 3060
aacagacttt tgcgtctcat cgttgatcac aacatcgccg attacatgac ggccaagaac 3120
aacgttgtta ttaattacaa agacatgaat cacacaaaca gttacggaat cataagagga 3180
cttcagtttg cctcgtttgt cacgcagtat tacggtttgg tattggattt gttagttctc 3240
ggcctacaga gggcgtccga aatggcgggc ccacccccaaa tgcccaacga ctttttgact 3300
tttcaagatg ttgcaacaga aagctgtcat ccaatcagat tgtactgcag atatgtcgac 3360
agaatacaca tgttctttag gttttctgct gaagaagcta aagacctgat tcaaaggtat 3420
ttaacagaac atcctgatcc aaacaacgaa aacatcgttg gttataacaa caaaaaatgc 3480
tggcccaggg acgctaggat gcgtcttatg aaacacgatg ttaacttggg cagggccgta 3540
ttctgggaca ttaaaaatcg tctaccgaga tctgtaacta ctatccaatg ggagaacagt 3600
tttgtcagcg tttattcaaa ggataatcca aatcttttgt ttaacatgtc cggatttgag 3660
tgtaggattt tgcctaaatg tcgcacgcaa cacgaagaat tcacccatcg cgatggtgtt 3720
tggaatttgc aacatgaagg cagtaaagaa agaaccgctc aatgtttctt gagagttgat 3780
gatgaatcaa tgtcaaggtt tcacaacaga gtccgacaga ttttgatggc ttctggatct 3840
accacgttca caaagatcgt caacaaatgg aacacagccc ttataggtct aatgacatat 3900
tttagagaag ctgtggtaaa cacgcaagaa ctgctagact tgttggtaaa gtgcgagaac 3960
aaaattcaaa ctcgtatcaa aatcggtctt aactctaaaa tgcccagcag attcccgccc 4020
gtggtgttct atactcccaa agaattgggc gggttgggta tgttgtcgat gggtcacgtt 4080
ttaattccgc agtccgattt aagatggtct aaacagaccg acgttggcat cacacatttt 4140
cgttcaggta tgagccacga cgaggaccag ctaattccca atttgtatcg ttacatacag 4200
ccgtgggagt cggagtttat cgactcccag cgtgtctggg ccgagtacgc actcaaaaga 4260
caggaagcaa atgcgcaaaa caggcgtttg acattggaag atttggaaga ttcctgggat 4320
cgcggtattc ccaggataaa cacgctcttc cagaaggaca ggcacacctt ggcttacgac 4380
aagggctgga gaatccgaac ggagttcaag cagtatcaag ttttgaaaca gaatccgttc 4440
tggtggactc accaaagaca cgatggcaaa ctttggaact tgaataacta cagaacagac 4500
atgattcaag cgttgggagg tgttgaaggt attctagaac acactttgtt caaaggtaca 4560
tattttccca cttgggaagg tcttttctgg gaaaaggctt ctggttttga ggaatccatg 4620
aagtacaaaa agctcaccaa cgcccaaaga tccggtttga accagattcc caatcgtaga 4680
tttacgcttt ggtggtcacc tacaattaac agggccaacg tttacgttgg atttcaggta 4740
caactcgatt tgaccggtat ttttatgcac ggtaaaattc caactctcaa aatctctttg 4800

attcaaatat tcagggctca cttgtggcag aaggtccatg agtcgatagt catggatctt 4860
tgtcaggtct ttgatcaaga attggacgct ttggaaattg aaacagttca aaaagaaaca 4920
atccatccca gaaagtcata caaaatgaac tcgtcttgtg ccgatatttt actgttctct 4980
gcttacaaat ggaatgtatc cagaccgtcc ttacttgcag acacaaaaga taccatggac 5040
aacacaacca cccaaaaata ctggatagac gtgcaactga gatggggtga ttacgattcc 5100
catgacgttg agcgttacgc ccgtgccaaa ttcttggatt acaccacaga caacatgtcc 5160
atctatccat caccaactgg tgtattgatc gccatagatt tggcctacaa cttgcacagc 5220
gcctacggaa actggttccc tggttgcaaa cctttgattc aacaggcgat ggcaaaaata 5280
atgaaggcga accccgcgct atatgtatta agagagcgta tccgtaaagc tctccaattg 5340
tactctagtg aacctactga accctacttg tcgagtcaaa attacggaga attgttctcg 5400
aatcaaatca tttggttcgt tgatgacacc aatgtgtacc gtgtcaccat ccacaagact 5460
tttgagggaa atttgacgac gaagccaatc aatggcgcta tatttatttt taaccccaga 5520
actggacagt tgttcttgaa aattattcat acttctgtat gggctggaca aaaacgtttg 5580
ggacaattgg caaaatggaa gaccgccgaa gaagtagccg ctctaattcg ttcgcttccc 5640
gtagaagaac aacccaaaca aatcatcgtt accagaaaag gaatgttgga tcctcttgaa 5700
gtgcatcttc ttgatttccc caacatcgtt atcaaaggat ctgaactaca actgcctttc 5760
caggcttgcc tcaagataga aaagttcggc gatttgattt tgaaagctac cgaacctcag 5820
atggttctgt tcaatcttta cgacgattgg ttgaagacca tttcgtctta caccgccttc 5880
tccaggttga ttttgatatt aagggcatta cacgtcaata cagaaagaac taaggttatt 5940
cttaaacccg acaaaaccac aattaccgag ccgcatcata tttggcctac gctttctgac 6000
gatgaatgga ttaaagttga agtgcaactt aaggatctta ttttggcgga ttacggaaaa 6060
aagaacaacg tgaacgttgc ttctctaacc caatcagaaa ttcgcgatat tattctcggt 6120
atggagatca gcgcgccatc tgctcagaga caacagatcg ccgagatcga aaagcaaacg 6180
aaggaacagt cgcaactcac tgctactact acaagaactg tcaacaaaca cggcgatgaa 6240
attattacca gcaccacgag caattacgag acgcagacgt tcaactcgaa gactgaatgg 6300
agagtgaggg caatttcagc aactaatttg catttgagga caaaccacat ttatgtcagt 6360
tcggatgata ttaaagagac tggatacacc tatattttgc ccaagaacgt cttgaagaag 6420
tttgtcacca tttccgatct tagagcacaa atttgcggct ttttgtatgg cgtcagcccg 6480
cccgacaatc ctcaagtgaa ggaactgcgc tgtttagtgc tgccacccca atggggcaca 6540
catcaaacgg ttcacatacc acacacgccg ccaaaccatc cgttcctcaa ggacatggaa 6600
cctctaggct ggatacacac acaacccaac gagttgcctc aactgtcacc tcaggatatc 6660
accaatcatg ccaaactgat ggccgataat cccgcttggg atggtgaaaa gaccgttatc 6720

attacatgct catttacgcc cggctcttgt tcgttgacag cgtataagtt aacgccctct 6780

gggttcgagt ggggcagaca aaatactgat aaaggtaaca atcccaaagg gtacttgccc 6840

agtcactacg aaaaggttca gatgttgctg tctgataggt ttctagggtt ctttatggta 6900

cctacgcagg ggtcttggaa ctacaacttt atgggtgtca gacacgaccc aagcatgaag 6960

tatgaattgc aattagcaaa tccaaaagag ttttaccatg aagttcacag gccggctcat 7020

ttcctcaact tttcgtcttt ggaagacgga gatggtgctg gtgccgatcg cgaagatgca 7080

ttcgcttaa 7089

<210> 6
<211> 2362
<212> PRT
<213> Meligethes aeneus

<400> 6

Met Ser Leu Pro Pro Tyr Leu Leu Gly Pro Asn Pro Trp Leu Met Ala
1               5                   10                  15

Gln Gln Gln Leu Ala Ala Ala His Ala Gln Ala Gln Ala Ala Ala Ala
            20                  25                  30

Gln Ala His Ala Gln Ala Leu Gln Gln Gln Ile Pro Pro Pro His Pro
        35                  40                  45

Lys Ser Asp Ile Ile Thr Glu Asp Lys Leu Gln Glu Lys Ala Gln Lys
    50                  55                  60

Trp His Gln Leu Gln Ser Lys Arg Phe Ala Asp Lys Arg Lys Leu Gly
65                  70                  75                  80

Phe Val Glu Ala Gln Lys Glu Asp Met Pro Pro Glu His Ile Arg Lys
                85                  90                  95

Ile Ile Arg Asp His Gly Asp Met Ser Ser Arg Lys Tyr Arg His Asp
            100                 105                 110

Lys Arg Val Tyr Leu Gly Ala Leu Lys Tyr Met Pro His Ala Val Met
        115                 120                 125

Lys Leu Leu Glu Asn Met Pro Met Pro Trp Glu Gln Ile Arg Asp Val
    130                 135                 140

Lys Val Leu Tyr His Ile Thr Gly Ala Ile Thr Phe Val Asn Glu Ile
145                 150                 155                 160

```
Pro Trp Val Val Glu Pro Ile Tyr Ile Ala Gln Trp Gly Thr Met Trp
                165                 170                 175

Ile Met Met Arg Arg Glu Lys Arg Asp Arg Arg His Phe Lys Arg Met
            180                 185                 190

Arg Phe Pro Pro Phe Asp Asp Glu Glu Pro Pro Leu Asp Tyr Ala Asp
        195                 200                 205

Asn Val Leu Asp Val Glu Pro Leu Glu Ala Ile Gln Ile Glu Leu Asp
    210                 215                 220

Ala Glu Glu Asp Ser Ala Ile Ala Lys Trp Phe Tyr Asp His Lys Pro
225                 230                 235                 240

Leu Val Gly Ser Lys Phe Val Asn Gly Ser Thr Tyr Arg Lys Trp Asn
                245                 250                 255

Leu Ser Leu Pro Met Met Ala Thr Leu Tyr Arg Leu Ala Asn Gln Leu
            260                 265                 270

Leu Thr Asp Leu Val Asp Thr Asn Tyr Phe Tyr Leu Phe Asp Thr Lys
        275                 280                 285

Ser Phe Phe Thr Ala Lys Ala Leu Asn Met Ala Ile Pro Gly Gly Pro
    290                 295                 300

Lys Phe Glu Pro Leu Ile Lys Asp Met Asn Pro Ala Asp Glu Asp Trp
305                 310                 315                 320

Asn Glu Phe Asn Asp Ile Asn Lys Ile Ile Ile Arg Gln Pro Ile Arg
                325                 330                 335

Thr Glu Tyr Arg Ile Ala Phe Pro Tyr Leu Tyr Asn Asn Met Pro His
            340                 345                 350

Phe Val His Leu Ser Trp Tyr His Ala Pro Asn Val Val Tyr Ile Lys
        355                 360                 365

Thr Glu Asp Pro Asp Leu Pro Ala Phe Tyr Phe Asp Pro Leu Ile Asn
    370                 375                 380

Pro Ile Ser His Arg His Ala Val Lys Ser Leu Glu Pro Leu Pro Glu
385                 390                 395                 400

Asp Asp Glu Glu Tyr Ile Leu Pro Glu Thr Val Gln Pro Phe Leu Gln
                405                 410                 415
```

```
Glu Thr Pro Leu Tyr Thr Asp Asn Thr Ala Asn Gly Ile Ala Leu Leu
            420                 425                 430

Trp Ala Pro Arg Pro Phe Asn Met Arg Ser Gly Arg Cys Arg Arg Ala
        435                 440                 445

Ile Asp Val Pro Leu Val Lys Ser Trp Tyr Met Glu His Val Pro Pro
    450                 455                 460

Gly Gln Pro Val Lys Val Arg Val Ser Tyr Gln Lys Leu Leu Lys Tyr
465                 470                 475                 480

Tyr Val Leu Asn Ala Leu Lys His Arg Ala Pro Lys Pro Gln Lys Lys
                485                 490                 495

Arg Tyr Leu Phe Arg Ser Phe Lys Ser Thr Lys Phe Phe Gln Thr Thr
            500                 505                 510

Thr Leu Asp Trp Val Glu Ala Gly Leu Gln Val Cys Arg Gln Gly Tyr
        515                 520                 525

Asn Met Leu Asn Leu Leu Ile His Arg Lys Asn Leu Asn Tyr Leu His
    530                 535                 540

Leu Asp Tyr Asn Phe Asn Leu Lys Pro Val Lys Thr Leu Thr Thr Lys
545                 550                 555                 560

Glu Arg Lys Lys Ser Arg Phe Gly Asn Ala Phe His Leu Cys Arg Glu
                565                 570                 575

Ile Leu Arg Leu Thr Lys Leu Ile Ile Asp Ser His Val Gln Tyr Arg
            580                 585                 590

Leu Asn Asn Val Asp Ala Phe Gln Leu Ser Asp Gly Leu Gln Tyr Ile
        595                 600                 605

Phe Ala His Val Gly Gln Leu Thr Gly Met Tyr Arg Tyr Lys Tyr Lys
    610                 615                 620

Leu Met Arg Gln Ile Arg Met Cys Lys Asp Leu Lys His Leu Val Tyr
625                 630                 635                 640

Tyr Arg Phe Asn Thr Gly Pro Val Gly Lys Gly Pro Gly Cys Gly Ile
                645                 650                 655

Trp Ala Pro Gly Trp Arg Val Trp Leu Phe Phe Met Arg Gly Ile Thr
            660                 665                 670
```

Pro Leu Leu Glu Arg Trp Leu Gly Asn Leu Leu Ser Arg Gln Phe Glu
675 680 685

Gly Arg His Ser Lys Gly Val Ala Lys Thr Val Thr Lys Gln Arg Val
690 695 700

Glu Ser His Phe Asp Leu Glu Leu Arg Ala Ser Val Met His Asp Ile
705 710 715 720

Val Asp Met Met Pro Glu Gly Ile Lys Gln Asn Lys Ala Arg Thr Ile
725 730 735

Leu Gln His Leu Ser Glu Ala Trp Arg Cys Trp Lys Ala Asn Ile Pro
740 745 750

Trp Lys Val Pro Gly Leu Pro Ile Pro Ile Glu Asn Met Ile Leu Arg
755 760 765

Tyr Val Lys Met Lys Ala Asp Trp Trp Thr Asn Thr Ala His Tyr Asn
770 775 780

Arg Glu Arg Ile Arg Arg Gly Ala Thr Val Asp Lys Thr Val Cys Lys
785 790 795 800

Lys Asn Leu Gly Arg Leu Thr Arg Leu Tyr Leu Lys Ala Glu Gln Glu
805 810 815

Arg Gln His Asn Tyr Leu Lys Asp Gly Pro Tyr Ile Ser Pro Glu Glu
820 825 830

Ala Val Ala Ile Tyr Thr Thr Thr Val His Trp Leu Glu Ser Arg Arg
835 840 845

Phe Ala Pro Ile Pro Phe Pro Pro Leu Ser Tyr Lys His Asp Thr Lys
850 855 860

Leu Leu Ile Leu Ala Leu Glu Arg Leu Lys Glu Ala Tyr Ser Val Lys
865 870 875 880

Ser Arg Leu Asn Gln Ser Gln Arg Glu Glu Leu Gly Leu Ile Glu Gln
885 890 895

Ala Tyr Asp Asn Pro His Glu Ala Leu Ser Arg Ile Lys Arg His Leu
900 905 910

Leu Thr Gln Arg Ala Phe Lys Glu Thr Gly Ile Glu Phe Met Asp Leu

```
        915                 920                 925

Tyr Ser His Leu Ile Pro Val Tyr Asp Val Glu Pro Leu Glu Lys Ile
    930                 935                 940

Thr Asp Ala Tyr Leu Asp Gln Tyr Leu Trp Tyr Glu Ala Asp Lys Arg
945                 950                 955                 960

Arg Leu Phe Pro Pro Trp Ile Lys Pro Ala Asp Thr Glu Pro Pro Pro
                965                 970                 975

Leu Leu Val Tyr Lys Trp Cys Gln Gly Ile Asn Asn Leu Gln Glu Val
            980                 985                 990

Trp Asp Val Asn Glu Gly Glu Cys  Asn Val Leu Leu Glu  Ser Lys Phe
        995                 1000                 1005

Glu Lys  Leu Tyr Glu Lys Ile  Asp Leu Thr Leu Leu  Asn Arg Leu
    1010                 1015                 1020

Leu Arg  Leu Ile Val Asp His  Asn Ile Ala Asp Tyr  Met Thr Ala
    1025                 1030                 1035

Lys Asn  Asn Val Val Ile Asn  Tyr Lys Asp Met Asn  His Thr Asn
    1040                 1045                 1050

Ser Tyr  Gly Ile Ile Arg Gly  Leu Gln Phe Ala Ser  Phe Val Thr
    1055                 1060                 1065

Gln Tyr  Tyr Gly Leu Val Leu  Asp Leu Leu Val Leu  Gly Leu Gln
    1070                 1075                 1080

Arg Ala  Ser Glu Met Ala Gly  Pro Pro Gln Met Pro  Asn Asp Phe
    1085                 1090                 1095

Leu Thr  Phe Gln Asp Val Ala  Thr Glu Ser Cys His  Pro Ile Arg
    1100                 1105                 1110

Leu Tyr  Cys Arg Tyr Val Asp  Arg Ile His Met Phe  Phe Arg Phe
    1115                 1120                 1125

Ser Ala  Glu Glu Ala Lys Asp  Leu Ile Gln Arg Tyr  Leu Thr Glu
    1130                 1135                 1140

His Pro  Asp Pro Asn Asn Glu  Asn Ile Val Gly Tyr  Asn Asn Lys
    1145                 1150                 1155
```

```
Lys Cys  Trp Pro Arg Asp Ala  Arg Met Arg Leu Met  Lys His Asp
    1160                 1165                 1170

Val Asn  Leu Gly Arg Ala Val  Phe Trp Asp Ile Lys  Asn Arg Leu
    1175                 1180                 1185

Pro Arg  Ser Val Thr Thr Ile  Gln Trp Glu Asn Ser  Phe Val Ser
    1190                 1195                 1200

Val Tyr  Ser Lys Asp Asn Pro  Asn Leu Leu Phe Asn  Met Ser Gly
    1205                 1210                 1215

Phe Glu  Cys Arg Ile Leu Pro  Lys Cys Arg Thr Gln  His Glu Glu
    1220                 1225                 1230

Phe Thr  His Arg Asp Gly Val  Trp Asn Leu Gln His  Glu Gly Ser
    1235                 1240                 1245

Lys Glu  Arg Thr Ala Gln Cys  Phe Leu Arg Val Asp  Asp Glu Ser
    1250                 1255                 1260

Met Ser  Arg Phe His Asn Arg  Val Arg Gln Ile Leu  Met Ala Ser
    1265                 1270                 1275

Gly Ser  Thr Thr Phe Thr Lys  Ile Val Asn Lys Trp  Asn Thr Ala
    1280                 1285                 1290

Leu Ile  Gly Leu Met Thr Tyr  Phe Arg Glu Ala Val  Val Asn Thr
    1295                 1300                 1305

Gln Glu  Leu Leu Asp Leu Leu  Val Lys Cys Glu Asn  Lys Ile Gln
    1310                 1315                 1320

Thr Arg  Ile Lys Ile Gly Leu  Asn Ser Lys Met Pro  Ser Arg Phe
    1325                 1330                 1335

Pro Pro  Val Val Phe Tyr Thr  Pro Lys Glu Leu Gly  Gly Leu Gly
    1340                 1345                 1350

Met Leu  Ser Met Gly His Val  Leu Ile Pro Gln Ser  Asp Leu Arg
    1355                 1360                 1365

Trp Ser  Lys Gln Thr Asp Val  Gly Ile Thr His Phe  Arg Ser Gly
    1370                 1375                 1380

Met Ser  His Asp Glu Asp Gln  Leu Ile Pro Asn Leu  Tyr Arg Tyr
    1385                 1390                 1395
```

Ile Gln Pro Trp Glu Ser Glu Phe Ile Asp Ser Gln Arg Val Trp
1400 1405 1410

Ala Glu Tyr Ala Leu Lys Arg Gln Glu Ala Asn Ala Gln Asn Arg
1415 1420 1425

Arg Leu Thr Leu Glu Asp Leu Glu Asp Ser Trp Asp Arg Gly Ile
1430 1435 1440

Pro Arg Ile Asn Thr Leu Phe Gln Lys Asp Arg His Thr Leu Ala
1445 1450 1455

Tyr Asp Lys Gly Trp Arg Ile Arg Thr Glu Phe Lys Gln Tyr Gln
1460 1465 1470

Val Leu Lys Gln Asn Pro Phe Trp Trp Thr His Gln Arg His Asp
1475 1480 1485

Gly Lys Leu Trp Asn Leu Asn Asn Tyr Arg Thr Asp Met Ile Gln
1490 1495 1500

Ala Leu Gly Gly Val Glu Gly Ile Leu Glu His Thr Leu Phe Lys
1505 1510 1515

Gly Thr Tyr Phe Pro Thr Trp Glu Gly Leu Phe Trp Glu Lys Ala
1520 1525 1530

Ser Gly Phe Glu Glu Ser Met Lys Tyr Lys Lys Leu Thr Asn Ala
1535 1540 1545

Gln Arg Ser Gly Leu Asn Gln Ile Pro Asn Arg Arg Phe Thr Leu
1550 1555 1560

Trp Trp Ser Pro Thr Ile Asn Arg Ala Asn Val Tyr Val Gly Phe
1565 1570 1575

Gln Val Gln Leu Asp Leu Thr Gly Ile Phe Met His Gly Lys Ile
1580 1585 1590

Pro Thr Leu Lys Ile Ser Leu Ile Gln Ile Phe Arg Ala His Leu
1595 1600 1605

Trp Gln Lys Val His Glu Ser Ile Val Met Asp Leu Cys Gln Val
1610 1615 1620

Phe Asp Gln Glu Leu Asp Ala Leu Glu Ile Glu Thr Val Gln Lys
1625 1630 1635

Glu Thr Ile His Pro Arg Lys Ser Tyr Lys Met Asn Ser Ser Cys
1640 1645 1650

Ala Asp Ile Leu Leu Phe Ser Ala Tyr Lys Trp Asn Val Ser Arg
1655 1660 1665

Pro Ser Leu Leu Ala Asp Thr Lys Asp Thr Met Asp Asn Thr Thr
1670 1675 1680

Thr Gln Lys Tyr Trp Ile Asp Val Gln Leu Arg Trp Gly Asp Tyr
1685 1690 1695

Asp Ser His Asp Val Glu Arg Tyr Ala Arg Ala Lys Phe Leu Asp
1700 1705 1710

Tyr Thr Thr Asp Asn Met Ser Ile Tyr Pro Ser Pro Thr Gly Val
1715 1720 1725

Leu Ile Ala Ile Asp Leu Ala Tyr Asn Leu His Ser Ala Tyr Gly
1730 1735 1740

Asn Trp Phe Pro Gly Cys Lys Pro Leu Ile Gln Gln Ala Met Ala
1745 1750 1755

Lys Ile Met Lys Ala Asn Pro Ala Leu Tyr Val Leu Arg Glu Arg
1760 1765 1770

Ile Arg Lys Ala Leu Gln Leu Tyr Ser Ser Glu Pro Thr Glu Pro
1775 1780 1785

Tyr Leu Ser Ser Gln Asn Tyr Gly Glu Leu Phe Ser Asn Gln Ile
1790 1795 1800

Ile Trp Phe Val Asp Asp Thr Asn Val Tyr Arg Val Thr Ile His
1805 1810 1815

Lys Thr Phe Glu Gly Asn Leu Thr Thr Lys Pro Ile Asn Gly Ala
1820 1825 1830

Ile Phe Ile Phe Asn Pro Arg Thr Gly Gln Leu Phe Leu Lys Ile
1835 1840 1845

Ile His Thr Ser Val Trp Ala Gly Gln Lys Arg Leu Gly Gln Leu
1850 1855 1860

Ala Lys Trp Lys Thr Ala Glu Glu Val Ala Ala Leu Ile Arg Ser

```
    1865                1870                1875

Leu Pro  Val Glu Glu Gln Pro  Lys Gln Ile Ile Val  Thr Arg Lys
    1880                1885                1890

Gly Met  Leu Asp Pro Leu Glu  Val His Leu Leu Asp  Phe Pro Asn
    1895                1900                1905

Ile Val  Ile Lys Gly Ser Glu  Leu Gln Leu Pro Phe  Gln Ala Cys
    1910                1915                1920

Leu Lys  Ile Glu Lys Phe Gly  Asp Leu Ile Leu Lys  Ala Thr Glu
    1925                1930                1935

Pro Gln  Met Val Leu Phe Asn  Leu Tyr Asp Asp Trp  Leu Lys Thr
    1940                1945                1950

Ile Ser  Ser Tyr Thr Ala Phe  Ser Arg Leu Ile Leu  Ile Leu Arg
    1955                1960                1965

Ala Leu  His Val Asn Thr Glu  Arg Thr Lys Val Ile  Leu Lys Pro
    1970                1975                1980

Asp Lys  Thr Thr Ile Thr Glu  Pro His His Ile Trp  Pro Thr Leu
    1985                1990                1995

Ser Asp  Asp Glu Trp Ile Lys  Val Glu Val Gln Leu  Lys Asp Leu
    2000                2005                2010

Ile Leu  Ala Asp Tyr Gly Lys  Lys Asn Asn Val Asn  Val Ala Ser
    2015                2020                2025

Leu Thr  Gln Ser Glu Ile Arg  Asp Ile Ile Leu Gly  Met Glu Ile
    2030                2035                2040

Ser Ala  Pro Ser Ala Gln Arg  Gln Gln Ile Ala Glu  Ile Glu Lys
    2045                2050                2055

Gln Thr  Lys Glu Gln Ser Gln  Leu Thr Ala Thr Thr  Thr Arg Thr
    2060                2065                2070

Val Asn  Lys His Gly Asp Glu  Ile Ile Thr Ser Thr  Thr Ser Asn
    2075                2080                2085

Tyr Glu  Thr Gln Thr Phe Asn  Ser Lys Thr Glu Trp  Arg Val Arg
    2090                2095                2100
```

Ala Ile Ser Ala Thr Asn Leu His Leu Arg Thr Asn His Ile Tyr
2105 2110 2115

Val Ser Ser Asp Asp Ile Lys Glu Thr Gly Tyr Thr Tyr Ile Leu
2120 2125 2130

Pro Lys Asn Val Leu Lys Lys Phe Val Thr Ile Ser Asp Leu Arg
2135 2140 2145

Ala Gln Ile Cys Gly Phe Leu Tyr Gly Val Ser Pro Pro Asp Asn
2150 2155 2160

Pro Gln Val Lys Glu Leu Arg Cys Leu Val Leu Pro Pro Gln Trp
2165 2170 2175

Gly Thr His Gln Thr Val His Ile Pro His Thr Pro Pro Asn His
2180 2185 2190

Pro Phe Leu Lys Asp Met Glu Pro Leu Gly Trp Ile His Thr Gln
2195 2200 2205

Pro Asn Glu Leu Pro Gln Leu Ser Pro Gln Asp Ile Thr Asn His
2210 2215 2220

Ala Lys Leu Met Ala Asp Asn Pro Ala Trp Asp Gly Glu Lys Thr
2225 2230 2235

Val Ile Ile Thr Cys Ser Phe Thr Pro Gly Ser Cys Ser Leu Thr
2240 2245 2250

Ala Tyr Lys Leu Thr Pro Ser Gly Phe Glu Trp Gly Arg Gln Asn
2255 2260 2265

Thr Asp Lys Gly Asn Asn Pro Lys Gly Tyr Leu Pro Ser His Tyr
2270 2275 2280

Glu Lys Val Gln Met Leu Leu Ser Asp Arg Phe Leu Gly Phe Phe
2285 2290 2295

Met Val Pro Thr Gln Gly Ser Trp Asn Tyr Asn Phe Met Gly Val
2300 2305 2310

Arg His Asp Pro Ser Met Lys Tyr Glu Leu Gln Leu Ala Asn Pro
2315 2320 2325

Lys Glu Phe Tyr His Glu Val His Arg Pro Ala His Phe Leu Asn
2330 2335 2340

Phe Ser Ser Leu Glu Asp Gly Asp Gly Ala Gly Ala Asp Arg Glu
2345 2350 2355

Asp Ala Phe Ala
2360

<210> 7
<211> 488
<212> DNA
<213> Diabrotica virgifera

<400> 7
caatttacaa gatgtgtggg atgtgaatga aggggagtgt aacgtgttac tggaatctaa 60

gtttgaaaaa ctatatgaaa agatcgattt gactctactt aacagacttc tccgattgat 120

agtggaccac aacatagctg attacatgac cgctaagaat aacgtcgtta taaactacaa 180

agatatgaat cacaccaaca gttacggaat tattcgagga ttgcagtttg cctcgttcat 240

tactcagtat tatggtctgg ttttggatct gctggtattg ggtctgcaga gagccagtga 300

aatggctggg ccacctcaaa tgcctaacga tttcttgacg ttccaagatg ttcaatccga 360

aacgtgccat cctattcggc tttactgcag atatgtggac agaattcata tgtttttcag 420

attttctgca gaagaagcca aagatttgat ccaaagatac ctaacagaac atccagatcc 480

taataatg 488

<210> 8
<211> 452
<212> DNA
<213> Diabrotica virgifera

<400> 8
cggcttaatc cgcggcctcc agttcagcag tttcatattc caatattatg ctctggtcat 60

agatcttctg attttagggc tgacgcgagc caatgaactt gccggcagta taggtggcgg 120

cggaggcgga ggtttcgcta atctcaaaga tcgcgaaacg gagataaaac atcccatccg 180

cttgtattgc cgatatatag atgaaatatg gatctgcttc aaattcacca aagaggagtc 240

tcgtagcttg attcaaaggt atttgacgga gaatccaacc gctagtcagc agctctccac 300

tgaagaaggc atcgactacc ccatcaaaaa gtgttggcct aaagactgcc gaatgagaaa 360

aatgaaattc gacgttaata tcggacgagc cgttttctgg gagattcaga aacgtctacc 420

gagaagttta gctgagctga gttggggcaa ag 452

<210> 9
<211> 336
<212> DNA
<213> Diabrotica virgifera

<400> 9

```
ctaagaataa cgtcgttata aactacaaag atatgaatca caccaacagt tacggaatta      60

ttcgaggatt gcagtttgcc tcgttcatta ctcagtatta tggtctggtt ttggatctgc     120

tggtattggg tctgcagaga gccagtgaaa tggctgggcc acctcaaatg cctaacgatt     180

tcttgacgtt ccaagatgtt caatccgaaa cgtgccatcc tattcggctt tactgcagat     240

atgtggacag aattcatatg tttttcagat tttctgcaga agaagccaaa gatttgatcc     300

aaagatacct aacagaacat ccagatccta ataatg                               336


<210>  10
<211>  120
<212>  DNA
<213>  Diabrotica virgifera

<400>  10
ctaagaataa cgtcgttata aactacaaag atatgaatca caccaacagt tacggaatta      60

ttcgaggatt gcagtttgcc tcgttcatta ctcagtatta tggtctggtt ttggatctgc     120


<210>  11
<211>  186
<212>  DNA
<213>  Diabrotica virgifera

<400>  11
tggctgggcc acctcaaatg cctaacgatt tcttgacgtt ccaagatgtt caatccgaaa      60

cgtgccatcc tattcggctt tactgcagat atgtggacag aattcatatg tttttcagat     120

tttctgcaga agaagccaaa gatttgatcc aaagatacct aacagaacat ccagatccta     180

ataatg                                                                186


<210>  12
<211>  385
<212>  DNA
<213>  Meligethes aeneus

<400>  12
aaatggaaga ccgccgaaga agtagccgct ctaattcgtt cgcttcccgt agaagaacaa      60

cccaaacaaa tcatcgttac cagaaaagga atgttggatc ctcttgaagt gcatcttctt     120

gatttcccca acatcgttat caaaggatct gaactacaac tgcctttcca ggcttgcctc     180

aagatagaaa agttcggcga tttgattttg aaagctaccg aacctcagat ggttctgttc     240

aatctttacg acgattggtt gaagaccatt tcgtcttaca ccgccttctc caggttgatt     300

ttgatattaa gggcattaca cgtcaataca gaaagaacta aggttattct taaacccgac     360

aaaaccacaa ttaccgagcc gcatc                                           385


<210>  13
<211>  24
<212>  DNA
```

<213> Artificial Sequence

<220>
<223> synthesized promoter oligonucleotide, T7 Promoter

<400> 13
ttaatacgac tcactatagg gaga 24

<210> 14
<211> 503
<212> DNA
<213> Artificial Sequence

<220>
<223> synthesized partial coding region, YFP

<400> 14
caccatgggc tccagcggcg ccctgctgtt ccacggcaag atcccctacg tggtggagat 60

ggagggcaat gtggatggcc acaccttcag catccgcggc aagggctacg gcgatgccag 120

cgtgggcaag gtggatgccc agttcatctg caccaccggc gatgtgcccg tgccctggag 180

caccctggtg accaccctga cctacggcgc ccagtgcttc gccaagtacg gccccgagct 240

gaaggatttc tacaagagct gcatgcccga tggctacgtg caggagcgca ccatcacctt 300

cgagggcgat ggcaatttca agacccgcgc cgaggtgacc ttcgagaatg gcagcgtgta 360

caatcgcgtg aagctgaatg gccagggctt caagaaggat ggccacgtgc tgggcaagaa 420

tctggagttc aatttcaccc cccactgcct gtacatctgg ggcgatcagg ccaatcacgg 480

cctgaagagc gccttcaaga tct 503

<210> 15
<211> 49
<212> DNA
<213> Artificial Sequence

<220>
<223> synthesized primer oligonucleotide

<400> 15
ttaatacgac tcactatagg gagacaattt acaagatgtg tgggatgtg 49

<210> 16
<211> 52
<212> DNA
<213> Artificial Sequence

<220>
<223> synthesized primer oligonucleotide

<400> 16
ttaatacgac tcactatagg gagacattat taggatctgg atgttctgtt ag 52

<210> 17
<211> 58

<212> DNA
<213> Artificial Sequence

<220>
<223> synthesized primer oligonucleotide

<400> 17
ttaatacgac tcactatagg gagacggctt aatccgcggc ctccagttca gcagtttc 58

<210> 18
<211> 51
<212> DNA
<213> Artificial Sequence

<220>
<223> synthesized primer oligonucleotide

<400> 18
ttaatacgac tcactatagg gagactttgc cccaactcag ctcagctaaa c 51

<210> 19
<211> 59
<212> DNA
<213> Artificial Sequence

<220>
<223> synthesized primer oligonucleotide

<400> 19
ttaatacgac tcactatagg gagactaaga ataacgtcgt tataaactac aaagatatg 59

<210> 20
<211> 53
<212> DNA
<213> Artificial Sequence

<220>
<223> synthesized primer oligonucleotide

<400> 20
ttaatacgac tcactatagg gagacattat taggatctgg atgttctgtt agg 53

<210> 21
<211> 47
<212> DNA
<213> Artificial Sequence

<220>
<223> synthesized primer oligonucleotide

<400> 21
ttaatacgac tcactatagg gagactaaga ataacgtcgt tataaac 47

<210> 22
<211> 50
<212> DNA
<213> Artificial Sequence

<220>
<223> Synthesized artificial sequence

<400> 22
ttaatacgac tcactatagg gagagcagat ccaaaaccag accataatac 50

<210> 23
<211> 44
<212> DNA
<213> Artificial Sequence

<220>
<223> synthesized primer oligonucleotide

<400> 23
ttaatacgac tcactatagg gagatggctg ggccacctca aatg 44

<210> 24
<211> 45
<212> DNA
<213> Artificial Sequence

<220>
<223> synthesized primer oligonucleotide

<220>
<221> misc_feature
<222> (393)..(393)
<223> n is a, c, g, or t

<220>
<221> misc_feature
<222> (394)..(394)
<223> n is a, c, g, or t

<220>
<221> misc_feature
<222> (395)..(395)
<223> n is a, c, g, or t

<400> 24
ttaatacgac tcactatagg gagagacatt attaggatct ggatg 45

<210> 25
<211> 43
<212> DNA
<213> Artificial Sequence

<220>
<223> Primer Ma-prp8_For

<400> 25
taatacgact cactataggg agaaaatgga agaccgccga aga 43

<210> 26
<211> 43
<212> DNA
<213> Artificial Sequence

<220>
<223> Primer Ma-prp8_Rev

<400> 26
taatacgact cactataggg agagatgcgg ctcggtaatt gtg 43

<210> 27
<211> 705
<212> DNA
<213> Artificial Sequence

<220>
<223> Synthesized artificial sequence, YFP protein coding sequence

<400> 27
atgtcatctg gagcacttct ctttcatggg aagattcctt acgttgtgga gatggaaggg 60

aatgttgatg gccacacctt tagcatacgt gggaaaggct acggagatgc ctcagtggga 120

aaggttgatg cacagttcat ctgcacaact ggtgatgttc ctgtgccttg gagcacactt 180

gtcaccactc tcacctatgg agcacagtgc tttgccaagt atggtccaga gttgaaggac 240

ttctacaagt cctgtatgcc agatggctat gtgcaagagc gcacaatcac ctttgaagga 300

gatggcaact tcaagactag ggctgaagtc acctttgaga atgggtctgt ctacaatagg 360

gtcaaactca atggtcaagg cttcaagaaa gatggtcatg tgttgggaaa gaacttggag 420

ttcaacttca ctccccactg cctctacatc tggggtgacc aagccaacca cggtctcaag 480

tcagccttca agatctgtca tgagattact ggcagcaaag gcgacttcat agtggctgac 540

cacacccaga tgaacactcc cattggtgga ggtccagttc atgttccaga gtatcatcac 600

atgtcttacc atgtgaaact ttccaaagat gtgacagacc acagagacaa catgtccttg 660

aaagaaactg tcagagctgt tgactgtcgc aagacctacc tttga 705

<210> 28
<211> 218
<212> DNA
<213> Diabrotica virgifera

<400> 28
tagctctgat gacagagccc atcgagtttc aagccaaaca gttgcataaa gctatcagcg 60

gattgggaac tgatgaaagt acaatmgtmg aaattttaag tgtmcacaac aacgatgaga 120

ttataagaat ttcccaggcc tatgaaggat tgtaccaacg mtcattggaa tctgatatca 180

aaggagatac ctcaggaaca ttaaaaaaga attattag 218

<210> 29
<211> 424
<212> DNA
<213> Diabrotica virgifera

<220>
<221> misc_feature
<222> (393)..(393)
<223> n is a, c, g, or t

<220>
<221> misc_feature
<222> (394)..(394)
<223> n is a, c, g, or t

<220>
<221> misc_feature
<222> (395)..(395)
<223> n is a, c, g, or t

<400> 29
ttgttacaag ctggagaact tctctttgct ggaaccgaag agtcagtatt taatgctgta 60

ttctgtcaaa gaaataaacc acaattgaat ttgatattcg acaaatatga agaaattgtt 120

gggcatccca ttgaaaaagc cattgaaaac gagttttcag gaaatgctaa acaagccatg 180

ttacacctta tccagagcgt aagagatcaa gttgcatatt tggtaaccag gctgcatgat 240

tcaatggcag gcgtcggtac tgacgataga actttaatca gaattgttgt ttcgagatct 300

gaaatcgatc tagaggaaat caaacaatgc tatgaagaaa tctacagtaa aaccttggct 360

gataggatag cggatgacac atctggcgac tannnaaaag ccttattagc cgttgttggt 420

taag 424

<210> 30
<211> 397
<212> DNA
<213> Diabrotica virgifera

<400> 30
agatgttggc tgcatctaga gaattacaca agttcttcca tgattgcaag gatgtactga 60

gcagaatagt ggaaaaacag gtatccatgt ctgatgaatt gggaagggac gcaggagctg 120

tcaatgccct tcaacgcaaa caccagaact tcctccaaga cctacaaaca ctccaatcga 180

acgtccaaca aatccaagaa gaatcagcta aacttcaagc tagctatgcc ggtgatagag 240

ctaaagaaat caccaacagg gagcaggaag tggtagcagc ctgggcagcc ttgcagatcg 300

cttgcgatca gagacacgga aaattgagcg atactggtga tctattcaaa ttctttaact 360

tggtacgaac gttgatgcag tggatggacg aatggac 397

<210> 31
<211> 490
<212> DNA
<213> Diabrotica virgifera

<400> 31
gcagatgaac accagcgaga aaccaagaga tgttagtggt gttgaattgt tgatgaacaa 60

ccatcagaca ctcaaggctg agatcgaagc cagagaagac aactttacgg cttgtatttc 120

tttaggaaag gaattgttga gccgtaatca ctatgctagt gctgatatta aggataaatt 180

ggtcgcgttg acgaatcaaa ggaatgctgt actacagagg tgggaagaaa gatgggagaa 240

cttgcaactc atcctcgagg tataccaatt cgccagagat gcggccgtcg ccgaagcatg 300

gttgatcgca caagaacctt acttgatgag ccaagaacta ggacacacca ttgacgacgt 360

tgaaaacttg ataaagaaac acgaagcgtt cgaaaaatcg gcagcggcgc aagaagagag 420

attcagtgct ttggagagac tgacgacgtt cgaattgaga gaaataaaga ggaaacaaga 480

agctgcccag 490

<210> 32
<211> 330
<212> DNA
<213> Diabrotica virgifera

<400> 32
agtgaaatgt tagcaaatat aacatccaag tttcgtaatt gtacttgctc agttagaaaa 60

tattctgtag tttcactatc ttcaaccgaa aatagaataa atgtagaacc tcgcgaactt 120

gcctttcctc caaaatatca agaacctcga caagtttggt tggagagttt agatacgata 180

gacgacaaaa aattgggtat tcttgagctg catcctgatg tttttgctac taatccaaga 240

atagatatta tacatcaaaa tgttagatgg caaagtttat atagatatgt aagctatgct 300

catacaaagt caagatttga agtgagaggt 330

<210> 33
<211> 320
<212> DNA
<213> Diabrotica virgifera

<400> 33
caaagtcaag atttgaagtg agaggtggag gtcgaaaacc gtggccgcaa aagggattgg 60

gacgtgctcg acatggttca attagaagtc cactttggag aggtggagga gttgttcatg 120

gaccaaaatc tccaacccct catttttaca tgattccatt ctacacccgt ttgctgggtt 180

tgactagcgc actttcagta aaatttgccc aagatgactt gcacgttgtg gatagtctag 240

atctgccaac tgacgaacaa agttatatag aagagctggt caaaagccgc ttttggggggt 300

ccttcttgtt ttatttgtag 320

<210> 34
<211> 47
<212> DNA
<213> Artificial Sequence

<220>
<223> synthesized primer oligonucleotide

<400> 34

ttaatacgac tcactatagg gagacaccat gggctccagc ggcgccc 47

<210> 35
<211> 23
<212> DNA
<213> Artificial Sequence

<220>
<223> synthesized primer oligonucleotide

<400> 35
agatcttgaa ggcgctcttc agg 23

<210> 36
<211> 23
<212> DNA
<213> Artificial Sequence

<220>
<223> synthesized primer oligonucleotide

<400> 36
caccatgggc tccagcggcg ccc 23

<210> 37
<211> 47
<212> DNA
<213> Artificial Sequence

<220>
<223> synthesized primer oligonucleotide

<400> 37
ttaatacgac tcactatagg gagaagatct tgaaggcgct cttcagg 47

<210> 38
<211> 46
<212> DNA
<213> Artificial Sequence

<220>
<223> synthesized primer oligonucleotide

<400> 38
ttaatacgac tcactatagg gagagctcca acagtggttc cttatc 46

<210> 39
<211> 29
<212> DNA
<213> Artificial Sequence

<220>
<223> synthesized primer oligonucleotide

<400> 39
ctaataattc ttttttaatg ttcctgagg 29

<210> 40
<211> 22
<212> DNA
<213> Artificial Sequence

<220>
<223> synthesized primer oligonucleotide

<400> 40
gctccaacag tggttcctta tc 22

<210> 41
<211> 53
<212> DNA
<213> Artificial Sequence

<220>
<223> synthesized primer oligonucleotide

<400> 41
ttaatacgac tcactatagg gagactaata attctttttt aatgttcctg agg 53

<210> 42
<211> 48
<212> DNA
<213> Artificial Sequence

<220>
<223> synthesized primer oligonucleotide

<400> 42
ttaatacgac tcactatagg gagattgtta caagctggag aacttctc 48

<210> 43
<211> 24
<212> DNA
<213> Artificial Sequence

<220>
<223> synthesized primer oligonucleotide

<400> 43
cttaaccaac aacggctaat aagg 24

<210> 44
<211> 24
<212> DNA
<213> Artificial Sequence

<220>
<223> synthesized primer oligonucleotide

<400> 44
ttgttacaag ctggagaact tctc 24

<210> 45
<211> 48
<212> DNA

<213> Artificial Sequence

<220>
<223> synthesized primer oligonucleotide

<400> 45
ttaatacgac tcactatagg gagacttaac caacaacggc taataagg 48

<210> 46
<211> 47
<212> DNA
<213> Artificial Sequence

<220>
<223> synthesized primer oligonucleotide

<400> 46
ttaatacgac tcactatagg gagaagatgt tggctgcatc tagagaa 47

<210> 47
<211> 22
<212> DNA
<213> Artificial Sequence

<220>
<223> synthesized primer oligonucleotide

<400> 47
gtccattcgt ccatccactg ca 22

<210> 48
<211> 23
<212> DNA
<213> Artificial Sequence

<220>
<223> synthesized primer oligonucleotide

<400> 48
agatgttggc tgcatctaga gaa 23

<210> 49
<211> 46
<212> DNA
<213> Artificial Sequence

<220>
<223> synthesized primer oligonucleotide

<400> 49
ttaatacgac tcactatagg gagagtccat tcgtccatcc actgca 46

<210> 50
<211> 46
<212> DNA
<213> Artificial Sequence

<220>

<223> synthesized primer oligonucleotide

<400> 50
ttaatacgac tcactatagg gagagcagat gaacaccagc gagaaa 46

<210> 51
<211> 22
<212> DNA
<213> Artificial Sequence

<220>
<223> synthesized primer oligonucleotide

<400> 51
ctgggcagct tcttgtttcc tc 22

<210> 52
<211> 22
<212> DNA
<213> Artificial Sequence

<220>
<223> synthesized primer oligonucleotide

<400> 52
gcagatgaac accagcgaga aa 22

<210> 53
<211> 46
<212> DNA
<213> Artificial Sequence

<220>
<223> synthesized primer oligonucleotide

<400> 53
ttaatacgac tcactatagg gagactgggc agcttcttgt ttcctc 46

<210> 54
<211> 51
<212> DNA
<213> Artificial Sequence

<220>
<223> synthesized primer oligonucleotide

<400> 54
ttaatacgac tcactatagg gagaagtgaa atgttagcaa atataacatc c 51

<210> 55
<211> 26
<212> DNA
<213> Artificial Sequence

<220>
<223> synthesized primer oligonucleotide

<400> 55

acctctcact tcaaatcttg actttg 26

<210> 56
<211> 27
<212> DNA
<213> Artificial Sequence

<220>
<223> synthesized primer oligonucleotide

<400> 56
agtgaaatgt tagcaaatat aacatcc 27

<210> 57
<211> 50
<212> DNA
<213> Artificial Sequence

<220>
<223> synthesized primer oligonucleotide

<400> 57
ttaatacgac tcactatagg gagaacctct cacttcaaat cttgactttg 50

<210> 58
<211> 50
<212> DNA
<213> Artificial Sequence

<220>
<223> synthesized primer oligonucleotide

<400> 58
ttaatacgac tcactatagg gagacaaagt caagatttga agtgagaggt 50

<210> 59
<211> 25
<212> DNA
<213> Artificial Sequence

<220>
<223> synthesized primer oligonucleotide

<400> 59
ctacaaataa aacaagaagg acccc 25

<210> 60
<211> 26
<212> DNA
<213> Artificial Sequence

<220>
<223> synthesized primer oligonucleotide

<220>
<221> misc_feature
<222> (22)..(22)

<223> n is a, c, g, or t

<400> 60
caaagtcaag atttgaagtg agaggt 26

<210> 61
<211> 49
<212> DNA
<213> Artificial Sequence

<220>
<223> synthesized primer oligonucleotide

<400> 61
ttaatacgac tcactatagg gagactacaa ataaaacaag aaggacccc 49

<210> 62
<211> 1150
<212> DNA
<213> Zea mays

<400> 62
caacggggca gcactgcact gcactgcaac tgcgaatttc cgtcagcttg gagcggtcca 60

agcgccctgc gaagcaaact acgccgatgg cttcggcggc ggcgtgggag ggtccgacgg 120

ccgcggagct gaagacagcg ggggcggagg tgattcccgg cggcgtgcga gtgaaggggt 180

gggtcatcca gtcccacaaa ggccctatcc tcaacgccgc ctctctgcaa cgctttgaag 240

atgaacttca aacaacacat ttacctgaga tggtttttgg agagagtttc ttgtcacttc 300

aacatacaca aactggcatc aaatttcatt ttaatgcgct tgatgcactc aaggcatgga 360

agaaagaggc actgccacct gttgaggttc ctgctgcagc aaaatggaag ttcagaagta 420

agccttctga ccaggttata cttgactacg actatacatt tacgacacca tattgtggga 480

gtgatgctgt ggttgtgaac tctggcactc cacaaacaag tttagatgga tgcggcactt 540

tgtgttggga ggatactaat gatcggattg acattgttgc cctttcagca aaagaaccca 600

ttcttttcta cgacgaggtt atcttgtatg aagatgagtt agctgacaat ggtatctcat 660

ttcttactgt gcgagtgagg gtaatgccaa ctggttggtt tctgcttttg cgtttttggc 720

ttagagttga tggtgtactg atgaggttga gagacactcg gttacattgc ctgtttggaa 780

acggcgacgg agccaagcca gtggtacttc gtgagtgctg ctggagggaa gcaacatttg 840

ctactttgtc tgcgaaagga tatccttcgg actctgcagc gtacgcggac ccgaacctta 900

ttgcccataa gcttcctatt gtgacgcaga agacccaaaa gctgaaaaat cctacctgac 960

tgacacaaag gcgccctacc gcgtgtacat catgactgtc ctgtcctatc gttgcctttt 1020

gtgtttgcca catgttgtgg atgtacgttt ctatgacgaa acaccatagt ccatttcgcc 1080

tgggccgaac agagatagct gattgtcatg tcacgtttga attagaccat tccttagccc 1140

tttttccccc 1150

<210> 63
<211> 22
<212> DNA
<213> Artificial Sequence

<220>
<223> synthesized primer oligonucleotide

<220>
<221> misc_feature
<222> (22)..(22)
<223> n is a, c, g, or t

<400> 63
ttttttttt ttttttttt vn 22

<210> 64
<211> 20
<212> DNA
<213> Artificial Sequence

<220>
<223> synthesized primer oligonucleotide

<400> 64
ttgtgatgtt ggtggcgtat 20

<210> 65
<211> 24
<212> DNA
<213> Artificial Sequence

<220>
<223> synthesized primer oligonucleotide

<400> 65
tgttaaataa aaccccaaag atcg 24

<210> 66
<211> 21
<212> DNA
<213> Artificial Sequence

<220>
<223> synthesized primer oligonucleotide

<400> 66
tgagggtaat gccaactggt t 21

<210> 67
<211> 24
<212> DNA
<213> Artificial Sequence

<220>
<223> synthesized primer oligonucleotide

<400> 67
gcaatgtaac cgagtgtctc tcaa 24

<210> 68
<211> 32
<212> DNA
<213> Artificial Sequence

<220>
<223> synthesized probe oligonucleotide

<400> 68
tttttggctt agagttgatg gtgtactgat ga 32

<210> 69
<211> 151
<212> DNA
<213> Escherichia coli

<400> 69
gaccgtaagg cttgatgaaa caacgcggcg agctttgatc aacgaccttt tggaaacttc 60

ggcttcccct ggagagagcg agattctccg cgctgtagaa gtcaccattg ttgtgcacga 120

cgacatcatt ccgtggcgtt atccagctaa g 151

<210> 70
<211> 69
<212> DNA
<213> Artificial Sequence

<220>
<223> Synthesized partial coding region

<400> 70
tgttcggttc cctctaccaa gcacagaacc gtcgcttcag caacacctca gtcaaggtga 60

tggatgttg 69

<210> 71
<211> 4233
<212> DNA
<213> Zea mays

<400> 71
agcctggtgt ttccggagga gacagacatg atccctgccg ttgctgatcc gacgacgctg 60

gacggcgggg gcgcgcgcag gccgttgctc ccggagacgg accctcgggg gcgtgctgcc 120

gccggcgccg agcagaagcg gccgccggct acgccgaccg ttctcaccgc cgtcgtctcc 180

gccgtgctcc tgctcgtcct cgtggcggtc acagtcctcg cgtcgcagca cgtcgacggg 240

caggctgggg gcgttcccgc gggcgaagat gccgtcgtcg tcgaggtggc cgcctcccgt 300

ggcgtggctg agggcgtgtc ggagaagtcc acggccccgc tcctcggctc cggcgcgctc 360

caggacttct cctggaccaa cgcgatgctg gcgtggcagc gcacggcgtt ccacttccag 420

cccccccaaga actggatgaa cggttagttg gacccgtcgc catcggtgac gacgcgcgga 480
tcgtttttttt cttttttcct ctcgttctgg ctctaacttg gttccgcgtt tctgtcacgg 540
acgcctcgtg cacatggcga tacccgatcc gccggccgcg tatatctatc tacctcgacc 600
ggcttctcca gatccgaacg gtaagttgtt ggctccgata cgatcgatca catgtgagct 660
cggcatgctg cttttctgcg cgtgcatgcg gctcctagca ttccacgtcc acgggtcgtg 720
acatcaatgc acgatataat cgtatcggta cagagatatt gtcccatcag ctgctagctt 780
tcgcgtattg atgtcgtgac attttgcacg caggtccgct gtatcacaag ggctggtacc 840
acctcttcta ccagtggaac ccggactccg cggtatgggg caacatcacc tggggccacg 900
ccgtctcgcg cgacctcctc cactggctgc acctaccgct ggccatggtg cccgatcacc 960
cgtacgacgc caacggcgtc tggtccgggt cggcgacgcg cctgcccgac ggccggatcg 1020
tcatgctcta cacgggctcc acggcggagt cgtcggcgca ggtgcagaac ctcgcggagc 1080
cggccgacgc gtccgacccg ctgctgcggg agtgggtcaa gtcggacgcc aacccggtgc 1140
tggtgccgcc gccgggcatc gggccgacgg acttccgcga cccgacgacg gcgtgtcgga 1200
cgccggccgg caacgacacg gcgtggcggg tcgccatcgg gtccaaggac cgggaccacg 1260
cggggctggc gctggtgtac cggacggagg acttcgtgcg gtacgacccg gcgccggcgc 1320
tgatgcacgc cgtgccgggc accggcatgt gggagtgcgt ggacttctac ccggtggccg 1380
cgggatcagg cgccgcggcg ggcagcgggg acgggctgga gacgtccgcg gcgccgggac 1440
ccggggtgaa gcacgtgctc aaggctagcc tcgacgacga caagcacgac tactacgcga 1500
tcggcaccta cgacccggcg acggacacct ggaccccccga cagcgcggag gacgacgtcg 1560
ggatcggcct ccggtacgac tatggcaagt actacgcgtc gaagaccttc tacgaccccg 1620
tccttcgccg gcgggtgctc tgggggtggg tcggcgagac cgacagcgag cgcgcggaca 1680
tcctcaaggg ctgggcatcc gtgcaggtac gtctcagggt ttgaggctag catggcttca 1740
atcttgctgg catcgaatca ttaatgggca gatattataa cttgataatc tgggttggtt 1800
gtgtgtggtg gggatggtga cacacgcgcg gtaataatgt agctaagctg gttaaggatg 1860
agtaatgggg ttgcgtataa acgacagctc tgctaccatt acttctgaca cccgattgaa 1920
ggagacaaca gtaggggtag ccggtagggt tcgtcgactt gccttttctt ttttcctttg 1980
ttttgttgtg gatcgtccaa cacaaggaaa ataggatcat ccaacaaaca tggaagtaat 2040
cccgtaaaac atttctcaag gaaccatcta gctagacgag cgtggcatga tccatgcatg 2100
cacaaacact agataggtct ctgcagctgt gatgttcctt tacatatacc accgtccaaa 2160
ctgaatccgg tctgaaaatt gttcaagcag agaggccccg atcctcacac ctgtacacgt 2220
ccctgtacgc gccgtcgtgg tctcccgtga tcctgccccg tcccctccac gcggccacgc 2280

ctgctgcagc gctctgtaca agcgtgcacc acgtgagaat ttccgtctac tcgagcctag 2340
tagttagacg ggaaaacgag aggaagcgca cggtccaagc acaacacttt gcgcgggccc 2400
gtgacttgtc tccggttggc tgagggcgcg cgacagagat gtatggcgcc gcggcgtgtc 2460
ttgtgtcttg tcttgcctat acaccgtagt cagagactgt gtcaaagccg tccaacgaca 2520
atgagctagg aaacgggttg gagagctggg ttcttgcctt gcctcctgtg atgtctttgc 2580
cttgcatagg gggcgcagta tgtagctttg cgttttactt cacgccaaag gatactgctg 2640
atcgtgaatt attattatta tatatatatc gaatatcgat ttcgtcgctc tcgtggggtt 2700
ttattttcca gactcaaact tttcaaaagg cctgtgtttt agttcttttc ttccaattga 2760
gtaggcaagg cgtgtgagtg tgaccaacgc atgcatggat atcgtggtag actggtagag 2820
ctgtcgttac cagcgcgatg cttgtatatg tttgcagtat tttcaaatga atgtctcagc 2880
tagcgtacag ttgaccaagt cgacgtggag ggcgcacaac agacctctga cattattcac 2940
ttttttttta ccatgccgtg cacgtgcagt caatccccag gacggtcctc ctggacacga 3000
agacgggcag caacctgctc cagtggccgg tggtggaggt ggagaacctc cggatgagcg 3060
gcaagagctt cgacggcgtc gcgctggacc gcggatccgt cgtgcccctc gacgtcggca 3120
aggcgacgca ggtgacgccg cacgcagcct gctgcagcga acgaactcgc gcgttgccgg 3180
cccgcggcca gctgacttag tttctctggc tgatcgaccg tgtgcctgcg tgcgtgcagt 3240
tggacatcga ggctgtgttc gaggtggacg cgtcggacgc ggcgggcgtc acggaggccg 3300
acgtgacgtt caactgcagc accagcgcag gcgcggcggg ccgggggcctg ctcggcccgt 3360
tcggccttct cgtgctggcg gacgacgact tgtccgagca gaccgccgtg tacttctacc 3420
tgctcaaggg cacggacggc agcctccaaa ctttcttctg ccaagacgag ctcaggtatg 3480
tatgttatga cttatgacca tgcatgcatg cgcatttctt agctaggctg tgaagcttct 3540
tgttgagttg tttcacagat gcttaccgtc tgctttgttt cgtatttcga ctaggcatcc 3600
aaggcgaacg atctggttaa gagagtatac gggagcttgg tccctgtgct agatggggag 3660
aatctctcgg tcagaatact ggtaagtttt tacagcgcca gccatgcatg tgttggccag 3720
ccagctgctg gtactttgga cactcgttct tctcgcactg ctcattattg cttctgatct 3780
ggatgcacta caaattgaag gttgaccact ccatcgtgga gagctttgct caaggcggga 3840
ggacgtgcat cacgtcgcga gtgtacccca cacgagccat ctacgactcc gcccgcgtct 3900
tcctcttcaa caacgccaca catgctcacg tcaaagcaaa atccgtcaag atctggcagc 3960
tcaactccgc ctacatccgg ccatatccgg caacgacgac ttctctatga ctaaattaag 4020
tgacggacag ataggcgata ttgcatactt gcatcatgaa ctcatttgta caacagtgat 4080
tgtttaattt atttgctgcc ttccttatcc ttcttgtgaa actatatggt acacacatgt 4140
atcattaggt ctagtagtgt tgttgcaaag acacttagac accagaggtt ccaggagtat 4200

cagagataag gtataagagg gagcagggag cag 4233

<210> 72
<211> 20
<212> DNA
<213> Artificial Sequence

<220>
<223> synthesized primer oligonucleotide

<400> 72
tgttcggttc cctctaccaa 20

<210> 73
<211> 22
<212> DNA
<213> Artificial Sequence

<220>
<223> synthesized primer oligonucleotide

<400> 73
caacatccat caccttgact ga 22

<210> 74
<211> 24
<212> DNA
<213> Artificial Sequence

<220>
<223> synthesized primer oligonucleotide

<400> 74
cacagaaccg tcgcttcagc aaca 24

<210> 75
<211> 18
<212> DNA
<213> Artificial Sequence

<220>
<223> synthesized primer oligonucleotide

<400> 75
tggcggacga cgacttgt 18

<210> 76
<211> 19
<212> DNA
<213> Artificial Sequence

<220>
<223> synthesized primer oligonucleotide

<400> 76
aaagtttgga ggctgccgt 19

<210> 77
<211> 26
<212> DNA
<213> Artificial Sequence

<220>
<223> synthesized primer oligonucleotide

<400> 77
cgagcagacc gccgtgtact tctacc 26

<210> 78
<211> 19
<212> DNA
<213> Artificial Sequence

<220>
<223> Synthesized primer oligonucleotide

<400> 78
cttagctgga taacgccac 19

<210> 79
<211> 19
<212> DNA
<213> Artificial Sequence

<220>
<223> Synthesized primer oligonucleotide

<400> 79
gaccgtaagg cttgatgaa 19

<210> 80
<211> 21
<212> DNA
<213> Artificial Sequence

<220>
<223> synthesized primer oligonucleotide

<400> 80
cgagattctc cgcgctgtag a 21

<210> 81
<211> 25
<212> DNA
<213> Artificial Sequence

<220>
<223> Synthesized primer oligonucleotide

<400> 81
gtatgtttct gcttctacct ttgat 25

<210> 82
<211> 29

<212> DNA
<213> Artificial Sequence

<220>
<223> Synthesized primer oligonucleotide

<400> 82
ccatgttttg gtcatatatt agaaaagtt 29

<210> 83
<211> 34
<212> DNA
<213> Artificial Sequence

<220>
<223> Synthesized primer oligonucleotide

<400> 83
agtaatatag tatttcaagt attttttca aaat 34

<210> 84
<211> 7370
<212> RNA
<213> Diabrotica virgifera

<400> 84
aaagaacaag cuuguuuucu auucugugau augcgcauug uuuuauaugu cauuugucag 60

uugucauauu guauuuacgu ugugugaacg uuuucgaagc auuuuuauau uuaauuuaag 120

uuuagauaua ugaaacgaca ucguaaaugu aaagaacagu aauuaaaagu uacaaugucu 180

uuaccucccu auuuguuggg gcccaauccu ugggccacga ugauggccca acaacaucua 240

gcagcggcuc augcucaggc ccaggcagcu gcugcucaag cucaugccca ugcuuuacaa 300

caacaaaugc caccaccuca uccuaagccg gauauuauaa cugaagauaa auugcaagaa 360

aaagcucuaa aauggcauca auuacaaucu aaaagauucg cugauaagag aaaguuggga 420

uucguggaag cucagaagga ggacaugccu ccagaacaua uuagaaaaau uauaagagac 480

cauggugaua ugaguagccg uaaauauaga caugauaaaa ggguuuauuu aggagcucuc 540

aaauauaugc cucaugcugu gaugaaacuu cuugaaaaca ugccuaugcc gugggagcag 600

auaagagaug uuaaaguauu guaccauauu acaggugcua uuacuuuugu gaaugaaauu 660

ccuuggguuu gugaaccuau uuacauugcu caaugggca ccaugugau uaugaugaga 720

agagaaaaga gagacagaag acacuuuaag agaaugcguu uuccaccauu ugaugaugag 780

gaaccuccuu uagauuacgc agauaacguu uuagauguag aaccuuuaga agcuauccag 840

auugagcugg acgcugauga agauucugcu auagcaaaau gguuuuauga ccacaagccg 900

cuaguuggaa ccaaauaugu aaaugggcua acauauagaa aauggaaucu uucuuuaccc 960

aucauggcua cccuauaccg uuuggcuaau cagcuauuga cagaucuggu agaugauaac 1020

uauuuuuauc uuuuugacac aaaaaguuuc uuuacugcca aagcucuuaa uauggcaauu 1080

ccaggaggac ccaaauuuga accacucaua aaagauauga auccugcgga ugaagauugg 1140
aacgaauuua augauaucaa uaaaauuaua auaagacaac caauuagaac agaauauaga 1200
auugcauuuc cauauuugua caauaauaug ccacauuuug uucacuuguc augguaccac 1260
gcaccaaaug uuguauacau caagacagaa gauccggauu uaccggccuu uuacuucgau 1320
ccauugauua aucccauauc ucacaggcau gccgucaaaa gucuggaacc ucuaccagau 1380
gacgacgaag aauauauuuu gccagaguuu guacaaccau ucuugcagga aacaccguug 1440
uauacagaua acacagcuaa uggaauuucu uuauuguggg cacccagacc guuuaauaug 1500
agaucagguc gauguagaag agcaauugac gucccucuag uaaaacccug guauauggaa 1560
cauuguccac caggccaacc uguaaaaguu agagucaguu accaaaaauu acugaaguau 1620
uacguauuga acgcucucaa acacaggccu ccuaaggcgc agaagaagag guacuuguuc 1680
agaucguuca agucuaccaa auucuuccaa acaacuacuu uggacugggu cgaagccgga 1740
cuacaaguuu gcaggcaagg uuauaacaug uugaaucuau ugauucaucg aaagaacuug 1800
aauuaccugc auuuggacua caacuuuaac uugaaaccag uuaagaccuu gacaacgaag 1860
gaaagaaaga agucucguuu uggaaaugcu uuccauuugu gcagagagau auuaagauua 1920
acaaaacuga uuauugacuc ccacguucaa uaucguuuga acaauguuga ugcuuuucaa 1980
uuggcagaug guuugcagua uauauuugcc cacguuggac aauugacugg aauguacaga 2040
uacaaauaca aacuuaugag acaaauuagg augugcaagg acuugaagca ucucaucuau 2100
uacagauuua acacuggacc ggugggcaaa ggaccggguu gcgguuuuug ggcgccugga 2160
uggagagucu gguuguucuu uaugaggggc auuacaccuc uuuuggaaag gugguuggga 2220
aaccuucugu cacgucaauu cgaaggaaga cacucgaaag gaguugcaaa aacugucaca 2280
aaacaaaggg uugagucuca cuuugaucuu gaacuuagag cuucgguuau gcacgauauu 2340
gucgacauga ugccugaagg uauaaagcag aacaaggcaa gaacuauacu ucaacauuua 2400
ucagaagccu ggagauguug gaaagcuaau auuccuugga aaguaccagg ucugccgaua 2460
ccuaucgaaa acaugauucu ucgauacgua aagaugaagg cugauuggug gacaaauacg 2520
gcccauuaca aucgcgagag gauccguaga ggagcaacug ucgauaaaac aguuugcaag 2580
aaaaaucuug gacggcuuac uagauuauau cuaaaagccg aacaagaaag acagcauaac 2640
uauuugaagg acgguccgua cauuucacca gaagaagcug uugccauuua caccaccacu 2700
guccauuggu uggaaucgag aagguuugca ccgauaccuu ucccaccucu gucauacaaa 2760
cacgacacca agcugcuuau uuuggcauua gaaagauuaa aagaagcuua caguguaaaa 2820
ucgcgucuga aucagaguca aagagaagaa uugggucuaa uugagcaggc uuaugauaau 2880
ccucacgaag cucuaucgag gauaaaacgu caucuuuuaa cacaaagagc uuucaaagag 2940

guagggauag aguucaugga uuuguacagu cauuugauac cuguguauga uguagaaccg 3000
cuagaaaaaa uaacugaugc guacuuagau caauaucuuu gguaugaagc ugacaaaaga 3060
cgacuauuuc cuccguggau caaaccagcu gauacggaac cuccuccauu acuuguuuau 3120
aaauggugcc aaggcauuaa caauuuacaa gaugugugg augugaauga aggggagugu 3180
aacguguuac uggaaucuaa guuugaaaaa cuauaugaaa agaucgauuu gacucuacuu 3240
aacagacuuc uccgauugau aguggaccac aacauagcug auuacaugac cgcuaagaau 3300
aacgucguua uaaacuacaa agauaugaau cacaccaaca guuacggaau uauucgagga 3360
uugcaguuug ccucguucau uacucaguau uauggucugg uuuuggaucu gcugguauug 3420
ggucugcaga gagccaguga aauggcuggg ccaccucaaa ugccuaacga uuucuugacg 3480
uuccaagaug uucaauccga aacgugccau ccuauucggc uuuacugcag auauguggac 3540
agaauucaua uguuuuucag auuuucugca gaagaagcca aagauuugau ccaaagauac 3600
cuaacagaac auccagaucc uaauaaugaa aacauugucg guuacaauaa uaaaaaaugc 3660
uggcccagag augcaagaau gcgucuaaug aagcacgaug uuaauuuggg aagagcagua 3720
uuuugggaca uuaaaaacag auugccgaga ucuguuacaa cuauucaaug ggagaacagc 3780
uuuguuagcg uguacucuaa ggauaauccc aaucuguugu uuaauauguc uggauuugaa 3840
uguagaauac uaccaaagug ccguacgcaa cacgaagaau ucacccauag ggacggagua 3900
uggaaccuuc aacaugaagg aaguaaagaa agaacggcuc aauguuucuu gcgaguagac 3960
gaugaaucca ugagucgauu ucauaauaga guucgacaga uucuuauggc uucagguuca 4020
acuacauuua cgaagauugu aaauaaaugg aacacagcuc uaauaggauu gaugacauau 4080
uuccgagaag ccgugguaaa cacccaggaa cuacuagauu uacucguaaa gugugaaaau 4140
aaaauacaaa cucguaucaa aaucggucuu aauucaaaaa ugccuagcag auucccucca 4200
gucguauuuu acacccccaa agaauugggu ggauugggua uguuauccau gggccacgug 4260
uugauccccc agucagacuu gagauggucu aagcagacgg auguaggaau cacucacuuc 4320
agaucuggua uaagucacga ugaagaucag uugauuccua auuuguacag auauauccaa 4380
ccgugggaau cugaguuuau agauucgcag agagugugg cugaguaugc ucugaaaagg 4440
caagaagcga acgcucagaa uagaaggcug acuuuggaag acuuggaaga uucuugggau 4500
agagguauac cuaggaucaa uacgcuuuuc cagaaagaua ggcauacuuu ggcguacgac 4560
aagggaugga gaauuaggac agaauucaaa caguaccaag uacuaaaaca aaauccguuc 4620
ugguggacgc aucaaagaca cgacggcaaa uuauggaacu ugaacaacua ccgaacugac 4680
augauccaag cucuuggagg uguagaaggu auucucgagc acacauuauu caaaggaacu 4740
uauuucccaa caugggaagg ucucuucugg gaaaaagcuu cugguuuuga ggagucaaug 4800
aaauauaaga aacuaaccaa ugcccaaaga ucugguuuga accagauucc aaaucgucgu 4860

uuuaccuuau gguggucacc uacaauaaac agagcuaacg uauauguugg uuuccaagua 4920
caauuggauu uaacugguau uuucaugcau gguaaaauac ccaccuugaa aauuucccuc 4980
auucagauuu ucagagcuca cuuguggcaa aaaguccaug aaucgauagu uauggauuug 5040
ugucagguau uugaucaaga auuggacgca uuagaaauug aaacugucca aaaagaaacu 5100
auccauccua gaaaaucaua caagaugaac ucaucuugug cggacauuuu acuguuuucg 5160
gcauauaaau ggaauguauc ccgaccguca uuauuagcag acacaaagga cacaauggau 5220
aauacaacga cucagaaaua cuggaucgau guucaacuua gaugggguga uuacgacucc 5280
cacgaugugg agagauaugc uagagccaaa uuuuuagauu auacaacuga uaauaugucu 5340
auauauccau cuccgacugg aguucuuauu gccauugauu uggcauacaa ucugcauagc 5400
gcuuauggca acugguuccc agguugcaaa ccauugaucc aacaagcuau ggcaaaaauc 5460
augaaggcca acccagcucu cuauguacuu cgagaacgca uacgaaaggc ucuacaauug 5520
uauuccagug aaccuaccga acccuaccuu ucgagucaga auuaugguga acuguucucg 5580
aaccaaauca uuugguucgu cgacgauacu aacguauaca gaguaacgau ucauaagacg 5640
uucgaaggca auuugacuac gaaaccuauc aauggagcua uauuuauuuu uaacccaagg 5700
acugggcagu uguucuugaa aauuauucau accucaguau gggcaggaca gaagcguuua 5760
ggacaguugg caaaauggaa aaccgcugaa gaaguggcag cucuuauccg uucgcuacca 5820
guugaagaac aaccgaaaca aauuauugua acaaggaaag gaauguugga uccucuugaa 5880
guacauuuac uagacuuccc uaauauuguc aucaaaggau ccgaacugca acuacccuuc 5940
caagcuuguu ugaaaauuga aaaguucggu gaucuuauuc uuaaagcuac agagccucag 6000
augguucuuu ucaacuugua cgaugauugg uugaagacua uuucuucaua uacggcauuu 6060
ucaagacuga uauuaauauu aagagccuug cacguuaaca cugaaagaac caaaguaaua 6120
uuaaaaccgg auaagacuac caucacggaa cuucaucaca uuuggccaac uuuaucagac 6180
gaugaaugga uuaaaguuga aguacagcuu aaggaucuaa uucuagcgga uuauggaaag 6240
aagaacaacg uaaauguugc aucucuaacc caaucagaaa uucgugauau caucuugggu 6300
auggaaauca gcgcuccauc ggcccagaga cagcaaaucg cagaaauuga aaagcagacu 6360
aaagagcagu cucagcuuac ugcgacgacu accaaaacag ucaacaaaca cggagacgaa 6420
auuauuacca gcacuaccag uaauuacgaa acgcaaacgu uuaguucgaa aaccgaaugg 6480
agaguuagag cuauuucugc uacuaauuua cauuugagaa ccaaccacau cuaugucagu 6540
ucugaugaua ucaaggaaac uggcuauacu uauauuuuac cgaagaaugu ccugaagaag 6600
uuuguaacga uuucagauuu gagagcacag auaugcgcgu uucuuuaugg agucagccca 6660
cccgauaauc cacaaguaaa agaacucaga uguuuaguuc uggcaccgca augggguacu 6720

caucaaacug uacacguucc uaacacaccg cccaaucauc cguuccuuaa agauauggaa 6780

ccacucggau ggauucacac ucaacccaac gaauuacccc aacuuucacc ccaggacauu 6840

accaaccaug ccaaacuuau gucagauaau acuacuuggg acggugaaaa gacuauuauu 6900

auuaccuguu cguuuacacc ugggucaugu ucguugacag cuuacaaauu gacgccuucu 6960

ggauuugaau ggggaaggca aaauacggac aaaggcaaua aucccaaagg auaucuaccc 7020

agucauuaug aaaaaguaca aauguuguua ucagacaggu ucuuaggauu cuuuagguu 7080

ccagcccaag gaucguggaa cuauaacuuu auggguguca ggcaugaccc caguaugaaa 7140

uaugaauuac aauuagcaaa uccaaaagaa uucuaccacg agguucacag accugcacau 7200

uuccucaacu ucuccgccuu agaagauggc gauggagcag gagcagauag agaagaugcu 7260

uuugcuuaga uuaguuuaua gauuauaaaa uaauugauug uauuauucga acauauauac 7320

cucauggaug uuguugauau agaauaauau acccuauucc acgaacauac 7370

<210> 85
<211> 9518
<212> RNA
<213> Diabrotica virgifera

<400> 85
ugaaagaauc gaucaccucc ccaaaaaaac acauaccugc uucccagauc ggaugaugau 60

cgucacccac uaugggaccg ucagcuccac aaggugcaag aacagucugu guuuuuggcc 120

gugaacuucu uugaggcgac cuguacgagu acgagagcgc ucccucacgu gggauuucgg 180

uuacaucguc cuuuaguccg caaaacgucg ucaccggaac uuuggaauga ggguugaugc 240

ucaaaaaucc acaauuauac gacaagcauu uaucuagacc aucguugacg uuuguguaau 300

ucgugugaug uccuuuugaa caugcauaaa gcauguuaag cacaggugug aaccccucuu 360

ucguugguag gcgcuccuua ggaauuacca augaacuuuc gccagaauuu ggguucgaaa 420

agauuguguc cgagaauuca cagcuaacaa auucagucgg auuaguaguc gucgcguuau 480

agcugaugaa gccgcauucc gggucaagag agcacguggc gagcgcgauc uaaggugaca 540

acuaugucgg agcagagucu uagagccuca cagauauugu cggcuuaucc ugcaauacga 600

uaaaucuuuu gcaacucuug aaacaacaua ccagaccuuu gagagauuuc cggcccguac 660

aggggacauc aacauucuuu aauacgagug augugaucuc uggaguuugg ggcucagucu 720

cgccauaaca agcgguacug aagacauaaa aagaggcuaa acugcgcauu gagcacacgc 780

gugucuugga caugaaggcc cgacaaauga ucuccgaagu ugagcuuuaa auauugugaa 840

ggcgggggau gagcucaaau gggccaggua guagcaagaa caugaauggc aggaagccgg 900

aaaugccucc agaggcucug aggaagauaa uugcagauca uggcgacaug aguagccgga 960

aguuucgcca agauaagaga guuuaccuug gagcgcugaa guauguaccc caugcuguuu 1020

acaaacucuu agagaaucua cccaugccuu gggagcaagu gagaaacgua aaagucuugu 1080

aucacacaac uggggcaauc ucuuuuguga acgagauacc uuggguaguc gagccgauuu 1140

uucuggccca guggggaaca auguggauaa ugaugcgacg ugagaaacgc gaucgccguc 1200

auuucaaacg uaugagauuu ccgccuuucg augacgaaga gccuccacuu gauuacgccg 1260

acaacauauu agaccaacag ccccucgacg caauacaaau ggagcuggac gcugaggaag 1320

acgcuccagu gauagacugg uuuuacgauc accaaccucu ccaauacgau ucuaauuacc 1380

ucgcaggucc caaauaccga agauggcguc ucgauuugaa ccaaaugagc guccuguaua 1440

gauuagccca ucaacuucug ucugauauca uugaugacaa uuacuuuuac cuauuugauc 1500

ugaaaucauu cuuuacagcc aaagcgcuaa accuugccau ucccgguggg ccaaaguuug 1560

agccccuggu ccgcgauguc gcugaugauu cggauuggaa cacauuuaau aacauugaca 1620

agauaaucgu ucggcauaaa auccguacgg aauauaaaau ugcauucccc uaucucuaca 1680

augacaggcc auucaaaguu ucuuugagua aauaucauuc uccgacugug guguuuguga 1740

agcaagagga ggucgaccaa ccugcauucu acuuugaccc ucuccuguau ccaauaccug 1800

ccuaucgaac uaaaaccgac aaguauuucu gccaaacuau cgaaaguuca auagacgaug 1860

acuuccuuca ggagcuuaac agcuuugcgu caagcgccag cgcaggcauu ggauccgcug 1920

auagucuacu ccagccgcuu uuguuugagg cgccuuugca gaccgacaca acauauggag 1980

guauaacauu gcugugggcu ccaagacccu ucaacauaag auccggguug accaggagag 2040

cucaagauau uccacuaguu caguccuggu uccgagagca cugcccaggu gcuucgaccu 2100

auccggugaa aguucgcguc ucuuaucaga agcuucucaa aacuugggua cugagccauc 2160

ucagaagucg uccgccuaag gcaaugaaga agcgcaaucu ccugagacua uuuaaaaaca 2220

ccaaauucuu ucaauguacu gaaacugauu ggguggaggu uggucugcac gugugccgcc 2280

aaggauauaa uaugcucaau cuccugauuc aucgccgaaa ucuaaacuac cuucaucugg 2340

auuauaauuu caaucugaag cccauuaaaa cauugaccac uaaagaacga aaaaagaguc 2400

guuucggaaa ugcguuccau cuaugucgcg agauucuacg ucucaccaaa uugauuguug 2460

acucucacgu ccaguaccgg cuggggaaua uagaugcaua ucaacuggca gauggcuuac 2520

aauacauauu cugccacguc ggucaauuga cauccaugua ucgauacaaa uaccggcuua 2580

ugcgacaggu ucggcugugc aaggaucuca agcaucuaau auauuacaga uucaacaccg 2640

gccaaguggg uaaaggccca ggcugcggau ucugguugcc cucauaucgu gucugguugu 2700

ucuuucugcg cgggauuuua ccuuuauugg agagaugguu ggguaaucua uuggcucguc 2760

aguuugaagg ucgaaacuug cgcggucaag caaaauccgu cacgaagcaa cgaguggaag 2820

ucuacuucga uuuagagcua cgagcugcug ugaugcauga ucugcuagau augaugccag 2880

aaggaauccg agcaaacaaa gccaaaauug uacuucagca ucucagcgaa gccuggagau 2940

guuggaaggc gaauauuccc uggaaggucg ccgggauucc agcuccggug gaaaacauua 3000
uucugagaua uguaaaacua aaaucugacu gguggacgaa ugccgcauau uucaaucggg 3060
agagaauuag acguggagca acuguggaca agacugugug caaaaagaac uuggggcggc 3120
ucacucguuu gugguugaag ucagagcaag aacgucaaca uggguacaug aaggaugguc 3180
ccuaucuaac cagugaggag gcgguggcga uuuacacuac aagguacau ugguuggauu 3240
ugcgaaaauu cacucauauc ccauuuccuc cauugaacua uaaacacgac acaaaacuuc 3300
ugauucucgc ucuggagcgc uugagggaca cauacgccgu gaagacacga cugaaucaag 3360
uucagcguga agaguugggu cuaaucgaac acgcguacga uaauccucau gaggccauau 3420
cgcgaauaaa acgacauuua uugacucaac gagccuucaa agacgccagu guugaguuca 3480
uggaucucua cucgcauuua guaccuguau acgagaucga uccacuagaa aaaaucaccg 3540
acgcuuaccu cgaccaguau uuaugguacg agucugaccu ccgccaccuc uucccaccgu 3600
ggauaaaacc gagcgaucac gagccucugc cucugcugcu cuauaaaugg ucaaacaaua 3660
uaaauaauuu ggacucgaua ugggaacaug acgacgguuc cugcguugcc augaugcaaa 3720
cgaaguugaa gaagauuuuc gagaaaauug aucucacccu ucucaauaga uugcugagau 3780
ugauaguuga ccauaaucuc gcugauuaca ugaccgcgaa aaacaacauu cggcugaucu 3840
ucaaggacau gucccauaca aauuauuacg gcuuaauccg cggccuccag uucagcaguu 3900
ucauauucca auauuaugcu cuggucauag aucuucugau uuuagggcug acgcgagcca 3960
augaacuugc cggcaguaua gguggcggcg gaggcggagg uuucgcuaau cucaaagauc 4020
gcgaaacgga gauaaaacau cccauccgcu uguauugccg auauauagau gaaauaugga 4080
ucugcuucaa auucaccaaa gaggagucuc guagcuugau ucaaagguau uugacggaga 4140
auccaaccgc uagucagcag cucuccacug aagaaggcau cgacuacccc aucaaaaagu 4200
guuggccuaa agacugccga augagaaaaa ugaaauucga cguuaauauc ggacgagccg 4260
uuuucuggga gauucagaaa cgucuaccga gaaguuuagc ugagcugagu uggggcaaag 4320
augcuggaga cucgacaucg uuugugucag ucuauagugu caauaacccc aaucuucugu 4380
uuagcauggg cggcuuugag guccgaaucc ugccaaaagu ucgaggugggg acuaguaugg 4440
gaacugggag caguucacaa ggcguauggc guuuacaaaa cuaucugacc aaggagacga 4500
cagcguauug uuacauuaga guuggugacg aagccauacg uaacuucgaa aaucgaauuc 4560
ggcagauucu gaugucaucc ggcucggcaa cguucacaaa gguggcaaac aaauggaaua 4620
cagcucugau cagccuugug aguuauuuca gagaggcgau aauauauacg gaggaucucc 4680
ucgaucuguu ggugaaaugu gaaaacaaaa uacaaacgag aaucaagauc gguuugaaua 4740
guaaaaugcc gucgagguuc ccccccguug uguucuacac gcccaaagag cucggcggcu 4800

ugggcaugcu uuccaugggg cacauccuua ucccucaauc ugacuugcgc uauaugaagc 4860
agaccaauga uuauaccauc acccauuucc gcucgggaau gacucacgac gaagaucagu 4920
ugauacccaa ucucuauaga uacauccaga caugggaaag ugaguucauc gacagucagc 4980
gaguuugguc ggaauauaac aucaagagau uugaagcaac cacuaacggc ggcgccgguu 5040
caaguggcgg cagcggcggg agucgcagac ugacuuugga agacguagag gagaacuggg 5100
aucaugguau uccccguauu aauacguugu uucagaaaga ucgacacacg cugugcuacg 5160
auaagggcug gagauuacgu caagaguuua agcaauauca gauccugcgg agcaauccau 5220
ucugguggac aaauaucaag cacgauggaa aauuguggaa ucucaacaac uauagaacug 5280
auaugaucca agcuuugggc ggaguugagg gcauuuugga acacacgcuu uucaaaggaa 5340
cuuacuucca gacaugggaa ggucuauucu gggaaaaguc uaguggcuuc gaggaaucca 5400
ugaaauauaa gaaguugaca aacgcgcaaa gaaguggguu aaaucaaaua ccuaaucgga 5460
gguucacccu cugguggagu ccaacgauca aucggucaaa uaucuauguu ggauuccaag 5520
uccaauuaga ucucacagga auuuucaugc acggcaaaau cccaacccuc aagaucagcu 5580
ugauucaaau cuuccgcgcg caucuuuggc agaagauuca ugagucaguu aucauggauc 5640
ucugucagau uuuggaucuc gaaauugaau cuuuaggaau ccacacaguu aagaaagaaa 5700
cuauccaucc ucgaaaaagu uacaagauga auagcucuug ugcagauauc auuuuguacu 5760
cgucguacaa auggaacauc agcaaugugc cuacacuucu aucagccaac gcaaacgcau 5820
cggccucauc aaccaccuca accauaaguu ggcuugaucu ucaacuccga ugggggggauu 5880
acgacucgca cgacaucgaa agauacugcc gguccaagua ucuugauuac gucaacgaca 5940
gcaugucuau uuauccgucg aauaccggag uucuucuggg cauagauuug gcuuacaaua 6000
uguacagcgg auuuggaaua uggauugacg gcuuaaagga auugguccgu acgggcaugc 6060
gcaagaucau caaaucgaau ccgaguuugu augucuugag agaacgaaua aggaaaggcu 6120
uacaacugua uagcucggag ccgacagagc caaaucuuga gucuucuaac uaugguggaac 6180
uguucaccuc uaacggcccc aauacuuggu ucgucgauga uacuaauguu uauaggguua 6240
caauucacaa aacuuucgag ggaaauuuaa caaccaagcc gacgaauggg gccauuguua 6300
ucaucaaccc agugacuggc caguuguuuc ugaagauuau acauacuagu guauggucag 6360
gucagaaacg cuugagucaa uuggcgaagu ggaagaccgc ugaggaaauc accagucuca 6420
uccggucuuu gccuauugaa gaacaaccca agcagauuau agugaccaga aagggcaugc 6480
uggaccccuu ggaaguacau cugcuagauu uuccuaacau cauaaucaaa gguuccgagu 6540
uggcauugcc auuccaaagu cucaugaagu uggagaaguu cucagaucuc auucuaaaag 6600
cuacaaaacc agauaugguu cucuuuaacc ucuaugauga uuggcuucaa aacauuucag 6660
cauacacugc auuuuccaga uugauucuuc uacuccgcuc auugcacgug aaucccgaga 6720

agaccaagau caucuugagg ccggauagau ccauuaucac caaaccacac cauauauggc 6780

cuaccauuaa gaaugaggac uggaagaaga uugaaguuca auugaccgac cuaauucuga 6840

cugauuacuc caaggcaaau aaugucgcua ucagcucacu cacccagaca gaaauacgug 6900

auaucauucu agguauggau cuccaaccac caagccugca gagacaacaa aucgccgaga 6960

ucggaggcga gacguccaac aauggagugg cguugucugc uucagguauc acugcaacga 7020

cuacgaguac uacuaauauc aguggugacg caaugaucgu cacuacccag aguccucaug 7080

aacaacagau guucuugagu aaaacugacu ggagaguucg ggcgaugaac agcggguccu 7140

uguauuugag agcugagaag auuuauaucg augaugacgc gagagaugag acgaucacug 7200

guacaucaag uacugcaacc ucggacggau uuacguauac uauuccacau aaucuuauua 7260

ggcuauuucu uggggccgcg gauuugagaa cucgaauugg cgcauacaua uuuggcacaa 7320

caucugccaa aaauccucuu gugaaagaga ucaagaccuu cguuaugguu ccgcaaucca 7380

auucacauga aaaaguggau uuugucgaca uguuaccaga ucauccuauu cucaaagaac 7440

uugaaccauu gggaugggua caaacuacug ccacuggauc aaagccaucu cuccacgaua 7500

ucacauucac agcugcucua cucucggacg guccauguca gaugccuagg cucgauccua 7560

augcuugugu aaugcuguuu gucgcuuuga cgcaaggaag uugcacguug agcgguuaca 7620

gauugacucc cgcagggcuc gagugggcua guggcauuac ggcaacaaua caggcggagg 7680

uagcuccuca guauauugag aaaacccaau ugcuggucuc ggauaauaca gccggauucu 7740

uuauggugcc agaugacgga uuuuggaauu ucgcuuucau gggcguaaga uucaacaaga 7800

aaaccccuua caauuuggua uugaacguuc cgaaauccuu cugugaugaa uugcaucgac 7860

cuaaucauuu cuugcaauuu gcucaacugg aagcgcugga ugaguccgau ggcguugaag 7920

ccgaagacug guuagauuag aucggacacg cgugugcgcg cgcaaauaua gauaaaugcg 7980

cguguugacu agauuuuugc cucuugccuc aguggcauuc gcagucaaug uugagccuuc 8040

gcaucaaguc augacgcaag auacuggagg agcuguauca aacgugcugg gaagcaucaa 8100

gagucgaucc aaacagcugg cccaaagcau ucccgggucg ucgauagcua gcuguuugac 8160

uuccucaaau ccggaacuuu gcaagaaaca gguucgcuuc gagcaugauu ugagaggacu 8220

cauguugaaa gguaccaccg aucuggcuuc caugcaaucu cucaagcaaa aauuaacggu 8280

gccuagcgcc uauggccugg acgccgcuca agcuaaugac auuuuucauc aacugauaaa 8340

ggagcuucac uuugaucagc aggccuacga auuggucacu aaugcagcaa aagcaacgac 8400

gccgaugagc ccgaguaucu cgcuuccgac aguggcaccc auaccgauca acgcaggugu 8460

gggcgcugcg gcagugaguc ccggcauagc gaccgcaauu agccccuucg ccacaacauc 8520

ggugagcaca uuggcucccu cuucuggagu cuuaaaugcu gcggcccuua cgaccgcggc 8580

gccgacggcg agcacacuga uugcaagugu cuccaccacu gccucgacgg cacacuaaau 8640

uucauuuuuu auuggaaagc uaauguucgu ugcucuaguu uacggaauca guucugcugc 8700

auuggugcug gaaacaaagg ggauuuugag agcuuguuca gacaaguuga agguucuggc 8760

cuuacaacag agcgucauag cguuaugcua cgugaucuug agcacuguga augcacacaa 8820

aaauggcaca cacggcucug gauuguggag uuuucaggac uucaaacgag cgauaccggu 8880

gacacuagcu uuucucagca ugcaggcaac ucagaugauu ugccucgcca auucgaguau 8940

ggguagcuac guggucgcga aagcaaguug ucugacauuu aauauacugc uguucggcug 9000

ucugauugug acaauuggcg uugugcuccc uguuuguaau agucgagcgc acugcacaaa 9060

gucuggguuu ugcgcgggcu ugaugucuuc ccuggcgcaa gcugcuuuca ugcuucuguc 9120

auccguugcg acuaaaagac auuuugcagc agcgccgaug aaacuccucg gucauuacac 9180

auucucggcu guuguaguau uaugggcuau ccucuggcuu cgugggguacu ccgaugauuc 9240

gacuugccag accagggggc uuuugacacg cauaaucugg uccgguauua ucaauguagu 9300

uguggccaug agcgcaaugc gauguuuaaa aaacagucau ccaguugcau ugaacaugau 9360

caguuucguc aaauccguuu uacagauuug cugcgcugcu uuguucuacg gagaccgccc 9420

caacagaaca gaaauaaugg gcguggcauu uguucuaggu ggaagugcag ucuacucgug 9480

cggccgauuu uucaucaaag aaacagacug agugcccu 9518

<210> 86
<211> 488
<212> RNA
<213> Diabrotica virgifera

<400> 86
caauuuacaa gaugugugg augugaauga aggggagugu aacguguuac uggaaucuaa 60

guuugaaaaa cuauaugaaa agaucgauuu gacucuacuu aacagacuuc uccgauugau 120

aguggaccac aacauagcug auuacaugac cgcuaagaau aacgucguua uaaacuacaa 180

agauaugaau cacaccaaca guuacggaau uauucgagga uugcaguuug ccucguucau 240

uacucaguau uauggucugg uuuuggaucu gcugguauug ggucugcaga gagccaguga 300

aauggcuggg ccaccucaaa ugccuaacga uuucuugacg uuccaagaug uucaauccga 360

aacgugccau ccuauucggc uuuacugcag auauguggac agaauucaua uguuuuucag 420

auuuucugca gaagaagcca aagauuugau ccaaagauac cuaacagaac auccagaucc 480

uaauaaug 488

<210> 87
<211> 452
<212> RNA
<213> Diabrotica virgifera

<400> 87
cggcuuaauc cgcggccucc aguucagcag uuucauauuc caauauuaug cucuggucau 60

agaucuucug auuuuagggc ugacgcgagc caaugaacuu gccggcagua uagguggcgg 120

cggaggcgga gguuucgcua aucucaaaga ucgcgaaacg gagauaaaac aucccauccg 180

cuuguauugc cgauauauag augaaauaug gaucugcuuc aaauucacca aagaggaguc 240

ucguagcuug auucaaaggu auuugacgga gaauccaacc gcuagucagc agcucuccac 300

ugaagaaggc aucgacuacc ccaucaaaaa guguuggccu aaagacugcc gaaugagaaa 360

aaugaaauuc gacguuaaua ucggacgagc cguuuucugg gagauucaga aacgucuacc 420

gagaaguuua gcugagcuga guuggggcaa ag 452

<210> 88
<211> 336
<212> RNA
<213> Diabrotica virgifera

<400> 88
cuaagaauaa cgucguuaua aacuacaaag auaugaauca caccaacagu uacggaauua 60

uucgaggauu gcaguuugcc ucguucauua cucaguauua uggucugguu uuggaucugc 120

ugguauuggg ucugcagaga gccagugaaa uggcugggcc accucaaaug ccuaacgauu 180

ucuugacguu ccaagauguu caauccgaaa cgugccaucc uauucggcuu uacugcagau 240

auguggacag aauucauaug uuuuucagau uuucugcaga agaagccaaa gauuugaucc 300

aaagauaccu aacagaacau ccagauccua auaaug 336

<210> 89
<211> 120
<212> RNA
<213> Diabrotica virgifera

<400> 89
cuaagaauaa cgucguuaua aacuacaaag auaugaauca caccaacagu uacggaauua 60

uucgaggauu gcaguuugcc ucguucauua cucaguauua uggucugguu uuggaucugc 120

<210> 90
<211> 186
<212> RNA
<213> Diabrotica virgifera

<400> 90
uggcugggcc accucaaaug ccuaacgauu ucuugacguu ccaagauguu caauccgaaa 60

cgugccaucc uauucggcuu uacugcagau auguggacag aauucauaug uuuuucagau 120

uuucugcaga agaagccaaa gauuugaucc aaagauaccu aacagaacau ccagauccua 180

auaaug 186

<210> 91
<211> 7089
<212> RNA
<213> Meligethes aeneus

<400> 91
augucuuugc caccuuauuu acugggccca aaucccuggu uaauggcuca acagcaguua 60

gcugcagcuc augcucaagc ucaggcugcu gcugcucaag cucaugccca agcuuuacaa 120

caacaaauuc cuccuccaca cccaaaauca gauauuauaa cagaggauaa acuucaagaa 180

aaagcccaaa aauggcauca guuacaauca aaaagguuug cagacaaaag aaaguugggu 240

uuuguagaag cccaaaaaga agauaugcca ccagagcaua uuagaaaaau uauaagggau 300

cauggggaua ugagcagucg uaaauacaga caugauaaaa gaguuuauuu aggagcuuua 360

aaauauaugc cccaugcagu uaugaaauua uuggaaaaua ugccuaugcc uugggagcaa 420

auuagagaug ugaaaguuuu auaucacauu acaggugcua uuacauuugu aaaugaaauu 480

ccuuggguug uugagccuau uuauauugcu caaugggua cuauguggau caugaugaga 540

agagaaaaac gggaccguag acauuuuaaa agaaugaggu uuccuccuuu ugaugaugaa 600

gaaccuccuc uagauuaugc ugauaauguu uuagauguug aaccucuaga agcaauucaa 660

auugaacuag augcugaaga agacucugca auugcaaagu gguucuacga ucacaaacca 720

cuuguugguu ccaaauuugu uaauggcucc acauacagaa aguggaauuu auccuugcca 780

augauggcua cuuuguaucg guuggcaaau caauuacuua cugauuuagu agauacaaau 840

uauuuuuauu uguuugauac aaaaaguuuu uuuacugcua aagcuuuaaa uauggcuaua 900

ccaggagggc caaaauuuga accccuuauu aaagauauga auccagcuga ugaagauugg 960

aaugaauuua augauaucaa caaaauuaua auucgucaac cuauuagaac ugaauauagg 1020

auagcuuuuc cuuacuugua uaauaauaug ccacauuuug uacaucuauc cugguaucau 1080

gcaccuaacg uuguuuacau aaaaaccgaa gauccugauu ugcccgcguu uuauuuugau 1140

ccccuuauca accccauauc ucauagacac gcggugaaga guuuagaacc uuuacccgag 1200

gaugacgagg aauauauuuu accugaaacg guacaaccau ucuugcaaga aacgccucug 1260

uauaccgaca auacugcuaa ugguaucgcu cuacuuuggg cgccacgacc guuuaauaug 1320

agaucaggca gaugcagaag ggcgauagac guaccauuag uaaaaucuug guacauggag 1380

cauguuccac caggucaacc ugugaaaguc cgcgucaguu accaaaaauu acugaaauau 1440

uacguacuua acgcuuuaaa acacagagcc cccaaaccuc agaaaaagcg guaucuguuu 1500

agaucauuua aaucaacaaa auucuuucaa accacaaccc uugauugggu ugaagcuggu 1560

uuacaagucu gcagacaagg uuauaacaug uuaaaucugu uaauucauag aaagaauuug 1620

aauuacuuac auuuggauua caauuucaac uugaaaccug uuaaaacauu gacaaccaag 1680

gagagaaaaa agucgcguuu uggaaacgcu uuccacuugu gccgugaaau ucuucgucua 1740

acaaaauuaa uaauugauuc ccacguacaa uauagauuaa auaacgugga cgcuuuucaa 1800
cugucugacg guuuacaaua cauauucgcc caugucggcc aacuuacugg aauguacaga 1860
uacaaauaca aacuuaugag gcaaauccgc augugcaaag aucuuaagca ucugguguac 1920
uaucguuuua acacaggucc aguugguaaa gguccugguu guggaaucug ggcuccaggu 1980
uggagagugu ggcuguucuu uaugaggggu auuacucccgc uucuugaaag auggcugggu 2040
aacuuguugu cgcgucaguu cgagggucgu cacucuaaag guguggcaaa aacggucacc 2100
aagcaaaggg uugagucuca cuuugaucuu gaguugagag caucuguuau gcaugauauu 2160
guugauauga ugccugaagg cauaaaacag aacaaggcua gaacaauuuu gcagcauuug 2220
uccgaagcuu ggagauguug gaaggcgaau auucccugga aaguaccugg gcuaccaauu 2280
cccaucgaaa acaugauccu ucguuacguu aaaaugaaag ccgauuggug gacaaacacc 2340
gcucacuaua acagagaaag aauacguaga ggagccacug ucgauaaaac cgucugcaaa 2400
aagaacuuag gucgcuuaac caggcuguac cuuaaagcug aacaagaaag acaacauaac 2460
uaccuuaaag acgguccuua cauuuccccu gaagaagccg uugccauuua uacaacuacu 2520
gugcauuggu uggaguccag aagauucgcu cccauaccuu uuccaccgcu uuccuauaaa 2580
cacgacacca aacugcuaau uuuggcuuug gaaaggcuua aagaagcaua cagcguaaaa 2640
uccagguuaa aucaaaguca aagagaagaa cuuggucuca uugaacaagc cuacgauaau 2700
ccacacgaag cuuuaucgag aaucaaacgu cauuuguuga cacaaagagc uuuuaaagaa 2760
acggggauug aauuuaugga uuuguauagc cacuugauac caguguauga uguugaaccc 2820
uuagaaaaaa uuacagaugc cuauuuagau caauaccuuu gguaugaagc cgauaaaaga 2880
agauuguuuc cgccauggau caaaccugca gacacggaac caccgccguu acuuguauac 2940
aaauggguguc aaggcauaaa caaccuucag gagguuuggg acguaaacga gggcgaaugu 3000
aacguuuugc uggaaucgaa auuugaaaaa cuuuaugaaa aaaucgauuu gacccugcug 3060
aacagacuuu ugcgucucau cguugaucac aacaucgccg auuacaugac ggccaagaac 3120
aacguuguua uuaauuacaa agacaugaau cacacaaaca guuacggaau cauaagagga 3180
cuucaguuug ccucguuugu cacgcaguau uacgguuugg uauuggauuu guuaguucuc 3240
ggccuacaga gggcguccga aauggcgggc ccaccccaaa ugcccaacga cuuuuugacu 3300
uuucaagaug uugcaacaga aagcugucau ccaaucagau uguacugcag auaugucgac 3360
agaauacaca uguucuuuag guuuucugcu gaagaagcua aagaccugau ucaaagguau 3420
uuaacagaac auccugaucc aaacaacgaa aacaucguug guuauaacaa caaaaaaugc 3480
uggcccaggg acgcuaggau gcgucuuaug aaacacgaug uuaacuuggg cagggccgua 3540
uucugggaca uuaaaaaucg ucuaccgaga ucuguaacua cuauccaaug ggagaacagu 3600

uuugucagcg uuuauucaaa ggauaaucca aaucuuuugu uuaacauguc cggauuugag 3660
uguaggauuu ugccuaaaug ucgcacgcaa cacgaagaau ucacccaucg cgauggugu u 3720
uggaauuugc aacaugaagg caguaaagaa agaaccgcuc aauguuucuu gagaguugau 3780
gaugaaucaa ugucaagguu ucacaacaga guccgacaga uuuugauggc uucuggaucu 3840
accacguuca caaagaucgu caacaaaugg aacacagccc uuauaggucu aaugacauau 3900
uuuagagaag cugugguaaa cacgcaagaa cugcuagacu uguugguaaa gugcgagaac 3960
aaaauucaaa cucguaucaa aaucggucuu aacucuaaaa ugcccagcag auucccgccc 4020
gugguguucu auacucccaa agaauugggc ggguugggua uguugucgau gggucacguu 4080
uuaauuccgc aguccgauuu aagauggucu aaacagaccg acguuggcau cacacauuuu 4140
cguucaggua ugagccacga cgaggaccag cuaauuccca auuuguaucg uuacauacag 4200
ccgugggagu cggaguuuau cgacucccag cgugucuggg ccgaguacgc acucaaaaga 4260
caggaagcaa augcgcaaaa caggcguuug acauuggaag auuuggaaga uuccugggau 4320
cgcgguauuc ccaggauaaa cacgcucuuc cagaaggaca ggcacaccuu ggcuuacgac 4380
aagggcugga gaauccgaac ggaguucaag caguaucaag uuuugaaaca gaauccguuc 4440
ugguggacuc accaaagaca cgauggcaaa cuuuggaacu ugaauaacua cagaacagac 4500
augauucaag cguugggagg uguugaaggu auucuagaac acacuuuguu caaagguaca 4560
uauuuuccca cuugggaagg ucuuuucugg gaaaaggcuu cugguuuuga ggaauccaug 4620
aaguacaaaa agcucaccaa cgcccaaaga uccgguuuga accagauucc caaucguaga 4680
uuuacgcuuu gguggucacc uacaauuaac agggccaacg uuuacguugg auuucaggua 4740
caacucgauu ugaccgguau uuuuaugcac gguaaaauuc caacucucaa aaucucuuug 4800
auucaaauau ucagggcuca cuuguggcag aagguccaug agucgauagu cauggaucuu 4860
ugucaggucu uugaucaaga auuggacgcu uuggaaauug aaacaguuca aaaagaaaca 4920
auccauccca gaaagucaua caaaaugaac ucgucuugug ccgauauuuu acuguucucu 4980
gcuuacaaau ggaauguauc cagaccgucc uuacuugcag acacaaaaga uaccauggac 5040
aacacaacca cccaaaaaua cuggauagac gugcaacuga gaugggguga uuacgauucc 5100
caugacguug agcguuacgc ccgugccaaa uucuuggauu acaccacaga caacauguccc 5160
aucuauccau caccaacugg uguauugauc gccauagauu uggccuacaa cuugcacagc 5220
gccuacggaa acugguuccc ugguugcaaa ccuuugauuc aacaggcgau ggcaaaaaua 5280
augaaggcga accccgcgcu auauguauua agagagcgua uccguaaagc ucuccaauug 5340
uacucuagug aaccuacuga acccuacuug ucgagucaaa auuacggaga auuguucucg 5400
aaucaaauca uuugguucgu ugaugacacc aauguguacc gugucaccau ccacaagacu 5460
uuugagggaa auuugacgac gaagccaauc aauggcgcua uauuuauuuu uaaccccaga 5520

acuggacagu uguucuugaa aauuauucau acuucuguau gggcuggaca aaaacguuug 5580

ggacaauugg caaaauggaa gaccgccgaa gaaguagccg cucuaauucg uucgcuuccc 5640

guagaagaac aacccaaaca aaucaucguu accagaaaag gaauguugga uccucuugaa 5700

gugcaucuuc uugauuuccc caacaucguu aucaaaggau cugaacuaca acugccuuuc 5760

caggcuugcc ucaagauaga aaaguucggc gauuugauuu ugaaagcuac cgaaccucag 5820

augguucugu ucaaucuuua cgacgauugg uugaagacca uuucgucuua caccgccuuc 5880

uccagguuga uuuugauauu aagggcauua cacgucaaua cagaaagaac uaagguuauu 5940

cuuaaacccg acaaaaccac aauuaccgag ccgcaucaua uuuggccuac gcuuucugac 6000

gaugaaugga uuaaaguuga agugcaacuu aaggaucuua uuuuggcgga uuacggaaaa 6060

aagaacaacg ugaacguugc uucucuaacc caaucagaaa uucgcgauau uauucucggu 6120

auggagauca gcgcgccauc ugcucagaga caacagaucg ccgagaucga aaagcaaacg 6180

aaggaacagu cgcaacucac ugcuacuacu acaagaacug ucaacaaaca cggcgaugaa 6240

auuauuacca gcaccacgag caauuacgag acgcagacgu ucaacucgaa gacugaaugg 6300

agagugaggg caauuucagc aacuaauuug cauuugagga caaaccacau uuaugucagu 6360

ucggaugaua uuaaagagac uggauacacc uauauuuugc ccaagaacgu cuugaagaag 6420

uuugucacca uuuccgaucu uagagcacaa auuugcggcu uuuuguaugg cgucagcccg 6480

cccgacaauc cucaagugaa ggaacugcgc uguuuagugc ugccacccca augggcaca 6540

caucaaacgg uucacauacc acacacgccg ccaaaccauc cguuccucaa ggacauggaa 6600

ccucuaggcu ggauacacac acaacccaac gaguugccuc aacugucacc ucaggauauc 6660

accaaucaug ccaaacugau ggccgauaau cccgcuuggg auggugaaaa gaccguuauc 6720

auuacaugcu cauuuacgcc cggcucuugu ucguugacag cguauaaguu aacgcccucu 6780

gggguucgagu ggggcagaca aaauacugau aaagguaaca aucccaaagg guacuugccc 6840

agucacuacg aaaagguuca gauguugcug ucugauaggu uucuaggguu cuuuauggua 6900

ccuacgcagg ggucuuggaa cuacaacuuu augggguguca gacacgaccc aagcaugaag 6960

uaugaauugc aauuagcaaa uccaaaagag uuuuaccaug aaguucacag gccggcucau 7020

uuccucaacu uuucgucuuu ggaagacgga gauggugcug gugccgaucg cgaagaugca 7080

uucgcuuaa 7089

<210> 92
<211> 385
<212> RNA
<213> Meligethes aeneus

<400> 92
aaauggaaga ccgccgaaga aguagccgcu cuaauucguu cgcuucccgu agaagaacaa 60

```
cccaaacaaa ucaucguuac cagaaaagga auguuggauc cucuugaagu gcaucuucuu      120
gauuucccca acaucguuau caaaggaucu gaacuacaac ugccuuucca ggcuugccuc      180
aagauagaaa aguucggcga uuugauuuug aaagcuaccg aaccucagau gguucuguuc      240
aaucuuuacg acgauugguu gaagaccauu ucgucuuaca ccgccuucuc cagguugauu      300
uugauauuaa gggcauuaca cgucaauaca gaaagaacua agguuauucu uaaacccgac      360
aaaaccacaa uuaccgagcc gcauc                                            385
```